(19) Europäisches Patentamt
European Patent Office
Office européen des brevets

(11) EP 4 314 829 B1

(12) # EUROPEAN PATENT SPECIFICATION

(45) Date of publication and mention of the grant of the patent:
**07.05.2025 Bulletin 2025/19**

(21) Application number: **22719581.5**

(22) Date of filing: **30.03.2022**

(51) International Patent Classification (IPC):
***G01N 33/569** (2006.01)*

(52) Cooperative Patent Classification (CPC):
**G01N 33/56972;** G01N 2800/28; G01N 2800/30

(86) International application number:
**PCT/EP2022/058485**

(87) International publication number:
**WO 2022/207748 (06.10.2022 Gazette 2022/40)**

(54) **CLASSIFICATION OF NEUROLOGICAL OR PSYCHIATRIC DISEASE MANIFESTATIONS USING MULTI-DIMENSIONAL CEREBROSPINAL FLUID ANALYSIS**

KLASSIFIZIERUNG VON NEUROLOGISCHEN ODER PSYCHIATRISCHEN KRANKHEITSBILDERN DURCH MEHRDIMENSIONALE ZEREBROSPINALE FLÜSSIGKEITSANALYSE

CLASSIFICATION DE MANIFESTATIONS DE MALADIES NEUROLOGIQUES OU PSYCHIATRIQUES À L’AIDE D’UNE ANALYSE MULTIDIMENSIONNELLE DU LIQUIDE CÉPHALORACHIDIEN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2021 US 202163168114 P**
**31.03.2021 EP 21166177**

(43) Date of publication of application:
**07.02.2024 Bulletin 2024/06**

(73) Proprietors:
- **Westfälische Wilhelms-Universität Münster**
  **48149 Münster (DE)**
- **The Regents of the University of California**
  **Oakland, CA 94607-5200 (US)**

(72) Inventors:
- **WIENDL, Heinz**
  **79106 Freiburg (DE)**
- **MEYER ZU HÖRSTE, Gerd**
  **48149 Münster (DE)**
- **GROSS, Catharina C.**
  **48149 Münster (DE)**
- **SCHULTE-MECKLENBECK, Andreas**
  **48149 Münster (DE)**
- **BARANZINI, Sergio E.**
  **San Francisco, California 94143-0435 (US)**
- **MADIREDDY, Lohith**
  **San Francisco, California 94158 (US)**

(74) Representative: **Schiweck Weinzierl Koch Patentanwälte Partnerschaft mbB**
**Ganghoferstraße 68 B**
**80339 München (DE)**

(56) References cited:
**WO-A1-2020/043693**

- **COOPER MEGAN A ET AL: "Isolation and characterization of human natural killer cell subsets", CURRENT PROTOCOLS IN IMMUNOLOGY, JOHN WILEY & SONS, INC, US, no. SUPPL. 60, 1 May 2004 (2004-05-01), pages 7.34.1 - 7.34.12, XP002536332, ISSN: 1934-3671**
- **RYAN D H ET AL: "Flow cytometry in the clinical laboratory", CLINICA CHIMICA ACTA, ELSEVIER BV, AMSTERDAM, NL, vol. 171, no. 2-3, 15 February 1988 (1988-02-15), pages 125 - 173, XP023967845, ISSN: 0009-8981, [retrieved on 19880215], DOI: 10.1016/0009-8981(88)90142-8**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- S. HAN ET AL: "Comprehensive Immunophenotyping of Cerebrospinal Fluid Cells in Patients with Neuroimmunological Diseases", THE JOURNAL OF IMMUNOLOGY, vol. 192, no. 6, 7 February 2014 (2014-02-07), US, pages 2551 - 2563, XP055218489, ISSN: 0022-1767, DOI: 10.4049/jimmunol.1302884
- HEMING MICHAEL ET AL: "Immune Cell Profiling of the Cerebrospinal Fluid Provides Pathogenetic Insights Into Inflammatory Neuropathies", FRONTIERS IN IMMUNOLOGY, vol. 10, 1 January 2019 (2019-01-01), pages 515, XP055842247, DOI: 10.3389/fimmu.2019.00515

## Description

### TECHNICAL FIELD OF THE INVENTION

The present invention relates to a method of stratifying a subject with neurological or psychiatric disease manifestation, preferably with neuro-inflammatory autoimmune diseases a) determining i) a level of B cells in a test cerebrospinal fluid (CSF) sample obtained from a subject; ii) a level of immune cells per μl in said test CSF sample obtained from said subject; iii) a level of NKT cells in said test CSF sample obtained from said subject; iv) a level of monocytes in said test CSF sample obtained from said subject, and v) a level of $CD56^{dim}$ $CD16^{+}$ NK cells in a test peripheral blood (PB) sample obtained from said subject, and b) stratifying said subject as suffering from neuro-inflammatory autoimmune diseases, if the following are fulfilled: i) the level of B cells is increased relative to corresponding levels of B cells in control CSF samples obtained from subjects not suffering from neuro-inflammatory autoimmune diseases; ii) the level of immune cells per μl is increased relative to corresponding levels of immune cells per μl in said control CSF samples obtained from said subjects not suffering from neuro-inflammatory autoimmune diseases; iii) the level of NKT cells is decreased relative to corresponding levels of NKT cells in said control CSF samples obtained from said subjects not suffering from neuro-inflammatory autoimmune diseases; iv) the level of monocytes is decreased relative to corresponding levels of monocytes in said control CSF samples obtained from said subjects not suffering from neuro-inflammatory autoimmune diseases; v) the level of $CD56^{dim}$ $CD16^{+}$ NK cells is decreased relative to corresponding levels of $CD56^{dim}$ $CD16^{+}$ NK cells in control PB samples obtained from said subjects not suffering from neuro-inflammatory autoimmune diseases, wherein the combination of said levels i) - v) of step b) is indicative for whether said subject is suspected to suffer from neuro-inflammatory autoimmune diseases or from another neurological or psychiatric disease manifestation other than neuro-inflammatory autoimmune disease. Further, the present invention relates to a data processing system comprising a processor configured to perform the method of the invention, a flow cytometry device capable of detecting the abovementioned levels and a computer program comprising instructions to cause the data processing system or the flow cytometry device to execute the steps of the method of the invention. Finally, the present invention relates to a kit comprising a fluorescently labeled binding partner for certain surface markers used in the method of the invention.

### BACKGROUND OF THE INVENTION

Neurological or psychiatric disorders, also called neurological or psychiatric disease manifestations include neuro-vascular (e.g. ischemic stroke), neuro-degenerative (e.g. Alzheimer's disease (AD), amyotrophic lateral sclerosis (ALS)), and neuro-inflammatory autoimmune diseases, together affecting hundreds of millions of people worldwide. Autoimmune inflammatory diseases of the CNS comprise a large and heterogeneous spectrum of disorders: some are characterized by disease-specific (auto-) antibodies (e.g. neuromyelitis optica spectrum disorders (NMOSD) (S. Jarius, B. Wildemann, F. Paul, Clin Exp Immunol 176, 149-164 (2014)), autoimmune encephalitides (AIEs) (J. Dalmauet al., Lancet Neurol 7, 1091-1098 (2008)], others feature alterations in the T-cell compartment (e.g. Susac syndrome (SuS) (C. C. Gross et al., Nat Commun10, 5779 (2019)), or alterations in both the adaptive (T- and B cells) and innate (NK cells, monocytes and dendritic cells) immune compartment (e.g. multiple sclerosis (MS) (H. Wiendl, Curr Opin Neurol 25, 302-305 (2012)). MS as the most prevalent autoimmune disease of the CNS with 2.3 million people affected worldwide is a paradigmatic example for a chronic neuro-inflammatory disease, comprising typical features of CNS autoimmunity including (i) a clinically heterogeneous disease course, (ii) continuous disease evolution over time, and (iii) putative pathophysiological heterogeneity within one disease (e.g. inflammatory versus neurodegenerative components).

Cerebrospinal fluid (CSF) is an ultrafiltrate of the serum that surrounds and protects the CNS parenchyma (J. J. Iliff et al., Sci Transl Med 4, 147ra111 (2012)). Under non-pathological conditions CSF contains 0.3% of serum proteins and distinct circulating immune-cell subsets playing an important role in immune surveillance of the CNS. Since pathophysiological changes in the CNS are reflected in the CSF (A. Lleo et al., Nat Rev Neurol 11, 41-55 (2015)), its analysis concomitant with radiological, neurophysiological, neuropsychological and other examinations facilitates diagnosis of neurological diseases. However, so far, conventional CSF parameters such as total cell counts, lactate, peripheral blood (PB) / CSF glucose quotient (QGluc), integrity of the blood-CSF barrier, total protein, and intrathecal Ig synthesis have been used for gross differentiation between infectious, autoimmune and degenerative CNS disorders (H. Tumani et al., DGNeurologie 2, 456-480 (2019)).

Whether a truly multi-dimensional CSF characterization including additional soluble and/or cellular parameters can support a more fine-grained distinction between clinically similar disease entities remains unknown. Multi-parameter flow cytometry is one of the platforms that enable such multi-dimensional characterization; although many other techniques have been applied to CSF (G. Meyer Zu Horste, et al. Trends Immunol 41, 341-354(2020)). Although several studies have performed multi-parameter analysis in the CSF, most focused on distinct neuro-immunological diseases. Moreover, many studies are limited by small sample sizes and lack of appropriate controls such as sufficient normative data on non-pathological conditions, explaining some of the contradictory results (S. Alvermann, et al. JAMA Neurol 71, 905-912 (2014)).

WO 2020/043693; S, Han et al. J Immunol 192(6), 2551-2563 (2014); and Heming, M. et al. Front Immunol 10, 5151 (2019) disclose cellular markers for neuro-autimmune diseases in CSF and methods of diagnostically using these.

Therefore, there is a need in the art to provide new, alternative methods of stratifying a subject with neurological or psychiatric disease manifestation, preferably with neuro-inflammatory autoimmune diseases using CSF and/or PB samples and determining certain cell levels therein, preferably bypassing costly differential diagnosis.

Therefore, the objective of the present invention is to comply with this need.

The solution of the present invention is described in the following, exemplified in the appended examples, illustrated in the figures and reflected in the claims.

## SUMMARY OF THE INVENTION

The present invention deals with a comprehensive study comparing disease related changes of standard and flow cytometry-derived parameters in the PB and/or in the CSF in a discovery cohort of 546 immunotherapy-naive patients with distinct neurological diseases (autoimmune, degenerative, vascular) of the CNS in comparison to non-inflammatory controls. By combining feature selection with dimensionality reduction and machine learning (ML), the present inventors identified particular levels of specific cells in the CSF (immune cells/μl, monocytes, NKT cells, B cells) and more strikingly in the PB ($CD56^{dim}$ NK cells), which are increased or decreased relative to corresponding levels of cells in control CSF and/or PB samples obtained from a cohort of healthy subjects, which are not suffering from neuro-inflammatory autoimmune disease. Those particular "markers" / "parameters" / "classifiers" (also called "pan-disease" marker/parameter/classifier) in the CSF and/or in the PB are altered (increased or decreased relative to corresponding cell levels in control samples as defined by step b) of the method of the present invention) across all autoimmune diseases of the CNS and are sensitive to immune-modulating therapies. Those alteration(s) as defined by step b) of the method refer(s) to the specific requirements for stratifying a subject with neuro-inflammatory autoimmune diseases. Thereby, it was found that a subject can be easily stratified with a neuro-inflammatory autoimmune disease based on one or more of these particular markers, bypassing costly differential diagnosis. If the preferred markers / cell levels mentioned above can be detected, but do not fulfil the particular requirements as defined by step b) of the method of the present invention in the subject being examined, it is indicative that said subject is suspected to suffer from another neurological or psychiatric disease manifestation other than neuro-inflammatory autoimmune diseases.

The additional occurrence of plasma cells concomitant with detected intrathecal immunoglobulin (Ig) synthesis and/or detected oligoclonal bands (OCBs) sub-differentiates variants of neuro-inflammatory autoimmune diseases (CNS-directed autoimmunity), thus representing so called "inter-autoimmunity" classifiers, which distinguish relapsing forms of MS (RMS) from other neuro-inflammatory autoimmune diseases. As in accordance with the pan-disease classifiers, also those classifiers are particularly altered (intrathecal immunoglobulin (Ig) synthesis and/or OCBs can be detected and the level of plasma cells is increased relative to corresponding levels of plasma cells in control samples) as will be defined in more detail below. Those alteration(s) refer(s) to the specific requirements for stratifying a subject with RMS. If said inter-autoimmunity classifiers as defined above do not fulfil the particular requirements of said classifiers for stratifying a subject as suffering from RMS, said method of the present invention comprising determining one or more pan-disease classifiers is also able to distinguish based on the additional occurrence of certain cell levels as defined elsewhere herein that said subject is suspected to suffer from NMOSD, SuS or AIE, which also refer to neuro-inflammatory autoimmune diseases. MS-specific changes including the occurrence of one or more pan-disease classifiers according to the method of the present invention as well as the additional occurrence of certain cell levels such as the level of CD4+ HLA-DR+ T cells as defined elsewhere herein form a continuum reflecting clinical disease development thereby supporting "intra-disease", preferably called "intra-MS" differentiation, which distinguishes whether said subject is suspected to suffer from progressive forms of MS (PMS). Again, if said inter-autoimmunity classifiers as defined above can be determined in a subject being examined, but the particular requirements of said classifiers for stratification of a subject as suffering from RMS are not fulfilled, said method of the present invention comprising determining one or more pan-disease classifiers is also able to distinguish based on the additional occurrence of certain cell levels as defined elsewhere herein that said subject is suspected to suffer from PMS.

Overall, the present invention reveals that cellular parameters in the PB and in the CSF contributing to shared and distinct pathophysiological immune-signatures can be employed to improve differential diagnosis between neurological or psychiatric disease manifestations and within neuro-inflammatory autoimmune diseases as well as within MS with a high prediction accuracy.

Thus, in a first aspect the present invention relates to a method of stratifying a subject with neurological or psychiatric disease manifestation, preferably with neuro-inflammatory autoimmune diseases, comprising a) determining i) a level of B cells in a test cerebrospinal fluid (CSF) sample obtained from a subject; ii) a level of immune cells per μl in said test CSF sample obtained from said subject; iii) a level of NKT cells in said test CSF sample obtained from said subject; iv) a level of monocytes in said test CSF sample obtained from said subject, and v) a level of $CDS6^{dim}$ $CD16^{+}$ NK cells in a test peripheral blood (PB) sample obtained from said subject, and b) stratifying said subject as suffering from neuro-inflammatory autoimmune diseases, if the following are fulfilled: i) the level of B cells is increased relative to corresponding levels of B cells in control CSF samples obtained from subjects not suffering from neuro-inflammatory autoimmune diseases; ii) the level of immune cells per μl is increased relative to corresponding levels of immune cells per μl in said control CSF samples obtained from said subjects not suffering from neuro-inflammatory autoimmune diseases; iii) the level of NKT cells is decreased relative to corresponding levels of NKT cells in said control CSF samples obtained from said subjects not suffering from neuro-inflammatory autoimmune diseases; iv) the level of monocytes is decreased relative to corresponding levels of monocytes in said control CSF samples obtained from said subjects not suffering from neuro-inflammatory autoimmune diseases; v) the level of $CD56^{dim}$ $CD16^{+}$ NK cells is decreased relative to corresponding levels of $CD56^{dim}$ $CD16^{+}$ NK cells in control PB samples obtained from said subjects not suffering from neuro-inflammatory autoimmune diseases, wherein the combination of said levels i) - v) of step b) is indicative for whether said subject is suspected to suffer from neuro-inflammatory autoimmune diseases or from another neurological or psychiatric disease manifestation other than neuro-inflammatory autoimmune disease.

Additionally, the present invention may also comprise the method as defined elsewhere herein, wherein determining the level of B cells comprises measuring one or more of the markers selected from the group consisting of CD1c, CD5, CD14, CD19, CD20, CD21, CD27, CD24, CD38, CD40, CD45, CD80, CD138, CXCR5, HLA-DR, IgD, IgM and TACI.

Further, the present invention may also encompass the method as defined elsewhere herein, wherein determining the level of NKT cells comprises measuring of one or more of the markers selected from the group consisting of CD1d, CD3, CD4, CD8, CD14, CD16, CD45, CD56, CD161 TCRγδ, Va24-Ja18, Vβ11, Vδ1 and Vδ2.

The present invention may also comprise the method as defined elsewhere herein, wherein determining the level of monocytes comprises measuring of one or more of the markers selected from the group consisting of CD11b, CD11c, CD14, CD16, CD45, CD62L, CD86, CD115, CD116, CD121, CD163, CD192, CD206, $CX_3CR1$ and HLA-DR.

In addition, the present invention may also envisage the method as defined elsewhere herein, wherein determining the level of $CD56^{dim}$ $CD16^{+}$ NK cells comprises measuring of one or more of the markers selected from the group consisting of CD14, CD16, CD45, CD56, CD94, CD122, CD159a, CD244, CD315, CD335, CD337, KIR, Tbet and EOMES.

Further, the present invention may also encompass the method as defined elsewhere herein, further comprising determining whether or not intrathecal immunoglobulin (Ig) synthesis can be detected in said simultaneously obtained test CSF and serum samples obtained from said subject.

Additionally, also comprised by the method as defined elsewhere herein is determining whether or not type 2 or type 3 oligoclonal bands can be detected in said simultaneously obtained test CSF and serum samples obtained from said subject.

Further, the present invention may also encompass the method as defined elsewhere herein, further comprising determining a level of plasma cells in said test CSF sample obtained from said subject, preferably wherein determining the level of plasma cells comprises measuring of one or more of the markers selected from the group consisting of BCMA, CD19, CD20, CD27, CD38, CD78, CD138, CD319, CXCR4 and HLA-DR.

Additionally, the present invention may also comprise the method as defined elsewhere herein, further stratifying said subject as suffering from relapsing forms of multiple sclerosis (RMS), if the following are fulfilled: i) intrathecal immunoglobulin (Ig) synthesis can be detected in said simultaneously obtained test CSF and serum samples obtained from said subject; and/or type 2 or type 3 oligoclonal bands can be detected in said simultaneously obtained test CSF and serum samples obtained from said subject; and ii) the determined level of plasma cells in said test CSF sample is increased relative to corresponding levels of plasma cells in control CSF samples obtained from subjects suffering from another neuro-inflammatory autoimmune disease other than RMS.

Further, the present invention may also comprise the method as defined elsewhere herein, further stratifying said subject as suffering from neuromyelitis optica spectrum disorders (NMOSD), if one or more of the following are fulfilled: i) a level of $CD56^{bright}$ $CD16^{dim/-}$ NK cells in said test PB sample is decreased relative to corresponding levels of $CD56^{bright}$ $CD16^{dim/-}$ NK cells in control PB samples obtained from subjects suffering from another neuro-inflammatory autoimmune disease other than NMOSD; ii) a level of lactate in said test CSF sample is increased relative to corresponding levels of lactate in control CSF samples obtained from subjects suffering from another neuro-inflammatory autoimmune disease other than NMOSD, preferably wherein determining the level of $CD56^{bright}$ $CD16^{dim/-}$ NK cells comprises measuring of one or more of the markers selected from the group consisting of CD14, CD16, CD25, CD45, CD56, CD94, CD122, CD159a, CD244, CD315, CD335, CD336, CD337, Tbet and EOMES.

Additionally, the present invention may also encompass the method as defined elsewhere herein, further stratifying said subject as suffering from Susac's syndrome (SuS), if one or more of the following are fulfilled: i) a level of $CD8^{+}$ $HLA\text{-}DR^{+}$ T cells in said test PB and/or in said test CSF sample is increased relative to corresponding levels of $CD8^{+}$ $HLA\text{-}DR^{+}$ T cells in control PB and/or control CSF samples obtained from subjects suffering from another neuro-inflammatory autoimmune disease other than SuS; ii) a level of $CD8^{+}$ T cells in said test PB and/or in said test CSF sample is increased relative to corresponding levels of $CD8^+$ T cells in control PB and/or control CSF samples obtained from subjects suffering from another neuro-inflammatory autoimmune disease other than SuS; iii) a level of $CD4^+$ T cells in said test PB and/or in said test CSF sample is decreased relative to corresponding levels of $CD4^+$ T cells in control PB and/or control CSF samples obtained from subjects suffering from another neuro-inflammatory autoimmune disease other than SuS, preferably wherein determining the level of $CD8^+$ HLA-$DR^+$ T cells comprises measuring of one or more of the markers selected from the group consisting of CD3, CD4, CD8, CD14, CD45, CD56 and HLA-DR; and wherein determining the level of $CD8^+$ T cells comprises measuring of one or more of the markers selected from the group consisting of CD3, CD4, CD8, CD14, CD45 and CD56; and wherein determining the level of $CD4^+$ T cells comprises measuring of one or more of the markers selected from the group consisting of CD3, CD4, CD8, CD14, CD45 and CD56.

The present invention may also comprise the method as defined elsewhere herein, further stratifying said subject as suffering from autoimmune encephalitis (AIE), if one or more of the following are fulfilled: i) a level of lymphocytes in said test CSF sample is decreased relative to corresponding levels of lymphocytes in control CSF samples obtained from subjects suffering from another neuro-inflammatory autoimmune disease other than AIE; ii) a level of NKT cells in said test CSF sample is increased relative to corresponding levels of NKT cells in control CSF samples obtained from subjects suffering from another neuro-inflammatory autoimmune disease other than AIE; iii) a level of monocytes in said test CSF sample is increased relative to corresponding levels of monocytes in control CSF samples obtained from subjects suffering from another neuro-inflammatory autoimmune disease other than AIE, preferably wherein determining the level of lymphocytes comprises measuring of one or more of the markers selected from the group consisting of CD14, CD15, CD45 and CD66b; and wherein determining the level of NKT cells comprises measuring of one or more of the markers selected from the group consisting of CD1d, CD3, CD4, CD8, CD14, CD16, CD45, CD56, CD161 TCRγδ, Vα24-Ja18, Vβ11, Vδ1 and Vδ2; and wherein determining the level of monocytes comprises measuring of one or more of the markers selected from the group consisting of CD11b, CD11c, CD14, CD16, CD45, CD62L, CD86, CD115, CD116, CD121, CD163, CD192, CD206, $CX_3CR1$ and HLA-DR.

The present invention may also envisage the method as defined elsewhere herein, further stratifying said subject as suffering from progressive forms of multiple sclerosis (PMS), if one or more of following are fulfilled: i) a level of $CD4^+$ HLA-$DR^+$ T cells in said test PB sample is increased relative to corresponding levels of $CD4^+$ HLA-$DR^+$ T cells in control PB samples obtained from subjects suffering from another neuro-inflammatory autoimmune disease other than PMS; ii) a level of monocytes in said test CSF sample is increased relative to corresponding levels of monocytes in said control CSF samples obtained from subjects suffering from another neuro-inflammatory autoimmune disease other than PMS.

In a second aspect, the present invention relates to a data processing system comprising a processor configured to perform the method of the invention, a flow cytometry device capable of detecting the abovementioned levels under the first aspect of the invention and a computer program comprising instructions to cause the data processing system or the flow cytometry device to execute the steps of the method of the invention.

In a third aspect, the present invention relates to a kit comprising a fluorescently labeled binding partner for CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and HLA-DR.

## BRIEF DESCRIPTION OF THE FIGURES

**Fig. 1: Study cohorts and design.**
(**A**) Data derived from patients undergoing routine as well as flow cytometric CSF analysis was investigated to identify factors assisting classification of neurological diseases. The results from this discovery cohort (first n numbers) were validated in a comparable validation cohort (second n numbers). From a total of 546/231 patients undergoing routine as well as flow cytometric CSF analysis, a group of 74/35 individuals was identified as non-inflammatory controls (upper box of the column entitled "Pan-disease"). Furthermore, 97/8 patients with neuro-vascular (ischemic stroke, black boxes of the column entitled "Pan-disease") and 93/156 patients with neuro-degenerative diseases (grey boxes of the column entitled "Pan-disease", Amyotrophic lateral sclerosis ALS, n =52/0; mild Alzheimer disease mAD, n = 41/156) were included in the study. 282/32 patients suffered from inflammatory (auto-) immune CNS diseases (lower box of the column entitled "Pan-disease"), which could be further differentiated into patients with multiple sclerosis (MS, n = 196/22, upper box of the column entitled "Inter-autoimmunity"), Neuromyelitis optica spectrum disease (NMOSD, n = 15/0, second box from top of the column entitled "Inter-autoimmunity"), Susac syndrome (SuS, n = 14/0, third box from top of the column entitled "Inter-autoimmunity"), or autoimmune encephalitis (AIE, n = 57/10, lower box of the column entitled "Inter-autoimmunity"). RRMS patients could be further divided into patients with radiologically or clinically isolated symptoms (RIS/CIS, n = 26/1), early relapsing-remitting MS (≤36 months since onset, n = 125/21), and RRMS >36 months since disease onset (n = 45/0) or into patients with active (n = 142/15) or inactive (n = 48/7) disease at the time of lumbar puncture. (**B**) Peripheral blood (PB), serum and CSF samples from patients included in the study were analyzed by routine CSF diagnostics and multi-parameter flow cytometry. 113 features characterizing the peripheral and intrathecal immune status derived from those analyses were investigated for co-linearity (center). Features with an absolute correlation of at least 0.9 with significant p-values were considered as correlated. From the correlating features only one was included in the further analyses. The remaining 34 parameters preserved the information of the initial 113 features describing the peripheral (top) and intrathecal (bottom) immune-profile of each patient. (**C**) Immune-profiles of patients with distinct neurological diseases and under homeostatic conditions from the discovery cohort were analyzed by combining feature selection with dimensionality reduction and machine learning approaches to identify disease-specific immune signatures. These signatures were summarized in composite scores to facilitate clinical practice and verified in a validation cohort.

**Fig. 2: Novel factors defining Inflammatory CNS diseases.**
(**A**) Patients from the discovery cohort under homeostatic conditions (non-inflammatory controls, circles, n = 74), inflammatory autoimmune CNS (inflammatory, triangles, n = 229: early RRMS incl. RIS/CIS, NMOSD, SuS, AIE), neuro-degenerative (squares, n = 93: ALS, mAD), or neuro-vascular (diamonds, n = 97) disorders were mapped based on immunological data from the peripheral blood (PB) and cerebrospinal fluid (CSF) following dimensionality reduction with UMAP. (**B**) Parameters represented in top 10 results are displayed as a heatmap for each condition visualizing the relative difference between the distinct neurological conditions with grey indicating the highest and black the lowest expression (left). Right: Mean and SD of prediction accuracy (PA) and area under the curve (AUC) from the top 10 results of up to 5 parameters as analyzed by logistic regression to differentiate patients with neuro-inflammatory autoimmune diseases from homeostatic, neuro-degenerative, and neuro-vascular conditions. (**C**) Volcano plots representing the median fold change of parameters between neuro-inflammatory and homoeostatic conditions (left), neuro-inflammatory and neuro-degenerative conditions (center), and neuro-inflammatory and neuro-vascular (right) conditions in the PB (top) and CSF (bottom). P-values were calculated by Mann-Whitney test. Only significantly ($p<0.05$) altered parameters are labelled. Dashed lines represent the 20% change of respective parameters. Consistent alterations across all analyses are highlighted by red bold font and underlined.

**Fig. 3: Identification of pan-disease parameters characterizing CNS neuro-Inflammation.**
(**A**) Heatmap (left) illustrating the relative changes and dot plot (right) displaying the fold changes of the 9 parameters identified in **Figure 2** in distinct inflammatory autoimmune diseases of the CNS. In the heatmap, grey indicates the highest expression, whereas black indicates the lowest expression. Parameter labeling provides information on the respective compartment (grey - PB; black - CSF). The black box highlights the 5 parameters simultaneously altered in all investigated neuro-inflammatory autoimmune diseases (early RRMS incl. RIS/CIS, arrow head up, n = 143; NMOSD, arrow head left, n = 15; Susac, arrow head right, n = 14; AIE, arrow head down, n = 57). (**B**) CD56$^{dim}$NK-cell frequencies in the PB of healthy donors (n = 71) and early RRMS patients (n = 115) from an independent cohort (NationMS cohort). Boxes indicate the median $\pm$IQR, while their error bars indicate the 10-90% percentile. P-values were calculated by Mann-Whitney test, *p<0.05, **p<0.01, ***p<0.001, ****p<0.0001. (**C**) Re-alignment plot illustrating the change in neuro-inflammatory parameters as a consequence of first line and escalation immune-therapies. The median height of each parameter within a) the non-inflammatory control, degenerative, and vascular cohort, b) RRMS patients treated with first line therapies (arrow head down; IFN-$\beta$ n = 9, glatiramer acetate n = 5, dimethyl fumarate n = 5), or c) escalation therapies (arrow head up; fingolimod n = 15, natalizumab n = 41, alemtuzumab (10-14 months post last injection) n = 30) was summed up and averaged as described for volcano plots. Medians were re-aligned by division of the result from the subtraction of the control median from the respective median by the result of the subtraction of the control median from the median of treatment-naive RRMS patients. For CD56$^{dim}$NK cells for example, controls were at 12.22%, naive RRMS at 10.55% and escalation therapies at 17.62%, resulting in (17.62% -12.22%)/(10.55%-12.22%) = -3.23, indicating an overcompensation (<0) beyond control levels. Thus, parameters overcompensated by immunotherapy are in the left part of the graph, whereas parameters with partial compensation for neuro-inflammatory alterations are in the middle part. Parameters exhibiting aggravated levels more different from control cohorts than untreated RRMS patients are represented in the right part of the graph. (**D**) Mean and SD of PA and AUC from the top 10 combinations of up to five neuro-inflammatory parameters as calculated by logistic regression for differentiating distinct inflammatory autoimmune diseases (RRMS - first triangle from left, NMOSD - second triangle from left, SuS - third triangle from left, AIE - fourth triangle from left) from non-inflammatory controls, neuro-degenerative, and neuro-vascular diseases. (**E**) Predictive score composed by division of parameters consistently increased by parameters decreased in neuro-inflammation (top). Receiver operating characteristic analysis (left) identified a cut-off of 2.8 distinguishing (right) patients with neuro-inflammatory diseases (infl, second column from left) from non-inflammatory controls (ctrl, first column from left), neuro-degenerative (deg, third column from left) and -vascular (vasc, fourth column from left) patients with an AUC of 83.5 and an odds ratio of 11.34. This composite score was verified in the validation cohort, resulting in the identification of neuro-inflammatory patients with an odds ratio of 15.45. (**F**) Neuro-inflammatory auto-immune disease parameters commonly altered in all neuro-inflammatory autoimmune diseases of the CNS in comparison to other neurological diseases as well homeostatic conditions.

**Fig. 4: Inter-autoimmunity classifiers characterizing distinct neuro-inflammatory diseases.**
(**A**) Best predicting parameters (left; CD56bright, $CD4^+$ T cells, $CD8^+$ T cells, $CD8^+$ HLA-$DR^+$; lymphocytes -PB; lactate, plasma cells, IgG synthesis, IgA synthesis, IgM synthesis, $CD8^+$ HLA-$DR^+$, plasma cells, lymphocytes, NKT cells, monocytes, $CD14^+CD16^+$ -CSF) included in the top 10 results of combinations of up to 5 parameters identified by machine learning differentiating early RRMS incl. RIS/CIS from NMOSD (first triangle from left), Susac (second triangle from left), and AIE (third triangle from left) in the discovery cohort as well as the respective mean and SD of prediction accuracy (PA) and area under the curve (AUC) (right). (**B**) Volcano plots showing the median fold change in parameters between early RRMS incl. RIS/CIS (n = 143) and NMOSD (left, n = 15), SuS (center, n = 14), and AIE (right, n = 57) in the PB (top) and CSF (bottom). Respective p-values were calculated by Mann-Whitney test. Only significantly ($p<0.05$) altered parameters are labelled. Consistent alterations across all analyses are highlighted by bold font and underlined. (**C**) PA and AUC of plasma cells occurrence and intrathecal IgG synthesis as parameters identified by exploratory data analysis determined by logistic regression for differentiating early RRMS incl. RIS/CIS from NMOSD (first triangle from left), Susac (second triangle from left), and AIE (third triangle from left). (**D**) Composite score (top left) derived by addition of plasma cell positivity (= 1) and intrathecal IgG synthesis (= 1) allowing differentiation (bottom) of RRMS patients from the discovery cohort from NMOSD, SuS, and AIE patients by a cut-off of 1.5 as determined by receiver operating characteristic analysis (top left) with an AUC of 84.0 and an odds ratio of 18.9. Inter-autoimmunity classification was verified in the validation cohort, showing classification with an odds ratio of 35.8. (**E**) inter autoimmunity classifiers distinguishing RRMS from other neuroinflammatory autoimmune diseases.

**Fig. 5: Factors describing RRMS disease evolution and activity.**
**(A)** Heatmap (left) illustrating the relative changes and dot plot (right) displaying the fold changes in pan- and inter-disease classifiers between patients with non-inflammatory diseases as well as patients from the discovery cohort with RIS/CIS (n = 26) or RRMS within (early, n = 125) and after 36 months (>36M, n = 45) from disease manifestation. Grey indicates the highest expression, whereas black indicates the lowest expression. Parameter labelling provides information on the respective compartment (grey - PB; black - CSF). **(B)** Volcano plots representing the median fold change in parameters between patients with RRMS without ("inactive", n = 48) and with ("active", n = 142) clinical and/or radiological disease activity within 4 weeks before lumbar puncture. P-values were calculated by Mann-Whitney test. Only significantly ($p<0.05$) altered parameters are labelled.

**Fig. 6:** Visualization of multi-level discrimination by flow-cytometric immune-profiling between (i) inflammatory auto-immune CNS diseases and other neurological diseases, (ii) RRMS and other inflammatory auto-immune CNS diseases as well as (iii) alteration of involved parameters during disease course.

**Fig. 7: Gating strategy.**
Immune cells in the peripheral blood (PB, top) and cerebrospinal fluid (CSF, bottom) were investigated by flow cytometry and identified by selecting leukocytes as CD45 expressing cells. Leukocyte subsets were separated into SSChighCD14- Granulocytes, SSCintCD14+ Monocytes, and SSClowCD14- Lymphocytes. Within total Lymphocytes, B cells and Plasma cells were identified as CD19highCD138- cells and CD19low CD138high cells, respectively. NK cells were selected as CD56+CD3- cells and further divided into CD56brightCD16dim/- ("CD56bright") and CD56dimCD16+ ("CD56dim") cells. Whereas NKT cells were identified as CD56+CD3+ cells, T cells were selected as CD3+CD56- cells and further differentiated based on CD4 and CD8 expression into CD4+CD8- ("CD4"), CD4+CD8+, and CD4-CD8+ ("CD8") T cells. CD4 and CD8 T cells were additionally investigated for expression of the activation marker HLA-DR. Monocyte subsets were identified as CD14highCD16-, CD14+CD16+, and CD14lowCD16high cells. Flow count fluorospheres ("Beads") were used as internal control.

**Fig. 8:** Volcano plot describing the distinct immune-cell composition in the CSF compared to PB. The median fold change in parameters between the PB and CSF of patients with non-inflammatory CNS diseases (n = 74) is plotted against the p-values calculated by Mann-Whitney test.

**Fig. 9:** Heatmap visualizing the relative difference in the investigated parameters between non-inflammatory controls (n = 74), neuro-inflammatory (n = 229), neuro-degenerative (n = 93), and neuro-vascular (n = 97) diseases in the discovery cohort. Grey indicates the highest expression, whereas black indicates the lowest expression. Parameter labelling provides information on the respective compartment (grey - PB; black - CSF).

**Fig. 10: (A)** Correlation analysis between sex (top) or age (bottom) and linear immunological parameters from the PB (grey) and CSF (back) from patients under homeostatic conditions from the discovery cohort. Only significant ($p<0.05$, Mann-Whitney test) correlation indices are shown. **(B)** Age-matched comparison of parameters (ctrl n = 24, infl n = 104, deg n = 20, vasc n = 47) affected by ageing and putatively related to differentiation of inflammatory from other CNS diseases. P-values were calculated by Kruskal-Wallis test with Dunn's post-test, *p<0.05, **p<0.01, ***p<0.001, ****p<0.0001.

**Fig. 11: Intra-autoimmunity classifiers characterizing relapsing-remitting versus primary-progressive MS. (A)** UMAP of non-inflammatory controls (circle, n = 74), RRMS (triangle up, RIS/CIS, early RRMS, RRMS >36M, n = 196) and PPMS (triangle down, n = 30) patients. **(B)** Top 10 combinations of up to 5 parameters (left; grey - PB, black - CSF) differentiating RRMS from PPMS patients as well as mean and SD of the respective prediction accuracy (PA) and area under the curve (AUC) calculated by logistic regression. **(C)** Volcano plots representing the median fold change in parameters between patients with RRMS and PPMS in the PB (left) and CSF (right). P-values were calculated by Mann-Whitney test. Only significantly ($p<0.05$) altered parameters are labelled with red labelling for parameters also identified by logistic regression. **(D)** Predictive score composed by division of parameters increased by parameters decreased in RRMS in comparison to PPMS (top). Receiver operating characteristic analysis (left) identified an optimal cut-off of 0.1 resulting in a differentiation (right) of both groups with an AUC of 76.9 and an odds ratio of 10.4. (E) Intra-autoimmunity classifiers distinguishing RRMS (left) from PPMS patients (right).

**Fig. 12: Composite score calculated from all five parameters showing the differences between distinct groups of patients with neurological and psychiatric disorders.**
Ctrl - non-inflammatory controls: Soma - somatoform disorders, IIH - idiopathic intracranial hypertension; Infl - inflammatory auto-immune CNS disorders: RIS - radiologically isolated syndrome, CIS - clinically isolated syndrome, RRMS - early RRMS <36 months since first symptoms, NMOSD - neuromyelitis optica spectrum disorder, SuS - Susac syndrome, AIE - autoimmune encephalitisPRMS - progressive RRMS >36 months since first symptoms, SPMS - secondary progressive MS, PPMS - primary progressive MS; Peri - neurological disorders of the peripheral nervous system: CIDP - Chronic inflammatory demyelinating polyneuropathy, GBS - Guillain-Barré syndrome; Deg - neuro-degenerativ disorders: AD - Alzheimer dementia, FTD - frontotemporal dementia, PSP - Progressive supranuclear palsy, ALS - amyotrophic lateral sclerosis; Vasc - neuro-vascular disorders: Stroke; Epi - epilepsy of non-inflammatory origin; Psych - schizophrenia; Rheuma - rheumatoid syndrome partially with CNS affection).

**TABLES.**

**Table 1: Patient demographics and routine CSF parameters.** Median (IQR) or percentage of patients for the respective parameters. Each first row shows information for the discovery cohort, whereas every second row provides information regarding the validation cohort. Na - not applicable.

**Table 2:** Top 10 results of up to 5 parameters (left column) for prediction accuracy (PA), area under the curve (AUC), sensitivity (SE), and specificity (SP) obtained by logistic regression including correction for age differentiating the indicated cohorts following an unsupervised (Machine learning) and supervised (exploratory) approach.

**Table 3:** Parameters replacing (left) co-linear parameters (right).

**Table 4: Peripheral and intrathecal cellular parameters of CNS diseases.** Median percentage, levels of significance (Mann-Whitney test: *p<0.05, **p<0.01, ***p<0.001, ****p<0.0001) including direction of alteration compared to non-inflammatory controls, as well as cells counts per μl for the discovery cohort (top) as well as for pan- and inter-CNS-autoimmunity classifiers from the validation cohort (bottom).

**DETAILED DESCRIPTION OF THE INVENTION**

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments described throughout the specification should not be construed to limit the present invention to only the explicitly described embodiments. This description should be understood to support and encompass embodiments which combine the explicitly described embodiments with any number of the disclosed and/or preferred elements. Furthermore, any permutations and combinations of all elements described herein should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. When used herein the term "comprising" can be substituted with the term "containing" or "including" or sometimes when used herein with the term "having". When used herein "consisting of" excludes any element, step, or ingredient not specified.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Unless otherwise indicated, the term "at least" preceding a series of elements is to be understood to refer to every element in the series. The term "at least one" refers to one or more such as one, two, three, four, five, six, seven, eight, nine, ten and more.

The term "and/or" wherever used herein includes the meaning of "and", "or" and "all or any other combination of the elements connected by said term".

When used herein "consisting of" excludes any element, step, or ingredient not specified in the claim element. When used herein, "consisting essentially of" does not exclude materials or steps that do not materially affect the basic and novel characteristics of the claim.

The term "including" means "including but not limited to". "Including" and "including but not limited to" are used interchangeably.

The term "about" means plus or minus 20%, preferably plus or minus 10%, more preferably plus or minus 5%, most preferably plus or minus 1%.

Throughout the description and claims of this specification, the singular encompasses the plural unless the context otherwise requires. In particular, where the indefinite article is used, the specification is to be understood as contemplating plurality as well as singularity, unless the context requires otherwise.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

A better understanding of the present invention and of its advantages will be gained from the examples, offered for illustrative purposes only. The examples are not intended to limit the scope of the present invention in any way.

***Method of the present invention***

As used herein, the method of the present invention may be applied to stratify a subject with a neurological or psychiatric disease manifestation. Such neurological or psychiatric disease manifestation relates *inter alia* to neuro-inflammatory autoimmune diseases, neuro-degenerative diseases, neuro-vascular diseases or non-inflammatory diseases as depicted in **Figure 1A.** The term "neuro-degenerative disease" as used herein refers to, but is not limited to, dementia such as Alzheimer's disease (short: AD) or frontotemporal lobe dementia (short: FTD), Lewy body disease, amyotrophic lateral sclerosis (ALS), parkinsons disease, atypical parkinson's syndromes, multisystem atrophies, and progressive supranuclear palsy (short: PSP). The term "neuro-vascular disease" as used herein refers to, but is not limited to, ischemic stroke, primary angiitis of the CNS, any other vasculitis affecting the vessels of the CNS and posterior reversible encephalopathy syndrome. The term "non-inflammatory disease" as used herein refers to, but is not limited to, somatisation disorders (short: Soma), idiopathic intercranial hypertension (short: IIH), normal pressure hydrocephalus (see **Fig. 12).** The term "neurological or psychiatric disease manifestation" may also refer to peripheral neuro-inflammatory disorders such as chronic inflammatory demyelinating polyradiculoneuropathy (short: CIDP) or Guillain-Barré syndrome (short: GBS); infectious and non-infectious meningites, meningoencephalitides, encephaltiides, epilepsia disorders (epilepsy), rheumatoid symptoms, cancer and cancer-associated disorders (e.g. paraneoplastic disorders, meningeosis), Rasmussen encephalitis, acute and chronic immune-mediated neuropathies, narcolepsia, polyneuropathy, toxic neuropathies, neurologic and psychiatric complications resulting from systemic inflammatory processes of different origin (infectious and non-infectious), psychosis, emotional disorders, schizophrenia, various types of encephalopathies, unclear radiological lesions/abnormalities upon imaging procedures (preferably Magentic resonsance imaging), pathological optic nerve manifestation, and inflammatory CSF syndromes (see **Fig. 12).** Preferably, the method of the present invention is used to stratify a subject with a suspicion for any or used to stratify a subject with a neuro-inflammatory autoimmune disease.

The term "neuro-inflammatory autoimmune disease" as used herein comprises, besides others, multiple sclerosis (short: MS), neuromyelitis optica spectrum disorders (short: NMOSD), Susac's syndrome (short: SuS), and autoimmune encephalitis (short: AIE) (see **Figure 1A).** Thus, said term is not limited to MS, NMOSD, SuS and AIE. Preferably, MS as one class of the neuro-inflammatory autoimmune diseases comprises relapsing forms of multiple sclerosis (short: RMS) or progressive forms of multiple sclerosis (PMS) as will be defined elsewhere herein. The term "inflammatory autoimmune CNS disease" can also be used interchangeably with the term "neuro-inflammatory autoimmune disease".

First, as step a) of the method of the present invention when a subject is being examined, one or more of the following classifiers need to be determined in said obtained samples of said subject: i) a level of B cells in a test cerebrospinal fluid (CSF) sample obtained from a subject; ii) a level of immune cells per μl in said test CSF sample obtained from said subject; iii) a level of natural killer T cells (NKT cells) in said test CSF sample obtained from said subject; iv) a level of monocytes in said test CSF sample obtained from said subject, and v) a level of $CD56^{dim}$ $CD16^{+}$ NK cells in a test peripheral blood (PB). Such one or more classifiers/markers/parameters refer to the term "pan-disease classifier/marker/parameter" as used throughout the present application. The term "marker" or "parameter" can be used interchangeably with the term "classifier". In this context, the term "immune cells per μl" may also refer to leukocytes per μl, thus comprising as another pan-disease classifier a level of leukocytes per μl in said test CSF sample obtained from said subject.

The term "detect" or "detecting" can be used interchangeably with the term "determine" or "determining" as used herein. The term "detect" or "detecting" as well as the term "determine" or "determining" when used herein in combination with the words "level", "amount" or "value", the words "detect", "detecting", "determine" or "determining" are understood to generally refer to a quantitative or a qualitative level. For example, when used in the context of detecting the level of a certain cell population, such as B cells, "detect", "detecting", "determine" or "determining" are understood to generally refer to a quantitative level. Accordingly, methods according to the invention that include a quantification of CD1c, CD5, CD14, CD19, CD20, CD21, CD27, CD24, CD38, CD40, CD45, CD80, CD138, CXCR5, HLA-DR, IgD, IgM and TACI means i.e. the amount or number of CD1cexpressing, CD5expressing, CD14expressing, CD19expressing, CD20expressing, CD21expressing, CD27expressing, CD24expressing, CD38expressing, CD40expressing, CD45expressing, CD80expressing, CD138expressing, CXCR5expressing, HLA-DRexpressing, IgDexpressing, IgMexpressing and TACIexpressing cells. In this regard the words "value," "amount" or "level" are used interchangeably herein. The invention involves detection / determination of the "level", i.e. number or (relative) amount of B cells. The level of specific cells may be expressed by the amount of specific cells being detected. It can also be expressed by the strength of a signal measured in the method of detecting the level of specific cells when immunofluorescence may be used, which may be combined with flow cytometry. The abovementioned can be applied to each cell level being determined by the method of the present invention.

Further, after the determination of said particular cell levels in said samples, for the stratification of said subject with a neurological or psychiatric disease manifestation, preferably with neuro-inflammatory autoimmune disease, as step b) of the method of the present invention each level of cells i) to v) of step a) need to be either increased or decreased relative to corresponding cell levels in control samples which have been obtained from a cohort of subjects which do not suffer from neuro-inflammatory autoimmune disease. Then, if the following requirements of step b) are fulfilled: i) the level of B cells is increased relative to corresponding levels of B cells in control CSF samples obtained from subjects not suffering from neuro-inflammatory autoimmune diseases; ii) the level of immune cells per μl is increased relative to corresponding levels of immune cells per μl in said control CSF samples obtained from said subjects not suffering from neuro-inflammatory autoimmune diseases; iii) the level of NKT cells is decreased relative to corresponding levels of NKT cells in said control CSF samples obtained from said subjects not suffering from neuro-inflammatory autoimmune diseases; iv) the level of monocytes is decreased relative to corresponding levels of monocytes in said control CSF samples obtained from said subjects not suffering from neuro-inflammatory autoimmune diseases; and v) the level of $CD56^{dim}$ $CD16^{+}$ NK cells is decreased relative to corresponding levels of $CD56^{dim}$ $CD16^{+}$ NK cells in control PB samples obtained from said subjects not suffering from neuro-inflammatory autoimmune diseases, it is indicative that said subject will suffer / is suspected to suffer from a neuro-inflammatory autoimmune disease.

For stratifying a subject with a neurological or psychiatric disease manifestation, preferably with neuro-inflammatory autoimmune disease, all of the pan-disease classifiers i) to v) in step a) of the method of the present invention need to be determined in said samples obtained from said subject being examined, which then either be increased or decreased as defined elsewhere herein relative to said corresponding levels of cells in the control samples. Thus, the combination of said particular cell levels as defined in i) to v) of step b) of the method of the present invention is indicative for whether said subject is suspected to suffer from a neuro-inflammatory autoimmune disease or from another neurological or psychiatric disease manifestation other than neuro-inflammatory autoimmune disease.

If the abovementioned pan-disease classifiers are determined in said obtained samples from said subject being examined, but the requirements for said classifiers in accordance with step b) of the method are not fulfilled, the subject will most likely not suffer from neuro-inflammatory autoimmune disease. Thus, the negative occurrence of said one or more classifiers i) to v), preferably of the combination of said classifiers i) to v) in said obtained samples of said subject, is an indication that said subject is suspected to suffer from another neurological or psychiatric disease manifestation other than neuro-inflammatory autoimmune disease. The method of the present invention thus makes it possible for a subject to be stratified with a neuro-inflammatory autoimmune disease if all of the pan-disease classifiers are being determined in said samples being obtained from said subject and if said requirements listed above under step b) of the method of the present invention are fulfilled, or being stratified with another neurological or psychiatric disease manifestation other than neuro-inflammatory autoimmune disease, if the samples being obtained from said subject comprise all of the pan-disease classifiers, but said classifiers do not fulfil the particular requirements as mentioned elsewhere herein.

The term "stratification / stratifying" as used herein and throughout the entire description refers to making a (risk) stratification that the subject will suffer / is suspected to suffer from neurological or psychiatric disease manifestation, preferably from neuro-inflammatory autoimmune disease as defined elsewhere herein in the near future, if said pan-disease classifiers are being determined in said samples being obtained from said subject. Thus, it comprises that there is a certain risk for the subject to suffer from said manifestation in the near future. Said term "stratify" / "stratifying" may thus refer that a subject is being suspected to suffer from neurological or psychiatric disease manifestation, preferably from neuro-inflammatory autoimmune diseases as defined elsewhere herein. In other words and also being comprised by said term, it refers to assuming that said subject being examined by using the method of the present invention might suffer from neurological or psychiatric disease manifestation, preferably from neuro-inflammatory autoimmune disease based on the general stratification test(s) available in the prior art (e.g., MRI), which has/have already been applied to said subject before the method of the present invention has been applied as a confirmation procedure.

According to the present invention, it may also be comprised that a subject not being stratified as defined herein with neurological or psychiatric disease manifestation, preferably with neuro-inflammatory autoimmune disease based on the general stratification test(s) available in the prior art (e.g., MRI), which has/have been applied to said subject before, may then be stratified with neurological or psychiatric disease manifestation, preferably with neuro-inflammatory autoimmune disease by applying the method of the present invention. Thus, the method of the present invention may also stratify a specific patient group, which has not been stratified with neurological or psychiatric disease manifestation, preferably with neuro-inflammatory autoimmune disease or which failed to be stratified with neurological or psychiatric disease manifestation, preferably with neuro-inflammatory autoimmune disease by applying classical stratification procedures known to a person skilled in the art.

Further the term "stratification / stratifying" as used herein and throughout the entire description may also relate to a diagnose or diagnosing that a subject suffers from neurological or psychiatric disease manifestation, preferably from neuro-inflammatory autoimmune disease or confirming a diagnosis that a subject suffers indeed from neurological or psychiatric disease manifestation, preferably from neuro-inflammatory autoimmune disease, which has been suspected to suffer from neurological or psychiatric disease manifestation, preferably from neuro-inflammatory autoimmune disease, before said method of the present invention has been applied to said subject. In this regard, a diagnosis is more concrete and reliable than a stratification of a subject as defined herein, since it is not associated with the risk of suffering from such disease in the near future, but a concrete detection or determination of such disease.

Thus, it is also comprised by the present invention that the method as defined comprising the determination of said pan-disease classifiers is being used to diagnose a subject with a neurological or psychiatric disease manifestation, preferably with neuro-inflammatory autoimmune disease.

The term "to have / having neurological or psychiatric disease manifestation, preferably a neuro-inflammatory autoimmune disease" can be used interchangeably with the term "to suffer from / suffering from neurological or psychiatric disease manifestation, preferably a neuro-inflammatory autoimmune disease". In general, when a subject suffers from a disease, said subject shows specific symptoms of the disease, whereas when a subject has a disease, said subject does not always have to show certain symptoms of the disease, but still is diagnosed with said disease. However, this general concept does not apply to neuro-inflammatory autoimmune diseases according to the present invention.

The term "subject" as used herein and throughout the entire description, also addressed as an individual, refers to a human or non-human animal, generally a mammal. A subject may be a mammalian species such as a rabbit, a mouse, a rat, a Guinea pig, a hamster, a dog, a cat, a pig, a cow, a goat, a sheep, a horse, a monkey, an ape or a human. Preferably, the subject being used in the present invention is a human. More preferably, said subject is an adult. Preferably, said adult is older than 18 years. More preferably, said adult is about 20 to 50 years, about 25 to 45 years, about 30 to 40 years, about 25 years, about 28 years, about 30 years, about 32 years old.

When a subject as defined herein is being examined and then stratified with neurological or psychiatric disease manifestation, preferably with neuro-inflammatory disease as mentioned above, a test CSF sample and/or a test PB sample is being obtained from said subject as known to the person skilled in the art. Those samples being obtained from said subject as defined herein are thus examined for said particular cell levels i) to v) of step a) of the method of the present invention. In more detail, said CSF sample is being examined for a level of B cells, a level of immune cells per $\mu$l, a level of NKT cells and a level of monocytes. Said PB sample is additionally examined in parallel for a level of CD56$^{dim}$ CD16$^{+}$ NK cells.

Typically, the sample is, essentially consists of, or includes CSF / PB from the subject. The term "essentially consists of" is understood to allow the presence of additional components in said CSF sample and PB sample or a composition that do not affect the properties of the sample or a composition. Examples of additional components may include, but are not limited to certain types of media, or any type of buffer.

The sample used in the method of the present invention is a CSF sample and a PB sample dependent on the fact that each classifier is determined in step a) of the method of the present invention as defined elsewhere herein. The CNS is enveloped and protected by CSF that provides a unique diagnostic option in clinical neurology. CSF is a clear, colorless body fluid found in the brain and spinal cord. It acts as a buffer for the brain, providing basic mechanical and immunological protection to the brain and serving a vital function in cerebral autoregulation of cerebral blood flow. CSF is derived from blood plasma and is largely similar to it. However, CSF is nearly protein-free compared with plasma and has some different electrolyte levels. In general, CSF is substantially free of red blood cells (erythrocytes), and at most contains a few white blood cells (leukocytes). The peripheral blood (PB) on the other hand is the flowing, circulating blood of the body. It is composed of erythrocytes, leukocytes and thrombocytes. These blood cells are suspended in blood plasma, through which the blood cells are circulated through the body.

In the present invention said CSF and said PB sample may refer to a "test" CSF and a "test" PB sample. This means that said sample(s) obtained from said subject according to the present invention is/are analyzed / examined by determining said specific cell levels as defined by step a) of the method of the present invention, wherein if the requirements of step b) of the method are fulfilled, it is indicative whether or not said subject may suffer from a neuro-inflammatory disease. The control CSF and the control PB sample(s) as comparison for whether said particular cell levels in the test samples as defined in step b) of the method of the present invention are either increased or decreased are taken from a patient cohort which have been diagnosed with not suffering from neuro-inflammatory autoimmune disease. Thus, this patient cohort may be considered as being healthy, thus not suffering from any other neurological or psychiatric disease manifestation other than neuro-inflammatory autoimmune disease or as not being healthy, thus suffering from another neurological or psychiatric disease manifestation other than neuro-inflammatory autoimmune disease. Patients of said cohort visiting the clinic over a course of about 10 years had been screened for eligibility, i.e. well-defined, treatment-naive, right diagnoses, complete data set, and subsequently included without further selection criteria.

The methods of the invention may include providing a sample from the subject or obtaining said sample from the subject. The CSF sample may be obtained by lumbar puncture. Lumbar puncture may be performed under sterile conditions by inserting a needle into the subarachnoid space, preferably between the third and fourth lumbar vertebrae. Then, CSF may be extracted through the needle, and tested. In a preferred embodiment a 20G Sprotte Canulae (Pajunk Medical) may be used for performing a lumbar puncture. The PB and serum samples may be obtained by venous puncture following typical procedures known to a person skilled in the art and then collected in particular tubes for said further determination of said particular cell levels or parameters.

The sample may have been taken at any desired point in time before carrying out the method of the invention. Generally, a time interval between taking the sample and carrying out the method of the invention is selected to allow analysis of viable cells. Cell viability can readily be determined using standard methods known in the art, e.g. neutral red uptake (NRU) or water soluble tetrazolium (WST-1). It is within the skilled artisan's experience to determine a respective time interval during which cells in a sample can be expected to remain viable.

It is envisaged by the invention that the sample may have been taken on the same or on the previous day, such as about 72 hours, about 48 hours, about 42 hours, about 36 hours, about 30 hours, about 28 hours, about 24 hours, about 18 hours, about 15 hours, about 12 hours, about 10 hours, about 8 hours, about 6 hours, about 4 hours, about 2 hours or less before the methods of the invention are being carried out. Preferably, it is envisaged by the invention that the sample is taken about 2 hours before the method of the invention is carried out. In other words, said sample preferably needs to be processed within about 2 hours after having obtained said sample. When the sample may be fixed and then analysed / examined as defined elsewhere herein, it may also be processed within about 72 hours (about 3 days). Also envisaged by the invention is that the sample may have been taken within a period of up to about 72 hours, i.e. 0 to about 72 hours, to about 48 hours, to about 42 hours, to about 36 hours, to about 30 hours, to about 28 hours, to about 24 hours, to about 18 hours, to about 15 hours or 0 to about 12 hours before the methods of the invention are being carried out. The subject, also addressed as a patient or an individual herein, from which/whom the sample has been obtained is an animal, generally a mammal as defined elsewhere herein.

The invention also contemplates that the sample from the individual may be a frozen sample. Generally, the sample may be frozen within the above detailed time intervals, e.g. 0 to about 72 or 0 to about 48 or 0 to about 42 hours as defined above, and/or at the above exemplified time points, such as about 72 hours, about 48 hours, about 36 hours or less, preferably about 2 hours, after the sample has been obtained from the individual. A frozen sample may be formed by freezing an obtained sample after adding a cryoprotective agent such as DMSO, glycerol and/or hydroxyethyl starch. As an illustrative example DMSO may be used in a final concentration in the range from about 2% to about 10 %, such as about 2%, about 4%, about 5% or about 10% DMSO. Typically, the sample is then frozen at a controlled rate to a temperature less than -50°C, whereafter the sample may for instance be stored, including long-term storage, at a temperature below -130°C such as -160°C, e.g. in liquid nitrogen for extended periods of time. Serum/CSF supernatant for determination of immunoglobulin levels, OCBs, lactate, glucose may also be cryoconserved.

The term "relative to" or just "to" as used within the present invention with regard to each stratification as defined herein, e.g. "the level of A" (relative) to "the level of B", generally means comparing "A" to "B". Thus, in the context of the present invention for example a level of B cells being determined in a test CSF sample being obtained from a subject to be examined is then compared to the levels of B cells in control CSF samples taken from a cohort of subjects which have been diagnosed to not suffer from neuro-inflammatory autoimmune disease, thus that the levels of B cells in the control samples can be considered as being corresponding to the level of B cells in the test sample obtained from said subject. Preferably, this comparison is carried out by comparing the median fold change in each classifier (each cell level determined by the method of the present invention for the different stratifications as defined herein), whereas p-values are preferably calculated by the Mann-Whitney test. Median fold changes and p-values cannot be determined from individual measurements but rather indicate systematic differences between groups of several individuals. (see f.e. **Fig. 2C, Fig. 4B** or **Fig. 11C).** The term "in comparison to" can be used interchangeably with the term "relative to" or just "to".

The term "increased" or "decreased" as used herein and throughout the entire description with regard to each stratification as defined herein, relates to an increased level of cells or a decreased level of cells concerning the quantity of the cells in view of their cell number. Thus, any elevation or reduction of the cell number can be considered as a relevant increase or decrease. According to the present invention, any recognizable alteration in form a higher or lower cell count in relation to corresponding levels of cells in said control samples may be considered as an increase or decrease. The increase or decrease of a cell number may be determined using flow cytometry as defined elsewhere herein. As an illustrative example, an increase or decrease may be in the range of at least about 0.4-fold, about 0.5-fold, about 0.6-fold, about 0.7-fold, 0.8-fold, 0.9-fold, 1-fold, about 1.1-fold, about 1.2-fold, about 1.3-fold, about 1.4-fold, about 1.5-fold, about 1.6-fold, about 1.7-fold, about 1.8-fold, about 1.9-fold, about 2-fold, or even about 3-fold.

Thus, it is further encompassed by the present invention that said level of B cells, said level of immune cells per μl, said level of NKT cells, said level of monocytes and said level of $CD56^{dim}$ $CD16^{+}$ NK cells are detected / determined using flow cytometry.

Flow cytometry-based analysis is typically combined with optical detection to identify and classify cells. This allows speed, sensitivity/specificity, and a non-invasive nature of the technique. Typically, fluorescent markers are used, which are compounds that bind to specific structures or molecules on the surface or within target cells. Such fluorescent markers are introduced into the mixture of cells, whereafter the mixture is rinsed to remove excess fluorescent markers. It is envisaged that flow cytometry may be combined with immunofluorescence. It is thus contemplated by the invention that the level of each specific cells defined above may be determined by detecting the specific surface molecules on the surface of each cells by using flow cytometry being combined with immunofluorescence. Therefore, in the present invention CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Va24-Ja18, Vβ11, Vδ1, Vδ2, KIR, Tbet, EOMES and/or HLA-DR may be detected on the surface of the specific cells in the samples using a flow cytometry based analysis.

Generally, the invention envisages that the detection of the biomarkers CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and/or HLA-DR can be carried out in a single step or can be carried out in more than one step, e.g. two steps, three steps or four steps. In preferred embodiments, the detection is carried out in a single step. A preferred method for the detection of said specific cell levels as defined above is flow cytometry such as FACS. The skilled person in the art knows that if detection of the biomarkers is to be carried out in more than one step, more than one sequences of steps can be selected. It is understood that the skilled person is able to recognize suitable sequences of steps for identifying the cell populations of interest. Also, if more than one step is carried out, the steps may partly involve enriching and/or isolating subpopulations. Again, the skilled person is able to recognize suitable sequences of steps and can judge whether those steps would reasonably involve an enrichment and/or isolation step for identifying the cell populations of interest.

Flow cytometry is a technique enabling counting, examining, and sorting microscopic particles such as biological cells suspended in a stream of fluid. It allows a simultaneous multiparametric analysis of the physical and chemical characteristics of single cells flowing through an optical detection device. An illustrative example of a well-established flow cytometry based analysis in the art is FACS. FACS allows sorting a heterogeneous mixture of cells into a plurality of containers, one cell at a time, based upon the specific light scattering and fluorescent characteristics of each cell. Thereby, FACS allows the sorting of subpopulations of cells of interest and their further use in *in vitro* and *in vivo* assays. FACS is often used in combination with monoclonal immunoglobulins as a reagent to detect cells as having a particular antigen, indicative of an expressed protein.

This technique allows the concurrent fast, objective and quantitative recording of fluorescent signals from individual cells and the physical separation of respective cells according to particular interest. Fluorescent signals used in flow cytometry, for instance when quantifying and/or sorting cells by any marker present on or in the cell, are typically fluorescently-tagged antibody preparations or fluorescently-tagged ligands for binding to antibodies or other antigen-, epitope- or ligand-specific agent, such as with biotin/avidin binding systems or fluorescently-labeled and optionally addressable beads (e.g. LUMINEX® microspheres). Depending of the equipment used, any desired detectable marker or combination of detectable markers can be detected by the optics of a flow cytometer. Current three-laser "multidimensional", flow cytometry devices enable up to 23 simultaneous single-cell measurements, such as two light scatter detectors and 21 fluorescence plus forward detectors allowing for example the detection of fluorescent surface/intracellular markers. Current three-laser, "multidimensional", FACS machines, if used as an illustrative example of flow cytometry, enable up to 14 simultaneous single-cell measurements, such as two light scatter detectors and 12 fluorescence plus forward detectors allowing for example the detection of fluorescent surface/intracellular markers. As an illustrative example, the three lasers of a flow cytometry device may be a krypton laser operating at 407 nm, an argon laser operating at 488 nm, and a dye laser operating at 595 nm. There are also devices with seven lasers and about 70 channels i.e. so-called spectral flow-cytometer, whereas about 40 channels may be realistic to use, which fall under the term "multi-dimensional" flow cytometers which may be used by the present invention.

The flow cytometry technique has been used extensively in relation to antigens expressed on the surface of cells, including cells that remain alive during, and after, flow cytometry. Similarly, the method has been used with intracellular reporter gene systems based on the expression of a detectably labeled gene product by the cell. Accordingly, the technique not only allows detecting the presence of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and/or HLA-DR on the cell surface, but also detecting the presence of RNA or DNA within the cell, for example RNA encoding CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Va24-Ja18, Vβ11, Vδ1, Vδ2, KIR, Tbet, EOMES and/or HLA-DR. Therefore, flow cytometry can also be used to determine the amount of nucleic acid formation from the genes, which encode CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Va24-Ja18, Vβ11, Vδ1, Vδ2, KIR, Tbet, EOMES and/or HLA-DR in cells of the sample from the subject.

In preferred embodiments when stratifying a subject with neurological or psychiatric disease manifestation, preferably with neuro-inflammatory disease, flow cytometry technique is used for detecting the level of B cells, the level of immune cells per μl, the level of NKT cells, the level of monocytes, and the level of $CD56^{dim}$ $CD16^+$ NK cells, preferably by detecting each specific cell surface marker / molecule such as CD1c, CD1d, CD3, CD4, CD5, CD8, CD11b, CD11c, CD14, CD16, CD19, CD20, CD21, CD27, CD24, CD38, CD40, CD45, CD56, CD62L, CD80, CD86, CD94, CD115, CD116, CD121, CD122, CD138, CD159a, CD161, CD163, CD192, CD206, CD335, CD337, CXCR5, $CX_3CR1$, IgD, IgM, TACI, TCRγδ, Vα24-Jα18, Vβ11, Vδ1, Vδ2, KIR, Tbet, EOMES and/or HLA-DR on the cell surface of each specific cells, which demonstrates a certain strength of a signal being measured.

In detail, determining the level of B cells comprises measuring one or more of the markers selected from CD1c, CD5, CD14, CD19, CD20, CD21, CD27, CD24, CD38, CD40, CD45, CD80, CD138, CXCR5, HLA-DR, IgD, IgM and TACI. The invention contemplates that *inter alia* the presence of CD19 (Cluster of Differentiation 19) and/or CD138 (Cluster of Differentiation 138) on the surface of a cell may be used to identify the cell as a B cell. B cells are also a type of white blood cell (leukocytes) of the lymphocyte subtype. They function in the adaptive immune system by secreting antibodies. Also B cells present antigens and secrete cytokines. They express B cell receptors (BCRs) on their cell surface, thereby allowing the B cell to bind specific antigens, against which the B cell will initiate an antibody response. By detecting further markers as defined herein being expressed by B cells, the determination of said cells in the sample is made more reliable.

According to the invention, identifying CD19+ cells in the sample serves in distinguishing CD19+ cells from other cells such as CD19- cells (such as monocytes, T cells, NK cells). CD19 also known as B-lymphocyte antigen CD19 is a transmembrane protein encoded in humans by the gene CD19 and is a biomarker for B lymphocyte development being widely expressed during all phases of B cell development until terminal differentiation into plasma cells. CD19 plays two major roles in human B cells: 1.) acting as an adaptor protein to recruit cytoplasmic signaling proteins to the membrane; 2.) working within the CD19/CD21 complex to decrease the threshold for B cell receptor signaling pathways. Further, identifying CD19+ CD138+ cells refer to B cells, also expressing CD138 (syndecan-1) which is a transmembrane proteoglycan preferably with a main cellular expression in stratified and simple epithelia and which is mainly confined to late stages of B-cell differentiation.

With regard to the level of immune cells per μl in said test CSF sample, preferably with regard to the level of leukocytes per μl in said test CSF sample, said cells are determined as defined herein by any cell marker known to a skilled person being expressed on said immune cells, preferably expressed on leukocytes, which comprise one or more of the markers selected from the group consisting of CD1c, CD1d, CD3, CD4, CD5, CD8, CD11b, CD11c, CD14, CD16, CD19, CD20, CD21, CD27, CD24, CD38, CD40, CD45, CD56, CD62L, CD80, CD86, CD94, CD115, CD116, CD121, CD122, CD138, CD159a, CD161, CD163, CD192, CD206, CD335, CD337, CXCR5 and $CX_3CR1$ IgD, IgM, TACI, TCRγδ, Va24-Ja18, Vβ11, Vδ1, Vδ2, KIR, Tbet, EOMES and/or HLA-DR. In the context of the present invention, distinct markers represent essential markers. Accordingly, CD45 can be considered as a pan leukocyte marker, which should be included everywhere. Further, CD45 can be considered as essential marker for the distinct subsets of cells, such as for B cells: *inter alia* CD19 and/or CD20; for NKT cells: *inter alia* CD3 and CD56; for monocytes: *inter alia* CD14 and CD16; and for $CD56^{dim}$ $CD16^{+}$ NK cells: *inter alia* CD56 and CD16. The present invention thus contemplates that the presence of the CD45 on the surface of a cell may be used to identify the cell as a leukocyte. CD45 (Cluster of Differentiation 45 or leukocyte common antigen) protein is a member of the protein tyrosine phosphatase (PTP) family and is expressed by all leukocytes. PTPs are known to be signaling molecules that regulate a variety of cellular processes including cell growth, differentiation, mitotic cycle, and oncogenic transformation. This PTP contains an extracellular domain, a single transmembrane segment and two tandem intracytoplasmic catalytic domains, and thus belongs to receptor type PTP. CD45 is a type I transmembrane protein that is in various forms present on all differentiated hematopoietic cells, except erythrocytes and plasma cells that assists in the activation of those cells (a form of co-stimulation).

Additionally, determining the level of NKT cells as another pan-disease classifier as defined herein comprises measuring of one or more of the markers selected from CD1d, CD3, CD4, CD8, CD14, CD16, CD45, CD56, CD161 TCRγδ, Vα24-Jα18, Vβ11, Vδ1, and Vδ2. NKT cells are a subset of T cells that co-express a αβ T-cell receptor, but also express a variety of molecular markers that are typically associated with NK cells such as CD16 and CD56 expression. Many of these cells recognize the non-polymorphic CD1d molecule, an antigen-presenting molecule that binds self and foreign lipids and glycolipids. There are three groups of NKT cells: type 1, type 2 NKT and NKT-like cells. Type 1 NKT cells refer to classical NKT cells and express CD1d as well as T cell receptor repertoire Va24-Ja18 and/or Vβ11. Type 2 NKT cells refer to non-classical NKT cells, which are also restricted to the expression of CD1d and express a diverse T-cell receptor repertoire. NKT-like cells express instead of CD1d MHCs and also a diverse T-cell receptor repertoire.

Further, determining the level of monocytes as another pan-disease classifier as defined herein comprises measuring of one or more of the markers selected from CD11b, CD11c, CD14, CD16, CD45, CD62L, CD86, CD115, CD116, CD121, CD163, CD192, CD206, $CX_3CR1$, and HLA-DR. Also the invention contemplates that the presence of CD14 (Cluster of Differentiation 14) and/or CD16 (Cluster of Differentiation 16) on the surface of a cell may be used to identify the cell as a monocyte. As mentioned elsewhere herein, CD16 may also be expressed not only on monocytes, but also on NK cells. Thus, another surface molecule may be determined to detect the level of monocytes, such as CD14. Monocytes represent a heterogeneous population of primary immune effector cells. They are the largest type of leukocyte and can differentiate into macrophages and myeloid lineage dendritic cells. There are three different subsets being distinguished based on their expression of CD14 and the low-affinity CD16 (FcγRIII): CD14++CD16- classical monocytes, which are characterized by high level expression of the CD14 cell surface receptor. CD14++CD16+ intermediate monocytes, which are characterized by high level expression of CD14 and low-level expression of CD16 and CD14+CD16++ non-classical monocytes, which are characterized by low level expression of CD14 and high co-expression of the CD16 receptor. According to the present invention, the level of monocytes as being determined in step a) of the method of the present invention preferably refers to CD14+CD16- monocytes which refer to classical monocytes as defined above.

Additionally, determining the level of $CD56^{dim}$ $CD16^{+}$ NK cells as another pan-disease classifier as defined herein comprises measuring of one or more of the markers selected from CD14, CD16, CD45, CD56, CD94, CD122, CD159a, CD244, CD315, CD335, CD337, KIR, Tbet, and EOMES. The invention also contemplates that the presence of the FcγRIII (CD16) and NCAM (neural cell adhesion molecule, also called CD56) on the surface of a cell may be used to identify the cell as a natural killer cell (NK cell). NK cells are a type of cytotoxic lymphocytes providing rapid responses to viral-infected cells and respond to tumor formation for example. They do not express T-cell antigen receptors (TCR) or pan T marker CD3, but they express the surface marker CD16 and CD56 in humans. According to the invention, identifying CD16+CD56+ cells in the sample serves in distinguishing CD16+ CD56+ cells from other cells such as CD16- CD56- cells. Antibodies that bind to antigens can be recognised by FcγRIII (CD16) receptors expressed on NK cells, resulting in NK activation, release of cytolytic granules and consequent cell apoptosis. CD16 may also be found not only on the surface of NK cells, but also on neutrophil polymorphonuclear leukocytes, monocytes and macrophages. Therefore, NK cells need to be distinguished from for example monocytes also expressing CD16 by another surface molecule such as CD56, which is most stringently associated with NK cells. CD16 has been identified as Fc receptors FcγRIIIa (CD16a) and FcγRIIIb (CD16b), which participate in signal transduction. While FcγRIIIa is expressed on mast cells, macrophages, and natural killer cells as a transmembrane receptor, FcγRIIIb is only expressed on neutrophils.

In the present invention NK cells were selected as $CD56^{+}CD3^{-}$ cells and further divided into $CD56^{bright}CD16^{dim/-}$ ("$CD56^{bright}$") and $CD56^{dim}CD16^{+}$ ("$CD56^{dim}$") cells. $CD56^{bright}$ NK cells are similar to T helper cells in exerting their influence by releasing cytokines. They are mainly found in bone marrow, secondary lymphoid tissue, liver, and skin, whereas $CD56^{dim}$ NK cells are primarily found in the peripheral blood, and are characterized by their cell killing ability. $CD56^{bright}$ NK cells may also be found in PB, but being decreased in comparison to $CD56^{dim}$ NK cells. $CD56^{dim}$ NK cells are always CD16 positive, whereas $CD56^{bright}$ can transition into $CD56^{dim}$ by acquiring CD16.

The term "measuring a surface marker / molecule" refers to measuring the signal of a detectable marker, which is attached to a binding partner that recognizes the specific surface marker / molecule expressed by the specific cell when using flow cytometry. Said signal may then be converted by the process called gating, which is known to a person skilled in the art, which then demonstrates the level (or relative amount) of cells being detected in said sample. The gating comprises among other factors measuring of distinct cell populations using forward scatter (FSC) and side scatter (SSC). Measuring FSC and SSC in combination allows to some extend the differentiation of a cell populations within heterogenous cell populations. The determination of FSC allows the discrimination of cells by size. The determination of SCS allows the determination of internal complexity of the cells (such as granularity). For example, intracellular granules and the nucleus increase SSC.

The measurement used is generally selected to be of a sensitivity of detection that allows detection of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and/or HLA-DR expressing cells in the range of a selected threshold value, in particular of a sensitivity of detection that allows determining whether CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Va24-Ja18, Vβ11, Vδ1, Vδ2, KIR, Tbet, EOMES and/or HLA-DR expressing cells are above the threshold.

It is understood that if detection is carried out using flow cytometry, a binding partner of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and/or HLA-DR may be used, preferably linked to excitable fluorescent dyes or proteins. Such a binding partner of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Va24-Ja18, Vβ11, Vδ1, Vδ2, KIR, Tbet, EOMES and/or HLA-DR has a detectable affinity and specificity for CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Ja18, Vβ11, Vδ1, Vδ2, KIR, Tbet, EOMES and/or HLA-DR. Typically, binding is considered specific when the binding affinity is higher than $10^{-6}$ M. A binding partner of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and/or HLA-DR, has in some embodiments an affinity of about $10^{-8}$ M or higher, or of about $10^{-9}$ M or higher. As indicated above, in some embodiments for example B cells in the sample are identified by the presence of the CD19 protein on their surface; or B cells may be enriched or isolated via the presence of the CD19 protein on their surface. Identification of CD19+ B cells may be carried out using spectroscopic, photochemical, photometric, fluorometric, radiological, enzymatic or thermodynamic means. Identification and enrichment or isolation of B cells may likewise be carried out by using a suitable binding partner of CD19+. Accordingly, the abovementioned applies *mutatis mutandis* to identifying and enriching or isolating all kinds of immune cells, NKT cells, classical and non-classical monocytes, $CD56^{dim}$ $CD16^{+}$ NK cells, plasma cells, $CD56^{bright}$ $CD16^{dim/-}$ NK cells, CD8+ HLA-DR+ T cells, CD8+ T cells, CD4+ T cells, lymphocytes in general, and/or CD4+ HLA-DR+ T cells by using a suitable binding partner of their specific surface molecules.

Further, in some embodiments for example T cells may be identified or isolated in a similar manner, using suitable surface proteins known in the art, for example the T cell receptor. In some embodiments a suitable binding partner of CD3 and a further suitable binding partner of a surface protein characteristic for T cells such as the T cell receptor are combined to identify CD3+ T cells. Accordingly, the abovementioned applies *mutatis mutandis* to identifying and enriching or isolating all kinds of B cells, immune cells, NKT cells, classical and non-classical monocytes, $CD56^{dim}$ $CD16^{+}$ NK cells, plasma cells, $CD56^{bright}$ $CD16^{dim/-}$ NK cells, and/or lymphocytes in general by using a suitable binding partner of their specific surface molecules and a further suitable binding partner of a surface protein characteristic for said cells.

Immunofluorescence being used in flow cytometry is generally achieved using a binding partner, which is linked to, or includes, a fluorophore as a detectable marker. Typically, a binding partner of CD19 f.e. may be used in combination with a detectable marker or the binding partner is functionally linked to a detectable marker. Likewise, a binding partner of CD1c, CD1d, CD3, CD4, CD5, CD8, CD11b, CD11c, CD14, CD16, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and/or HLA-DR may be used in combination with a detectable marker or the binding partner is functionally linked to a detectable marker.

In some embodiments suitable binding partners of any one of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES or HLA-DR are combined to identify cells expressing a combination of biomarkers selected from CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Va24-Ja18, Vβ11, Vδ1, Vδ2, KIR, Tbet, EOMES and HLA-DR. In an illustrative example, suitable binding partners of any one of CD1c, CD5, CD14, CD19, CD20, CD21, CD27, CD24, CD38, CD40, CD45, CD80, CD138, CXCR5, HLA-DR, IgD, IgM or TACI are combined to identify B cells. In another illustrative example, suitable binding partners of any one of CD1d, CD3, CD4, CD8, CD14, CD16, CD45, CD56, CD161 TCRγδ, Va24-Ja18, Vβ11, Vδ1 or Vδ2 are combined to identify NKT cells. In a further illustrative example, suitable binding partners of any one of CD11b, CD11c, CD14, CD16, CD45, CD62L, CD86, CD115, CD116, CD121, CD163, CD192, CD206, $CX_3CR1$ or HLA-DR are combined to identify monocytes. In another illustrative example, suitable binding partners of any one of CD14, CD16, CD45, CD56, CD94, CD122, CD159a, CD244, CD315, CD335, CD337, KIR, Tbet or EOMES are combined to identify $CD56^{dim}$ $CD16^{+}$ NK cells. In another illustrative example, suitable binding partners of any one of BCMA, CD19, CD20, CD27, CD38, CD78, CD138, CD319, CXCR4 or HLA-DR are combined to identify plasma cells. In another illustrative example, suitable binding partners of any one of CD14, CD16, CD25, CD45, CD56, CD94, CD122, CD159a, CD244, CD315, CD335, CD336, CD337, Tbet or EOMES are combined to identify $CD56^{bright}$ $CD16^{dim/-}$ NK cells. In another illustrative example, suitable binding partners of any one of CD3, CD4, CD8, CD14, CD45, CD56 or HLA-DR are combined to identify $CD8^{+}$ $HLA\text{-}DR^{+}$ T cells. In another illustrative example, suitable binding partners of any one of CD3, CD4, CD8, CD14, CD45 or CD56 are combined to identify $CD8^{+}$ T cells. In another illustrative example, suitable binding partners of any one of CD3, CD4, CD8, CD14, CD45 or CD56 are combined to identify $CD4^{+}$ T cells. In another illustrative example, suitable binding partners of any one of CD14, CD15, CD45 or CD66b are combined to identify lymphocytes. In another illustrative example, suitable binding partners of any one of CD3, CD4, CD8, CD14, CD45, CD56 or HLA-DR are combined to identify $CD4^{+}$ $HLA\text{-}DR^{+}$ T cells. The abovementioned binding partners may be functionally linked to a detectable marker.

In a preferred embodiment of the present invention, a suitable binding partner of CD19 and a suitable binding partner of CD20 are combined to identify B cells. In a further preferred embodiment of the invention, a suitable binding partner of CD3 and a suitable binding partner of CD56 are combined to identify NKT cells. Further preferred according to the invention, a suitable binding partner of CD14 and a suitable binding partner of CD16 are combined to identify monocytes. In a further preferred embodiment of the invention, a suitable binding partner of CD56 and a suitable binding partner of CD16 are combined to identify $CD56^{dim}$ CD16+ NK cells.

A respective binding partner of e.g. CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Va24-Ja18, Vβ11, Vδ1, Vδ2, KIR, Tbet, EOMES and/or HLA-DR as well as a binding partner for another selected cell-characteristic protein, may be an immunoglobulin, a fragment thereof or a proteinaceous binding molecule with immunoglobulin-like functions. An antibody fragment generally contains an antigen binding or variable region. Examples of (recombinant) antibody fragments are immunoglobulin fragments such as Fab fragments, Fab' fragments, Fv fragments, single-chain Fv fragments (scFv), diabodies or domain antibodies (Holt, L.J., et al., Trends Biotechnol. (2003), 21, 11, 484-490). An example of a proteinaceous binding molecule with immunoglobulin-like functions is a mutein based on a polypeptide of the lipocalin family (WO 03/029462, Beste et al., Proc Nat. Acad Sci 1999; 96:1898-1903). Lipocalins, such as the bilin binding protein, the human neutrophil gelatinase-associated lipocalin, human Apolipoprotein D or glycodelin, possess natural ligand-binding sites that can be modified so that they bind to selected small protein regions known as haptens. Examples of other proteinaceous binding molecules are the so-called glubodies (see e.g. international patent application WO 96/23879 or Napolitano et al., Chemistry & Biology 1996; 3(5):359-367), proteins based on the ankyrin scaffold (Mosavi et al., Protein Science 2004; 13(6):1435-1448) or crystalline scaffold (e.g. internation patent application WO 01/04144), the proteins described in Skerra, J. Mol. Recognit. 2000; 13:167-187, AdNectins, tetranectins and avimers. Avimers contain so called A-domains that occur as strings of multiple domains in several cell surface receptors (Silverman et al., Nature Biotechnology 2005; 23:1556-1561). Adnectins, derived from a domain of human fibronectin, contain three loops that can be engineered for immunoglobulin-like binding to targets (Gill & Damle, Current Opinion in Biotechnology 2006; 17:653-658). Tetranectins, derived from the respective human homotrimeric protein, likewise contain loop regions in a C-type lectin domain that can be engineered for desired binding. Peptoids, which can act as protein ligands, are oligo(N-alkyl) glycines that differ from peptides in that the side chain is connected to the amide nitrogen rather than the $\alpha$ carbon atom. Peptoids are typically resistant to proteases and other modifying enzymes and can have a much higher cell permeability than peptides (see e.g. Kwon and Kodadek, J. Am. Chem. Soc. 2007; 129:1508-1509). A suitable antibody may in some embodiments also be a multispecific antibody that includes several immunoglobulin fragments.

An immunoglobulin or a proteinaceous binding molecule with immunoglobulin-like functions may be PEGylated or hyperglycosylated if desired. In some embodiments a proteinaceous binding molecule with immunoglobulin-like functions is a fusion protein of one of the exemplary proteinaceous binding molecules above and an albumin-binding domain, for instance an albumin-binding domain of streptococcal protein G. In some embodiments, a proteinaceous binding molecule with immunoglobulin-like functions is a fusion protein of an immunoglobulin fragment, such as a single-chain diabody, and an immunoglobulin binding domain, for instance a bacterial immunoglobulin binding domain. As an illustrative example, a single-chain diabody may be fused to domain B of staphylococcal protein A as described by Unverdorben et al., Protein Engineering, Design & Selection 2012; 25:81-88.

An immunoglobulin may be monoclonal or polyclonal. The term "polyclonal" refers to immunoglobulins that are heterogenous populations of immunoglobulin molecules derived from the sera of animals immunized with an antigen or an antigenic functional derivative thereof. For the production of polyclonal immunoglobulins, one or more of various host animals may be immunized by injection with the antigen. Various adjuvants may be used to increase the immunological response, depending on the host species. "Monoclonal immunoglobulins", also called "monoclonal antibodies", are substantially homogenous populations of immunoglobulins to a particular antigen. They may be obtained by any technique which provides for the production of immunoglobulin molecules by continuous cell lines in culture. Monoclonal immunoglobulins may be obtained by methods well known to those skilled in the art (see for example, Köhler et al., Nature (1975) 256, 495-497, and U.S. Patent No. 4,376,110). An immunoglobulin or immunoglobulin fragment with specific binding affinity only for said particular cell surface markers as defined elsewhere herein can be isolated, enriched, or purified from a prokaryotic or eukaryotic organism. Routine methods known to those skilled in the art enable production of both immunoglobulins or immunoglobulin fragments and proteinaceous binding molecules with immunoglobulin-like functions, in both prokaryotic and eukaryotic organisms.

In more detail, an immunoglobulin may be isolated by comparing its binding affinity to a protein of interest, e.g. CD19, with its binding affinity to other polypeptides. Humanized forms of the antibodies of the present invention may be generated using one of the procedures known in the art such as chimerization or CDR grafting. In general, techniques for preparing monoclonal antibodies and hybridomas are well known in the art. Any animal such as a goat, a mouse or a rabbit that is known to produce antibodies can be immunized with the selected polypeptide, e.g. CD19. Methods for immunization are well known in the art. Such methods include subcutaneous or intraperitoneal injection of the polypeptide. One skilled in the art will recognize that the amount of polypeptide used for immunization and the immunization regimen will vary based on the animal which is immunized, including the species of mammal immunized, its immune status and the body weight of the mammal, as well as the antigenicity of the polypeptide and the site of injection.

As indicated above, a detectable marker may be coupled to a binding partner of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCR$\gamma\delta$, V$\alpha$24-J$\alpha$18, V$\beta$11, V$\delta$1, V$\delta$2, KIR, Tbet, EOMES and/or HLA-DR as the case may be, or a molecule that forms a complex with the binding partner of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Va24-Ja18, Vβ11, Vδ1, Vδ2, KIR, Tbet, EOMES and/or HLA-DR. In some embodiments, a detectable marker being coupled to a binding partner may refer to a "fluorescently labelled binding partner". A respective detectable marker, which may be coupled to a binding partner of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and/or HLA-DR, or a molecule that forms a complex therewith, may be an optically detectable label, a fluorophore, or a chromophore. Examples of suitable labels include, but are not limited to, an organic molecule, an enzyme, a radioactive, fluorescent, and/or chromogenic moiety, a luminescent moiety, a hapten, digoxigenin, biotin, a metal complex, a metal and colloidal gold. Accordingly an excitable fluorescent dye, a radioactive amino acid, a fluorescent protein or an enzyme may for instance be used to detect e.g. the level of CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Ja18, Vβ11, Vδ1, Vδ2, KIR, Tbet, EOMES and/or HLA-DR. Examples of suitable fluorescent dyes include, but are not limited to, Krome Orange (KrO), fluorescein (FITC), fluorescein isothiocyanate, 5,6-carboxymethyl fluorescein, Cascade Blue®, Oregon Green®, Texas red, nitrobenz-2-oxa-1,3-diazol-4-yl, coumarin, dansyl chloride, rhodamine, amino-methyl coumarin, DAPI, Eosin, Erythrosin, BODIPY®, pyrene, lissamine, xanthene, acridine, a fluorescent brightener (PB), an oxazine, phycoerythrin, a Cy dye such as Cy3, Cy3.5, Cy5, Cy5PE, Cy5.5, Cy7, Cy7PE or Cy7APC, an Alexa dye such as Alexa 647, Alexa 750 or Alexa 700, and NBD (Naphthol basic dye). Examples of suitable fluorescent protein include, but are not limited to, EGFP, emerald, EYFP, a phycobiliprotein such as phycoerythrin (PE) or allophycocyanin (APC), Monomeric Red Fluorescent Protein (mRFP), mOrange, mPlum and mCherry. In some embodiments a reversibly photoswitchable fluorescent protein such as Dronpa, bsDronpa and Padron may be employed (Andresen, M., et al., Nature Biotechnology (2008) 26, 9, 1035). Regarding suitable enzymes, alkaline phosphatase, soybean peroxidase, or horseradish peroxidase may serve as a few illustrative examples. In a further embodiment, tandem conjugates such as PE-Cy5.5 or PE-Cy7 may also be used. In some embodiments a method of detection may include electrophoresis, HPLC, flow cytometry, fluorescence correlation spectroscopy or a modified form of these techniques. Some or all of these steps may be part of an automated separation/detection system.

In a preferred example, KrO may be used as a detectable marker (fluorescent dye) of the binding partner for CD45. PE-Cy5.5 may be used as a detectable marker (fluorescent dye) of the binding partner for CD3. APC may be used as a detectable marker (fluorescent dye) of the binding partner for CD4. PB may be used as a detectable marker (fluorescent dye) of the binding partner for CD8. FITC may be used as a detectable marker (fluorescent dye) of the binding partner for CD14. AF750 may be used as a detectable marker (fluorescent dye) of the binding partner for CD16. AF700 may be used as a detectable marker (fluorescent dye) of the binding partner for CD19. PE-Cy7 may be used as a detectable marker (fluorescent dye) of the binding partner for CD56. PE may be used as a detectable marker (fluorescent dye) of the binding partner for CD138.

The present inventors further generated a so called "basic composite score" (also called "composite score 1") for stratifying a subject with neuro-inflammatory autoimmune disease by dividing particular determined cell levels of said pan-disease classifiers which are consistently increased by other particular determined cell levels of said pan-disease classifiers which are consistently decreased (see the Example section hereto). In other words, a basic composite score may be generated by the ratio of said determined level of immune cells per μl in said test CSF sample multiplied with said determined level of B cells in said test CSF sample and then also multiplied by 100 to said determined level of $CD56^{dim}$ $CD16^{+}$ NK cells in said test PB sample multiplied with said determined level of monocytes in said test CSF sample multiplied with said determined level of NKT cells in said test CSF sample. In this context, the term "ratio" refers to the division of said multiplication products of said increased levels of cells as defined elsewhere herein by said multiplications products of said decreased levels of cells as defined elsewhere (see **Fig. 3E).** Thus, it is also comprised herein the method of the present invention as defined by step a) mentioned elsewhere herein, wherein the stratifying step b) instead comprises multiplying i) said determined level of immune cells per μl in said test CSF sample with said determined level of B cells in said test CSF sample and with the value of 100, thereby obtaining a multiplication product 1; ii) said determined level of $CD56^{dim}$ $CD16^{+}$ NK cells in said test PB sample with said determined level of monocytes in said test CSF sample and with said determined level of NKT cells in said test CSF sample, thereby obtaining another multiplication product 2; and dividing said multiplication product 1 of step i) by said multiplication product 2 of step ii), thereby obtaining a basic composite score.

The term "basic composite score" or "composite score 1" or "composite score for stratifying a subject with neuro-inflammatory autoimmune disease" refers to a score which is based on five specific but different cell levels of the pan-disease classifiers as defined elsewhere herein and which is calculated as mentioned above by the method of the invention.

Further, the method of the present invention may additionally comprise the further step of comparing said basic composite score determined with a reference value.

The term "reference value" is understood to generally refer to a qualitative or quantitative value. Thus, if the reference value is understood to refer to a qualitative value, said basic composite score may be above said reference value. Also comprised herein is, if the reference value is understood to refer to a quantitative value, said basic composite score may deviate from said reference value. In another embodiment, if the reference value is understood to refer to a quantitative value, said basic composite score may deviate from said reference value by about 0.1%, about 0.2% about 0.3%, about 0.4%, about 0.5%, about 0.6%, about 0.7%, about 0.8%, about 0.9%, about 1%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10%, about 15%, about 20%, about 25%, about 30%, about 35%, about 40%, about 45%, about 50%, about 60%, about 70%, about 80%, about 90%, or about 100%. The greater the deviation of said basic composite score from said reference value, the higher the possibility that a subject of the present invention is suspected to suffer from neuro-inflammatory autoimmune diseases.

In comparison, a threshold value is understood to generally refer to a qualitative value. If said basic composite score may be above said specific value, being compared to, the term "threshold value" may be used interchangeably with the term "reference value". The term "a reference" may be used interchangeably with the term "a reference value". The term "a threshold" may be used interchangeably with the term "a threshold value".

Selecting a diagnostic reference / threshold involves, among other things, consideration of the probability of disease, distribution of true and false diagnoses at different test references / thresholds, and estimates of the consequences of treatment (or a failure to treat) based on the diagnosis. Suitable references / thresholds may be determined in a variety of ways.

For example, population studies may be used to select a reference / threshold value. Receiver Operating Characteristic ("ROC") is often used to select a reference / threshold able to best distinguish a well responding subpopulation (a control sample not being stratified with neuro-inflammatory autoimmune diseases) from a poorly responding subpopulation (a test sample being stratified with neuro-inflammatory autoimmune diseases). A false positive in this case occurs when a person tests positive (poor responder or stratified with neuro-inflammatory autoimmune diseases), but actually is a good responder and thus will not suffer from neuro-inflammatory autoimmune diseases. A false negative, on the other hand, occurs when the person tests negative (good responder or not being stratified with neuro-inflammatory autoimmune diseases), when it actually will suffer from neuro-inflammatory autoimmune diseases. To draw a ROC curve, the true positive rate (TPR) and false positive rate (FPR) are determined as the decision reference / threshold is varied continuously. Since TPR is equivalent with sensitivity and FPR is equal to 1 - specificity, the ROC graph is sometimes called the sensitivity vs (1 - specificity) plot. A perfect test will have an area under the ROC curve (AUC) of 1.0; a random test will have an area of 0.5. Preferably, the tests described herein provide a ROC curve area greater than 0.5, preferably at least 0.6, more preferably 0.7, still more preferably at least 0.8, even more preferably at least 0.9, and most preferably at least 0.95. A reference / threshold value is selected to provide an acceptable level of specificity and sensitivity. A reference / threshold that can provide an acceptable level of specificity and sensitivity in separating a population of subjects into "bins" such as a "first" subpopulation (e.g., which is stratified with neuro-inflammatory autoimmune diseases) and a "second" subpopulation which is not stratified with neuro-inflammatory autoimmune diseases. A reference / threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

According to the present invention, stratifying a subject with neuro-inflammatory autoimmune diseases by determining the basic composite score as defined herein with regard to the ratio of said particular determined cell levels of the pan-disease classifiers being consistently increased to said particular determined cell levels of the pan-disease classifiers being consistently decreased, may demonstrate a very sensitive and specific test (AUC: 0.835, see **Fig. 3E).**

In addition to reference / threshold comparisons, other methods for correlating assay results to a patient selection or classification (e.g. likelihood of being a good responder or a poor responder) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1.

The comparison to a reference / threshold value may be carried out manually, semi-automatically or in a fully automated manner. In some embodiments, the comparison may be computer assisted. A computer assisted comparison may employ values stored in a database as a reference for comparing an obtained value or a determined amount, for example via a computer implemented algorithm. Likewise, the comparison to a reference measurement may be carried out manually, semi-automatically or in a fully automated manner, including in a computer assisted manner.

According to the present invention, if said basic composite score may be above the reference value, it may be indicative for whether or not said subject is suspected to suffer from neuro-inflammatory autoimmune diseases. The present invention also comprises that if said basic composite score may deviate from a reference value, it may be indicative for whether or not said subject may be suspected to suffer from neuro-inflammatory autoimmune diseases. In a preferred embodiment, if said basic composite score may deviate from a reference value by about 0.1%, about 0.2% about 0.3%, about 0.4%, about 0.5%, about 0.6%, about 0.7%, about 0.8%, about 0.9%, about 1%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10%, about 15%, about 20%, about 25%, about 30%, about 35%, about 40%, about 45%, about 50%, about 60%, about 70%, about 80%, about 90%, or by about 100%, it may be indicative for whether or not said subject may be suspected to suffer from neuro-inflammatory autoimmune diseases. The greater the deviation of said basic composite score from said reference value, the higher the possibility that the subject may be suspected to suffer from neuro-inflammatory autoimmune diseases.

In a preferred embodiment, the reference value may be at least about 2.5, at least about 2.6, at least about 2.7, at least about 2.8, at least about 2.9, at least about 3.0, at least about 3.1, at least about 3.2, at least about 3.3, at least about 3.4, at least about 3.5, at least about 3.6, at least about 3.7, at least about 3.8, at least about 3.9, at least about 4.0, at least about 4.1, at least about 4.2, at least about 4.3, at least about 4.4, at least about 4.5, at least about 4.6, at least about 4.7, at least about 4.8, at least about 4.9, at least about 5.0, at least about 5.1, at least about 5.2, at least about 5.3, at least about 5.4, at least about 5.5, at least about 5.6, at least about 5.7, at least about 5.8, at least about 5.9, at least about 6.0, or be any number in the range of about 2.5 to about 6.0, from about 2.5 to about 5.9, from about 2.5 to about 5.8, from about 2.5 to about 5.7, from about 2.5 to about 5.6, from about 2.5 to about 5.5, from about 2.5 to about 5.4, from about 2.5 to about 5.3, from about 2.5 to about 5.2, from about 2.5 to about 5.1, from about 2.5 to about 5.0, from about 2.5 to about 4.9, from about 2.5 to about 4.8, from about 2.5 to about 4.7, from about 2.5 to about 4.6, from about 2.5 to about 4.5, from about 2.5 to about 4.4, from about 2.5 to about 4.3, from about 2.5 to about 4.2, from about 2.5 to about 4.1, from about 2.5 to about 4.0, from about 2.5 to about 3.9, from about 2.5 to about 3.8, from about 2.5 to about 3.7, from about 2.5 to about 3.6, from about 2.5 to about 3.5, from about 2.5 to about 3.4, from about 2.5 to about 3.3, from about 2.5 to about 3.2, from about 2.5 to about 3.1, from about 2.5 to about 3.0, or from about 2.6 to about 6.0, from about 2.6 to about 5.9, from about 2.6 to about 5.8, from about 2.6 to about 5.7, from about 2.6 to about 5.6, from about 2.6 to about 5.5, from about 2.6 to about 5.4, from about 2.6 to about 5.3, from about 2.6 to about 5.2, from about 2.6 to about 5.1, from about 2.6 to about 5.0, from about 2.6 to about 4.9, from about 2.6 to about 4.8, from about 2.6 to about 4.7, from about 2.6 to about 4.6, from about 2.6 to about 4.5, from about 2.6 to about 4.4, from about 2.6 to about 4.3, from about 2.6 to about 4.2, from about 2.6 to about 4.1, from about 2.6 to about 4.0, from about 2.6 to about 3.9, from about 2.6 to about 3.8, from about 2.6 to about 3.7, from about 2.6 to about 3.6, from about 2.6 to about 3.5, from about 2.6 to about 3.4, from about 2.6 to about 3.3, from about 2.6 to about 3.2, from about 2.6 to about 3.1, from about 2.6 to about 3.0, or from about 2.7 to about 6.0, from about 2.7 to about 5.9, from about 2.7 to about 5.8, from about 2.7 to about 5.7, from about 2.7 to about 5.6, from about 2.7 to about 5.5, from about 2.7 to about 5.4, from about 2.7 to about 5.3, from about 2.7 to about 5.2, from about 2.7 to about 5.1, from about 2.7 to about 5.0, from about 2.7 to about 4.9, from about 2.7 to about 4.8, from about 2.7 to about 4.7, from about 2.7 to about 4.6, from about 2.7 to about 4.5, from about 2.7 to about 4.4, from about 2.7 to about 4.3, from about 2.7 to about 4.2, from about 2.7 to about 4.1, from about 2.7 to about 4.0, from about 2.7 to about 3.9, from about 2.7 to about 3.8, from about 2.7 to about 3.7, from about 2.7 to about 3.6, from about 2.7 to about 3.5, from about 2.7 to about 3.4, from about 2.7 to about 3.3, from about 2.7 to about 3.2, from about 2.7 to about 3.1, from about 2.7 to about 3.0, or from about 2.8 to about 6.0, from about 2.8 to about 5.9, from about 2.8 to about 5.8, from about 2.8 to about 5.7, from about 2.8 to about 5.6, from about 2.8 to about 5.5, from about 2.8 to about 5.4, from about 2.8 to about 5.3, from about 2.8 to about 5.2, from about 2.8 to about 5.1, from about 2.8 to about 5.0, from about 2.8 to about 4.9, from about 2.8 to about 4.8, from about 2.8 to about 4.7, from about 2.8 to about 4.6, from about 2.8 to about 4.5, from about 2.8 to about 4.4, from about 2.8 to about 4.3, from about 2.8 to about 4.2, from about 2.8 to about 4.1, from about 2.8 to about 4.0, from about 2.8 to about 3.9, from about 2.8 to about 3.8, from about 2.8 to about 3.7, from about 2.8 to about 3.6, from about 2.8 to about 3.5, from about 2.8 to about 3.4, from about 2.8 to about 3.3, from about 2.8 to about 3.2, from about 2.8 to about 3.1, from about 2.8 to about 3.0, or from about 2.9 to about 6.0, from about 2.9 to about 5.9, from about 2.9 to about 5.8, from about 2.9 to about 5.7, from about 2.9 to about 5.6, from about 2.9 to about 5.5, from about 2.9 to about 5.4, from about 2.9 to about 5.3, from about 2.9 to about 5.2, from about 2.9 to about 5.1, from about 2.9 to about 5.0, from about 2.9 to about 4.9, from about 2.9 to about 4.8, from about 2.9 to about 4.7, from about 2.9 to about 4.6, from about 2.9 to about 4.5, from about 2.9 to about 4.4, from about 2.9 to about 4.3, from about 2.9 to about 4.2, from about 2.9 to about 4.1, from about 2.9 to about 4.0, from about 2.9 to about 3.9, from about 2.9 to about 3.8, from about 2.9 to about 3.7, from about 2.9 to about 3.6, from about 2.9 to about 3.5, from about 2.9 to about 3.4, from about 2.9 to about 3.3, from about 2.9 to about 3.2, from about 2.9 to about 3.1, from about 2.9 to about 3.0, preferably about 2.5, about 2.6, about 2.7, about 2.8, about 2.9, about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, most preferably about 2.8. It is understood that ranges herein include all values in between such as natural numbers, rational numbers or real numbers. In an even more preferred embodiment, the reference value for the basic composite score may be (at least) about 2.8. Thus, if said basic composite score may be above the reference (or threshold) value of about 2.8, it may be indicative that the subject is suspected to suffer from neuro-inflammatory autoimmune diseases. Put it differently, if said basic composite score may be above the reference (or threshold) value of about 2.8, the subject may be stratified with neuro-inflammatory autoimmune diseases. Vice versa, if said basic composite score may be below the reference (or threshold) value of about 2.8, the subject may not be stratified with neuro-inflammatory autoimmune diseases, but be stratified with another neurological or psychiatric disease manifestation.

In this context, the term "above a certain reference (or threshold) value" means any number, preferably being rounded up to two digits behind the comma, which is above (not equal to) the indicated number of the reference (or threshold) value (e.g. for example about 2.5). As an illustrative example, if said number may be about 2.51, preferably being rounded up to two digits behind the comma, said number may be above the reference value of about 2.5.

*RMS*

Further, according to the present invention, the method of the invention further comprises whether or not intrathecal immunoglobulin (Ig) synthesis can be detected in said simultaneously obtained test CSF and serum samples obtained from said subject. Said detection of the synthesis is determined by a multi-step process as defined herein and is based on the determination of the immunoglobulin (Ig) quotient and the albumin quotient by using the Reiber scheme as known to a person skilled in the art. The term "Reiber scheme" can be used interchangeably with the term "Reiber diagram". In other words, first the Ig quotient derived from immunoglobulin levels as defined in more detail below needs to be determined in said simultaneously obtained test CSF and serum samples obtained from said subject. Second, the albumin quotient as defined in more detail below also needs to be determined in said simultaneously obtained test CSF and serum samples obtained from said subject. By using the Reiber scheme and taking both quotients (the Ig and the albumin quotient) into account as known to a person skilled in the art, it can be determined whether there is Ig synthesis or not in said subject as defined further herein. Thus, to decide whether specific intrathecal Ig synthesis occurs, the result is judged with the help of the Reiber scheme, thereby integrating further information like albumin quotient and age. Commercially available clinical assay kits known to the person skilled in the art may be used to perform these analyses. The immunoglobulin quotients are determined by nephelometry or turbidimetry from simultaneously obtained serum and CSF samples, i.e. by an independent technique other than flow cytometry.

In this context, "simultaneously obtained samples" refer to a test CSF and a test serum sample, which are obtained from the same subject being examined at the same time. Thus, said CSF sample already being obtained from said subject has already been examined for said pan-disease classifiers as defined herein and is now further examined for the determination of said Ig synthesis and/or said OCBs as defined below. At the same time when obtaining said CSF sample, if the Ig synthesis and/or OCBs need to be determined as well, a serum sample also needs to be obtained from said same subject. In other words, those samples are "paired" samples. Thus, it is also comprised herein that the method of the present invention further comprises determining whether or not intrathecal immunoglobulin (Ig) synthesis can be detected in said paired CSF/serum samples. This also applies *mutatis mutandis* to the determination of type 2 or type 3 oligoclonal bands (OCBs) as defined below. The definition for "obtaining said CSF sample" as made elsewhere herein is also applicable in these contexts. The serum sample being simultaneously obtained from the same subject is obtained by venous puncture as it is done for said PB sample following typical procedures known to a person skilled in the art and then further collected in particular tubes for further determination. The tubes used for said serum sample comprises however different additives as the tubes used for said PB sample.

Normally, proteins should not accumulate in the CSF. However, proteins such as Igs passaging from the blood into the CSF may be due to barrier disturbances of the blood-brain-barrier. The more permeable the blood-brain-barrier is, the more proteins (e.g., albumin) may be detected in the CSF. Another reason for detecting proteins such as Igs in the CSF is due to pathological processes of certain diseases including MS. However, if Igs are increased in said CSF and also in the serum, albumin concentration may also be detected to observe whether Igs may passage from the blood due to barrier disturbances of the blood-brain barrier or whether Igs may be produced in the CSF and serum, which would correlate to a pathological process of certain diseases including, but not limited to, MS.

Therefore, the Ig synthesis being additionally detected in the method of the present invention may be determined by taking into account the Ig-quotient and the albumin quotient using the Reiber scheme. As it may be done for determining the albumin quotient, the Ig quotient may be determined by measuring the concentration / amount / level of Igs in the CSF and the concentration / amount / level of Igs in the blood serum simultaneously in said samples obtained from said subject as defined herein. Then, the Ig concentration / amount / level of CSF is divided by the Ig concentration / amount / level of the blood serum, thereby obtaining the Ig quotient. In other words, the Ig quotient as well as the albumin quotient are determined by analyzing the Ig concentrations / albumin concentrations in said simultaneously obtained serum and CSF samples. By dividing said concentrations as defined above, the Ig quotient (also called "Ig CSF/serum quotient") and the albumin quotient (also called "albumin CSF/serum quotient") may be determined / calculated.

In some embodiments, the IgG quotient is of most importance in the method of the present invention. Thus, in a preferred embodiment, the concentration / amount / level of IgGs in the CSF and the concentration / amount / level of IgGs in the blood serum is measured. Then, the IgG concentration / amount / level of CSF is divided by the IgG concentration / amount / level of the blood serum, thereby obtaining the IgG quotient. Elevation / increase of said IgG levels in the CSF relative to the serum of patients with a neuro-inflammatory autoimmune disease, such as MS, is due to local central nervous system (CNS) synthesis of said IgGs.

The present invention also comprises further determining, as described similarly as for the IgG quotient, the IgA quotient or the IgM quotient. By taking into account the Ig quotient, preferably the IgG-, IgA-, IgM quotient, more preferably the IgG quotient as defined herein, and the albumin quotient, Ig synthesis, preferably IgG synthesis, can then be determined using the Reiber diagram known to a person skilled in the art. Further, the age of said subject may also be taken into account besides both determined quotients in order to detect Ig synthesis. After taking both quotients into account in the Reiber scheme, whether Ig synthesis can be detected is readable from said diagram. If the value to be determined, after having taken into account both quotients in the Reiber scheme, is above a certain reference range / area, Ig synthesis can be detected. In this context, the term "detected" (or "determined") means that if a CNS-specific Ig synthesis occurs / can be detected, a value of 1 is counted with regard to the composite score as defined elsewhere herein. If no Ig synthesis can be detected, because the value to be determined from said scheme, after taking both quotients into account in the Reiber scheme, is within said reference range, a value of 0 is counted. Thus, the relative extent of the synthesis is not relevant at all, rather it is a yes (value of 1) or no (value of 0) decision. There are different reference ranges / areas which can be applied in said scheme, which are dependent whether the IgG, IgA or IgM quotient is determined as known to a person skilled in the art. Depending on which reference range /area is used, a value above this range, after having taken into account the Ig quotient as defined herein and the albumin quotient as also defined herein in the Reiber scheme, indicates that Ig synthesis is present or is indicative that Ig synthesis can be detected (see Deutsche Gesellschaft für Liquordiagnostik und Klinische Neurochemie e.V. in der Deutschen Gesellschaft für Neurologie, "Ausgewählte Methoden der Liquordiagnostik und Klinische Neurochemie", 2020, pp. 20-25).

In a further preferred embodiment of the present invention, the method further comprises determining whether or not type 2 or type 3 oligoclonal bands (OCBs) can be detected in said simultaneously obtained test CSF and serum samples obtained from said subject. OCB are bands of immunoglobulins, in particular oligoclonal immunoglobulins, that are seen when a patient's blood serum and cerebrospinal fluid (CSF) may be analyzed. Two methods of analysis for detecting OCB are possible, but not limited to: (a) protein electrophoresis, a method of analyzing the composition of fluids, also known as "agarose gel electrophoresis/Coomassie Blue staining"; and (b) the combination of protein separation by isoelectric focusing followed by immunoblotting (such as silver staining). In a preferred embodiment, the second method (b) for detecting OCB is applied in the present invention. In MS, normally only OCBs made of immunoglobulin G antibodies are considered, though sometimes other proteins can be taken into account, like lipid-specific immunoglobulin M. The presence of these IgM OCBs is associated with a more severe course of MS (Ferraro et al. 2015, Neuroimmunology. 283: 64-69). Because of the high sensitivity of CSF OCB in MS as well as its high specificity in the appropriate clinical setting, examination of CSF for OCB of IgG class may be highly recommended to support the stratification of MS, preferably of RMS. In general, more than 95% of patients with multiple sclerosis (MS) have CSF OCB of IgG class, thereby providing powerful evidence for the stratification of MS, preferably of RMS. In fact, such immunoglobulins are thought to be produced locally by clonally expanded B cells in the CSF or meninges in MS and may recognize ubiquitous self-proteins. Thus, an importance of B cells in MS may be suggested by the presence of oligoclonal immunoglobulins (OCB). In brief, concerning the OCB detection, the non-cellular fraction (i.e. protein) of the CSF and serum is separated on a gel and stained. Then one looks for bands indicating expanded proteins of one size which represent oligoclonal immunoglobulins. By definition, results are classified as:

- type 1, no bands in CSF and serum;
- type 2, oligoclonal IgG bands in CSF,
- type 3, oligoclonal bands in CSF and serum with additional bands in CSF;
- type 4, identical oligoclonal bands in CSF and serum,
- type 5, monoclonal bands in CSF and serum,
- type 6, presence of a single band limited to the CSF.

According to the present invention, only type 2 and/or type 3 OCBs are important for the detection within the method of the present invention. In this context, the term "detected" (or "determined") means that if type 2 and/or type 3 OCBs occur(s) / can be detected, a value of 1 is counted with regard to the composite score as defined elsewhere herein. If no OCBs can be detected in said samples, a value of 0 is counted with regard to the composite score as defined elsewhere herein.

In a preferred embodiment of the present invention, the method further comprises determining a level of plasma cells in said test CSF sample being obtained from said subject which is examined and from whom the already obtained CSF sample has already been examined for the pan-disease classifiers as defined herein. In a further preferred embodiment of the invention, it is foreseen that detecting the level of plasma cells comprises measuring of one or more of the markers selected from BCMA, CD19, CD20, CD27, CD38, CD78, CD138, CD319, CXCR4, and HLA-DR. In preferred embodiment when stratifying a subject with RMS as defined below, flow cytometry technique as defined elsewhere herein is used for detecting the level of plasma cells, preferably by detecting each specific cell surface marker / molecule such as BCMA, CD19, CD20, CD27, CD38, CD78, CD138, CD319, CXCR4 and HLA-DR on the cell surface of each specific cells, which demonstrates a certain strength of a signal being measured.

A further subject-matter of the present invention is therefore directed to the method as described before, further stratifying said subject as suffering from relapsing forms of multiple sclerosis (RMS), if the following are fulfilled: i) intrathecal immunoglobulin (Ig) synthesis can be detected in said simultaneously obtained test CSF and serum samples obtained from said subject and/or type 2 or type 3 oligoclonal bands can be detected in said test CSF and test serum samples being simultaneously obtained from said subject; and ii) a level of plasma cells is increased relative to corresponding levels of plasma cells in said control CSF samples obtained from subjects suffering from another neuro-inflammatory autoimmune disease other than RMS. Thus, if said requirements of said so called "inter-autoimmunity classifiers" are additionally fulfilled to said pan-disease classifiers as defined herein, it is indicative for whether said subject is suspected to suffer from RMS.

As a comparison the control CSF samples (see the requirement of an increased level of plasma cells in said test CSF sample) are obtained from a cohort of subjects who have been diagnosed with not suffering from RMS, but have been diagnosed with suffering from another neuro-inflammatory autoimmune disease other than RMS. Thus, said control samples do not fulfill the abovementioned requirements outlined for said inter-autoimmunity classifiers, but fulfill the requirements for being stratified as suffering from a neuro-inflammatory autoimmune disease other than RMS. However, in the test samples of said subject being stratified with RMS as defined herein, all of the pan-disease classifiers as defined herein are detected, which fulfill the requirements listed in step b) of the method of the present invention. Additionally, in said test samples of said subject being stratified with RMS as defined herein also all of the inter-autoimmunity classifiers are detected, which fulfill the requirements for said RMS stratification. For said stratification of RMS the requirements of i) (detection of Ig synthesis) and ii) are additionally fulfilled. Also for said stratification of RMS the requirements of i) (detection of OCBs) and ii) may additionally be fulfilled. Preferably, for said stratification of RMS the requirements of i) (detection of Ig synthesis and detection of OCBs) and ii) are additionally fulfilled.

In general, the term "multiple sclerosis (MS)" as used herein refers to a chronic, inflammatory central nervous system (CNS) disease, characterized pathologically by demyelination. MS has also been classified as a neuro-inflammatory autoimmune disease. It refers to a demyelinating disease in which the insulating covers of nerve cells in the brain and spinal cord are damaged. MS disease activity can be monitored by cranial scans, including magnetic resonance imaging (MRI) of the brain, accumulation of disability, as well as rate and severity of relapses. There are six distinct disease stages and/or types of MS, namely, (1) radiologically isolated syndrome (RIS); (2) clinically isolated syndrome (CIS); (3) relapsing-remitting multiple sclerosis (RRMS); (4) secondary progressive multiple sclerosis (SPMS); (5) progressive relapsing multiple sclerosis (PRMS); and (6) primary progressive multiple sclerosis (PPMS). However, RIS and CIS are pre-stages / pre-types of MS.

As an illustrative example for revision of MS diagnostic criteria see the scenarios below. Here it should be mentioned that CIS refers to a first episode suggestive of MS. It is a first episode of neurologic symptoms that lasts at least about 24 hours and is caused by inflammation or demyelination (loss of the myelin that covers the nerve cells) in the central nervous system (CNS). Some reviews describe CIS as "the prodromal stage of MS" (Dupont et al. 2017, International Journal of Molecular Sciences. 18 (4): 785), an early sign or symptom (or set of signs and symptoms), which often indicate the onset of a disease before more diagnostically specific signs and symptoms develop. Dissemination in time (DIT) is what distinguishes CIS from RRMS as mentioned below. If a subject may be examined with a first demyelinating event and dissemination in space (DIS) on an MRI scan, said subject may still have / suffer from CIS, since DIT criteria have not been fulfilled:

Scenario **(A)** first event and exclusion of differential diagnoses: if no DIS may be examined, the subject may then be diagnosed with CIS or none of the other four stages of MS;
Scenario **(B)** first event and exclusion of differential diagnoses: if DIS, but no DIT may be examined, the subject may then be diagnosed with CIS;
Scenario **(C)** First event and exclusion of differential diagnoses: if DIS and DIT may be examined, the subject may then be diagnosed with RRMS;
Scenario **(D)** Many years of RRMS, but now slowly progressing, the subject may then be diagnosed with SPMS;
Scenario **(E)** exclusion of differential diagnoses: if relapses have never occurred, but slowly progressing from the start, the subject may then be diagnosed with PPMS.

RMS as used herein for the further stratification of said subject within neuro-inflammatory autoimmune diseases comprises relapsing-remitting MS (short: RRMS) (see Scenario C above) and secondary progressive MS (short: SPMS) (see Scenario D above). Thus, it is also comprised by the present invention the method as defined elsewhere herein, further being able to stratify said subject as suffering from RRMS or SPMS, if said requirements of said inter-autoimmunity classifiers as defined elsewhere herein are additionally fulfilled as defined herein. In a preferred embodiment of the present invention, said method is further being able to further stratify said subject as suffering from RRMS, if said requirements of said inter-autoimmunity classifiers as defined elsewhere herein are additionally fulfilled as defined herein.

The present inventors further generated a so called "composite score 1a" for stratifying a subject with RMS by summing up a particular determined Ig synthesis as defined herein with a particular determined plasma cell level of said inter-autoimmunity classifiers which is consistently increased (see the Example section hereto). The same composite score 1a may be also generated for stratifying a subject with RMS by summing up the detected type 2 or 3 OCBs with a particular determined plasma cell level of said inter-autoimmunity classifiers which is consistently increased (see the Example section hereto). In other words, a composite score 1a may be generated by the addition of said particular determined Ig synthesis (value of "1" as defined above) in said simultaneously obtained test CSF and serum samples with a particular determined plasma cell level in said simultaneously obtained test CSF and serum samples or by the addition of said detected type 2 or type 3 OCBs in said simultaneously obtained test CSF and serum samples with a particular determined plasma cell level in said simultaneously obtained test CSF and serum samples (see **Fig. 4D).** Thus, it is also comprised herein the method of the present invention as defined by step a) mentioned elsewhere herein, wherein the stratifying step b) instead comprises summing up said detected intrathecal immunoglobulin (Ig) synthesis or said detected OCBs in said simultaneously obtained test CSF and serum samples with a determined plasma cell level in said simultaneously obtained test CSF and serum samples, thereby obtaining the composite score 1a.

The term "composite score 1a" or "composite score for stratifying a subject with RMS" refers to a score which is based on more than one, such as preferably two, specific inter-autoimmunity classifiers defined elsewhere herein and which is calculated as mentioned above by the method of the invention. The difference between the basic composite score and the composite score 1a lies in the independent use, the first for stratifying a subject with neuro-inflammatory autoimmune disease, the second for stratifying a subject with RMS after already having stratified said subject with neuro-inflammatory autoimmune disease based on applying the method of the present invention for generating the basic composite score (composite score 1). In other words, for stratifying a subject with RMS, first the basic composite score has to be generated for the stratification whether a subject is suspected to suffer from neuro-inflammatory autoimmune diseases and then the composite score 1a has to be generated afterwards to clearly stratify said subject with RMS within the neuro-inflammatory autoimmune diseases. The composite score 1a is based as defined above on the detection step of intrathecal immunoglobulin (Ig) synthesis or oligoclonal bands and on the additional occurrence of plasma cells. Thus, the basic composite score and the composite score 1a are independent from each other and are applied consecutively to the results of said test sample. As mentioned earlier, if the Ig synthesis (preferably IgG synthesis) is thereby assessed or there is detection of the type 2 or type 3 OCBs, the value of 1 is counted for said addition, otherwise counting the value of 0, if no Ig synthesis (preferably IgG synthesis) or OCBs can be assessed.

According to the present invention, stratifying a subject with RMS by determining the composite score 1a as defined herein with regard to the addition of said particular determined Ig synthesis in said simultaneously obtained test CSF and serum samples with a particular determined plasma cell level in said simultaneously obtained test CSF and serum samples, or by the addition of said detected type 2 or type 3 OCBs in said simultaneously obtained test CSF and serum samples with a particular determined plasma cell level in said simultaneously obtained test CSF and serum samples, may demonstrate a very sensitive and specific test (AUC: 0.84, see Fig. 4D).

According to the present invention, if said composite score 1a may be above the reference value as defined in more detail below, it may be indicative for whether or not said subject is suspected to suffer from RMS. The present invention also comprises that if said composite score 1a may deviate from said reference value, it may be indicative for whether or not said subject may be suspected to suffer from RMS. In a preferred embodiment, if said composite score 1a may deviate from said reference value by about 0.1%, about 0.2% about 0.3%, about 0.4%, about 0.5%, about 0.6%, about 0.7%, about 0.8%, about 0.9%, about 1%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10%, about 15%, about 20%, about 25%, about 30%, about 35%, about 40%, about 45%, about 50%, about 60%, about 70%, about 80%, about 90%, or by about 100%, it may be indicative for whether or not said subject may be suspected to suffer from RMS as defined herein. The greater the deviation of said composite score 1a from said reference value, the higher the possibility that the subject may be suspected to suffer from RMS.

In a preferred embodiment, the reference value may be at least about 1.2, at least about 1.3, at least about 1.4, at least about 1.5, at least about 1.6, at least about 1.7, at least about 1.8, at least about 1.9, at least about 2.0, at least about 2.1, at least about 2.2, at least about 2.3, at least about 2.4, at least about 2.5, at least about 2.6, at least about 2.7, at least about 2.8, at least about 2.9, at least about 3.0, or be any number in the range of about 1.2 to about 3.0, from about 1.2 to about 2.9, from about 1.2 to about 2.8, from about 1.2 to about 2.7, from about 1.2 to about 2.6, from about 1.2 to about 2.5, from about 1.2 to about 2.4, from about 1.2 to about 2.3, from about 1.2 to about 2.2, from about 1.2 to about 2.1, from about 1.2 to about 2.0, from about 1.2 to about 1.9, from about 1.2 to about 1.8, from about 1.2 to about 1.7, from about 1.2 to about 1.6, preferably about 1.2, about 1.3 about 1.4, about 1.5, about 1.6, about 1.7, about 1.8, about 1.9, about 2.0, about 2.1, about 2.2, about 2.3, about 2.4, about 2.5, about 2.6, about 2.7, about 2.8, about 2.9, about 3.0, most preferably about 1.5. It is understood that ranges herein include all values in between such as natural numbers, rational numbers or real numbers. In an even more preferred embodiment, the reference value for the composite score 1a may be (at least) about 1.5. Thus, if said composite score 1a may be above the reference (or threshold) value of about 1.5, it may be indicative that the subject is suspected to suffer from RMS. Put it differently, if said composite score 1a may be above the reference (or threshold) value of about 1.5, the subject may be stratified with RMS. Vice versa, if said composite score 1a may be below the reference (or threshold) value of about 1.5, the subject may not be stratified with RMS, but be stratified with another neuro-inflammatory autoimmune disease other than RMS.

The abovementioned disclosure regarding the reference value / threshold value / ROCs and the associated methods used for accuracy mentioned with regard to the basic composite score are also applicable to said composite score 1a.

*NMOSD*

In another preferred embodiment of the present invention, the method further comprises determining a level of $CD56^{bright}$ $CD16^{dim/-}$ NK cells in said test PB sample being obtained from said subject which is examined and from whom the already obtained PB sample has already been examined for the pan-disease classifiers as defined herein. In a further preferred embodiment of the invention, it is foreseen that detecting the level of $CD56^{bright}$ $CD16^{dim/-}$ NK cells comprises measuring of one or more of the markers selected from CD14, CD16, CD25, CD45, CD56, CD94, CD122, CD159a, CD244, CD315, CD335, CD336, CD337, Tbet and EOMES. In a preferred embodiment when further stratifying a subject with NMOSD, flow cytometry technique is used for detecting the level of $CD56^{bright}$ $CD16^{dim/-}$ NK cells, preferably by detecting each specific cell surface marker / molecule such as CD14, CD16, CD25, CD45, CD56, CD94, CD122, CD159a, CD244, CD315, CD335, CD336, CD337, Tbet and/or EOMES on the cell surface of each specific cells, which demonstrates a certain strength of a signal being measured. In another preferred embodiment of the present invention, the method further comprises determining a level of lactate in said test CSF sample being obtained from said subject which is examined and from whom the already obtained CSF sample has already been examined for the pan-disease classifiers as defined herein. In a further preferred embodiment of the invention, it is foreseen that detecting the level of lactate comprises pipetting said cell-free CSF sample, preferably a 20μl cell-free CSF sample, into the red sample segment of a SuperGL compact (Hitado).

A further subject-matter of the present invention is thus directed to the method as described before, further stratifying said subject as suffering from neuromyelitis optica spectrum disorders (NMOSD), if one or more of the following are fulfilled: i) a level of $CD56^{bright}$ $CD16^{dim/-}$NK cells in said PB sample is decreased relative to corresponding levels of $CD56^{bright}$ $CD16^{dim/-}$NK cells in control PB samples which are obtained from a cohort of subjects suffering from another neuro-inflammatory autoimmune disease other than NMOSD; ii) a level of lactate is increased in said test CSF sample relative to corresponding levels of lactate in control CSF samples obtained from subjects suffering from another neuro-inflammatory autoimmune disease other than NMOSD. Thus, if one or more of said requirements of said classifiers are additionally fulfilled to said pan-disease classifiers and the requirements of said abovementioned inter-autoimmunity classifiers are not fulfilled as defined herein, it is indicative for whether said subject is suspected to suffer from NMOSD.

As a comparison the control PB samples (see the requirement of a decreased level of $CD56^{bright}$ $CD16^{dim/-}$ NK cells in the test PB sample) and/or the control CSF samples (see the requirement of an increased level of lactate in the test CSF sample) are obtained from a cohort of subjects who have been diagnosed with not suffering from NMOSD, but have been diagnosed with suffering from another neuro-inflammatory autoimmune disease other than NMOSD. Thus, said control samples do not fulfill the abovementioned requirements outlined with regard to a decreased level of $CD56^{bright}$ $CD16^{dim/-}$ NK cells and/or an increased level of lactate, but fulfill the requirements for being stratified as suffering from a neuro-inflammatory autoimmune disease other than NMOSD. However, in the test sample of said subject being stratified with NMOSD as defined herein, all of the pan-disease classifiers as defined herein are detected, which fulfill the requirements listed in step b) of the method of the present invention. Additionally, in said test sample of said subject being stratified with NMOSD as defined herein also one or more, preferably all of the classifiers as mentioned for the NMOSD stratification can also be detected, which fulfill one or more of the requirements for said NMOSD stratification. Preferably, for said stratification of NMOSD the requirements of i) (decreased level of $CD56^{bright}$ $CD16^{dim/-}$ NK cells) and ii) (increased level of lactate) are additionally fulfilled. In said test sample of said subject being stratified with NMOSD as defined herein the inter-autoimmunity classifiers can be detected in said sample, but do not fulfil the particular requirements of said inter-autoimmunity classifiers as defined elsewhere herein.

SuS

In another preferred embodiment of the present invention, the method further comprises determining a level of $CD8^+$ HLA-$DR^+$ T cells in said test CSF and/or in said PB sample being obtained from said subject which is examined and from whom the already obtained CSF and/or PB sample has already been examined for the pan-disease classifiers as defined herein. In a further preferred embodiment of the invention, it is foreseen that detecting the level of $CD8^+$ HLA-$DR^+$ T cells comprises measuring of one or more of the markers selected from CD3, CD4, CD8, CD14, CD45, CD56 and HLA-DR. In another preferred embodiment of the present invention, the method further comprises determining a level of $CD8^+$ T cells in said test CSF and/or in said PB sample being obtained from said subject which is examined and from whom the already obtained CSF and/or PB sample has already been examined for the pan-disease classifiers as defined herein. In a further preferred embodiment of the invention, it is foreseen that detecting the level of $CD8^+$ T cells comprises measuring of one or more of the markers selected from CD3, CD4, CD8, CD14, CD45 and CD56. In another preferred embodiment of the present invention, the method further comprises determining a level of $CD4^+$ T cells in said test CSF and/or in said PB sample being obtained from said subject which is examined and from whom the already obtained CSF and/or PB sample has already been examined for the pan-disease classifiers as defined herein. In a further preferred embodiment of the invention, it is foreseen that detecting the level of $CD4^+$ T cells comprises measuring of one or more of the markers selected from CD3, CD4, CD8, CD14, CD45 and CD56. In preferred embodiment when stratifying a subject with SuS, flow cytometry technique is used for detecting the level of $CD8^+$ $HLA\text{-}DR^+$ T cells, the level of $CD8^+$ T cells and/or the level of $CD4^+$ T cells, preferably by detecting each specific cell surface marker / molecule such as CD3, CD4, CD8, CD14, CD45, CD56 and/or HLA-DR on the cell surface of each specific cells, which demonstrates a certain strength of a signal being measured.

In general, T cells are known to the skilled artisan as lymphocytes, i.e. nucleated blood cells that are also called white blood cells. T cells mature in the thymus and can be distinguished from other lymphocytes in that they have the T cell receptor on their cell surface. The main known role of the T cell is recognition of antigens bound to major histocompatibility complex (MHC) molecules. The T cell receptor (TCR) is a heterodimer, which consists of a 34 kD $\alpha$-chain, linked by a disulphide bond to a 34 kD $\beta$-chain in about 95 % of T cells. Both chains span the plasma membrane and have accordingly an extracellular portion, each of which includes a variable region, termed Va and V $\beta$, respectively. About 5 % of T cells have a T cell receptor that consists of a $\gamma$- and a $\delta$-chain instead of an $\alpha$ - and a $\beta$-chain, which likewise have extracellular variable regions. T cell receptors can, like immunoglobulins, recognize a very large number of different epitopes. As the T cell receptor has variable regions it may, nevertheless, be advantageous to use another cell surface protein to identify a T cell. An example of suitable protein in this regard is a T cell co-receptor. An illustrative example of a co-receptor of the T cell receptor is the protein complex CD3 (Cluster of Differentiation 3). CD3 has four chains, which are in mammals one CD3$\gamma$ chain (e.g. human CD3$\gamma$ of the UniProt accession number P09693, version 147 of 7 January 2015 or corresponding mRNA of the GenBank Accession number X04145, version X04145.1 GI:37021 of 18 April 2005), one CD3$\delta$ chain (e.g. human CD35 of the UniProt accession number P04234, version 157 of 7 January 2015 or corresponding mRNA of the GenBank Accession number BC039035, version BC039035.1 GI:25058311 of 19 June 2006), and two CD3$\varepsilon$ chains (e.g. human CD3$\varepsilon$ of the UniProt accession number P07766, version 167 of 7 January 2015 or corresponding mRNA of the GenBank Accession number X03884, version X03884.1 GI:37039 of 07 April 1994). These chains associate with a molecule known as the T-cell receptor and at least one T-cell surface glycoprotein CD3 zeta chain also known as T-cell receptor T3 zeta chain or CD247 (Cluster of Differentiation 247) (e.g. human CD3 zeta chain of the UniProt accession number P20963, version 165 of 7 January 2015 or corresponding mRNA of the GenBank Accession number J04132, version J04132.1 GI:623041 of 12 January 1995). The complex of TCR, CD247 and CD3 can generate an activation signal in T lymphocytes. The TCR, $\zeta$-chain(s), and CD3 molecule together define the TCR complex. In practicing the methods according to the invention identifying the presence of CD3 on a particular cell or plurality of cells is often a convenient way of identifying T cells. According to the invention, identifying CD3+ T cells in the sample serves in distinguishing CD3+ T cells from other cells such as CD3- cells or non-T cells (such as NK cells and/or B cells).

In the methods according to the invention, the level of T cells in the sample that is CD4 positive (CD4+) are detected. identifying CD4+ T cells typically serves in distinguishing CD4+ T cells from other cells such as CD4- T cells. CD4+ T cells in addition to CD3 have the CD4 (Cluster of Differentiation 4) protein on their surface, a glycoprotein consisting of four extracellular immunoglobulin domains, termed D1 to D4, and a small cytoplasmic region (e.g. human CD4 of the UniProt accession number P01730, version 180 of 7 January 2015 or corresponding mRNA of the GenBank Accession number M12807, version M12807.1 GI:179141 of 27 April 1993). CD4+ T cells can be classified into a variety of cell populations with different functions and should thus not be taken to define a unitary set of cells. Typical examples of a CD4+ T cell are naïve CD4+ T-cells (naive), "central" memory CD4+ T-cells (TCM), or "effector" memory CD4+ T cells (TEM).

In the method according to the invention, the level of T cells in the sample being CD8 positive (CD8+) may also be detected. Identifying CD8+ T cells typically serves in distinguishing CD8+ T cells from other cells such as CD8- T cells. CD8+ T cells in addition to CD3 have the CD8 (Cluster of Differentiation 8) protein on their surface, a transmembrane glycoprotein consisting of a pair of CD8 chains, most commonly composed of a CD8- $\alpha$ and CD8- $\beta$ chain having a molecular weight of about 34 kDa. The CD8 co-receptor is predominantly expressed on the surface of cytotoxic T cells. Like the TCR, CD8 interacts with the MHC molecule, but is specific for the Class I MHC molecule (in particular it interacts with $\alpha_3$ portion of the Class I MHC molecule).

A further subject-matter of the present invention is thus directed to the method as described before, further stratifying said subject as suffering from Susac's syndrome (SuS), if one or more of the following are fulfilled: i) a level of $CD8^+$ $HLA\text{-}DR^+$ T cells in said test PB and/or in said test CSF sample is increased relative to corresponding levels of $CD8^+$ $HLA\text{-}DR^+$ T cells in control PB and/or control CSF samples obtained from subjects suffering from another neuro-inflammatory autoimmune disease other than SuS; ii) a level of $CD8^+$ T cells in said test PB and/or in said test CSF sample is increased relative to corresponding levels of $CD8^+$ T cells in control PB and/or control CSF samples obtained from subjects suffering from another neuro-inflammatory autoimmune disease other than SuS; iii) a level of $CD4^+$ T cells in said test PB and/or in said test CSF sample is decreased relative to corresponding levels of $CD4^+$ T cells in control PB and/or control CSF samples obtained from subjects suffering from another neuro-inflammatory autoimmune disease other than SuS. Thus, if one or more of said requirements of said classifiers are additionally fulfilled to all pan-disease classifiers and the requirements of said abovementioned inter-autoimmunity classifiers are not fulfilled as defined herein, it is indicative for whether said subject is suspected to suffer from SuS.

As a comparison the control PB and/or CSF samples are obtained from a cohort of subjects who have been diagnosed with not suffering from SuS, but have been diagnosed with suffering from another neuro-inflammatory autoimmune disease other than SuS. Thus, said control samples do not fulfill the abovementioned requirements outlined with regard to an increased level of $CD8^+$ $HLA\text{-}DR^+$ T cells, an increased level of $CD8^+$ T cells and/or a decreased level of $CD4^+$ T cells, but fulfill the requirements for being stratified as suffering from a neuro-inflammatory autoimmune disease other than SuS. However, in the test sample(s) of said subject being stratified with SuS as defined herein, all of the pan-disease classifiers as defined herein are detected, which fulfill the requirements listed in step b) of the method of the present invention. Additionally, in said test sample(s) of said subject being stratified with SuS as defined herein also one or more, preferably all of the classifiers as mentioned for the SuS stratification can also be detected, which fulfill one or more of the requirements for said SuS stratification. Preferably, for said stratification of SuS, the requirements of i) and ii) are additionally fulfilled. Also preferably, for said stratification of SuS, the requirements of i) and iii) are additionally fulfilled. Also preferably, for said stratification of SuS, the requirements of ii) and iii) are additionally fulfilled. Most preferably, for said stratification of SuS, the requirements of i) and ii) and iii) are additionally fulfilled. In said test sample(s) of said subject being stratified with SuS as defined herein the inter-autoimmunity classifiers can be detected in said sample, but do not fulfil the particular requirements of said inter-autoimmunity classifiers as defined elsewhere herein.

*AIE*

In another preferred embodiment of the present invention, the method further comprises determining a level of lymphocytes in said test CSF sample being obtained from said subject which is examined and from whom the already obtained CSF sample has already been examined for the pan-disease classifiers as defined herein. In a further preferred embodiment of the invention, it is foreseen that detecting the level of lymphocytes comprises measuring of one or more of the markers selected from CD14, CD15, CD45 and CD66b. In another preferred embodiment of the present invention, the method further comprises determining again the level of NKT cells in said test CSF sample being obtained from said subject which is examined and from whom the already obtained CSF sample has already been examined for the pan-disease classifiers as defined herein. In a further preferred embodiment of the invention, it is foreseen that detecting the level of NKT cells comprises measuring of, as already defined elsewhere herein, one or more of the markers selected from CD1d, CD3, CD4, CD8, CD14, CD16, CD45, CD56, CD161 TCRγδ, Va24-Ja18, Vβ11, Vδ1 and Vδ2. In another preferred embodiment of the present invention, the method further comprises determining again the level of monocytes in said test CSF sample being obtained from said subject which is examined and from whom the already obtained CSF sample has already been examined for the pan-disease classifiers as defined herein. In a further preferred embodiment of the invention, it is foreseen that detecting the level of monocytes comprises measuring of, as already defined elsewhere herein, one or more of the markers selected from CD11b, CD11c, CD14, CD16, CD45, CD62L, CD86, CD115, CD116, CD121, CD163, CD192, CD206, $CX_3CR1$ and HLA-DR. In a preferred embodiment when further stratifying a subject with AIE, flow cytometry technique is used for detecting the level of lymphocytes, the level of NKT cells and/or the level of monocytes, preferably by detecting each specific cell surface marker / molecule such as CD1d, CD3, CD4, CD8, CD11b, CD11c, CD14, CD16, CD45, CD56, CD62L, CD66b, CD86, CD115, CD116, CD121, CD161, CD163, CD192, CD206, TCRγδ, $CX_3CR1$, HLA-DR, Vα24-Jα18, Vβ11, Vδ1 and/or Vδ2 on the cell surface of each specific cells, which demonstrates a certain strength of a signal being measured.

A further subject-matter of the present invention is thus directed to the method as described before, further stratifying said subject as suffering from autoimmune encephalitis (AIE), if one or more of the following are fulfilled: i) a level of lymphocytes in said test CSF sample is decreased relative to corresponding levels of lymphocytes in control CSF samples obtained from subjects suffering from another neuro-inflammatory autoimmune disease other than AIE; ii) a level of NKT cells in said test CSF sample is increased relative to corresponding levels of NKT cells in control CSF samples obtained from subjects suffering from another neuro-inflammatory autoimmune disease other than AIE; iii) a level of monocytes in said test CSF sample is increased relative to corresponding levels of monocytes in control CSF samples obtained from subjects suffering from another neuro-inflammatory autoimmune disease other than AIE. Thus, if one or more of said requirements of said classifiers are additionally fulfilled to said pan-disease classifiers and the requirements of said abovementioned inter-autoimmunity classifiers are not fulfilled as defined herein, it is indicative for whether said subject is suspected to suffer from AIE.

As a comparison the control CSF samples are obtained from a cohort of subjects who have been diagnosed with not suffering from AIE, but have been diagnosed with suffering from another neuro-inflammatory autoimmune disease other than AIE. Thus, said control samples do not fulfill the abovementioned requirements outlined with regard to a decreased level of lymphocytes, an increased level of NKT cells and/or an increased level of monocytes, but fulfill the requirements for being stratified as suffering from a neuro-inflammatory autoimmune disease other than AIE. However, in the test sample of said subject being stratified with AIE as defined herein, all of the pan-disease classifiers as defined herein are detected, which fulfill the requirements listed in step b) of the method of the present invention. Additionally, in said test sample of said subject being stratified with AIE as defined herein also one or more, preferably all of the classifiers as mentioned for the AIE stratification can also be detected, which fulfill one or more of the requirements for said AIE stratification. Preferably, for said stratification of AIE, the requirements of i) and ii) are additionally fulfilled. Also preferably, for said stratification of AIE, the requirements of i) and iii) are additionally fulfilled. Also preferably, for said stratification of AIE, the requirements of ii) and iii) are additionally fulfilled. Most preferably, for said stratification of AIE, the requirements of i) and ii) and iii) are additionally fulfilled. In said test sample of said subject being stratified with AIE as defined herein the inter-autoimmunity classifiers can be detected in said sample, but do not fulfil the particular requirements of said inter-autoimmunity classifiers as defined elsewhere herein.

*PMS*

In another preferred embodiment of the present invention, the method further comprises determining a level of $CD4^+$ $HLA\text{-}DR^+$ T cells in said test PB sample being obtained from said subject which is examined and from whom the already obtained PB sample has already been examined for the pan-disease classifiers as defined herein. In a further preferred embodiment of the invention, it is foreseen that detecting the level of $CD4^+$ $HLA\text{-}DR^+$ T cells comprises measuring of one or more of the markers selected from CD3, CD4, CD8, CD14, CD45, CD56 and the activation marker HLA-DR. In another preferred embodiment of the present invention, the method further comprises again determining the level of monocytes in said test CSF sample being obtained from said subject which is examined and from whom the already obtained CSF sample has already been examined for the pan-disease classifiers as defined herein. In a further preferred embodiment of the invention, it is foreseen that detecting the level of monocytes comprises measuring of, as already defined elsewhere herein, one or more of the markers selected from CD11b, CD11c, CD14, CD16, CD45, CD62L, CD86, CD115, CD116, CD121, CD163, CD192, CD206, $CX_3CR1$ and HLA-DR. In a preferred embodiment when stratifying a subject with PMS, flow cytometry technique is used for detecting the level of CD4+ HLA-DR+ T cells, and/or the level of monocytes, preferably by detecting each specific cell surface marker / molecule such as CD3, CD4, CD8, CD11b, CD11c, CD14, CD16, CD45, CD56, CD62L, CD86, CD115, CD116, CD121, CD163, CD192, CD206, $CX_3CR1$ and/or HLA-DR on the cell surface of each specific cells, which demonstrates a certain strength of a signal being measured.

A further subject-matter of the present invention is thus directed to the method as described before, further stratifying said subject as suffering from progressive forms of multiple sclerosis (PMS), if one or more of the following are fulfilled: i) a level of $CD4^+$ $HLA\text{-}DR^+$ T cells in said test PB sample is increased relative to corresponding levels of $CD4^+$ $HLA\text{-}DR^+$ T cells in said control PB sample obtained from subjects suffering from another neuro-inflammatory autoimmune disease other than PMS; ii) a level of monocytes in said test CSF sample is increased relative to corresponding levels of monocytes in said control CSF samples obtained from subjects suffering from another neuro-inflammatory autoimmune disease other than PMS. Thus, if one or more of said requirements of said so called intra-MS classifiers are additionally fulfilled to said pan-disease classifiers and the requirements of said abovementioned inter-autoimmunity classifiers are not fulfilled as defined herein, it is indicative for whether said subject is suspected to suffer from PMS.

As a comparison, the control PB samples (see requirement of increased level of $CD4^+$ $HLA\text{-}DR^+$ T cells in said test PB sample) and/or the control CSF samples (see requirement of increased level of monocytes in said test CSF sample) are obtained from a cohort of subjects who have been diagnosed with not suffering from PMS, but have been diagnosed with suffering from another neuro-inflammatory autoimmune disease other than PMS. Thus, said control samples do not fulfill the abovementioned requirements outlined for said intra-MS classifiers, but fulfill the requirements for being stratified as suffering from a neuro-inflammatory autoimmune disease other than PMS. However, in the test sample of said subject being stratified with PMS as defined herein, all of the pan-disease classifiers as defined herein are detected, which fulfill the requirements listed in step b) of the method of the present invention. Additionally, in said test sample of said subject being stratified with PMS as defined herein also one or more, preferably all of the intra-MS classifiers can also be detected, which fulfill one or more of the requirements for said PMS stratification. Preferably, for said stratification of PMS, the requirements of i) and ii) of said intra-MS classifiers are additionally fulfilled as mentioned above. In said test sample of said subject being stratified with PMS as defined herein the inter-autoimmunity classifiers can be detected in said sample, but do not fulfil the particular requirements of said inter-autoimmunity classifiers as defined elsewhere herein.

PMS as used herein for the stratification of said subject within neuro-inflammatory autoimmune diseases comprises progressive relapsing multiple sclerosis (PRMS) and primary progressive multiple sclerosis (PPMS). Thus, it is also comprised by the present invention the method as defined elsewhere herein, further being able to stratify said subject as suffering from PRMS or PPMS, if one or more of said requirements of said intra-MS classifiers as defined elsewhere herein are additionally fulfilled as defined herein. In a preferred embodiment of the present invention, said method is further being able to stratify said subject as suffering from PPMS, if one or more of said requirements of said intra-MS classifiers as defined elsewhere herein are additionally fulfilled as defined herein.

The present inventors further generated a so called "composite score 1b" for stratifying a subject with PMS by dividing a particular determined level of plasma cells of said inter-autoimmunity classifier multiplied with a determined Ig synthesis of said inter-autoimmunity classifier and also multiplied with a determined level of immune cells per µl which are consistently increased by other particular determined cell levels of said intra-MS classifiers which are also consistently increased (see the Example section hereto). In other words, a composite score 1b may be generated by the ratio of said determined level of plasma cells in said test CSF sample multiplied with said determined Ig synthesis as defined elsewhere herein in said simultaneously obtained test CSF and serum samples and then also multiplied by said determined level of immune cells per µl in said test CSF sample to said determined level of monocytes in said test CSF sample multiplied with said determined level of $CD4^+$ HLA-$DR^+$ T cells in said test PB sample. Again, in this context, the term "ratio" refers to the division of said first multiplication product by said second multiplications product as defined elsewhere herein (see **Fig. 11D).** Thus, it is also comprised herein the method of the present invention as defined by step a) mentioned elsewhere herein, wherein the stratifying step b) instead comprises multiplying i) said determined level of plasma cells in said test CSF sample with said determined Ig synthesis as defined elsewhere herein in said simultaneously obtained test CSF and serum samples and with said determined level of immune cells per µl in said test CSF sample, thereby obtaining a multiplication product 1; ii) said determined level of monocytes in said test CSF sample with said determined level of $CD4^+$ HLA-$DR^+$ T cells in said test PB sample, thereby obtaining another multiplication product 2; and dividing said multiplication product 1 of step i) by said multiplication product 2 of step ii), thereby obtaining a composite score 1b.

The term "composite score 1b" or "composite score for stratifying a subject with PMS" refers to another score which is based on more than one, such as preferably five, specific classifiers from the inter-autoimmunity and the intra-MS classifiers defined elsewhere herein and which is calculated as mentioned above by the method of the invention. The difference between the basic composite score and the composite score 1b lies in the independent use, the first for stratifying a subject with neuro-inflammatory autoimmune disease, the second for stratifying a subject with PMS after already having stratified said subject with neuro-inflammatory autoimmune disease based on applying the method of the present invention for generating the basic composite score (composite score 1). In other words, for stratifying a subject with PMS, first the basic composite score has to be generated for the stratification whether a subject is suspected to suffer from neuro-inflammatory autoimmune diseases and then the composite score 1b has to be generated afterwards to clearly stratify said subject with PMS within the neuro-inflammatory autoimmune diseases. Thus, the basic composite score and the composite score 1b are independent from each other and are applied consecutively to the results of said test sample.

According to the present invention, stratifying a subject with PMS by determining the composite score 1b as defined herein with regard to the, may demonstrate a very sensitive and specific test (AUC: 0.772, see **Fig. 11D).**

According to the present invention, if said composite score 1b may be above the reference value as defined in more detail below, it may be indicative for whether or not said subject is suspected to suffer from PMS. The present invention also comprises that if said composite score 1b may deviate from said reference value, it may be indicative for whether or not said subject may be suspected to suffer from PMS. In a preferred embodiment, if said composite score 1b may deviate from said reference value by about 0.1%, about 0.2% about 0.3%, about 0.4%, about 0.5%, about 0.6%, about 0.7%, about 0.8%, about 0.9%, about 1%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10%, about 15%, about 20%, about 25%, about 30%, about 35%, about 40%, about 45%, about 50%, about 60%, about 70%, about 80%, about 90%, or by about 100%, it may be indicative for whether or not said subject may be suspected to suffer from PMS as defined herein. The greater the deviation of said composite score 1b from said reference value, the higher the possibility that the subject may be suspected to suffer from PMS.

In a preferred embodiment, the reference value may be at least about 0.05, at least about 0.06, at least about 0.07, at least about 0.08, at least about 0.09, at least about 0.1, at least about 0.2, at least about 0.3, at least about 0.4, at least about 0.5, at least about 0.6, at least about 0.7, at least about 0.8, at least about 0.9, at least about 1.0, or be any number in the range of about 0.05 to about 1.0, from about 0.05 to about 0.9, from about 0.05 to about 0.8, from about 0.05 to about 0.7, from about 0.05 to about 0.6, from about 0.05 to about 0.5, from about 0.05 to about 0.4, from about 0.05 to about 0.3, from about 0.05 to about 0.2, or from about 0.07 to about 0.8, from about 0.07 to about 0.7, from about 0.07 to about 0.6, from about 0.07 to about 0.5, from about 0.07 to about 0.4, from about 0.07 to about 0.3, or from about 0.09 to about 0.6, from about 0.09 to about 0.5, from about 0.09 to about 0.4, from about 0.09 to about 0.3, from about 0.09 to about 0.2, about 0.05 about 0.06, about 0.07, about 0.08, about 0.09, about 0.1, about 0.2, about 0.3, about 0.4, about 0.5, about 0.6, about 0.7, about 0.8, about 0.9, about 1.0, most preferably about 0.1. It is understood that ranges herein include all values in between such as natural numbers, rational numbers or real numbers. In an even more preferred embodiment, the reference value for the composite score 1b may be (at least) about 0.1. Thus, if said composite score 1b may be above the reference (or threshold) value of about 0.1, it may be indicative that the subject is suspected to suffer from PMS. Put it differently, if said composite score 1b may be above the reference (or threshold) value of about 0.1, the subject may be stratified with PMS. Vice versa, if said composite score 1b may be below the reference (or threshold) value of about 0.1, the subject may not be stratified with PMS, but be stratified with another neuro-inflammatory autoimmune disease other than PMS.

The abovementioned disclosure regarding the reference value / threshold value / ROCs and the associated methods used for accuracy mentioned with regard to the basic composite score are also applicable to said composite score 1b.

*Computer-implemented features*

The subject-matter of the defined method steps of the present invention may also fully be carried out by computer program instructions running on means which, in the context of the invention, provide generic data processing functions. Such means may, for example, be embedded in a personal computer, smartphone, printer. A computer-implemented invention may therefore be one which involves the use of a computer, computer network or other programmable apparatus, where one or more features are realised wholly or partly by means of a computer program.

Thus, the present invention comprises a data processing system comprising a processor configured to perform a method comprising the steps of: a) obtaining: i) the detected level of B cells from a test CSF sample obtained from a subject; ii) the detected level of immune cells per μl from said test CSF sample obtained from said subject; iii) the detected level of NKT cells from said test CSF sample obtained from said subject; iv) the detected level of monocytes from said test CSF sample obtained from said subject; and v) the detected level of $CD56^{dim}$ $CD16^{+}$ NK cells from a test PB sample obtained from said subject; b) stratifying said subject as suffering from neurological or psychiatric disease manifestation, preferably from neuro-inflammatory autoimmune diseases, if the following are fulfilled: i) the detected level of B cells is increased relative to corresponding levels of B cells in control CSF samples obtained from subjects not suffering from neuro-inflammatory autoimmune diseases; ii) the detected level of immune cells per μl is increased relative to corresponding levels of immune cells per μl in said control CSF samples obtained from said subjects not suffering from neuro-inflammatory autoimmune diseases; iii) the detected level of NKT cells is decreased relative to corresponding levels of NKT cells in said control CSF samples obtained from said subjects not suffering from neuro-inflammatory autoimmune diseases; iv) the detected level of monocytes is decreased relative to corresponding levels of monocytes in said control CSF samples obtained from said subjects not suffering from neuro-inflammatory autoimmune diseases; and v) the detected level of $CD56^{dim}$ $CD16^{+}$ NK cells is decreased relative to corresponding levels of $CD56^{dim}$ $CD16^{+}$ NK cells in control PB samples obtained from said subjects not suffering from neuro-inflammatory autoimmune diseases, wherein the combination of said levels i) - v) of step b) is indicative for whether said subject is suspected to suffer from neuro-inflammatory autoimmune diseases or from another neurological or psychiatric disease manifestation other than neuro-inflammatory autoimmune disease as defined elsewhere herein.

Further, the present invention comprises the data processing system comprising a processor configured to perform the abovementioned method further comprising the steps of c) obtaining vi) the detected Ig synthesis in said simultaneously obtained test CSF and serum samples and/or the detected type 2 or type 3 oligoclonal bands in said simultaneously obtained test CSF and serum samples obtained from said subject; and vii) the detected level of plasma cells in said test CSF sample obtained from said subject; d) stratifying said subject as suffering from RMS, if the following are fulfilled: vi) intrathecal immunoglobulin (Ig) synthesis can be detected in said simultaneously obtained test CSF and serum samples obtained from said subject and/or type 2 or type 3 oligoclonal bands can be detected in said simultaneously obtained test CSF and serum samples obtained from said subject, and vii) the detected level of plasma cells is increased relative to corresponding levels of plasma cells in said control CSF samples obtained from subjects suffering from another neuro-inflammatory autoimmune disease other than RMS.

Further, the present invention comprises the data processing system comprising a processor configured to perform the abovementioned method further comprising the steps of c) obtaining vi) the detected level of $CD56^{bright}$ $CD16^{dim/-}$ NK cells in said test PB sample obtained from said subject; and/or vii) the detected level of lactate in said test CSF sample obtained from said subject; d) stratifying said subject as suffering from NMOSD, if one or more of the following are fulfilled: vi) the detected level of $CD56^{bright}$ $CD16^{dim/-}$ NK cells in said test PB sample is decreased relative to corresponding levels of $CD56^{bright}$ $CD16^{dim/-}$ NK cells in control PB samples obtained from subjects suffering from another neuro-inflammatory autoimmune disease other than NMOSD, vii) the detected level of lactate in said test CSF sample is increased relative to corresponding levels of lactate in control CSF samples obtained from subjects suffering from another neuro-inflammatory autoimmune disease other than NMOSD.

Further, the present invention comprises the data processing system comprising a processor configured to perform the abovementioned method further comprising the steps of c) obtaining vi) the detected level of $CD8^{+}$ HLA-$DR^{+}$ T cells in said test PB and/or in said test CSF sample obtained from said subject; vii) the detected level of $CD8^{+}$ T cells in said test PB and/or in said test CSF sample obtained from said subject; and/or viii) the detected level of $CD4^{+}$ T cells in said test PB and/or in said test CSF sample obtained from said subject; d) stratifying said subject as suffering from SuS, if one or more of the following are fulfilled: vi) the detected level of $CD8^{+}$ HLA-$DR^{+}$ T cells in said test PB and/or in said test CSF sample is increased relative to corresponding levels of $CD8^{+}$ HLA-$DR^{+}$ T cells in control PB and/or control CSF samples obtained from subjects suffering from another neuro-inflammatory autoimmune disease other than SuS, vii) the detected level of $CD8^{+}$ T cells in said test PB and/or in said test CSF sample is increased relative to corresponding levels of $CD8^{+}$ T cells in control PB and/or control CSF samples obtained from subjects suffering from another neuro-inflammatory autoimmune disease other than SuS, viii) the detected level of $CD4^{+}$ T cells in said test PB and/or in said test CSF sample is decreased relative to corresponding levels of $CD4^+$ T cells in control PB and/or control CSF samples obtained from subjects suffering from another neuro-inflammatory autoimmune disease other than SuS.

Further, the present invention comprises the data processing system comprising a processor configured to perform the abovementioned method further comprising the steps of c) obtaining vi) the detected level of lymphocytes in said test CSF sample obtained from said subject; vii) the detected level of NKT cells in said test CSF sample obtained from said subject; and/or viii) the detected level of monocytes in said test CSF sample obtained from said subject; d) stratifying said subject as suffering from AIE, if one or more of the following are fulfilled: vi) the detected level of lymphocytes in said test CSF sample is decreased relative to corresponding levels of lymphocytes in control CSF samples obtained from subjects suffering from another neuro-inflammatory autoimmune disease other than AIE, vii) the detected level of NKT cells in said test CSF sample is increased relative to corresponding levels of NKT cells in control CSF samples obtained from subjects suffering from another neuro-inflammatory autoimmune disease other than AIE, viii) the detected level of monocytes in said test CSF sample is increased relative to corresponding levels of monocytes in control CSF samples obtained from subjects suffering from another neuro-inflammatory autoimmune disease other than AIE.

Further, the present invention comprises the data processing system comprising a processor configured to perform the abovementioned method further comprising the steps of c) obtaining vi) the detected level of $CD4^+$ $HLA\text{-}DR^+$ T cells in said test PB sample obtained from said subject; and/or vii) the detected level of monocytes in said test CSF sample obtained from said subject; d) stratifying said subject as suffering from PMS, if one or more of the following are fulfilled: vi) the detected level of $CD4^+$ $HLA\text{-}DR^+$ T cells in said test PB sample is increased relative to corresponding levels of $CD4^+$ $HLA\text{-}DR^+$ T cells in control PB samples obtained from subjects suffering from another neuro-inflammatory autoimmune disease other than PMS, vii) the detected level of monocytes in said test CSF sample is increased relative to corresponding levels of monocytes in said control CSF samples obtained from subjects suffering fromanother neuro-inflammatory autoimmune disease other than PMS.

According to the present invention, said specific cell levels as defined above may be detected in a test CSF and/or PB sample obtained from a subject according to the present invention using flow cytometry, in particular using a flow cytometry device. The detected level of specific cells being expressed in terms a decimal value or a percentage as described elsewhere herein may be entered into said data processing system comprising a processor which is configured to perform the abovementioned steps. In some embodiments, the step of obtaining said detected cell levels as defined above may be an optional step. As used herein, by using the term "obtaining / obtain the detected level of specific cells" in this context means that the data for the detected level (in percentage) of defined cells being detected in a flow cytometry device as defined elsewhere herein is entered into the data processing system by any way known to a person skilled in the art. The step of comparing the detected cell levels to the corresponding levels in the control samples for said particular stratifications is performed as defined elsewhere herein within the processor.

For the step of comparing said basic composite score, said composite score 1a or 1b with a reference value (or threshold value) as used herein, it is of importance whether said basic composite score, said composite score 1a or 1b is above or below the reference value (or threshold value). If said basic composite score, said composite score 1a or 1b may be above the reference value (or threshold value) being defined elsewhere herein, it may be indicative that said subject is suspected to suffer from neuro-inflammatory autoimmune diseases, RMS or PMS. If said basic composite score, said composite score 1a or 1b may be below the reference value (or threshold value) being defined elsewhere herein, it may be indicative that said subject is not suspected to suffer from neuro-inflammatory autoimmune diseases, but from another neurological or psychiatric disease manifestations as defined elsewhere herein or it may be indicative that said subject is not suspected to suffer from RMS, but from another neuro-inflammatory autoimmune disease other than RMS or it may be indicative that said subject is not suspected to suffer from PMS, but from another neuro-inflammatory autoimmune disease other than PMS. Also the deviation of said basic composite score, said composite score 1a or 1b being defined elsewhere herein from the reference value may be considered for the comparing step as defined above. The greater the deviation of said basic composite score, said composite score 1a or 1b from said reference value, the higher the possibility that the subject may be suspected to suffer from neuro-inflammatory autoimmune diseases, RMS or PMS.

Also comprised by the present invention is a data processing system comprising a processor configured to perform a method comprising the steps of

1.) comparing a basic composite score determined from a test CSF and a test PB sample obtained from a subject, wherein said score is being calculated by

a) multiplying

i) said determined level of immune cells per μl in said test CSF sample with said determined level of B cells in said test CSF sample and with the value of 100, thereby obtaining a multiplication product 1;

ii) said determined level of $CD56^{dim}$ $CD16^+$ NK cells in said test PB sample with said determined level of monocytes in said test CSF sample and with said determined level of NKT cells in said test CSF sample, thereby obtaining another multiplication product 2; and

b) dividing said multiplication product 1 of step a i) by said multiplication product 2 of step a ii), thereby obtaining a basic composite score,

with a reference value (or threshold value); and
2.) indicating / stratifying whether or not said subject is suspected to suffer from neuro-inflammatory autoimmune diseases.

Also comprised by the present invention is a data processing system comprising a processor configured to perform a method comprising the steps of

1.) comparing a composite score 1a determined from a test CSF sample obtained from a subject, wherein said score is being calculated by

a) summing up

i) said determined immunoglobulin (Ig) synthesis or said detected type 2 or type 3 oligoclonal bands in said simultaneously obtained test CSF and serum samples obtained from said subject; with
ii) a determined plasma cell level in said simultaneously obtained test CSF and serum samples, thereby obtaining a composite score 1a; and

2.) indicating / stratifying whether or not said subject is suspected to suffer from RMS.

Also comprised by the present invention is a data processing system comprising a processor configured to perform a method comprising the steps of

1.) comparing a composite score 1b determined from a test CSF and a test PB sample obtained from a subject, wherein said score is being calculated by

a) multiplying

i) said determined level of plasma cells in said test CSF sample with said determined Ig synthesis or said detected type 2 or type 3 oligoclonal bands as defined elsewhere herein in said simultaneously obtained test CSF and serum samples and with said determined level of immune cells per μl in said test CSF sample, thereby obtaining a multiplication product 1;
ii) said determined level of monocytes in said test CSF sample with said determined level of $CD4^+$ $HLA\text{-}DR^+$ T cells in said test PB sample, thereby obtaining a multiplication product 2; and

b) dividing said multiplication product 1 of step a i) by said multiplication product 2 of step a ii), thereby obtaining a composite score 1b.

with a reference value (or threshold value); and
2.) indicating / stratifying whether or not said subject is suspected to suffer from PMS.

The definitions made above for the other data processing system may also apply *mutatis mutandis* to this defined data processing system. As mentioned earlier, first a data processing system comprising a processor configured to perform a method comprising the steps as defined for said basic composite score need to be applied and then afterwards performing the method comprising the steps as defined for said composite score 1a, when stratifying a subject with RMS. The same applies for stratifying a subject with PMS, thus first a data processing system comprising a processor configured to perform a method comprising the steps as defined for said basic composite score need to be applied and then afterwards performing the method comprising the steps as defined for said composite score 1b.

A more meaningful characterization of modern digital data processing systems is the functional classification as either computational or input/output ("I/O") oriented. As the classifying labels imply, computational oriented data processing systems are designed primarily for performing long, complicated calculations. I/O oriented data processing systems are designed to handle large quantities of digital data, thereby requiring extensive I/O operations. Both data processing systems may be comprised by the present invention. The data processing system of the present invention may also be utilized as a peripheral subsystem of a larger computational computer. The structural design of a data processing system may necessarily directly be related to the functional use to which the data processing system is put. A data processing system of the present invention may refer to a programmable or programmed device / apparatus, where one or more features are realised wholly or partly by means of a computer program. A data processing system may include, but are not limited to, a computer, smartphone, tablet, chip.

As used herein, the term "processor" may refer to one functional element being a physical unit of a data processing system, which is configured by a computer program as defined elsewhere herein to perform the specified steps mentioned above.

The present invention further comprises the interaction between the data processing steps and other technical means such as a flow cytometry device.

Thus, the present invention comprises a flow cytometry device capable of detecting the following: i) the level of B cells in a test CSF sample obtained from a subject; ii) the level of immune cells per μl in said test CSF sample obtained from said subject; iii) the level of NKT cells in said test CSF sample obtained from said subject; iv) the level of monocytes in said test CSF sample obtained from said subject, and v) the level of $CD56^{dim}$ $CD16^{+}$ NK cells in a test PB sample obtained from said subject; optionally vi) the level of plasma cells in said test CSF sample obtained from said subject; vii) the level of $CD56^{bright}$ $CD16^{dim/-}$ NK cells in said test PB sample obtained from said subject; viii) the level of $CD8^{+}$ $HLA\text{-}DR^{+}$ T cells in said test PB and/or in said test CSF sample obtained from said subject; ix) the level of $CD8^{+}$ T cells in said test PB and/or in said test CSF sample obtained from said subject; x) the level of $CD4^{+}$ T cells in said test PB and/or in said test CSF sample obtained from said subject; xi) the level of lymphocytes in said test CSF sample obtained from said subject; and/or xii) the level of $CD4^{+}$ $HLA\text{-}DR^{+}$ T cells in said test PB sample obtained from said subject; comprising the data processing system of the present invention as defined elsewhere herein.

As defined above, said specific cell levels may be detected by using a flow cytometry device, preferably using flow cytometry analysis being combined with immunofluorescence as described elsewhere herein. As an illustrative example, said flow cytometry device as defined above may be a FACS. In some embodiments said flow cytometry such as for example FACS may be connected to a computer, tablet, smartphone or any other technical device / apparatus being used as a further means for performing said flow cytometry analysis.

The present invention also envisages a computer program comprising instructions to cause the data processing system as defined above or the flow cytometry device as defined above to execute the steps of a) obtaining: i) the detected level of B cells from a test CSF sample obtained from a subject; ii) the detected level of immune cells per μl from said test CSF sample obtained from said subject; iii) the detected level of NKT cells from said test CSF sample obtained from said subject; iv) the detected level of monocytes from said test CSF sample obtained from said subject; and v) the detected level of $CD56^{dim}$ $CD16^{+}$ NK cells from a test PB sample obtained from said subject; b) stratifying said subject as suffering from neurological or psychiatric disease manifestation, preferably from neuro-inflammatory autoimmune diseases, if the following are fulfilled: i) the detected level of B cells is increased relative to corresponding levels of B cells in control CSF samples obtained from subjects not suffering from neuro-inflammatory autoimmune diseases; ii) the detected level of immune cells per μl is increased relative to corresponding levels of immune cells per μl in said control CSF samples obtained from said subjects not suffering from neuro-inflammatory autoimmune diseases; iii) the detected level of NKT cells is decreased relative to corresponding levels of NKT cells in said control CSF samples obtained from said subjects not suffering from neuro-inflammatory autoimmune diseases; iv) the detected level of monocytes is decreased relative to corresponding levels of monocytes in said control CSF samples obtained from said subjects not suffering from neuro-inflammatory autoimmune diseases; and v) the detected level of $CD56^{dim}$ $CD16^{+}$ NK cells is decreased relative to corresponding levels of $CD56^{dim}$ $CD16^{+}$ NK cells in control PB samples obtained from said subjects not suffering from neuro-inflammatory autoimmune diseases, wherein the combination of said detected levels i) - v) of step b) is indicative for whether said subject is suspected to suffer from neuro-inflammatory autoimmune diseases or from another neurological or psychiatric disease manifestation other than neuro-inflammatory autoimmune disease.

Further, also comprised by the present invention is a computer program comprising instructions to cause the data processing system as defined above or the flow cytometry device as defined above to further execute the steps of c) obtaining vi) the detected immunoglobulin (Ig) synthesis in said simultaneously obtained test CSF and serum samples and/or the detected type 2 or type 3 oligoclonal bands in said simultaneously obtained test CSF and serum samples obtained from said subject; and vii) the detected level of plasma cells in said test CSF sample obtained from said subject; d) stratifying said subject as suffering from RMS, if the following are fulfilled: vi) the immunoglobulin (Ig) synthesis can be detected in said simultaneously obtained test CSF and serum samples and/or type 2 or type 3 oligoclonal bands can be detected in said simultaneously obtained test CSF and serum samples obtained from said subject, and vii) the detected level of plasma cells is increased relative to corresponding levels of plasma cells in said control CSF samples obtained from subjects suffering from another neuro-inflammatory autoimmune disease other than RMS.

Further, also comprised by the present invention is a computer program comprising instructions to cause the data processing system as defined above or the flow cytometry device as defined above to further execute the steps of c) obtaining vi) the detected level of $CD56^{bright}$ $CD16^{dim/-}$ NK cells in said test PB sample obtained from said subject; and/or vii) the detected level of lactate in said test CSF sample obtained from said subject; d) stratifying said subject as suffering from NMOSD, if one or more of the following are fulfilled: vi) the detected level of $CD56^{bright}$ $CD16^{dim/-}$ NK cells in said PB sample is decreased relative to corresponding levels of $CD56^{bright}$ $CD16^{dim/-}$ NK cells in control PB samples obtained from subjects suffering from another neuro-inflammatory autoimmune disease other than NMOSD, vii) the detected level of lactate is increased relative to corresponding levels of lactate in control CSF samples obtained from subjects suffering from another neuro-inflammatory autoimmune disease other than NMOSD.

Further, also comprised by the present invention is a computer program comprising instructions to cause the data processing system as defined above or the flow cytometry device as defined above to further execute the steps of c) obtaining vi) the detected level of $CD8^+$ HLA-$DR^+$ T cells in said test PB and/or in said test CSF sample obtained from said subject; vii) the detected level of $CD8^+$ T cells in said test PB and/or in said test CSF sample obtained from said subject; and/or viii) the detected level of $CD4^+$ T cells in said test PB and/or in said test CSF sample obtained from said subject; d) stratifying said subject as suffering from SuS, if one or more of the following are fulfilled: vi) the detected level of $CD8^+$ HLA-$DR^+$ T cells is increased relative to corresponding levels of $CD8^+$ HLA-$DR^+$ T cells in control PB and/or control CSF samples obtained from subjects suffering from another neuro-inflammatory autoimmune disease other than SuS, vii) the detected level of $CD8^+$ T cells is increased relative to corresponding levels of $CD8^+$ T cells in control PB and/or control CSF samples obtained from subjects suffering from another neuro-inflammatory autoimmune disease other than SuS, viii) the detected level of $CD4^+$ T cells is decreased relative to corresponding levels of $CD4^+$ T cells in control PB and/or control CSF samples obtained from subjects suffering from another neuro-inflammatory autoimmune disease other than SuS.

Further, also comprised by the present invention is a computer program comprising instructions to cause the data processing system as defined above or the flow cytometry device as defined above to further execute the steps of c) obtaining vi) the detected level of lymphocytes in said test CSF sample obtained from said subject; vii) the detected level of NKT cells in said test CSF sample obtained from said subject; and/or viii) the detected level of monocytes in said test CSF sample obtained from said subject; d) stratifying said subject as suffering from AIE, if one or more of the following are fulfilled: vi) the detected level of lymphocytes is decreased relative to corresponding levels of lymphocytes in control CSF samples obtained from subjects suffering from another neuro-inflammatory autoimmune disease other than AIE, vii) the detected level of NKT cells is increased relative to corresponding levels of NKT cells in control CSF samples obtained from subjects suffering from another neuro-inflammatory autoimmune disease other than AIE, viii) the detected level of monocytes is increased relative to corresponding levels of monocytes in control CSF samples obtained from subjects suffering from another neuro-inflammatory autoimmune disease other than AIE.

Further, also comprised by the present invention is a computer program comprising instructions to cause the data processing system as defined above or the flow cytometry device as defined above to further execute the steps of c) obtaining vi) the detected level of $CD4^+$ HLA-$DR^+$ T cells in said test PB sample obtained from said subject; and/or vii) the detected level of monocytes in said test CSF sample obtained from said subject; d) stratifying said subject as suffering from PMS, if one or more of the following are fulfilled: vi) the detected level of $CD4^+$ HLA-$DR^+$ T cells is increased relative to corresponding levels of $CD4^+$ HLA-$DR^+$ T cells in control PB samples obtained from subjects suffering from another neuro-inflammatory autoimmune disease other than PMS, vii) the detected level of monocytes is increased relative to corresponding levels of monocytes in said control CSF samples obtained from subjects suffering fromanother neuro-inflammatory autoimmune disease other than PMS.

Again, in some embodiments, the step of obtaining said detected cell levels as defined above may be an optional step. Everything being defined above for said data processing system may apply *mutatis mutandis* to the corresponding computer program as defined herein.

According to the present invention a computer program as mentioned above may refer to a program listing written in a programming language to implement an algorithm, and to binary code loaded in a computer-based apparatus, encompassing the accompanying documentation. A computer program as defined above may include, but is not limited to any software or any downloadable internet link known to a person skilled in the art comprising instructions to cause the data processing system as defined elsewhere herein to execute the defined steps.

Also comprised herein is a computer program comprising instructions to cause the data processing system as defined above or the flow cytometry device as defined above to execute the steps of

1.) comparing a basic composite score determined from a test CSF and a test PB sample obtained from a subject, wherein said score is being calculated by

a) multiplying

i) said determined level of immune cells per µl in said test CSF sample with said determined level of B cells in said test CSF sample and with the value of 100, thereby obtaining a multiplication product 1;

ii) said determined level of $CD56^{dim}$ $CD16^+$ NK cells in said test PB sample with said determined level of monocytes in said test CSF sample and with said determined level of NKT cells in said test CSF sample, thereby obtaining another multiplication product 2; and

b) dividing said multiplication product 1 of step a i) by said multiplication product 2 of step a ii), thereby obtaining a basic composite score,

with a reference value (or threshold value); and
2.) indicating / stratifying whether or not said subject is suspected to suffer from neuro-inflammatory autoimmune diseases.

Also comprised herein is a computer program comprising instructions to cause the data processing system as defined above or the flow cytometry device as defined above to execute the steps of

1.) comparing a composite score 1a determined from a test CSF sample obtained from a subject, wherein said score is being calculated by

a) summing up

i) said determined immunoglobulin (Ig) synthesis or said detected type 2 or type 3 oligoclonal bands in said simultaneously obtained test CSF and serum samples obtained from said subject; with
ii) a determined plasma cell level in said simultaneously obtained test CSF and serum samples, thereby obtaining a composite score 1a; and

2.) indicating / stratifying whether or not said subject is suspected to suffer from RMS.

Also comprised herein is a computer program comprising instructions to cause the data processing system as defined above or the flow cytometry device as defined above to execute the steps of

1.) comparing a composite score 1b determined from a test CSF and a test PB sample obtained from a subject, wherein said score is being calculated by

a) multiplying

i) said determined level of plasma cells in said test CSF sample with said determined Ig synthesis or said detected type 2 or type 3 oligoclonal bands as defined elsewhere herein in said simultaneously obtained test CSF and serum samples and with said determined level of immune cells per μl in said test CSF sample, thereby obtaining a multiplication product 1;
ii) said determined level of monocytes in said test CSF sample with said determined level of $CD4^+$ $HLA\text{-}DR^+$ T cells in said test PB sample, thereby obtaining a multiplication product 2; and

b) dividing said multiplication product 1 of step a i) by said multiplication product 2 of step a ii), thereby obtaining a composite score 1b.

with a reference value (or threshold value); and
2.) indicating / stratifying whether or not said subject is suspected to suffer from PMS.

Also comprised by the present invention is a computer-readable medium having stored thereon one of the computer programs as defined above. A computer-readable medium having stored thereon all of the computer programs as defined above may also be comprised herein. As used herein, a computer-readable medium having stored thereon the computer program may include, but is not limited to, a USB stick, a disc, DVD, CD, CD-ROM.

***Kit* of *the present invention***

Also comprised by the present invention is a kit comprising a fluorescently labeled binding partner for CD1c, CD1d, CD3, CD4, CD5, CD8, CD11b, CD11c, CD14, CD16, CD19, CD20, CD21, CD27, CD24, CD38, CD40, CD45, CD56, CD62L, CD80, CD86, CD94, CD115, CD116, CD121, CD122, CD138, CD159a, CD161, CD163, CD192, CD206, CD335, CD337, CXCR5 and $CX_3CR1$ IgD, IgM, TACI, TCRγδ, Vα24-Jα18, Vβ11, Vδ1, Vδ2, KIR, Tbet, EOMES and HLA-DR.

Preferably, also comprised by the present invention is a kit comprising a fluorescently labeled binding partner for CD1c, CD1d, CD3, CD4, CD5, CD8, CD11b, CD11c, CD14, CD16, CD19, CD20, CD21, CD27, CD24, CD38, CD40, CD45, CD56, CD62L, CD78, CD80, CD86, CD94, CD115, CD116, CD121, CD122, CD138, CD159a, CD161, CD163, CD192, CD206, CD319, CD335, CD337, CXCR4, CXCR5 and $CX_3CR1$ IgD, IgM, TACI, TCRγδ, Vα24-Jα18, Vβ11, Vδ1, Vδ2, KIR, Tbet, EOMES, BCMA and HLA-DR.

Also preferably and comprised by the present invention is a kit comprising a fluorescently labeled binding partner for CD1c, CD1d, CD3, CD4, CD5, CD8, CD11b, CD11c, CD14, CD16, CD19, CD20, CD21, CD27, CD24, CD25, CD38, CD40, CD45, CD56, CD62L, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5 and $CX_3CR1$ IgD, IgM, TACI, TCRγδ, Vα24-Jα18, Vβ11, Vδ1, Vδ2, KIR, Tbet, EOMES, BCMA and HLA-DR.

Also preferably and comprised by the present invention is a kit comprising a fluorescently labeled binding partner for CD1c, CD1d, CD3, CD4, CD5, CD8, CD11b, CD11c, CD14, CD15, CD16, CD19, CD20, CD21, CD27, CD24, CD38, CD40, CD45, CD56, CD62L, CD66b, CD78, CD80, CD86, CD94, CD115, CD116, CD121, CD122, CD138, CD159a, CD161, CD163, CD192, CD206, CD319, CD335, CD337, CXCR4, CXCR5 and $CX_3CR1$ IgD, IgM, TACI, TCRγδ, Vα24-Jα18, Vβ11, Vδ1, Vδ2, KIR, Tbet, EOMES, BCMA and HLA-DR.

Mostly preferred and comprised by the present invention is a kit comprising a fluorescently labeled binding partner for CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and HLA-DR.

According to the present invention, the fluorescently labeled binding partner for CD1c, CD1d, CD3, CD4, CD5, CD8, CD11b, CD11c, CD14, CD16, CD19, CD20, CD21, CD27, CD24, CD38, CD40, CD45, CD56, CD62L, CD80, CD86, CD94, CD115, CD116, CD121, CD122, CD138, CD159a, CD161, CD163, CD192, CD206, CD335, CD337, CXCR5 and $CX_3CR1$, IgD, IgM, TACI, TCRγδ, Vα24-Jα18, Vβ11, Vδ1, Vδ2, KIR, Tbet, EOMES and HLA-DR are preferably provided in one or more containers or vials in a kit (pharmaceutical pack), which may be associated with a notice in the form prescribed by a governmental agency regulating the manufacture, use or sale of pharmaceuticals or biological products, reflecting approval by the agency of the manufacture, use or sale of the product for human administration or diagnostics. The same applies *mutatis mutandis* to the preferred abovementioned kits as well.

Thus, the present invention may comprise a kit comprising one vial or container comprising all of the specific fluorescently labeled binding partners mentioned above for each kit. Put it differently, said kit may comprise a preparation comprising a mixture of all of the fluorescently labeled binding partners defined above for each kit.

Also comprised by the present invention, is a kit comprising one vial for each fluorescently labeled binding partner, thereby comprising vials: one vial comprising a binding partner for CD1c, one vial comprising a binding partner for CD1d, one vial or container comprising a binding partner for CD3, one vial comprising a binding partner for CD4, one vial or container comprising a binding partner for CD5, one vial comprising a binding partner for CD8, one vial comprising a binding partner for CD11b, one vial comprising a binding partner for CD11c, one vial or container comprising a binding partner for CD14, one vial or container comprising a binding partner for CD16, one vial comprising a binding partner for CD19, one vial comprising a binding partner for CD20, one vial comprising a binding partner for CD21, one vial comprising a binding partner for CD24, one vial or container comprising a binding partner for CD27, one vial comprising a binding partner for CD38, one vial comprising a binding partner for CD40, one vial comprising a binding partner for CD45, one vial comprising a binding partner for CD56, one vial comprising a binding partner for CD62L, one vial comprising a binding partner for CD80, one vial comprising a binding partner for CD86, one vial comprising a binding partner for CD94, one vial comprising a binding partner for CD115, one vial comprising a binding partner for CD116, one vial comprising a binding partner for CD121, one vial comprising a binding partner for CD122, one vial or container comprising a binding partner for CD138, one vial comprising a binding partner for CD159a, one vial or container comprising a binding partner for CD161, one vial comprising a binding partner for CD163, one vial comprising a binding partner for CD192, one vial comprising a binding partner for CD206, one vial comprising a binding partner for CD335, one vial comprising a binding partner for CD337, one vial comprising a binding partner for CXCR5, one vial comprising a binding partner for $CX_3CR1$, one vial or container comprising a binding partner for IgD, one vial or container comprising a binding partner for IgM, one vial or container comprising a binding partner for TACI, one vial or container comprising a binding partner for TCRγδ, one vial or container comprising a binding partner for Vα24-Jα18, one vial or container comprising a binding partner for Vβ11, one vial or container comprising a binding partner for Vδ1, one vial or container comprising a binding partner for Vδ2, one vial or container comprising a binding partner for KIR, one vial or container comprising a binding partner for Tbet, one vial or container comprising a binding partner for EOMES, one vial or container comprising a binding partner for HLA-DR. The same applies *mutatis mutandis* to the preferred abovementioned kits as well, thus also comprising one vial or container comprising a binding partner for each marker listed in said kits.

Further comprised by the present invention is a kit comprising at least one vial or container comprising a mixture of at least one fluorescently labeled binding partner for B cells, at least one fluorescently labeled binding partner for NKT cells, at least one vial or container comprising a mixture of at least one fluorescently labeled binding partner for monocytes, at least one vial or container comprising a mixture of at least one fluorescently labeled binding partner for $CD56^{dim}$ $CD16^{+}$ NK cells, preferably comprising at least one vial or container comprising a mixture of at least one fluorescently labeled binding partner for plasma cells.

In this context, further comprised by the present invention is said kit mentioned above further comprising at least one vial or container comprising a mixture of at least one fluorescently labeled binding partner for $CD56^{bright}$ $CD16^{dim/-}$ NK cells.

Further comprised by the present invention is said kit mentioned above further comprising at least one vial or container comprising a mixture of at least one fluorescently labeled binding partner for $CD8^+$ HLA-$DR^+$ T cells, at least one vial or container comprising a mixture of at least one fluorescently labeled binding partner for $CD8^+$ T cells, and/or at least one vial or container comprising a mixture of at least one fluorescently labeled binding partner for $CD4^+$ T cells.

Further comprised by the present invention is said kit mentioned above further comprising at least one vial or container comprising a mixture of at least one fluorescently labeled binding partner for lymphocytes.

Further comprised by the present invention is said kit mentioned above further comprising at least one vial or container comprising a mixture of at least one fluorescently labeled binding partner for $CD4^+$ HLA-$DR^+$ T cells.

According to the present invention, the kit may further comprise a carrier, independent of how the kit is arranged. If the kit is arranged in having only one vial or container comprising a mixture of all of the fluorescently labeled binding partners defined for each kit, said carrier may also be comprised in said one vial or container. If the kit is arranged in having only one vial or container for each fluorescently labeled binding partner being used for each cell marker of said kit as defined above, said carrier may also be comprised in each vial or container for each fluorescently labeled binding partner. If the kit is arranged in having one vial or container comprising a mixture of at least one fluorescently labeled binding partner for each specific cell, said carrier may also be comprised in said one vial or container.

The term "carrier" refers to a diluent, adjuvant, or vehicle with which the binding partner is compounded / mixed in the kit. Such carriers can be sterile liquids, such as water, buffer including PBS and oils including those of petroleum, animal, vegetable or synthetic origin, such as peanut oil, soybean oil, mineral oil, sesame oil and the like. Water or PBS is a preferred carrier.

## EXAMPLES OF THE INVENTION

The following Examples illustrate the invention, but are not to be construed as limiting the scope of the invention.

### Material and Methods

### Experimental design.

The study aimed to identify cellular and/or molecular parameters in CSF in comparison to PB that improve diagnosis of neurological diseases **(Fig. 6).** Therefore, data from a discovery cohort of in total 546 patients were investigated **(Fig. 1A).** After elimination co-linear parameters, missing values were calculated **(Fig. 1B).** The resulting data set of the discovery cohort was analyzed by a combination of dimensionality reduction, exploratory analysis, and machine learning to identify "pan-disease" and "inter-autoimmunity" classifiers as well as their meaning during disease propagation and activity **(Fig. 1C).** "Pan-disease" and "interautoimmunity" classifiers were further summarized in composite scores to facilitate clinical application. The power of these composite scores to differentiate patients with CNS-autoimmunity from non-inflammatory controls, neuro-degenerative and -vascular patients was tested ultimately verified in an independent validation cohort **(Fig. 1C).**

### Patient characteristics.

3424 files of patients who received routine CSF diagnostics and multi-parameter flow cytometry were retrospectively screened. Among these 546 manually curated patients were selected for the discovery cohort including (i) 74 non-inflammatory controls; (ii) 282 patients with neuroinflammatory diseases including 196 relapsing multiple sclerosis (MS) patients, 15 NMO spectrum disorder (NMOSD) patients, 14 Susac syndrome (SuS) patients, and 57 autoimmune encephalitis (AIE) patients; (iii) 93 patients with neuro-degenerative diseases including 52 amyotrophic lateral sclerosis (ALS) patients and 41 mild Alzheimer disease (mAD) patients, and (iv) 97 patients with an ischemic stroke exemplary for a neuro-vascular disease **(Fig. 1A).** In addition, an independent validation cohort of 231 patients ((i) 35; (ii) 32; (iii) 156; (iv) 8) was identified to verify results generated based on data from the discovery cohort **(Fig. 1A).** All patients were treatment-naive for immune-modulating drugs at the time of sampling. Patients were diagnosed according to current guidelines (60-65) by experienced neurologists. Non-inflammatory controls included patients with somatoform disorders or patients who donated CSF during the course of spinal anesthesia. Non-inflammatory controls presented with intrathecal leukocyte counts <5 cells/μl, intrathecal lactate <2mmol/l, intact blood-CSF-barrier as identified by age-adjusted albumin quotient, no intrathecal Ig-synthesis according to Reiber criteria, as well as oligoclonal band pattern type 1. MS disease activity at time point of lumbar puncture was defined as a clinical relapse within the preceding 4 weeks and/or new T2 or Gadolinium enhanced lesions in brain MRI within the preceding 12 weeks. MRI scans were performed with 1.5 or 3.0 Tesla field strength. In addition to 576 untreated patients, 105 RRMS patients under disease-modifying therapy (interferon-β n = 9, glatiramer acetate n = 5, dimethyl fumarate n = 5, fingolimod n = 15, natalizumab n = 41, alemtuzumab 10-14 months post last injection n = 30) were included. To investigate the specificity of composite scores in a broader clinical setting, untreated patients with further neurological and psychiatric disease manifestations were included **(Fig. 12).**

**Biomaterial.**

Cerebrospinal fluid (CSF) was collected by lumbar puncture and processed within one hour simultaneously to serum collected in serum-gel tubes (Sarstedt), and to peripheral blood (PB) collected in EDTA tubes (Sarstedt).

**Routine diagnostics.**

All laboratory diagnostics, including flow cytometry, were performed in a certified clinical laboratory by experienced technicians following documented standard operating procedures (SOPs). CSF cells were counted using a Fuchs-Rosenthal chamber following a 5:1 dilution with methylviolett-solution (Waldeck). Total protein, albumin, and immunoglobulin concentrations were analyzed by nephelometry (BN ProSpec, Siemens) according to the manufacturer's guidelines. Composite parameters like blood/CSF barrier integrity and intrathecal Ig-synthesis (Reiber) are calculated from the results. The oligoclonal band pattern was analyzed by electrophoresis and subsequent silver staining. Therefore, 5μl serum and 100μl cell-free CSF are diluted to 25mg IgG/l with IgG sample diluter (Serva) and 20μl sample are transferred into the wells of FocusGel 6-11 24S (Serva) placed on a Multiphor II cooling plate (Pharmacia Biotech). Following electrophoresis with 30W (15-50mA, 250-1700V) for 135min using an EPS 350XL power supply (Pharmacia Biotech), the gel is carefully transferred to a Processor plus gel stainer (GE Healthcare), fixed, and covered. After running the automated staining process, the gel is dried at room temperature and sealed with Mylar preservation sheets (GE Healthcare). Results are analyzed in comparison to samples with known type 1 and type 2 patterns. Glucose and lactate concentrations were analyzed in cell-free CSF and serum by pipetting 20μl sample into the red sample segment of a SuperGL compact (Hitado).

**Multi-parameter flow cytometry.**

Cells from the CSF were analyzed in parallel to EDTA-blood samples. Therefore, CSF was spun down at 290g for 15min. The supernatant was removed and CSF cells were re-suspended in parallel to 100μl EDTA-blood in 1ml VersaLyse (Beckman Coulter) to lyse erythrocytes. Following 10min of incubation, cells were washed twice with flow cytometry buffer (FC buffer: PBS, 2% heat-inactivated FCS, 2mM EDTA; 300g 4min). Cells were stained in 100μl FC buffer supplemented with fluorochrome-conjugated antibodies directed against CD3 (UCHT1), CD4 (13B8.2), CD8 (B9.11), CD14 (RM052), CD16 (3G8), CD19 (J3-119), CD45 (J33), CD56 (N901), CD138 (B-A38), and HLA-DR (Immu-357) (all Beckman Coulter, clones in brackets). After washing, cells were re-suspended in FC buffer, 20μl flow count fluorospheres (Beckman Coulter) were added, and samples were acquired using a Navios flow cytometer (Beckman Coulter). Resulting files were analyzed using Kaluza 2.1 software (Beckman Coulter). Resulting files were analyzed using Kaluza 2.1 software (Beckman Coulter) according to the gating strategy described in **Fig. 7.**

**Data processing.**

Samples with more than 30% missing values were systematically removed. As a result, the missingness in the remaining data set **(Fig. 1A, Tab. 1)** was below 1%. The inventors computed pair-wise correlations using complete observations and calculated their statistical significance using the *corr.test* function from *psych* package in R3.5.2 via RStudio 1.1.442 including adjustment for multiple comparisons using the 'Holm' approach. Variables showing a high absolute correlation (>0.9) with statistically significant p-values (<0.05) were considered as correlated **(Fig. 9).** From correlated variables only one was included in the further analyses **(Table 3).** Furthermore, from biological closely related features (e.g. $CD4^+$ T cells/ml and $CD4^+$ T cells as percentage of lymphocytes) only one of the features was retained. Features excluded in one compartment were also excluded in the respective other compartment (e.g. replacement of $CD4^+$ T cells/ml in PB and CSF). With the non-collinear variables, missing data was imputed group-wise, because observations from different groups could widely differ across variables, using median imputation method. Therefore, the median of each variable at each group was calculated and the missing values for that variable and group were assigned their median value adding a tiny jitter to make these imputed values different from each other. For categorical parameters, imputed values were rounded to the next categorical value. For machine learning approaches as well as UMAP dimensionality reduction, the resulting data was additionally centered and scaled along the variables to bring all the variables to a similar scale and set the data to a normal distribution. Data processing is illustrated in **Figure 1B.**

**Machine learning.**

The inventors implemented logistic regression as a way to classify samples in two groups. First, they randomly split the data into training (75%) and test samples (25%). Next, they identified variables in each group that were statistically significant ($p<0.05$) using a two-sided t-test. They then took these significant variables and ran logistic regression using the *MLeam* function in *MLInterfaces* (1.68.0) BioConductor package in R, thus developing models containing up to 5 variables. They computed several performance metrics by *MLInterfaces* and *ROCR* (1.0-11) including Prediction Accuracy (PA), Area Under the Curve (AUC), Sensitivity (SE), and Specificity (SP).

**UMAP.**

Uniform manifold approximation and prediction (UMAP) arranges data points based on differences with high dimensional information in two dimensions in an unsupervised manner. UMAP analysis of centered and scaled data was performed by Rstudio (3.5.3) using umap package (0.2.4.0) as well as ggplot2 (3.2.1) and ggrepel (0.8.1) packages with Euclidean metric.

**Volcano plots.**

Volcano plots provide a tool to visualize differences between two groups while incorporating statistical information. Volcano plots are constructed by plotting log2 values of the relative difference between the medians (continuous parameters) or means (categorical parameters) against the p-values calculated by Mann-Whitney t-test. In case of division by zero, the relative difference is assumed as 1+X, whereas in case of division of zero, the log2 of the relative difference is set to -1.

**Heatmaps.**

Heatmaps were created by calculating the median for continuous and the mean for categorical parameters for each group. Resulting values were 0/1 normalized and plotted using package pheatmap (1.0.12) in Rstudio. For pooling distinct diseases with strongly divergent numbers **(Fig. 1),** first the median/mean within each disease was calculated, before summarizing the results as mean in order to adequately reflect the characteristics of each disease.

**Re-alignment plot.**

The re-alignment plot (Fig. 3C) describes changes in parameters relative to differences between two distinct levels defining the scale for each parameter. Therefore, medians from different groups (X) are re-aligned by division of the result from the subtraction of the negative control median (here: non-inflammatory groups) from the respective median by the result of the subtraction of the control median from the median of positive control median (here treatment naïve RRMS)

$$Y = \frac{X - negctrl}{posctrl - negctrl}$$

Y values below zero indicate a change beyond negative control levels, between zero and one a partial effect, and above one an even stronger deviation from negative control levels.

**Composite scores.**

In general, composite scores assisting differentiation of distinct pathologies on "pan-" or "interdisease" level were created by dividing parameters identified to be consistently increased by parameters exhibiting reduced frequencies. Parameters frequently taking a value of zero - e.g. categorical parameters - were additionally added by one to avoid errors while preserving information content.

**ROC analyses.**

Receiver operating characteristic (ROC) analyses were performed in Rstudio using the plot.roc function from the pROC (1.16.2) package.

**Correlation analyses.**

Correlation analyses of the effect of aging on the investigated linear parameters were performed in Rstudio by function cor and package ggcorrplot (0.1.3). Only significantly ($p<0.05$) correlated indices were illustrated.

**Statistical analysis.**

Statistical analyses were performed using R3.5.2 via RStudio 1.1.442, GraphPad Prism V6.01, and Excel 2016. Tests used are indicated in the respective figure legend.

**Results**

**Example 1: Strategy for identification of peripheral and intrathecal Immune signatures in neurological diseases.**

To identify potential novel cellular and/or molecular parameters in CSF in comparison to PB that improve diagnosis of neurological diseases **(Fig. 6),** a cohort of 546 patients (discovery) from our local clinical database was retrospectively identified fitting into four disease categories (autoimmune inflammatory, neuro-degenerative, neuro-vascular, and non-inflammatory conditions) and receiving no immune-modulating drugs at time point of sampling **(Fig. 1A, Tab. 1).** All patients underwent a lumbar puncture and assessment of standard CSF parameters such as cell count, total CSF protein (TPC), QAlb (as an indicator for the blood/CSF barrier integrity), intrathecal IgG, IgA, and IgM synthesis, oligoclonal bands (OCBs), QGluc, and lactate **(Fig. 1B).** Standard CSF parameters showed disease-specific changes within expected limits **(Tab. 1).** Additionally, the cellular composition in the PB and CSF of each patient was analyzed by multi-parameter flow cytometry covering the major immune-cell subsets and activation status of T-cells **(Fig. 1B, Fig. 7).** As expected from the literature, cellular parameters analyzed by flow cytometry strongly differentiated between PB and CSF under non-inflammatory conditions **(Fig. 8),** which constitutes a first "sanity check" for our approach and dataset. By combining conventional CSF analysis with multi-parameter flow cytometry, the inventors obtained a total of 113 cellular and molecular features in the PB and CSF per patient was obtained **(Fig. 1B).** To eliminate co-linear features while retaining as much information as possible, a pair-wise correlation analysis with all parameters was performed **(Fig. 9).** Based on this analysis, correlated parameters ($r2 > 90\%$, p-value $< 0.05$) were condensed into a single feature for further analysis. Thereby, the number of features was reduced to 34 **(Fig. 1B, Table 2),** a sufficient number to accurately define patient-specific profiles. The patient-specific immune profiles defined by the 34 parameters were then investigated in the discovery cohort **(Fig. 1C)** to identify "pan-disease" parameters specific for all neurological disease categories, "inter-autoimmunity" classifiers differentiating variants of CNS directed autoimmunity, and "intra-disease" parameters describing disease progression and activation **(Fig. 6).** "Pan-disease" parameters and "inter-autoimmunity" classifiers were verified in a validation cohort of 231 independent patients **(Fig 1C).**

**Example 2: Identification of "pan-disease" parameters.**

Dimensionality reduction by unsupervised cluster analysis employing UMAP (uniform manifold approximation and prediction) revealed that patients with inflammatory CNS autoimmune diseases tend to cluster together **(Fig. 2A),** suggesting that their profiles generally differ from patients with neuro-degenerative, neuro-vascular and non-inflammatory conditions **(Fig. 9).** To identify parameters defining this specific profile in an unbiased manner, machine learning (ML) and exploratory data analysis were combined, which revealed differences of parameters between distinct patient cohorts. Specifically, logistic regression identified 10 cellular and molecular parameters in the PB and CSF. The top ten distinct combinations of up to 5 of these parameters successfully differentiated neuro-inflammatory autoimmune diseases from all the other groups with a prediction accuracy (PA) of up to 0.82 and an area under the curve (AUC) of up to 0.89 **(Fig. 2B, Table 2).** Although combinations of those parameters can be used to identify and differentiate neuro-inflammatory conditions in general, further analysis revealed that from the parameters identified by ML only pleocytosis in the CSF (cells/$\mu$l CSF) and alterations in the intrathecal B-cell compartment (B cells, plasma cells, and intrathecal IgG synthesis) consistently distinguished neuro-inflammatory autoimmune diseases from all other examined neurological conditions **(Fig. 2C,** underlined). Moreover, exploratory data analysis identified diminished proportions of $CD56^{dim}$ NK cells in PB, increased proportions of lymphocytes and decreased proportions of CD3+CD56+ NKT cells and monocytes in the CSF, and an increased intrathecal IgM synthesis as further differentiating parameters **(Fig. 2C).** Among these, diminished proportions of PB $CD56^{dim}$ NK cells, increased immune-cell numbers in the CSF (cells/$\mu$l), decreased proportions of intrathecal monocytes and NKT cells, and increased proportions of intrathecal B-cells were identified as the five "pan-disease" parameters consistently altered across all investigated inflammatory CNS autoimmune diseases **(Fig. 3A, Table 2).** Alteration of the "pan-disease" blood parameter $CD56^{dim}$ NK cells was confirmed in an independent validation cohort of 115 RRMS patients, exemplary for neuro-inflammatory autoimmune diseases, in comparison to 71 healthy individuals **(Fig. 3B).** While these changes were already expected for MS and are used as a well described disease paradigm, relevance of those "pan-disease" parameters as a general feature of neuro-inflammation is novel information and hints towards their pathophysiological role in CNS autoimmunity.

Next the impact of immune-modulating therapies on the "pan-disease" parameters was investigated using MS as a paradigmatic example for neuro-inflammation **(Fig. 3C).** A shift of the "pan-disease" parameters towards non-inflammatory levels as a consequence of first line and to higher extent escalation therapies further highlighted their potential pathophysiological role. Moreover, the identified parameters differentiated distinct CNS autoimmune disease with high PA and AUC **(Fig. 3D, Table 2)** and combination of the identified parameters in a composite score facilitates use in differential diagnosis **(Fig. 3E).** Receiver operating characteristic analysis (ROC) provided a cut-off of 2.8 differentiating inflammatory from other conditions with odds ratios of 11.34 and 15.45 in the discovery and the validation cohort, respectively. Of note, age had an impact on B cells and NKT cells in the CSF under non-inflammatory conditions **(Fig. 10A)** and patients with neuro-degenerative and -vascular diseases were in average older than those with neuro-inflammatory conditions as expected **(Table 1).** Differences between these parameters still existed in a smaller age-matched cohort **(Fig. 10B).** To investigate the specificity of the identified pan-disease parameters in a broader clinical setting, untreated patients with further neurological and psychiatric disease manifestations were investigated **(Fig. 12).** Composite scores clearly differed between inflammatory and all other types of non-inflammatory disorders.

Overall, the inventors identified five cellular "pan-disease" parameters defining a common immune signature differentiating autoimmune inflammatory neurological diseases **(Fig. 3F).** It is a novel approach that these findings can be generalized across different autoimmune diseases of the CNS and thus, indicating common immunepathogenetic traits shared by those diseases and responding to effective immune-modulating drugs. Moreover, the data indicate that cellular parameters in the PB could support the diagnosis of neuro-inflammatory disorders.

**Example 3: Identification of "inter-disease" parameters defining distinct neuro-inflammatory autoimmune diseases.**

Up to this point, distinct peripheral and intrathecal cellular "pan-disease" parameters specifically altered across all neuro-inflammatory diseases have been identified **(Fig. 3F).** Next it was aimed to identify "inter-autoimmunity" parameters better differentiating RRMS as the prototype of an autoimmune disease of the CNS from its differential diagnoses NMOSD, SuS, and AIE **(Fig. 1A).** A notable example is provided by NMOSD, which in the absence of aquaporin 4 (AQP4) antibodies, can be indistinguishable from MS in its clinical presentation, despite a very distinct disease mechanism (Toledano et al. 2015, Curr Neurol Neurosci Rep 15, 57). Logistic regression identified cellular and molecular "interautoimmunity" classifiers differentiating RRMS from other neuro-inflammatory diseases with high PAs (NMOSD: 90.1, SuS: 98.1, AIE: 90.9) and AUCs (NMOSD: 92.4, SuS: 94.9, AIE: 82.6) **(Fig. 4A, Table 2).** While plasma cells in the CSF and intrathecal Ig syntheses consistently differentiated RRMS from all analyzed autoimmune CNS diseases, additional peripheral and intrathecal parameters improved differential diagnosis. Further exploratory analysis validated this result by revealing that in accordance with the literature (Cepok et al. 2001, Brain 124; Han et al. 2014, J Immunol 192; Schafflick et al. 2020 Nat Commun 11) occurrence of plasma cells in the CSF together with intrathecal IgG synthesis is an immunepathogenetic trait characteristic for RRMS **(Fig. 4B).** Strikingly, those two "interautoimmunity" classifiers were sufficient to distinguish RRMS from the other neuroinflammatory disease with a comparably high PA (NMOSD: 87.3, SuS: 95.3, AIE: 89.4) and AUC (NMOSD: 91.5, SuS: 90.7, AIE: 82.7) **(Fig. 4C, Table 2),** as compared to the additional parameters. ROC analysis of a composite score derived by the combination of intrathecal plasma cells and IgG synthesis revealed a cut-off of 1.5 differentiating RRMS from NMOSD, SuS, and AIE with odds ratios of 18.9 and 35.8 in the discovery and validation cohort, respectively **(Fig. 4D).** Taken together, the inventors identified occurrence of intrathecal plasma cells concomitant with IgG synthesis as "inter-disease" parameters defining RRMS-specific immune-pathogenetic traits **(Fig. 4E).**

**Example 4: Identification of "intra-disease" parameters distinguishing relapsing (RRMS) from primary progressive MS (PPMS).**

MS is an autoimmune disease with a heterogeneous pathophysiology. While the majority of MS patients display a relapsing-remitting disease course (RRMS), some experience accrual of disability in the absence of bouts and steady progression without relapses referred to as primary progressive (PP)MS **(Fig. 1A).** Although decreased efficacy of immune-modulatory drugs suggests that neuro-degenerative mechanisms may predominate in PPMS, continuous presence of inflammatory cells particularly in the meninges of PPMS patients is suggestive for an inflammatory component (in addition to several other arguments including identical genetic susceptibility factors). Unsupervised cluster analysis by UMAP revealed that PPMS patients cluster in-between non-pathological controls and RRMS patients **(Fig. 11A),** suggesting that PPMS patients partially share "pan-disease" parameters of neuro-inflammation, but are also characterized by "intra-disease" parameters specific for this progressive form of MS. Both ML **(Fig. 11B)** and exploratory data analysis **(Fig. 11C)** identified five parameters including intrathecal cell numbers, monocytes, occurrence of plasma cells concomitant with IgG synthesis, and $CD4^+$ T-cell activation in the PB that distinguish PPMS from RRMS patients with high PA of up to 0.90 and AUC of up to 0.91 **(Fig. 11B).** Notably, occurrence of plasma cells in the CSF of RRMS patients correlates with disease activity, further highlighting the potential pathophysiological relevance of this cell population. The identified parameters were combined in a composite score to assess whether those findings could help in a diagnostic scenario **(Fig. 11D**). ROC analysis revealed a cut-off of 0.1 differentiating RRMS from PPMS with an odds ratio of 10.4. Overall, the analysis suggests that alterations in the B-cell compartment are more specific for RRMS, whereas changes of $CD4^+$ T-cell activation and monocytes seem to be more prominent in PPMS **(Fig. 11E**). Importantly, among those parameters, plasma cells concomitant with IgG synthesis were consistently found to differentiate RRMS from both its differential diagnoses and its progressive form.

**Example 5: Identification of "intra-disease" parameters characterizing early RRMS propagation and activation.**

MS is characterized by dissemination of inflammatory demyelinating lesions in space and time. Thus, the inventors reasoned that this may be a paradigmatic example to study how immune-profiles develop during disease evolution as a trajectory of time since disease manifestation. Alterations in cellular parameters involved in differentiating inflammatory autoimmune CNS diseases were analyzed in therapy naive RIS/CIS patients, early RRMS patients (disease onset ≤ 36 months), and RRMS patients at later stages (disease onset > 36 months) **(Fig. 5A).** Strikingly, while "pan-disease" and "inter-autoimmune" parameters were present at all stages of disease propagation and therefore can add to diagnostic precision, disease-related alterations of circulating proportions of $CD56^{dim}$ NK cells as well as intrathecal B cells, plasma cells, and IgG synthesis gradually increased during disease propagation. These data suggest that disease propagation from earliest stages of clinical and/or radiological disease manifestation (RIS/CIS) is marked by gradual changes in immune-pathogenetic traits in the periphery and CSF **(Fig. 5A)** that evolve during the diseases course. Dichotomization of RRMS patients based on disease activity revealed minor effects on intrathecal proportions of lymphocytes, CD4+ T cells, CD4+ HLA-DR+ T cells and lactate levels **(Fig. 5B).** In contrast, changes in peripheral and intrathecal CD4+CD8+ T cells as well as intrathecal plasma cells were more pronounced **(Fig. 5B).** The correlation of intrathecal plasma cells with disease activity suggests that this factor drives disease propagation also via acute inflammatory processes, whereas other "pan-disease" and "inter-autoimmune" parameters might mainly affect continuous processes underlying disease pathophysiology.

**Discussion**

The present invention revealed that distinct cellular parameters in the PB and CSF improve differential diagnosis with a high prediction accuracy and provided insights into shared and distinct pathophysiological processes allowing classification of neurological diseases.

By combining feature selection with dimensionality reduction and ML, the inventors identified pan-disease parameters in the CSF (cells/μl, monocytes, NKT cells, B cells) and more strikingly in the PB ($CD56^{dim}$ NK cells) that are altered across all investigated inflammatory autoimmune diseases of the CNS and susceptible for immune-modulating therapies, while discriminating from neurodegenerative and neurovascular conditions. While these changes were already expected for MS (Kraus J, et al. 2000, Acta Neurol Scand), relevance of those pan-disease parameters as common immune-pathogenic traits of neuro-inflammation is novel information such as a yet unknown relevance of intrathecal NKT cells in AIE and a potential role of $CD56^{dim}$ NK cells in NMOSD. This suggests a pathophysiological role of these cell-subsets in CNS autoimmunity. $CD56^{dim}$ NK cells have been shown to play a crucial role in controlling activation of antigen-specific T cells in MS (Gross CC, et al. 2016, Proc Natl Acad Sci U S A) and it is therefore tempting to speculate that impaired immune-regulation by NK cells may be a general feature driving neuro-inflammation. Likewise, the present invention did not only validate several reports demonstrating disease-specific changes in the intrathecal B-cell compartment of MS patients, but also highlighted enhanced frequencies of intrathecal B cells as a common immune-pathogenic trait driving neuro-inflammation. To facilitate differential diagnosis of patients with CNS-autoimmune disease from other neurological disorders, especially in cases where MRI and neurophysiological parameters are non-pathological, the inventors constructed a composite score of the pan-disease parameters. This may be relevant to differentiate either patients with demyelinating white matter MRI lesions of unclear inflammatory (e.g. MS, NMOSD, SuS) from ischemic (i.e. stroke) origin, or patients with non-demyelinating grey matter lesions of inflammatory (i.e. AIE) from degenerative (e.g. mAD) origin. The identified pan-disease parameters discriminate neuro-inflammatory CNS diseases from other CNS conditions with a PA of 76%, i.e. from 1,000 patients with suspicion of an inflammatory CNS disease 760 patients would be correctly assigned without any further diagnostic information. Of note, the strategy ensures that this is true for each of the investigated neuro-inflammatory CNS diseases.

Differential diagnosis between distinct CNS autoimmune diseases can be hampered by specific clinical and/or MRI features and the lack of specific biomarkers. A notable example is antibody-seronegative NMOSD, which can be indistinguishable from MS in its clinical presentation, despite distinct disease mechanisms (Toledano M, Weinshenker BG, Solomon AJ. Curr Neurol Neurosci Rep. 2015;15:57). Another example is SuS, in which the classical clinical triad of encephalopathy, branch-retinal-artery-occlusions, and sensory-neural hearing loss is only seen at disease onset in 13% of cases (Dorr J, Krautwald S, Wildemann B, et al. Nat Rev Neurol. 2013;9:307-16). Misdiagnosis as MS can be detrimental, since some MS therapeutics such as Inteferon-β, dimethyl fumarate, fingolimod, natalizumab, and alemtuzumab in NMOSD (reviewed in Jarius et al. Nat Rev Dis Primers. 2020;6:85) and Interferon-β in SuS (Igahtani H, Shirah B, Amin M, Altarazi E, Almarzouki H. Neuroradiol J. 2018;31:207-212) have an adverse effect. The inventors were able to demonstrate that established parameters including occurrence of intrathecal plasma cells and IgG synthesis are sufficient to differentiate MS patients from patients suffering from NMOSD, SuS, and AIE with PAs of 92%, 91%, and 83%, respectively. In addition, the inventors identified distinct cellular parameters improving differential diagnosis with implications for personalized therapeutic decisions. For instance, differential diagnosis between SuS and MS can be improved by including HLA-DR expression on CD8 T cells in the PB and CSF as additional parameters.

Taken together the present invention demonstrates the power of multidimensional CSF analysis for classification of neurological diseases and endophenotypes within a disease spectrum (e.g. MS) with implications for personalized medicine.

**Tab. 1**

| | Non-infl. ctrl | Relapsing MS | RIS/CIS | Early RRMS | RRMS >36 months | NMOSD | Susac | AIE | Degenerative | Vascular |
|---|---|---|---|---|---|---|---|---|---|---|
| **Group size** | 74<br>35 | 196<br>22 | 26<br>1 | 125<br>21 | 45<br>0 | 15<br>0 | 14<br>0 | 57<br>10 | 93<br>156 | 97<br>8 |
| **Age [years]** | 34 (26-47)<br>39 (28-54) | 32 (25-43)<br>27 (25-51) | 32 (19-43)<br>18 | 31 (25-42)<br>28 (25-52) | 35 (29-46)<br>na | 42 (37-55)<br>na | 32 (25-40)<br>na | 50 (38-62)<br>59 (44-69) | 68 (59-72)<br>72 (63-78) | 57 (48-73)<br>63 (47-77) |
| **Sex [female]** | 62.2%<br>82.9% | 73.0%<br>72.0% | 73.1%<br>100% | 71.2%<br>70.8% | 77.8%<br>na | 73.3%<br>na | 78.6%<br>na | 63.2%<br>60.0% | 51.6%<br>55.1% | 39.2%<br>50.0% |
| **Relapse at time of sampling** | na<br>na | 74.7%<br>68.2% | 66.7%<br>100% | 87.1%<br>66.7% | 44.4%<br>na | na<br>na | na<br>na | na<br>na | na<br>na | na<br>na |
| **Months since disease onset** | na<br>na | 2 (0-37)<br>1 (0-4.5) | 1 (0-1)<br>0 | 1 (0-6)<br>1 (0-4.5) | 89 (54-118)<br>na | na<br>na | na<br>na | na<br>na | na<br>na | na<br>na |
| **EDSS** | na<br>na | 2 (1-2.5)<br>1.5 (1-2) | 1 (0-2)<br>1.5 | 2 (1-2)<br>1 (1-2) | 2.5 (1-3)<br>na | na<br>na | na<br>na | na<br>na | na<br>na | na<br>na |
| **Glucocorticoid treatment** | 0% | 0% | 0% | 0% | 0% | 40.0% | 71.4% | 17.5% | na | na |
| | 0% | 0% | 0% | 0% | na | na | na | 40.0% | na | na |
| **Leukocytes [$\mu l^{-1}$]** | 0.5 (0-1)<br>0 (0-1) | 4 (2-9)<br>3 (1-10.5) | 2.5 (1-4)<br>6 | 5 (2-9)<br>3 (1-11) | 3 (1-8)<br>na | 3 (1-12)<br>na | 1 (0-2.5)<br>na | 1 (0-2)<br>1 (0.75-2.75) | 1 (0-1)<br>1 (0-1) | 1 (0-2)<br>1 (0-2) |
| **Total protein [mg $ml^{-1}$]** | 360 (313-409)<br>335 (267-424) | 418 (344-539)<br>383 (283-578) | 418 (366-578)<br>292 | 421 (335-536)<br>387 (279-579) | 415 (355-528)<br>na | 528 (371-776)<br>na | 389 (367-480)<br>na | 419 (329-510)<br>423 (298-509) | 513 (376-641)<br>521 (404-664) | 459 (360-585)<br>667 (494-952) |
| **$Q_{Alb}$** | 4.2 (3.6-4.8)<br>4.0 (3.2-4.5) | 4.7 (3.8-6.0)<br>4.5 (2.9-7.6) | 5.1 (3.9-6.0)<br>2.8 | 4.6 (3.5-6.0)<br>4.6 (3.2-7.7) | 4.8 (4.1-5.9)<br>na | 8.1 (4.9-11.1)<br>na | 4.7 (4.5-5.5)<br>na | 4.9 (3.9-6.5)<br>5.6 (3.8-6.2) | 6.4 (4.5-8.6)<br>6.3 (4.7-8.0) | 5.8 (4.5-7.7)<br>8.8 (5.9-14.6) |
| **B/CSF-Barrier dysfunction [%]** | 0<br>0 | 22<br>32 | 27<br>0 | 22<br>33 | 20<br>na | 47<br>na | 7<br>na | 12<br>0 | 27<br>16 | 42<br>50 |
| **Quantitative intrathecal Ig synthesis (Reiber) [%]** | | | | | | | | | | |

(continued)

| | | Non-infl. ctrl | Relapsing MS | RIS/CIS | Early RRMS | RRMS >36 months | NMOSD | Susac | AIE | Degenerative | Vascular |
|---|---|---|---|---|---|---|---|---|---|---|---|
| | **IgG** | 0<br>0 | 52<br>59 | 35<br>100 | 55<br>52 | 53<br>na | 13<br>na | 0<br>na | 11<br>0 | 3<br>1 | 0<br>0 |
| | **IgA** | 0<br>0 | 6<br>5 | 4<br>0 | 7<br>5 | 4<br>na | 0<br>na | 0<br>na | 4<br>0 | 0<br>0 | 0<br>0 |
| | **IgM** | 0<br>0 | 10<br>18 | 8<br>100 | 11<br>14 | 7<br>na | 0<br>na | 0<br>na | 7<br>0% | 3<br>1 | 2<br>0 |
| **Qualitative Intrathecal IgG synthesis [%]** | | | | | | | | | | | |
| | **CSF specific (OCB type 2/3)** | 0<br>0 | 83<br>72 | 58<br>100 | 88<br>76 | 84<br>na | 20<br>na | 0<br>na | 25<br>0 | 4<br>3 | 19<br>0 |
| | **Mirrored (OCB type 4)** | 0<br>0 | 2<br>9 | 4<br>0 | 2<br>9 | 0<br>na | 7<br>na | 7<br>na | 9<br>10 | 8<br>12 | 11<br>25 |
| **$Q_{Gluc}$** | | 0.64 (0.58-0.70)<br>0.68 (0.58-0.75) | 0.65 (0.58-0.71)<br>0.63 (0.52-0.72) | 0.67 (0.60-0.79)<br>0.73 | 0.65 (0.56-0.70)<br>0.62 (0.52-0.71) | 0.66 (0.60-0.72)<br>na | 0.56 (0.47-0.71)<br>na | 0.66 (0.53-0.69)<br>na | 0.65 (0.52-0.80)<br>0.66 (0.54-0.71) | 0.60 (0.50-0.70)<br>0.64 (0.51-0.73) | 0.62 (0.52-0.72)<br>0.67 (0.46-0.76) |
| **Lactate [mmol $l^{-1}$]** | | 1.56 (1.45-1.67)<br>1.63 (1.49-1.76) | 1.69 (1.50-1.94)<br>1.83 (1.68-2.43) | 1.81 (1.48-1.95)<br>1.37 | 1.69 (1.52-1.92)<br>1.83 (1.71-2.44) | 1.63 (1.45-1.90)<br>na | 2.12 (1.68-2.63)<br>na | 1.63 (1.48-1.85)<br>na | 1.73 (1.55-2.10)<br>1.72 (1.56-2.17) | 1.79 (1.62-2.03)<br>1.78 (1.62-1.97) | 1.71 (1.56-1.91)<br>1.91 (1.51-2.31) |

**Tab. 2**

| **Figure 3: neuro-inflammatory vs other neurological diseases machine learning approach** | **PA** | **AUC** | **SE** | **SP** |
|---|---|---|---|---|
| plasma cells, B cells (CSF), $CD14^{low}CD16^{high}$ (CSF), IgG synthesis, lymphocytes (PB) | 0.8120403 | 0.8913736 | 0.7391207 | 0.8761212 |
| plasma cells, B cells (CSF), $CD14^{low}CD16^{high}$ (CSF), IgG synthesis, monocytes (PB) | 0.8158145 | 0.8899211 | 0.7328793 | 0.888697 |
| plasma cells, B cells (CSF), $CD4^{+}CD8^{+}$ (CSF), IgG synthesis, lymphocytes (PB) | 0.816121 | 0.8890992 | 0.7402414 | 0.882803 |
| plasma cells, B cells (CSF), $CD14^{low}CD16^{high}$ (CSF), $CD4^{+}CD8^{+}$ (CSF), IgG synthesis | 0.812250 | 0.8884119 | 0.7262414 | 0.8878334 |
| plasma cells, B cells (CSF), $CD14^{low}CD16^{high}$ (CSF), IgA synthesis, IgG synthesis | 0.8126048 | 0.8879781 | 0.7247586 | 0.8898031 |
| cells/µl, plasma cells, B cells (CSF), $CD14^{low}CD16^{high}$ (CSF), IgG synthesis | 0.8123145 | 0.8878859 | 0.7221207 | 0.8915758 |
| plasma cells, B cells (CSF), $CD14^{low}CD16^{high}$ (CSF), IgG synthesis | 0.8123468 | 0.8875933 | 0.7263966 | 0.8878788 |
| plasma cells, B cells (CSF), IgA synthesis, IgG synthesis, lymphocytes (PB) | 0.8154677 | 0.8874287 | 0.7346035 | 0.8865303 |
| plasma cells, B cells (CSF), $CD14^{low}CD16^{high}$ (CSF), $CD4^{+}$ HLA-$DR^{+}$ (CSF), IgG synthesis | 0.8111048 | 0.8872814 | 0.7258965 | 0.8859848 |
| B cells (CSF), $CD14^{low}CD16^{high}$ (CSF), plasma cells, IgG synthesis, lymphocytes (PB) | 0.8073307 | 0.8872628 | 0.7484828 | 0.8590454 |
| **exploratory data analysis: pan-disease parameters** | **PA** | **AUC** | **SE** | **SP** |
| cells/µl, B cells (CSF), monocytes (CSF) | 0.7618548 | 0.8537325 | 0.703362 | 0.8132576 |
| cells/µl, B cells (CSF), monocytes (CSF), $CD56^{dim}$ (PB) | 0.7610645 | 0.8518798 | 0.7013103 | 0.8135757 |
| cells/µl, B cells (CSF) | 0.7438387 | 0.8514665 | 0.6796035 | 0.8002879 |
| cells/µl, B cells (CSF), monocytes (CSF), NKT cells (CSF) | 0.7616129 | 0.8513561 | 0.7022069 | 0.8138182 |
| cells/µl, B cells (CSF), $CD56^{dim}$ (PB) | 0.749871 | 0.8495758 | 0.6832759 | 0.808394 |
| cells/µl, B cells (CSF), monocytes (CSF), NKT cells (CSF), $CD56^{dim}$ (PB) | 0.7606936 | 0.8495548 | 0.7003965 | 0.8136818 |
| cells/µl, B cells (CSF), NKT cells (CSF) | 0.7453306 | 0.8489378 | 0.6818793 | 0.8010909 |
| cells/µl, B cells (CSF), NKT cells (CSF), $CD56^{dim}$ (PB) | 0.7503064 | 0.8471988 | 0.6849655 | 0.8077273 |
| B cells (CSF), monocytes (CSF) | 0.7625484 | 0.8451346 | 0.7254138 | 0.7951818 |
| B cells (CSF), monocytes (CSF), $CD56^{dim}$ (PB) | 0.7606129 | 0.8441097 | 0.7257414 | 0.7912576 |
| **Figure 3: RRMS vs other neurological diseases** | | | | |
| **Pan-disease parameters** | **PA** | **AUC** | **SE** | **SP** |
| cells/µl, B cells (CSF), monocytes (CSF), NKT cells (CSF) | 0.840127451 | 0.919923401 | 0.756 | 0.886015152 |
| cells/µl, B cells (CSF), monocytes (CSF), NKT cells (CSF), $CD56^{dim}$ (PB) | 0.839754902 | 0.919625421 | 0.748861111 | 0.889333333 |
| cells/µl, B cells (CSF), monocytes (CSF) | 0.838264706 | 0.919297138 | 0.742305556 | 0.890606061 |
| cells/µl, B cells (CSF), monocytes (CSF), $CD56^{dim}$ (PB) | 0.839921569 | 0.918701599 | 0.738861111 | 0.895045455 |
| B cells (CSF), monocytes (CSF), NKT cells (CSF) | 0.841284314 | 0.917279882 | 0.771694444 | 0.879242424 |

(continued)

| Figure 3: RRMS vs other neurological diseases | | | | |
|---|---|---|---|---|
| **Pan-disease parameters** | **PA** | **AUC** | **SE** | **SP** |
| B cells (CSF), monocytes (CSF), NKT cells (CSF), CD56$^{dim}$ (PB) | 0.841323529 | 0.916835859 | 0.769055556 | 0.880742424 |
| B cells (CSF), monocytes (CSF) | 0.838176471 | 0.915517256 | 0.7525 | 0.884909091 |
| B cells (CSF), monocytes (CSF), CD56$^{dim}$ (PB) | 0.837519608 | 0.915011364 | 0.751666667 | 0.884348485 |
| cells/μl, B cells (CSF), NKT cells (CSF), CD56$^{dim}$ (PB) | 0.8255 | 0.912762626 | 0.711444444 | 0.887712121 |
| cells/μl, B cells (CSF), NKT cells (CSF) | 0.814911765 | 0.911657828 | 0.692361111 | 0.881757576 |
| **Figure 3: NMOSD vs other neurological diseases** | | | | |
| **Pan-disease parameters** | **PA** | **AUC** | **SE** | **SP** |
| cells/μl, NKT cells (CSF), CD56$^{dim}$ (PB) | 0.9461 | 0.693890152 | 0.1685 | 0.993227273 |
| cells/μl, monocytes (CSF), CD56$^{dim}$ (PB) | 0.945928571 | 0.711640152 | 0.1585 | 0.993651515 |
| cells/μl, monocytes (CSF), NKT cells (CSF), CD56$^{dim}$ (PB) | 0.945785714 | 0.683863636 | 0.162 | 0.993287879 |
| cells/μl, CD56$^{dim}$ (PB) | 0.945728571 | 0.736738636 | 0.1615 | 0.993257576 |
| cells/μl, monocytes (CSF) | 0.945014286 | 0.753344697 | 0.15 | 0.99319697 |
| cells/μl, monocytes (CSF), NKT cells (CSF) | 0.944928571 | 0.721920455 | 0.15525 | 0.992787879 |
| cells/μl, NKT cells (CSF) | 0.944842857 | 0.724026515 | 0.1565 | 0.992621212 |
| cells/μl | 0.944371429 | 0.767708333 | 0.14975 | 0.992530303 |
| cells/μl, B cells (CSF), NKT cells (CSF) | 0.943628571 | 0.761287879 | 0.1985 | 0.988787879 |
| cells/μl, B cells (CSF), NKT cells (CSF), CD56$^{dim}$ (PB) | 0.943185714 | 0.731238636 | 0.18825 | 0.988939394 |
| **Figure 3: SuS vs other neurological diseases** | | | | |
| **Pan-disease parameters** | **PA** | **AUC** | **SE** | **SP** |
| cells/μl, B cells (CSF), monocytes (CSF), NKT cells (CSF) | 0.9345 | 0.846609848 | 0.01875 | 0.99 |
| B cells (CSF), monocytes (CSF), NKT cells (CSF) | 0.934585714 | 0.853424242 | 0.01825 | 0.990121212 |
| cells/μl, B cells (CSF), monocytes (CSF), NKT cells (CSF), CD56$^{dim}$ (PB) | 0.935785714 | 0.83292803 | 0.04075 | 0.990030303 |
| cells/μl, B cells (CSF), monocytes (CSF), CD56$^{dim}$ (PB) | 0.936057143 | 0.83292803 | 0.0065 | 0.992393939 |
| B cells (CSF), monocytes (CSF), NKT cells (CSF), CD56$^{dim}$ (PB) | 0.936557143 | 0.839162879 | 0.0385 | 0.990984848 |
| cells/μl, monocytes (CSF), NKT cells (CSF) | 0.936757143 | 0.844787879 | 0.00125 | 0.993454545 |
| monocytes (CSF), NKT cells (CSF) | 0.937157143 | 0.851215909 | 0.001 | 0.993893939 |
| B cells (CSF), monocytes (CSF), CD56$^{dim}$ (PB) | 0.9372 | 0.838227273 | 0.00375 | 0.993772727 |
| cells/μl, B cells (CSF), monocytes (CSF) | 0.937914286 | 0.845113636 | 0.00475 | 0.994469697 |
| cells/μl, monocytes (CSF), NKT cells (CSF), CD56$^{dim}$ (PB) | 0.937928571 | 0.833056818 | 0.01825 | 0.993666667 |
| **Figure 3: AIE vs other neurological diseases** | | | | |
| **Pan-disease parameters** | **PA** | **AUC** | **SE** | **SP** |
| B cells (CSF) | 0.839283951 | 0.730926263 | 0.168266667 | 0.991787879 |
| B cells (CSF), monocytes (CSF) | 0.838790123 | 0.715340404 | 0.1666 | 0.991560606 |
| B cells (CSF), NKT cells (CSF) | 0.838691358 | 0.713768687 | 0.168066667 | 0.991106061 |
| B cells (CSF), CD56$^{dim}$ (PB) | 0.838481481 | 0.722014141 | 0.166 | 0.991318182 |

(continued)

| Figure 3: AIE vs other neurological diseases | | | | |
|---|---|---|---|---|
| **Pan-disease parameters** | **PA** | **AUC** | **SE** | **SP** |
| B cells (CSF), monocytes (CSF), NKT cells (CSF) | 0.83837037 | 0.698594949 | 0.167133333 | 0.990924242 |
| B cells (CSF), NKT cells (CSF), $CD56^{dim}$ (PB) | 0.838259259 | 0.705019192 | 0.1674 | 0.990727273 |
| B cells (CSF), monocytes (CSF), $CD56^{dim}$ (PB) | 0.837962963 | 0.707581818 | 0.164 | 0.991136364 |
| B cells (CSF), monocytes (CSF), NKT cells (CSF), $CD56^{dim}$ (PB) | 0.837703704 | 0.690966667 | 0.165133333 | 0.990560606 |
| cells/μl, B cells (CSF), NKT cells (CSF) | 0.834567901 | 0.709882828 | 0.169133333 | 0.98580303 |
| cells/μl, B cells (CSF) | 0.834444444 | 0.726192929 | 0.167933333 | 0.985924242 |
| **Figure 4: RRMS vs NMOSD** | | | | |
| **machine learning approach** | **PA** | **AUC** | **SE** | **SP** |
| IgG synthesis, IgM synthesis, lactate | 0.924975 | 0.9094722 | 0.9770833 | 0.45600 |
| IgA synthesis, IgG synthesis, lactate | 0.925075 | 0.9091389 | 0.9770833 | 0.45700 |
| IgA synthesis, IgG synthesis, IgM synthesis, lactate | 0.923275 | 0.9084375 | 0.9750556 | 0.45725 |
| IgA synthesis, IgG synthesis, lactate, $CD56^{bright}$ (PB) | 0.928075 | 0.9051945 | 0.9761667 | 0.49525 |
| plasma cells, IgG synthesis, IgM synthesis, lactate | 0.920325 | 0.9050834 | 0.9731389 | 0.44500 |
| plasma cells, IgA synthesis, IgG synthesis, lactate | 0.918150 | 0.9044861 | 0.9703611 | 0.44825 |
| plasma cells, IgA synthesis, IgG synthesis, IgM synthesis, lactate | 0.917950 | 0.9041042 | 0.9701945 | 0.44775 |
| IgG synthesis, lactate | 0.926350 | 0.9037847 | 0.9788333 | 0.45400 |
| IgG synthesis, IgM synthesis, lactate, $CD56^{bright}$ (PB) | 0.930925 | 0.9030347 | 0.9781944 | 0.50550 |
| IgA synthesis, IgG synthesis, IgM synthesis, lactate, $CD56^{bright}$ (PB) | 0.929425 | 0.9021389 | 0.9765278 | 0.50550 |
| **exploratory data analysis: inter-disease parameters** | **PA** | **AUC** | **SE** | **SP** |
| plasma cells, IgG synthesis | 0.915225 | 0.8734444 | 0.9737778 | 0.38825 |
| **Figure 4: RRMS vs SuS** | | | | |
| **machine learning approach** | **PA** | **AUC** | **SE** | **SP** |
| plasma cells, IgG synthesis, $CD4^+$ (PB), $CD8^+$ $HLA\text{-}DR^+$ (PB) | 0.946800 | 0.9819688 | 0.9763611 | 0.68075 |
| plasma cells, IgG synthesis, IgM synthesis, $CD4^+$ (PB), $CD8^+$ $HLA\text{-}DR^+$ (PB) | 0.946650 | 0.9818819 | 0.9761944 | 0.68075 |
| plasma cells, IgA synthesis, IgG synthesis, $CD4^+$ (PB), $CD8^+$ $HLA\text{-}DR^+$ (PB) | 0.946450 | 0.9818264 | 0.9759722 | 0.68075 |
| plasma cells, IgG synthesis, IgM synthesis, $CD4^+$ (PB) | 0.951800 | 0.9814028 | 0.9725278 | 0.76525 |
| plasma cells, IgG synthesis, $CD4^+$ (PB) | 0.951725 | 0.9813611 | 0.9724444 | 0.76525 |
| plasma cells, IgA synthesis, IgG synthesis, $CD4^+$ (PB) | 0.951675 | 0.9812986 | 0.9723889 | 0.76525 |
| plasma cells, IgA synthesis, IgG synthesis, IgM synthesis, $CD4^+$ (PB) | 0.951675 | 0.9812708 | 0.9723889 | 0.76525 |
| plasma cells, $CD8^+$ $HLA\text{-}DR^+$ (CSF), IgG synthesis, $CD8^+$ (PB) | 0.947850 | 0.9796493 | 0.9795833 | 0.66225 |
| plasma cells, $CD8^+$ $HLA\text{-}DR^+$ (CSF), IgG synthesis | 0.946200 | 0.979375 | 0.9736667 | 0.69900 |

(continued)

| Figure 4: RRMS vs SuS<br>**machine learning approach** | **PA** | **AUC** | **SE** | **SP** |
|---|---|---|---|---|
| plasma cells, $CD8^+$ $HLA\text{-}DR^+$ (CSF), IgG synthesis, IgM synthesis | 0.946200 | 0.9793403 | 0.9736667 | 0.69900 |
| **exploratory data analysis: inter-disease parameters** | **PA** | **AUC** | **SE** | **SP** |
| plasma cells, IgG synthesis | 0.90645 | 0.9531389 | 0.9449444 | 0.56 |
| **Figure 4: RRMS vs AIE**<br>**machine learning approach** | **PA** | **AUC** | **SE** | **SP** |
| lymphocytes (CSF), monocytes (CSF), NKT cells (CSF), IgG synthesis, lymphocytes (PB) | 0.8313333 | 0.9137889 | 0.9034445 | 0.6582667 |
| $CD14^+CD16^+$ (CSF), lymphocytes (CSF), NKT cells (CSF), IgG synthesis, lymphocytes (PB) | 0.8265294 | 0.9115704 | 0.9073889 | 0.6324667 |
| plasma cells, lymphocytes (CSF), monocytes (CSF), IgG synthesis, lymphocytes (PB) | 0.8255686 | 0.9103944 | 0.9031944 | 0.6392667 |
| lymphocytes (CSF), NKT cells (CSF), IgG synthesis, lymphocytes (PB) | 0.8310784 | 0.9088593 | 0.9139722 | 0.6321333 |
| plasma cells, lymphocytes (CSF), IgG synthesis, lymphocytes (PB) | 0.8282353 | 0.9082278 | 0.9185833 | 0.611400 |
| lymphocytes (CSF), IgG synthesis, lymphocytes (PB) | 0.8185098 | 0.9082093 | 0.9156389 | 0.585400 |
| lymphocytes (CSF), monocytes (CSF), IgG synthesis, lymphocytes (PB) | 0.8186079 | 0.9080278 | 0.9018334 | 0.6188667 |
| plasma cells, $CD14^+CD16^+$ (CSF), lymphocytes (CSF), IgG synthesis, lymphocytes (PB) | 0.8214314 | 0.9069889 | 0.9103056 | 0.6081333 |
| plasma cells, lymphocytes (CSF), NKT cells (CSF), IgG synthesis, lymphocytes (PB) | 0.8357843 | 0.9069371 | 0.9171667 | 0.6404667 |
| $CD14^+CD16^+$ (CSF), lymphocytes (CSF), IgG synthesis, lymphocytes (PB) | 0.818451 | 0.9065111 | 0.9141111 | 0.5888667 |
| **exploratory data analysis: inter-disease parameters** | **PA** | **AUC** | **SE** | **SP** |
| plasma cells, IgG synthesis | 0.8272941 | 0.8942 | 0.9117222 | 0.6246667 |

**Tab. 3**

| Retained parameters | Parameters replaced by retained parameters |
|---|---|
| Blood/CSF-barrier dysfunction | total protein<br>albumin CSF<br>albumin Serum<br>albumin ratio |
| IgG synthesis | IgG CSF<br>IgG serum |
| | IgG ratio<br>OCB type 2/3 status |
| IgA synthesis | IgA CSF<br>IgA serum<br>IgA ratio |
| IgM synthesis | IgM CSF<br>IgM serum |

(continued)

| Retained parameters | Parameters replaced by retained parameters |
|---|---|
| | IgM ratio |
| $Q_{Gluc}$ | - |
| lactate | - |
| lymphocytes/monocytes | granulocytes |
| subsets as % of lymphocytes | subsets as % of mother population (T cells, $CD4^+$ T cells, $CD8^+$ T cells, NK cells), CD4/CD8 ratio |
| $CD14^{low}CD16^{high}$ monocytes, $CD14^+CD16^+$ monocytes | $CD14^{high}CD16^-$ monocytes |
| plasma cells yes/no (>1/μl) | total plasma cells |
| plasma cells yes/no | plasma cells |
| lymphocytes/monocytes + subsets as % of lymphocytes/monocytes | total cell counts PB |
| cells/μl + lymphocytes/monocytes + subsets as % of lymphocytes/monocytes | total cell counts CSF |

Tab. 4

| | | | Non-infl. ctrl (n = 74/35) | Relapsing MS (n = 196/22) | RIS/CIS (n = 26/1) | Early RRMS (n = 125/21) | RRMS >36 months (n = 45/0) | NMOSD (n = 15/0) | Susac (n = 14/0) | AIE (n = 57/10) | Degenerative (n = 93/156) | Vascular (n = 97/8) |
|---|---|---|---|---|---|---|---|---|---|---|---|---|
| **lymphocytes** | | | | | | | | | | | | |
| PB | | % | 24.05 | 21.18 | 19.62 | 21.07 | 21.43 | 10.29 | 14.82 | 19.09 | 23.56 | 20.48 |
| | | p | | ↓** | ↓ | ↓** | ↓* | ↓** | ↓** | ↓**** | = | ↓*** |
| | | µl$^{-1}$ | 1310 | 1233 | 1270 | 1321 | 1098 | 1009 | 823 | 988 | 1201 | 1271 |
| CSF | | % | 63.00 | 86.00 | 81.01 | 87.48 | 83.31 | 82.08 | 71.16 | 56.08 | 64.09 | 60.79 |
| | | p | | ↑**** | ↑** | ↑**** | ↑*** | ↑*** | ↑ | = | = | = |
| | | µl$^{-1}$ | 0.33 | 2.27 | 1.42 | 2.78 | 2.49 | 1.75 | 0.76 | 0.36 | 0.39 | 0.45 |
| **T-cell compartment** | | | | | | | | | | | | |
| PB | CD4 | % | 43.16 | 51.14 | 53.62 | 50.82 | 51.21 | 53.99 | 42.52 | 50.90 | 48.37 | 51.24 |
| | | P | | ↑**** | ↑* | ↑**** | ↑*** | ↑* | = | ↑* | ↑* | ↑** |
| | | µl$^{-1}$ | 569 | 603 | 587 | 629 | 554 | 594 | 369 | 534 | 584 | 586 |
| | CD8 | % | 12.84 | 10.37 | 9.47 | 10.82 | 10.24 | 11.22 | 15.71 | 9.48 | 11.05 | 9.57 |
| | | p | | ↓* | ↓ | ↓ | ↓* | | ↑* | ↓* | ↓ | ↓** |
| | | µl$^{-1}$ | 147 | 126 | 119 | 134 | 123 | 79 | 128 | 101 | 135 | 99 |
| | CD4 HLA-DR* | % | 2.53 | 3.25 | 3.52 | 3.02 | 3.40 | 3.25 | 3.52 | 3.55 | 3.97 | 3.79 |
| | | p | | ↑*** | ↑** | ↑** | ↑*** | ↑* | ↑ | ↑*** | ↑**** | ↑**** |
| | | µl$^{-1}$ | 30 | 38 | 40 | 38 | 39 | 34 | 25 | 40 | 54 | 49 |
| | CD8 HLA-DR* | % | 1.04 | 1.08 | 1.17 | 1.04 | 1.17 | 1.30 | 3.40 | 1.13 | 1.91 | 1.40 |
| | | p | | | ↑ | | ↑ | ↑ | ↑*** | ↑ | ↑**** | ↑ |
| | | µl$^{-1}$ | 12 | 13 | 16 | 14 | 12 | 13 | 20 | 13 | 23 | 17 |
| CSF | **CD4** | % | 68.35 | 69.46 | 69.51 | 70.23 | 67.05 | 67.62 | 61.73 | 68.95 | 65.15 | 67.74 |
| | | p | | = | = | = | = | = | ↓* | = | = | = |
| | | µl$^{-1}$ | 0.25 | 1.59 | 1.12 | 1.88 | 1.75 | 1.14 | 0.42 | 0.25 | 0.26 | 0.29 |
| | **CD8** | % | 19.93 | 18.32 | 17.50 | 18.38 | 18.64 | 20.43 | 27.17 | 18.55 | 20.90 | 20.64 |
| | | P | | ↓* | | ↓* | | | ↑** | | | = |
| | | | 0.07 | 0.40 | 0.24 | 0.45 | 0.49 | 0.27 | 0.21 | 0.08 | 0.07 | 0.09 |
| | **CD4 HLA-DR$^{+}$** | % | 5.15 | 7.21 | 6.57 | 7.18 | 8.06 | 9.99 | 6.48 | 7.11 | 10.80 | 7.83 |
| | | P | | ↑**** | ↑* | ↑*** | ↑**** | ↑* | ↑ | ↑** | ↑**** | ↑**** |
| | | µl$^{-1}$ | 0.02 | 0.16 | 0.10 | 0.19 | 0.17 | 0.09 | 0.05 | 0.02 | 0.04 | 0.04 |

(continued)

| | | | | | | | | | | | | |
|---|---|---|---|---|---|---|---|---|---|---|---|---|
| **T-cell compartment** | | | | | | | | | | | | |
| | **CD8 HLA-DR+** | % | 5.41 | 6.50 | 6.18 | 6.29 | 7.40 | 6.71 | 12.05 | 7.17 | 8.85 | 7.05 |
| | | P | | ↑** | ↑ | ↑ | ↑** | ↑ | ↑*** | ↑↑ | ↑**** | ↑* |
| | | $\mu l^{-1}$ | 0.02 | 0.16 | 0.08 | 0.17 | 0.17 | 0.13 | 0.09 | 0.03 | 0.03 | 0.03 |
| **B-cell compartment** | | | | | | | | | | | | |
| **PB** | **B cells** | % | 11.57 | 11.36 | 12.23 | 11.45 | 10.11 | 8.76 | 10.79 | 11.02 | 9.72 | 9.60 |
| | | p | | | | | | ↓ | | | ↓ | ↓* |
| | | $\mu l^{-1}$ | 128 | 130 | 172 | 131 | 109 | 72 | 89 | 103 | 128 | 126 |
| **CSF** | **B cells** | % | 0.80 | 2.40 | 1.61 | 2.58 | 2.52 | 1.53 | 1.32 | 1.89 | 0.76 | 0.81 |
| | | p | | ↑**** | ↑*** | ↑**** | ↑**** | ↑** | ↑* | ↑**** | = | = |
| | | $\mu l^{-1}$ | 0.00 | 0.05 | 0.02 | 0.07 | 0.05 | 0.05 | 0.01 | 0.01 | 0.00 | 0.00 |
| | **plasma cells** | pos. | 0% | 73% | 38% | 81% | 73% | 33% | 0% | 28% | 8% | 7% |
| | | p | | ↑**** | ↑**** | ↑**** | ↑**** | ↑**** | = | ↑**** | ↑* | ↑* |
| **NK-cell compartment** | | | | | | | | | | | | |
| **PB** | CD56^dlm | % | 12.49 | 10.40 | 10.93 | 10.55 | 9.50 | 9.19 | 10.73 | 11.72 | 11.83 | 12.26 |
| | | p | | ↑** | ↓ | ↓** | ↓* | ↓* | ↓ | ↓ | = | = |
| | | $\mu l^{-1}$ | 152 | 124 | 115 | 134 | 98 | 76 | 93 | 120 | 156 | 125 |
| | CD56^bright | % | 0.69 | 0.66 | 0.68 | 0.67 | 0.58 | 0.52 | 0.34 | 0.68 | 0.54 | 0.59 |
| | | p | | | | | ↓ | ↓* | ↓ | | ↓** | ↓* |
| | | $\mu l^{-1}$ | 10 | 8 | 11 | 8 | 7 | 3 | 4 | 7 | 7 | 7 |
| **CSF** | **CD56^dim** | % | 0.35 | 0.29 | 0.22 | 0.29 | 0.30 | 0.24 | 0.18 | 0.32 | 0.36 | 0.28 |
| | | p | | = | ↓* | = | | ↓ | ↓ | | = | |
| | | $\mu l^{-1}$ | 0 | 0.01 | 0.00 | 0.01 | 0.01 | 0.00 | 0.00 | 0.00 | 0.00 | 0.00 |
| | **CD56^bright** | % | 1.51 | 1.30 | 1.16 | L39 | 1.25 | 0.90 | 0.70 | 1.48 | 1.59 | 1.82 |
| | | p | | = | y | = | = | ↓ | ↓** | = | = | ↑ |
| | | $\mu l^{-1}$ | 0.01 | 0.03 | 0.02 | 0.03 | 0.03 | 0.03 | 0.00 | 0.01 | 0.01 | 0.01 |
| **NKT cells** | | | | | | | | | | | | |
| **PB** | | % | 2.10 | 2.10 | 1.96 | 2.06 | 2.37 | 1.13 | 2.29 | 1.90 | 2.19 | 2.12 |
| | | p | | = | | = | = | ↓* | = | = | = | = |
| | | $\mu l^{-1}$ | 24 | 26 | 27 | 25 | 30 | 10 | 22 | 19 | 29 | 25 |

(continued)

| | | | | | | | | | | | | |
|---|---|---|---|---|---|---|---|---|---|---|---|---|
| **NKT cells** | | | | | | | | | | | | |
| **CSF** | | % | 1.38 | 0.57 | 0.91 | 0.49 | 0.67 | 0.75 | 1.21 | 1.18 | 1.72 | 1.43 |
| | | P | | ↓**** | ↓* | ↓**** | ↓** | ↓* | | ↓ | ↑* | = |
| | | µl$^{-1}$ | 0.01 | 0.02 | 0.01 | 0.02 | 0.01 | 0.01 | 0.01 | 0.01 | 0.01 | 0.01 |
| **monocytes** | | | | | | | | | | | | |
| **PB** | monocytes | % | 6.13 | 6.05 | 5.98 | 5.89 | 6.25 | 4.54 | 4.47 | 6.03 | 6.99 | 7.36 |
| | | P | | | = | = | = | ↓ | ↓* | = | ↑** | ↑*** |
| | | µl$^{-1}$ | 319 | 372 | 390 | 370 | 333 | 305 | 268 | 387 | 383 | 466 |
| | $CD14^+CD16^+$ | % | 5.98 | 7.32 | 6.53 | 7.09 | 9.10 | 7.30 | 8.86 | 9.76 | 10.63 | 11.48 |
| | | P | | ↑* | ↑ | ↑ | ↑** | ↑ | ↑ | ↑** | ↑**** | ↑**** |
| | | µl$^{-1}$ | 21 | 28 | 27 | 27 | 30 | 32 | 24 | 32 | 40 | 53 |
| | $CD14^{low}CD16^{high}$ | % | 4.11 | 4.83 | 4.37 | 4.82 | 4.92 | 2.80 | 3.60 | 4.72 | 4.51 | 6.43 |
| | | p | | ↑* | = | ↑* | ↑ | ↓ | | ↑ | = | ↑**** |
| | | µl$^{-1}$ | 12 | 17 | 15 | 18 | 17 | 12 | 11 | 17 | 18 | 27 |
| **CSF** | **monocytes** | % | 16.06 | 5.55 | 8.38 | 5.14 | 6.37 | 8.51 | 12.12 | 12.52 | 17.86 | 19.52 |
| | | p | | ↓**** | ↓** | ↓**** | ↓**** | ↓* | ↓ | ↓ | = | ↑ |
| | | µl$^{-1}$ | 0.09 | 0.16 | 0.16 | 0.17 | 0.17 | 0.20 | 0.11 | 0.14 | 0.11 | 0.18 |
| | **$CD14^+CD16^+$** | % | 48.71 | 35.77 | 37.77 | 34.92 | 36.67 | 46.21 | 35.66 | 55.00 | 46.12 | 45.61 |
| | | p | | 1 | | ↓** | ↓* | | y | = | = | |
| | | µl$^{-1}$ | 0.04 | 0.05 | 0.04 | 0.05 | 0.05 | 0.10 | 0.04 | 0.07 | 0.05 | 0.07 |
| | **$CD14^{low}CD16^{high}$** | % | 1.16 | 0.96 | 0.66 | 1.12 | 0.84 | 1.38 | 0.99 | 0.93 | 0 | 0.52 |
| | | p | | = | ↓ | | ↓ | = | = | ↓ | ↓**** | ↓ |
| | | µl$^{-1}$ | 0.00 | 0.00 | 0.00 | 0.00 | 0.00 | 0.00 | 0.00 | 0.00 | 0.00 | 0.00 |
| **Validation cohort** | | | | | | | | | | | | |
| **PB** | **$CD56^{dim}$** | % | 9.64 | 9.56 | 6.03 | 10.36 | na | na | na | 12.82 | 13.24 | 10.21 |
| | | µl$^{-1}$ | 71 | 84 | 144 | 86 | | | | 80 | 117 | 53 |
| | **B cells** | % | 0.68 | 2.87 | 4.54 | 2.82 | na | na | na | 2.06 | 0.79 | 0.69 |
| | | µl$^{-1}$ | 0.00 | 0.02 | 0.45 | 0.07 | | | | 0.01 | 0.00 | 0.00 |

(continued)

| Validation cohort | | | | | | | | | | | | |
|---|---|---|---|---|---|---|---|---|---|---|---|---|
| CSF | NKT cells | % | 1.04 | 0.64 | 0.42 | 0.64 | na | | na | 0.84 | 2.18 | 2.19 |
| | | $\mu l^{-1}$ | 0.00 | 0.01 | 0.04 | 0.01 | | na | | 0.00 | 0.01 | 0.00 |
| | monocytes | % | 16.95 | 6.46 | 2.49 | 6.86 | na | na | na | 18.20 | 26.15 | 21.61 |
| | | $\mu l^{-1}$ | 0.08 | 0.19 | 0.27 | 0.19 | | | | 0.18 | 0.17 | 0.08 |
| | plasma cells | pos. | 0% | 60% | 100% | 58% | na | na | na | 30% | 6% | 13% |

## Claims

1. A method of stratifying a subject with neurological or psychiatric disease manifestation, preferably with neuro-inflammatory autoimmune diseases, comprising

   a) determining

      i) a level of B cells in a test cerebrospinal fluid (CSF) sample obtained from a subject;
      ii) a level of immune cells per μl in said test CSF sample obtained from said subject;
      iii) a level of natural killer T (NKT) cells in said test CSF sample obtained from said subject;
      iv) a level of monocytes in said test CSF sample obtained from said subject, and
      v) a level of $CD56^{dim}$ $CD16^{+}$ natural killer (NK) cells in a test peripheral blood (PB) sample obtained from said subject, and

   b) stratifying said subject as suffering from neuro-inflammatory autoimmune diseases, if the following are fulfilled:

      i) the level of B cells is increased relative to corresponding levels of B cells in control CSF samples obtained from subjects not suffering from neuro-inflammatory autoimmune diseases;
      ii) the level of immune cells per μl is increased relative to corresponding levels of immune cells per μl in said control CSF samples obtained from said subjects not suffering from neuro-inflammatory autoimmune diseases;
      iii) the level of NKT cells is decreased relative to corresponding levels of NKT cells in said control CSF samples obtained from said subjects not suffering from neuro-inflammatory autoimmune diseases;
      iv)the level of monocytes is decreased relative to corresponding levels of monocytes in said control CSF samples obtained from said subjects not suffering from neuro-inflammatory autoimmune diseases;
      v) the level of $CD56^{dim}$ $CD16^{+}$ NK cells is decreased relative to corresponding levels of $CD56^{dim}$ $CD16^{+}$ NK cells in control PB samples obtained from said subjects not suffering from neuro-inflammatory autoimmune diseases,

   wherein the combination of said levels i) - v) of step b) is indicative for whether said subject is suspected to suffer from neuro-inflammatory autoimmune diseases or from another neurological or psychiatric disease manifestation other than neuro-inflammatory autoimmune disease.

2. The method of claim 1, wherein

   i) determining the level of B cells comprises measuring one or more of the markers selected from the group consisting of CD1c, CD5, CD14, CD19, CD20, CD21, CD27, CD24, CD38, CD40, CD45, CD80, CD138, CXCR5, HLA-DR, IgD, IgM and TACI, and/or
   ii) determining the level of NKT cells comprises measuring of one or more of the markers selected from the group consisting of CD1d, CD3, CD4, CD8, CD14, CD16, CD45, CD56, CD161 TCRγδ, Vα24-Jα18, Vβ11, Vδ1 and Vδ2, and/or
   iii) determining the level of monocytes comprises measuring of one or more of the markers selected from the group consisting of CD11b, CD11c, CD14, CD16, CD45, CD62L, CD86, CD115, CD116, CD121, CD163, CD192, CD206, $CX_3CR1$ and HLA-DR, and/or
   iv) determining the level of $CD56^{dim}$ $CD16^{+}$ NK cells comprises measuring of one or more of the markers selected from the group consisting of CD14, CD16, CD45, CD56, CD94, CD122, CD159a, CD244, CD315, CD335, CD337, KIR, Tbet and EOMES.

3. The method of any one of the preceding claims, further comprising

   i) determining whether or not intrathecal immunoglobulin (Ig) synthesis can be detected in said simultaneously obtained test CSF and serum samples obtained from said subject, and/or
   ii) determining whether or not type 2 or type 3 oligoclonal bands can be detected in said simultaneously obtained test CSF and serum samples obtained from said subject.

4. The method of any one of the preceding claims, further comprising determining a level of plasma cells in said test CSF sample obtained from said subject, preferably wherein determining the level of plasma cells comprises measuring of one or more of the markers selected from the group consisting of BCMA, CD19, CD20, CD27, CD38, CD78, CD138, CD319, CXCR4 and HLA-DR.

**5.** The method of any one of the preceding claims, further stratifying said subject as suffering from relapsing forms of multiple sclerosis (RMS), if the following are fulfilled:

i) intrathecal immunoglobulin (Ig) synthesis can be detected in said simultaneously obtained test CSF and serum samples obtained from said subject; and/or type 2 or type 3 oligoclonal bands can be detected in said simultaneously obtained test CSF and serum samples obtained from said subject; and
ii) the determined level of plasma cells in said test CSF sample is increased relative to corresponding levels of plasma cells in control CSF samples obtained from subjects suffering from another neuro-inflammatory autoimmune disease other than RMS.

**6.** The method of any one of the preceding claims, further stratifying said subject as suffering from neuromyelitis optica spectrum disorders (NMOSD), if one or more of the following are fulfilled:

i) a level of $CD56^{bright}$ $CD16^{dim/-}$ NK cells in said test PB sample is decreased relative to corresponding levels of $CD56^{bright}$ $CD16^{dim/-}$ NK cells in control PB samples obtained from subjects suffering from another neuro-inflammatory autoimmune disease other than NMOSD;
ii) a level of lactate in said test CSF sample is increased relative to corresponding levels of lactate in control CSF samples obtained from subjects suffering from another neuro-inflammatory autoimmune disease other than NMOSD,

preferably wherein determining the level of $CD56^{bright}$ $CD16^{dim/-}$ NK cells comprises measuring of one or more of the markers selected from the group consisting of CD14, CD16, CD25, CD45, CD56, CD94, CD122, CD159a, CD244, CD315, CD335, CD336, CD337, Tbet and EOMES.

**7.** The method of any one of claims 1-5, further stratifying said subject as suffering from Susac's syndrome (SuS), if one or more of the following are fulfilled:

i) a level of $CD8^+$ HLA-$DR^+$ T cells in said test PB and/or in said test CSF sample is increased relative to corresponding levels of $CD8^+$ HLA-$DR^+$ T cells in control PB and/or control CSF samples obtained from subjects suffering from another neuro-inflammatory autoimmune disease other than SuS;
ii) a level of $CD8^+$ T cells in said test PB and/or in said test CSF sample is increased relative to corresponding levels of $CD8^+$ T cells in control PB and/or control CSF samples obtained from subjects suffering from another neuro-inflammatory autoimmune disease other than SuS;
iii) a level of $CD4^+$ T cells in said test PB and/or in said test CSF sample is decreased relative to corresponding levels of $CD4^+$ T cells in control PB and/or control CSF samples obtained from subjects suffering from another neuro-inflammatory autoimmune disease other than SuS.

**8.** The method of claim 7, wherein determining the level of $CD8^+$ HLA-$DR^+$ T cells comprises measuring of one or more of the markers selected from the group consisting of CD3, CD4, CD8, CD14, CD45, CD56 and HLA-DR; and wherein determining the level of $CD8^+$ T cells comprises measuring of one or more of the markers selected from the group consisting of CD3, CD4, CD8, CD14, CD45 and CD56; and wherein determining the level of $CD4^+$ T cells comprises measuring of one or more of the markers selected from the group consisting of CD3, CD4, CD8, CD14, CD45 and CD56.

**9.** The method of any one of claims 1-5, further stratifying said subject as suffering from autoimmune encephalitis (AIE), if one or more of the following are fulfilled:

i) a level of lymphocytes in said test CSF sample is decreased relative to corresponding levels of lymphocytes in control CSF samples obtained from subjects suffering from another neuro-inflammatory autoimmune disease other than AIE;
ii) a level of NKT cells in said test CSF sample is increased relative to corresponding levels of NKT cells in control CSF samples obtained from subjects suffering from another neuro-inflammatory autoimmune disease other than AIE;
iii) a level of monocytes in said test CSF sample is increased relative to corresponding levels of monocytes in control CSF samples obtained from subjects suffering from another neuro-inflammatory autoimmune disease other than AIE.

**10.** The method of claim 9, wherein determining the level of lymphocytes comprises measuring of one or more of the markers selected from the group consisting of CD14, CD15, CD45 and CD66b; and wherein determining the level of NKT cells comprises measuring of one or more of the markers selected from the group consisting of CD1d, CD3, CD4, CD8, CD14, CD16, CD45, CD56, CD161 TCR$\gamma\delta$, V$\alpha$24-J$\alpha$18, V$\beta$11, V$\delta$1 and V$\delta$2; and wherein determining the level of monocytes comprises measuring of one or more of the markers selected from the group consisting of CD11b, CD11c, CD14, CD16, CD45, CD62L, CD86, CD115, CD116, CD121, CD163, CD192, CD206, $CX_3CR1$ and HLA-DR.

**11.** The method of any one of claims 1-5, further stratifying said subject as suffering from progressive forms of multiple sclerosis (PMS), if one or more of following are fulfilled:

i) a level of $CD4^+$ HLA-$DR^+$ T cells in said test PB sample is increased relative to corresponding levels of $CD4^+$ HLA-$DR^+$ T cells in control PB samples obtained from subjects suffering from another neuro-inflammatory autoimmune disease other than PMS;
ii) a level of monocytes in said test CSF sample is increased relative to corresponding levels of monocytes in said control CSF samples obtained from subjects suffering fromanother neuro-inflammatory autoimmune disease other than PMS.

**12.** A data processing system comprising a processor configured to perform a method comprising the steps of:

a) obtaining:

i) the detected level of B cells from a test CSF sample obtained from a subject;
ii) the detected level of immune cells per $\mu$l from said test CSF sample obtained from said subject;
iii) the detected level of NKT cells from said test CSF sample obtained from said subject;
iv) the detected level of monocytes from said test CSF sample obtained from said subject; and
v) the detected level of $CD56^{dim}$ $CD16^+$ NK cells from a test PB sample obtained from said subject;

b) stratifying said subject as suffering from neurological or psychiatric disease manifestation, preferably from neuro-inflammatory autoimmune diseases, if the following are fulfilled:

i) the detected level of B cells is increased relative to corresponding levels of B cells in control CSF samples obtained from subjects not suffering from neuro-inflammatory autoimmune diseases;
ii) the detected level of immune cells per $\mu$l is increased relative to corresponding levels of immune cells per $\mu$l in said control CSF samples obtained from said subjects not suffering from neuro-inflammatory autoimmune diseases;
iii) the detected level of NKT cells is decreased relative to corresponding levels of NKT cells in said control CSF samples obtained from said subjects not suffering from neuro-inflammatory autoimmune diseases;
iv) the detected level of monocytes is decreased relative to corresponding levels of monocytes in said control CSF samples obtained from said subjects not suffering from neuro-inflammatory autoimmune diseases; and
v) the detected level of $CD56^{dim}$ $CD16^+$ NK cells is decreased relative to corresponding levels of $CD56^{dim}$ $CD16^+$ NK cells in control PB samples obtained from said subjects not suffering from neuro-inflammatory autoimmune diseases,

wherein the combination of said levels i) - v) of step b) is indicative for whether said subject is suspected to suffer from neuro-inflammatory autoimmune diseases or from another neurological or psychiatric disease manifestation other than neuro-inflammatory autoimmune disease.

**13.** A flow cytometry device capable of detecting the following: i) the level of B cells in a test CSF sample obtained from a subject; ii) the level of immune cells per $\mu$l in said test CSF sample obtained from said subject; iii) the level of NKT cells in said test CSF sample obtained from said subject; iv) the level of monocytes in said test CSF sample obtained from said subject, and v) the level of $CD56^{dim}$ $CD16^+$ NK cells in a test PB sample obtained from said subject, comprising the data processing system of claim 12.

**14.** A computer program comprising instructions to cause the data processing system of claim 12 or the flow cytometry device of claim 13 to execute the steps of

a) obtaining:

i) the detected level of B cells from a test CSF sample obtained from a subject;
ii) the detected level of immune cells per µl from said test CSF sample obtained from said subject;
iii) the detected level of NKT cells from said test CSF sample obtained from said subject;
iv) the detected level of monocytes from said test CSF sample obtained from said subject; and
v) the detected level of $CD56^{dim}$ $CD16^{+}$ NK cells from a test PB sample obtained from said subject;

b) stratifying said subject as suffering from neurological or psychiatric disease manifestation, preferably from neuro-inflammatory autoimmune diseases, if the following are fulfilled:

i) the detected level of B cells is increased relative to corresponding levels of B cells in control CSF samples obtained from subjects not suffering from neuro-inflammatory autoimmune diseases;
ii) the detected level of immune cells per µl is increased relative to corresponding levels of immune cells per µl in said control CSF samples obtained from said subjects not suffering from neuro-inflammatory autoimmune diseases;
iii) the detected level of NKT cells is decreased relative to corresponding levels of NKT cells in said control CSF samples obtained from said subjects not suffering from neuro-inflammatory autoimmune diseases;
iv) the detected level of monocytes is decreased relative to corresponding levels of monocytes in said control CSF samples obtained from said subjects not suffering from neuro-inflammatory autoimmune diseases; and
v) the detected level of $CD56^{dim}$ $CD16^{+}$ NK cells is decreased relative to corresponding levels of $CD56^{dim}$ $CD16^{+}$ NK cells in control PB samples obtained from said subjects not suffering from neuro-inflammatory autoimmune diseases,

wherein the combination of said detected levels i) - v) of step b) is indicative for whether said subject is suspected to suffer from neuro-inflammatory autoimmune diseases or from another neurological or psychiatric disease manifestation other than neuro-inflammatory autoimmune disease.

**15.** A kit comprising a fluorescently labeled binding partner for CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES and HLA-DR.

## Patentansprüche

**1.** Verfahren zum Stratifizieren eines Subjekts mit neurologischer oder psychiatrischer Krankheitsmanifestation, vorzugsweise mit neuro-inflammatorischen Autoimmunerkrankungen, umfassend

a) Bestimmen

i) einer Menge an B-Zellen in einer Test-Zerebrospinalflüssigkeits- (CSF) Probe erhalten von einem Subjekt;
ii) einer Menge an Immunzellen pro µl in der Test-CSF Probe erhalten von dem Subjekt;
iii) einer Menge an natürlichen Killer-T (NKT) Zellen in der Test-CSF Probe erhalten von dem Subjekt;
iv) einer Menge an Monozyten in der Test-CSF Probe erhalten von dem Subjekt; und
v) einer Menge an $CD56^{dim}$ $CD16^{+}$ natürlichen Killer (NK) Zellen in einer Testprobe des peripheren Blutes (PB) erhalten von dem Subjekt; und

b) Stratifizieren des Subjekts als an neuro-inflammatorischen Autoimmunerkrankungen leidend, falls die folgenden Bedingungen erfüllt sind:

i) die Menge an B-Zellen ist im Verhältnis zu den entsprechenden Mengen an B-Zellen in Kontroll-CSF Proben erhalten von Subjekten, die nicht an neuro-inflammatorischen Autoimmunerkrankungen leiden, erhöht;
ii) die Menge an Immunzellen pro µl ist im Verhältnis zu den entsprechenden Mengen an Immunzellen pro µl in den Kontroll-CSF Proben erhalten von den Subjekten, die nicht an neuro-inflammatorischen Autoimmunerkrankungen leiden, erhöht;
iii) die Menge an NKT-Zellen ist im Verhältnis zu den entsprechenden Mengen an NKT-Zellen in den Kontroll-CSF Proben erhalten von den Subjekten, die nicht an neuro-inflammatorischen Autoimmunerkrankungen leiden, verringert;
iv) die Menge an Monozyten ist im Verhältnis zu den entsprechenden Mengen an Monozyten in den Kontroll-CSF Proben erhalten von den Subjekten, die nicht an neuro-inflammatorischen Autoimmunerkrankungen leiden, verringert;
v) die Menge an $CD56^{dim}$ $CD16^{+}$ NK-Zellen ist im Verhältnis zu den entsprechenden Mengen an $CD56^{dim}$ $CD16^{+}$ NK-Zellen in Kontroll-PB Proben erhalten von den Subjekten, die nicht an neuro-inflammatorischen Autoimmunerkrankungen leiden, verringert,

wobei die Kombination der Mengen i) - v) des Schritts b) darauf hindeuten, ob das Subjekt im Verdacht steht, an neuro-inflammatorischen Autoimmunerkrankungen oder an einer anderen neurologischen oder psychiatrischen Krankheitsmanifestation als einer neuro-inflammatorischen Autoimmunerkrankung zu leiden.

**2.** Verfahren nach Anspruch 1, wobei

i) Bestimmen der Menge an B-Zellen das Messen eines oder mehrerer der Marker ausgewählt aus der Gruppe bestehend aus CD1c, CD5, CD14, CD19, CD20, CD21, CD27, CD24, CD38, CD40, CD45, CD80, CD138, CXCR5, HLA-DR, IgD, IgM und TACI umfasst, und/oder
ii) Bestimmen der Menge an NKT-Zellen das Messen eines oder mehrerer der Marker ausgewählt aus der Gruppe bestehend aus CD1d, CD3, CD4, CD8, CD14, CD16, CD45, CD56, CD161 TCRγδ, Vα24-Jα18, Vβ11, Vδ1 und Vδ2 umfasst, und/oder
iii) Bestimmen der Menge an Monozyten das Messen eines oder mehrerer der Marker ausgewählt aus der Gruppe bestehend aus CD11b, CD11c, CD14, CD16, CD45, CD62L, CD86, CD115, CD116, CD121, CD163, CD192, CD206, CX3CR1 und HLA-DR umfasst, und/oder
iv) Bestimmen der Menge an CD56dim $CD16^{+}$ NK-Zellen das Messen eines oder mehrerer der Marker ausgewählt aus der Gruppe bestehend aus CD14, CD16, CD45, CD56, CD94, CD122, CD159a, CD244, CD315, CD335, CD337, KIR, Tbet und EOMES umfasst.

**3.** Verfahren nach einem der vorhergehenden Ansprüche, ferner umfassend

i) Bestimmen, ob oder ob nicht intrathekale Immunglobulin (Ig) Synthese in den gleichzeitig erhaltenen Test-CSF und Serumproben erhalten von dem Subjekt nachgewiesen werden kann, und/oder
ii) Bestimmen, ob oder ob nicht Typ 2 oder Type 3 oligoklonale Banden in den gleichzeitig erhaltenen Test-CSF und Serumproben erhalten von dem Subjekt nachgewiesen werden können.

**4.** Verfahren nach einem der vorhergehenden Ansprüche, ferner umfassend das Bestimmen einer Menge an Plasmazellen in der Test-CSF Probe erhalten von dem Subjekt, vorzugsweise, wobei das Bestimmen der Menge an Plasmazellen das Messen eines oder mehrerer der Marker ausgewählt aus der Gruppe bestehend aus BCMA, CD19, CD20, CD27, CD38, CD78, CD138, CD319, CXCR4 und HLA-DR umfasst.

**5.** Verfahren nach einem der vorhergehenden Ansprüche, ferner Stratifizieren des Subjekts als an rezidivierenden Formen von Multipler Sklerose (RMS) leidend, falls die folgenden Bedingungen erfüllt sind:

i) intrathekale Immunglobulin (Ig) Synthese kann in den gleichzeitig erhaltenen Test-CSF und Serumproben erhalten von dem Subjekt nachgewiesen werden; und/oder Typ 2 oder Typ 3 oligoklonale Banden können in den gleichzeitig erhaltenen Test-CSF und Serumproben erhalten von dem Subjekt nachgewiesen werden; und
ii) die bestimmte Menge an Plasmazellen ist in der Test-CSF Probe im Verhältnis zu den entsprechenden Mengen an Plasmazellen in Kontroll-CSF Proben erhalten von Subjekten, die an einer anderen neuro-inflammatorischen Autoimmunerkrankung als RMS leiden, erhöht.

**6.** Verfahren nach einem der vorhergehenden Ansprüche, ferner Stratifizieren des Subjekts als an Neuromyelitis-optica-Spektrum-Störungen (NMOSD) leidend, falls die folgenden Bedingungen erfüllt sind:

i) eine Menge an $CD56^{bright}$ $CD16^{dim/-}$ NK-Zellen ist in der Test-PB Probe im Verhältnis zu den entsprechenden Mengen an $CD56^{bright}$ $CD16^{dim/-}$ NK-Zellen in Kontroll-PB Proben erhalten von Subjekten, die an einer anderen neuro-inflammatorischen Autoimmunerkrankung als NMOSD leiden, verringert;
ii) eine Menge an Laktat ist in der Test-CSF Probe im Verhältnis zu den entsprechenden Mengen an Laktat in Kontroll-CSF Proben erhalten von Subjekten, die an einer anderen neuro-inflammatorischen Autoimmunerkrankung als NMOSD leiden, erhöht,

vorzugsweise wobei das Bestimmen der Menge an $CD56^{bright}$ $CD16^{dim/-}$ NK-Zellen das Messen eines oder mehrerer der Marker ausgewählt aus der Gruppe bestehend aus CD14, CD16, CD25, CD45, CD56, CD94, CD122, CD159a, CD244, CD315, CD335, CD336, CD337, Tbet und EOMES umfasst.

**7.** Verfahren nach einem der Ansprüche 1 bis 5, ferner Stratifizieren des Subjekts als an Susac-Syndrom (SuS) leidend, falls die folgenden Bedingungen erfüllt sind:

i) eine Menge an $CD8^+$ HLA-$DR^+$ T-Zellen ist in der Test-PB und/oder in der Test-CSF Probe im Verhältnis zu den entsprechenden Mengen an $CD8^+$ HLA-$DR^+$ T-Zellen in Kontroll-PB und/oder Kontroll-CSF Proben erhalten von Subjekten, die an einer anderen neuro-inflammatorischen Autoimmunerkrankung als SuS leiden, erhöht;
ii) eine Menge an $CD8^+$ T-Zellen ist in der Test-PB und/oder in der Test-CSF Probe im Verhältnis zu den entsprechenden Mengen an $CD8^+$ T-Zellen in Kontroll-PB und/oder Kontroll-CSF Proben erhalten von Subjekten, die an einer anderen neuro-inflammatorischen Autoimmunerkrankung als SuS leiden, erhöht;
iii) eine Menge an $CD4^+$ T-Zellen ist in dem Test-PB und/oder in der Test-CSF Probe im Verhältnis zu den entsprechenden Mengen an $CD4^+$ T-Zellen in Kontroll-PB und/oder Kontroll-CSF Proben erhalten von Subjekten, die an einer anderen neuro-inflammatorischen Autoimmunerkrankung als SuS leiden, verringert.

**8.** Verfahren nach Anspruch 7, wobei das Bestimmen der Menge an $CD8^+$ HLA-$DR^+$ T-Zellen das Messen eines oder mehrerer der Marker ausgewählt aus der Gruppe bestehend aus CD3, CD4, CD8, CD14, CD45, CD56 und HLA-DR umfasst; und wobei das Bestimmen der Menge an $CD8^+$ T-Zellen das Messen eines oder mehrerer der Marker ausgewählt aus der Gruppe bestehend aus CD3, CD4, CD8, CD14, CD45 und CD56 umfasst; und wobei das Bestimmen der Menge an $CD4^+$ T-Zellen das Messen eines oder mehrerer der Marker ausgewählt aus der Gruppe bestehend aus CD3, CD4, CD8, CD14, CD45 und CD56 umfasst.

**9.** Verfahren nach einem der Ansprüche 1 bis 5, ferner Stratifizieren des Subjekts als an Autoimmunenzephalitis (AIE) leidend, falls die folgenden Bedingungen erfüllt sind:

i) eine Menge an Lymphozyten ist in der Test-CSF Probe im Verhältnis zu den entsprechenden Mengen an Lymphozyten in Kontroll-CSF Proben erhalten von Subjekten, die an einer anderen neuro-inflammatorischen Autoimmunerkrankung als AIE leiden, verringert;
ii) eine Menge an NKT-Zellen ist in der Test-CSF Probe im Verhältnis zu den entsprechenden Mengen an NKT-Zellen in Kontroll-CSF Proben erhalten von Subjekten, die an einer anderen neuro-inflammatorischen Autoimmunerkrankung als AIE leiden, erhöht;
iii) eine Menge an Monozyten ist in der Test-CSF Probe im Verhältnis zu den entsprechenden Mengen an Monozyten in Kontroll-CSF Proben erhalten von Subjekten, die an einer anderen neuro-inflammatorischen Autoimmunerkrankung als AIE leiden, erhöht.

**10.** Verfahren nach Anspruch 9, wobei das Bestimmen der Menge an Lymphozyten das Messen eines oder mehrerer der Marker ausgewählt aus der Gruppe bestehend aus CD14, CD15, CD45 und CD66b umfasst, und wobei das Bestimmen der Menge an NKT-Zellen das Messen eines oder mehrerer der Marker ausgewählt aus der Gruppe bestehend aus CD1d, CD3, CD4, CD8, CD14, CD16, CD45, CD56, CD161, TCR$\gamma\delta$, V$\alpha$24-J$\alpha$18, V$\beta$11, V$\delta$1 und V$\delta$2 umfasst; und wobei das Bestimmen der Menge an Monozyten das Messen eines oder mehrerer der Marker ausgewählten aus der Gruppe bestehend aus CD11b, CD11c, CD14, CD16, CD45, CD62L, CD86, CD115, CD116, CD121, CD163, CD192, CD206, $CX_3CR1$ und HLA-DR umfasst.

**11.** Verfahren nach einem der Ansprüche 1 bis 5, ferner Stratifizieren des Subjekts als an progressiven Formen von Multipler Sklerose (PMS) leidend, falls die folgenden Bedingungen erfüllt sind:

i) eine Menge an $CD4^+$ HLA-$DR^+$ T-Zellen ist in der Test-PB Probe im Verhältnis zu den entsprechenden Mengen an $CD4^+$ HLA-$DR^+$ T-Zellen in Kontroll-PB Proben erhalten von Subjekten, die an einer anderen neuro-inflammatorischen Autoimmunerkrankung als PMS leiden, erhöht;
ii) eine Menge an Monozyten ist in der Test-CSF Probe im Verhältnis zu den entsprechenden Mengen an Monozyten in den Kontroll-CSF Proben erhalten von Subjekten, die aneineranderen neuro-inflammatorischen Autoimmunerkrankung als PMS leiden, erhöht.

**12.** Datenverarbeitungssystem umfassend einen Prozessor konfiguriert um ein Verfahren die folgenden Schritte umfassend auszuführen:

a) Erhalten von:

i) der nachgewiesenen Menge an B-Zellen aus einer Test-CSF Probe erhalten von einem Subjekt;
ii) der nachgewiesenen Menge an Immunzellen pro μl aus der Test-CSF Probe erhalten von dem Subjekt;
iii) der nachgewiesenen Menge an NKT-Zellen aus der Test-CSF Probe erhalten von dem Subjekt;
iv) der nachgewiesenen Menge an Monozyten aus der Test-CSF Probe erhalten von dem Subjekt; und
v) der nachgewiesenen Menge an $CD56^{dim}$ $CD16^{+}$ NK-Zellen aus einer Test-PB Probe erhalten von dem Subjekt.

b) Stratifizieren des Subjekts als an neurologischer oder psychiatrischer Krankheitsmanifestation leidend, vorzugsweise an neuro-inflammatorischen Autoimmunerkrankungen, falls die folgenden Bedingungen erfüllt sind:

i) die nachgewiesene Menge an B-Zellen ist im Verhältnis zu den entsprechenden Mengen an B-Zellen in Kontroll-CSF Proben erhalten von Subjekten, die nicht an neuro-inflammatorischen Autoimmunerkrankungen leiden, erhöht;
ii) die nachgewiesene Menge an Immunzellen pro μl ist im Verhältnis zu den entsprechenden Mengen an Immunzellen pro μl in den Kontroll-CSF Proben erhalten von den Subjekten, die nicht an neuro-inflammatorischen Autoimmunerkrankungen leiden, erhöht;
iii) die nachgewiesene Menge an NKT-Zellen ist im Verhältnis zu den entsprechenden Mengen an NKT-Zellen in den Kontroll-CSF Proben erhalten von den Subjekten, die nicht an neuro-inflammatorischen Autoimmunerkrankungen leiden, verringert;
iv) die nachgewiesene Menge an Monozyten ist im Verhältnis zu den entsprechenden Mengen an Monozyten in den Kontroll-CSF Proben erhalten von den Subjekten, die nicht an neuro-inflammatorischen Autoimmunerkrankungen leiden, verringert;
v) die nachgewiesene Menge an $CD56^{dim}$ $CD16^{+}$ NK-Zellen ist im Verhältnis zu den entsprechenden Mengen an $CD56^{dim}$ $CD16^{+}$ NK-Zellen in Kontroll-PB Proben erhalten von den Subjekten, die nicht an neuro-inflammatorischen Autoimmunerkrankungen leiden, verringert,

wobei die Kombination der Mengen i) - v) des Schritts b) darauf hindeuten, ob das Subjekt im Verdacht steht, an neuro-inflammatorischen Autoimmunerkrankungen oder an einer anderen neurologischen oder psychiatrischen Krankheitsmanifestation als einer neuro-inflammatorischen Autoimmunerkrankung zu leiden.

**13.** Durchflusszytometrie-Gerät, das in der Lage ist, das Folgende nachzuweisen: i) die Menge an B-Zellen in einer Test-CSF Probe erhalten von einem Subjekt; ii) die Menge an Immunzellen pro μl in der Test-CSF Probe erhalten von dem Subjekt; iii) die Menge an NKT Zellen in der Test-CSF Probe erhalten von dem Subjekt; iv) die Menge an Monozyten in der Test-CSF Probe erhalten von dem Subjekt; und v) die Menge an $CD56^{dim}$ $CD16^{+}$ NK in einer Test-PB Probe erhalten von dem Subjekt, umfassend das Datenverarbeitungssystem nach Anspruch 12.

**14.** Computerprogramm umfassend Anweisungen, um das Datenverarbeitungssystem nach Anspruch 12 oder das Durchflusszytometrie-Gerät nach Anspruch 13 zu veranlassen, die folgenden Schritte auszuführen:

a) Erhalten von:

i) der nachgewiesenen Menge an B-Zellen aus einer Test-CSF Probe erhalten von einem Subjekt;
ii) der nachgewiesenen Menge an Immunzellen pro μl aus der Test-CSF Probe erhalten von dem Subjekt;
iii) der nachgewiesenen Menge an NKT-Zellen aus der Test-CSF Probe erhalten von dem Subjekt;
iv) der nachgewiesenen Menge an Monozyten aus der Test-CSF Probe erhalten von dem Subjekt; und
v) der nachgewiesenen Menge an $CD56^{dim}$ $CD16^{+}$ NK-Zellen aus einer Test-PB Probe erhalten von dem Subjekt.

b) Stratifizieren des Subjekts als an neurologischer oder psychiatrischer Krankheitsmanifestation leidend, vorzugsweise an neuro-inflammatorischen Autoimmunerkrankungen, falls die folgenden Bedingungen erfüllt sind:

i) die nachgewiesene Menge an B-Zellen ist im Verhältnis zu den entsprechenden Mengen an B-Zellen in Kontroll-CSF Proben erhalten von Subjekten, die nicht an neuro-inflammatorischen Autoimmunerkrankungen leiden, erhöht;

ii) die nachgewiesene Menge an Immunzellen pro µl ist im Verhältnis zu den entsprechenden Mengen an Immunzellen pro µl in den Kontroll-CSF Proben erhalten von den Subjekten, die nicht an neuro-inflammatorischen Autoimmunerkrankungen leiden, erhöht;
iii) die nachgewiesene Menge an NKT-Zellen ist im Verhältnis zu den entsprechenden Mengen an NKT-Zellen in den Kontroll-CSF Proben erhalten von den Subjekten, die nicht an neuro-inflammatorischen Autoimmunerkrankungen leiden, verringert;
iv) die nachgewiesene Menge an Monozyten ist im Verhältnis zu den entsprechenden Mengen an Monozyten in den Kontroll-CSF Proben erhalten von den Subjekten, die nicht an neuro-inflammatorischen Autoimmunerkrankungen leiden, verringert;
v) die nachgewiesene Menge an $CD56^{dim}$ $CD16^{+}$ NK-Zellen ist im Verhältnis zu den entsprechenden Mengen an $CD56^{dim}$ $CD16^{+}$ NK-Zellen in Kontroll-PB Proben erhalten von den Subjekten, die nicht an neuro-inflammatorischen Autoimmunerkrankungen leiden, verringert,

wobei die Kombination der nachgewiesenen Mengen i) - v) des Schritts b) darauf hindeuten, ob das Subjekt im Verdacht steht, an neuro-inflammatorischen Autoimmunerkrankungen oder an einer anderen neurologischen oder psychiatrischen Krankheitsmanifestation als einer neuro-inflammatorischen Autoimmunerkrankung zu leiden.

**15.** Kit umfassend einen fluoreszenzmarkierten Bindungspartner für CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES und HLA-DR.

## Revendications

**1.** Méthode de stratification d'un sujet présentant des manifestations de maladies neurologiques ou psychiatriques, de préférence des maladies auto-immunes neuro-inflammatoires, consistant à

a) déterminer

i) un niveau de cellules B dans un échantillon de liquide céphalorachidien (LCR) prélevé sur un sujet;
ii) un niveau de cellules immunitaires par µl dans l'échantillon de LCR prélevé sur le sujet;
iii) un niveau de cellules T tueuses naturelles (NKT) dans ledit échantillon de LCR prélevé sur le sujet;
iv) un niveau de monocytes dans ledit échantillon de LCR obtenu dudit sujet, et
v) un niveau de cellules tueuses naturelles (NK) $CD56^{dim}$ $CD16^{+}$ dans un échantillon de sang périphérique (PB) prélevé sur ledit sujet, et

b) stratifier ledit sujet comme souffrant de maladies auto-immunes neuro-inflammatoires, si les conditions suivantes sont remplies:

i) le niveau des cellules B est augmenté par rapport aux niveaux correspondants de cellules B dans des échantillons de LCR de contrôle obtenus à partir de sujets ne souffrant pas de maladies auto-immunes neuro-inflammatoires;
ii) le niveau de cellules immunitaires par µl est augmenté par rapport aux niveaux correspondants de cellules immunitaires par µl dans lesdits échantillons de LCR de contrôle obtenus à partir desdits sujets ne souffrant pas de maladies neuro-inflammatoires auto-immunes;
iii) le niveau des cellules NKT est réduit par rapport aux niveaux correspondants de cellules NKT dans lesdits échantillons de LCR de contrôle obtenus à partir desdits sujets ne souffrant pas de maladies auto-immunes neuro-inflammatoires;
iv) le niveau de monocytes est réduit par rapport aux niveaux correspondants de monocytes dans lesdits échantillons de LCR de contrôle obtenus à partir desdits sujets ne souffrant pas de maladies auto-immunes neuro-inflammatoires,
v) le niveau des cellules NK $CD56^{dim}$ $CD16^{+}$ est diminué par rapport aux niveaux correspondants de cellules NK $CD56^{dim}$ $CD16^{+}$ dans les échantillons de PB de contrôle obtenus à partir desdits sujets ne souffrant pas de maladies auto-immunes neuro-inflammatoires,

la combinaison des niveaux i) à v) de l'étape b) indique si le sujet est suspecté de souffrir d'une maladie auto-immune neuro-inflammatoire ou d'une autre maladie neurologique ou psychiatrique autre qu'une maladie auto-immune neuro-inflammatoire.

**2.** Méthode de la revendication 1, dans laquelle

i) la détermination du niveau des cellules B comprend la mesure d'un ou de plusieurs marqueurs choisis dans le groupe consistant en CD1c, CD5, CD14, CD19, CD20, CD21, CD27, CD24, CD38, CD40, CD45, CD80, CD138, CXCR5, HLA-DR, IgD, IgM et TACI, et/ou
ii) la détermination du niveau des cellules NKT comprend la mesure d'un ou de plusieurs marqueurs choisis dans le groupe consistant en CD1d, CD3, CD4, CD8, CD14, CD16, CD45, CD56, CD161 TCRγδ, Vα24-Jα18, Vβ11, Vδ1 et Vδ2, et/ou
iii) la détermination du niveau de monocytes comprend la mesure d'un ou de plusieurs marqueurs choisis dans le groupe consistant en CD11b, CD11c, CD14, CD16, CD45, CD62L, CD86, CD115, CD116, CD121, CD163, CD192, CD206, $CX_3CR1$ et HLA-DR, et/ou
iv) la détermination du niveau de cellules NK $CD56^{dim}$ CD16+ comprend la mesure d'un ou de plusieurs marqueurs choisis dans le groupe consistant en CD14, CD16, CD45, CD56, CD94, CD122, CD159a, CD244, CD315, CD335, CD337, KIR, Tbet et EOMES.

**3.** Méthode de l'une quelconque des revendications précédentes, comprenant en outre

i) déterminer si la synthèse d'immunoglobulines (Ig) intrathécales peut être détectée dans les échantillons de LCR et de sérum obtenus simultanément à partir dudit sujet, et/ou
ii) déterminer si des bandes oligoclonales de type 2 ou de type 3 peuvent être détectées dans les échantillons de LCR et de sérum obtenus simultanément.

**4.** Méthode de l'une quelconque des revendications précédentes, comprenant en outre la détermination d'un niveau de cellules plasmatiques dans ledit échantillon de LCR obtenu dudit sujet, de préférence dans laquelle la détermination du niveau de cellules plasmatiques comprend la mesure d'un ou de plusieurs marqueurs choisis dans le groupe constitué de BCMA, CD19, CD20, CD27, CD38, CD78, CD138, CD319, CXCR4 et HLA-DR.

**5.** Méthode de l'une quelconque des revendications précédentes, stratifiant en outre ledit sujet comme souffrant de formes rémittentes de sclérose en plaques (RMS), si les conditions suivantes sont remplies:

i) une synthèse intrathécale d'immunoglobulines (Ig) peut être détectée dans les échantillons de LCR et de sérum obtenus simultanément chez ce sujet; et/ou des bandes oligoclonales de type 2 ou 3 peuvent être détectées dans les échantillons de LCR et de sérum obtenus simultanément chez ce sujet; et
ii) le niveau déterminé de plasmocytes dans ledit échantillon de LCR testé est augmenté par rapport aux niveaux correspondants de plasmocytes dans les échantillons de LCR de contrôle obtenus à partir de sujets souffrant d'une autre maladie auto-immune neuro-inflammatoire autre que le RMS.

**6.** Méthode de l'une des revendications précédentes, stratifiant en outre ledit sujet comme souffrant de troubles du spectre de la neuromyélite optique (NMOSD), si l'une ou plusieurs des conditions suivantes sont remplies:

i) un niveau de cellules NK $CD56^{bright}$ $CD16^{dim/-}$ dans ledit échantillon de PB test est diminué par rapport aux niveaux correspondants de cellules NK $CD56^{bright}$ $CD16^{dim/-}$ dans les échantillons de PB de contrôle obtenus à partir de sujets souffrant d'une autre maladie auto-immune neuro-inflammatoire autre que la NMOSD;
ii) un niveau de lactate dans ledit échantillon de LCR testé est augmenté par rapport aux niveaux correspondants de lactate dans les échantillons de LCR de contrôle obtenus à partir de sujets souffrant d'une autre maladie auto-immune neuro-inflammatoire autre que la NMOSD,

de préférence, la détermination du niveau de cellules NK $CD56^{bright}$ $CD16^{dim/-}$comprend la mesure d'un ou de plusieurs marqueurs choisis dans le groupe constitué de CD14, CD16, CD25, CD45, CD56, CD94, CD122, CD159a, CD244, CD315, CD335, CD336, CD337, Tbet et EOMES.

**7.** Méthode de l'une des revendications 1 à 5, stratifiant en outre ledit sujet comme souffrant du syndrome de Susac (SuS), si l'un ou plusieurs des critères suivants sont remplis:

i) un niveau de cellules T $CD8^+$ HLA-$DR^+$ dans ladite PB de test et/ou dans ledit échantillon de LCR de test est augmenté par rapport aux niveaux correspondants de cellules T $CD8^+$ HLA-$DR^+$ dans la PB de contrôle et/ou les échantillons de LCR de contrôle obtenus à partir de sujets souffrant d'une autre maladie auto-immune neuro-inflammatoire autre que la maladie de SuS;
ii) un niveau de cellules T $CD8^+$ dans ladite PB de test et/ou dans ledit échantillon de LCR de test est augmenté par rapport aux niveaux correspondants de cellules T $CD8^+$ dans la PB de contrôle et/ou les échantillons de LCR de contrôle obtenus à partir de sujets souffrant d'une autre maladie auto-immune neuro-inflammatoire autre que la maladie de SuS;
iii) un niveau de cellules T $CD4^+$ dans ladite PB de test et/ou dans ledit échantillon de LCR de test est diminué par rapport aux niveaux correspondants de cellules T $CD4^+$ dans la PB de contrôle et/ou les échantillons de LCR de contrôle obtenus à partir de sujets souffrant d'une autre maladie auto-immune neuro-inflammatoire autre que la maladie de SuS.

**8.** Méthode de la revendication 7, dans laquelle la détermination du niveau de cellules T $CD8^+$ HLA-$DR^+$ comprend la mesure d'un ou de plusieurs marqueurs choisis dans le groupe constitué de CD3, CD4, CD8, CD14, CD45, CD56 et HLA-DR; et dans lequel la détermination du niveau de cellules T $CD8^+$ comprend la mesure d'un ou de plusieurs marqueurs choisis dans le groupe consistant en CD3, CD4, CD8, CD14, CD45 et CD56; et dans lequel la détermination du niveau de cellules T $CD4^+$ comprend la mesure d'un ou de plusieurs marqueurs choisis dans le groupe consistant en CD3, CD4, CD8, CD14, CD45 et CD56.

**9.** Méthode de l'une des revendications 1 à 5, stratifiant en outre ledit sujet comme souffrant d'encéphalite auto-immune (AIE), si l'une ou plusieurs des conditions suivantes sont remplies:

i) un niveau de lymphocytes dans ledit échantillon de LCR testé est diminué par rapport aux niveaux correspondants de lymphocytes dans des échantillons de LCR de contrôle obtenus à partir de sujets souffrant d'une autre maladie auto-immune neuro-inflammatoire autre que l'AIE;
ii) un niveau de cellules NKT dans ledit échantillon de LCR testé est augmenté par rapport aux niveaux correspondants de cellules NKT dans des échantillons de LCR de contrôle obtenus à partir de sujets souffrant d'une autre maladie auto-immune neuro-inflammatoire autre que l'AIE;
iii) un niveau de monocytes dans ledit échantillon de LCR testé est augmenté par rapport aux niveaux correspondants de monocytes dans les échantillons de LCR de contrôle obtenus à partir de sujets souffrant d'une autre maladie auto-immune neuro-inflammatoire autre que l'AIE.

**10.** Méthode de la revendication 9, dans laquelle la détermination du niveau de lymphocytes comprend la mesure d'un ou de plusieurs marqueurs choisis dans le groupe consistant en CD14, CD15, CD45 et CD66b; et dans lequel la détermination du niveau des cellules NKT comprend la mesure d'un ou de plusieurs marqueurs choisis dans le groupe constitué de CD1d, CD3, CD4, CD8, CD14, CD16, CD45, CD56, CD161 TCR$\gamma\delta$, V$\alpha$24-J$\alpha$18, V$\beta$11, V$\delta$1 et V$\delta$2; et dans lequel la détermination du niveau de monocytes comprend la mesure d'un ou de plusieurs marqueurs choisis dans le groupe consistant en CD11b, CD11c, CD14, CD16, CD45, CD62L, CD86, CD115, CD116, CD121, CD163, CD192, CD206, $CX_3CR1$ et HLA-DR.

**11.** Méthode de l'une des revendications 1 à 5, stratifiant en outre ledit sujet comme souffrant de formes progressives de sclérose en plaques (PMS), si l'une ou plusieurs des conditions suivantes sont remplies:

i) un niveau de cellules T $CD4^+$ HLA-$DR^+$ dans ledit échantillon de PB test est augmenté par rapport aux niveaux correspondants de cellules T $CD4^+$ HLA-$DR^+$ dans les échantillons de PB de contrôle obtenus à partir de sujets souffrant d'une autre maladie auto-immune neuro-inflammatoire autre que le PMS;
ii) un niveau de monocytes dans ledit échantillon de LCR testé est augmenté par rapport aux niveaux correspondants de monocytes dans lesdits échantillons de LCR de contrôle obtenus à partir de sujets souffrant d'uneautre maladie auto-immune neuro-inflammatoire autre que le PMS.

**12.** Système de traitement de données comprenant un processeur configuré pour exécuter une méthode comprenant les étapes suivantes:

a) obtenir:

i) le niveau détecté de cellules B dans un échantillon de LCR prélevé sur un sujet;
ii) le niveau détecté de cellules immunitaires par $\mu$l de l'échantillon de LCR prélevé sur le sujet;

iii) le niveau détecté de cellules NKT dans ledit échantillon de LCR obtenu dudit sujet;
iv) le niveau détecté de monocytes dans ledit échantillon de LCR obtenu dudit sujet; et
v) le niveau détecté de cellules NK CD56$^{dim}$ CD16$^{+}$ à partir d'un échantillon de PB test obtenu dudit sujet;

b) stratifier ledit sujet comme souffrant d'une maladie neurologique ou psychiatrique, de préférence d'une maladie auto-immune neuro-inflammatoire, si les conditions suivantes sont remplies:

i) le niveau détecté de cellules B est augmenté par rapport aux niveaux correspondants de cellules B dans des échantillons de LCR de contrôle obtenus à partir de sujets ne souffrant pas de maladies auto-immunes neuro-inflammatoires;
ii) le niveau détecté de cellules immunitaires par μl est accru par rapport aux niveaux correspondants de cellules immunitaires par μl dans lesdits échantillons de LCR de contrôle obtenus à partir desdits sujets ne souffrant pas de maladies neuro-inflammatoires auto-immunes;
iii) le niveau détecté de cellules NKT est réduit par rapport aux niveaux correspondants de cellules NKT dans lesdits échantillons de LCR de contrôle obtenus à partir desdits sujets ne souffrant pas de maladies auto-immunes neuro-inflammatoires;
iv) le niveau détecté de monocytes est réduit par rapport aux niveaux correspondants de monocytes dans lesdits échantillons de LCR de contrôle obtenus à partir desdits sujets ne souffrant pas de maladies auto-immunes neuro-inflammatoires; et
v) le niveau détecté de cellules NK CD56$^{dim}$ CD16$^{+}$ est réduit par rapport aux niveaux correspondants de cellules NK CD56$^{dim}$ CD16$^{+}$ dans les échantillons de PB de contrôle obtenus à partir desdits sujets ne souffrant pas de maladies auto-immunes neuro-inflammatoires,

la combinaison des niveaux i) à v) de l'étape b) indique si le sujet est suspecté de souffrir d'une maladie auto-immune neuro-inflammatoire ou d'une autre maladie neurologique ou psychiatrique autre qu'une maladie auto-immune neuro-inflammatoire.

**13.** Dispositif de cytométrie en flux capable de détecter les éléments suivants : i) le niveau de cellules B dans un échantillon de LCR test obtenu à partir d'un sujet ; ii) le niveau de cellules immunitaires par μl dans ledit échantillon de LCR test obtenu à partir dudit sujet ; iii) le niveau de cellules NKT dans ledit échantillon de LCR test obtenu à partir dudit sujet ; iv) le niveau de monocytes dans ledit échantillon de LCR test obtenu à partir dudit sujet, et v) le niveau de cellules NK CD56$^{dim}$ CD16$^{+}$ dans un échantillon de PB test obtenu à partir dudit sujet, comprenant le système de traitement de données de la revendication 12.

**14.** Programme d'ordinateur comprenant des instructions pour amener le système de traitement des données de la revendication 12 ou le dispositif de cytométrie en flux de la revendication 13 à exécuter les étapes suivantes

a) obtenir:

i) le niveau détecté de cellules B dans un échantillon de LCR prélevé sur un sujet;
ii) le niveau détecté de cellules immunitaires par μl de l'échantillon de LCR prélevé sur le sujet;
iii) le niveau détecté de cellules NKT dans ledit échantillon de LCR obtenu dudit sujet;
iv) le niveau détecté de monocytes dans ledit échantillon de LCR obtenu dudit sujet; et
v) le niveau détecté de cellules NK CD56$^{dim}$ CD16$^{+}$ à partir d'un échantillon de PB test obtenu dudit sujet;

b) stratifier ledit sujet comme souffrant d'une maladie neurologique ou psychiatrique, de préférence d'une maladie auto-immune neuro-inflammatoire, si les conditions suivantes sont remplies:

i) le niveau détecté de cellules B est augmenté par rapport aux niveaux correspondants de cellules B dans des échantillons de LCR de contrôle obtenus à partir de sujets ne souffrant pas de maladies auto-immunes neuro-inflammatoires;
ii) le niveau détecté de cellules immunitaires par μl est accru par rapport aux niveaux correspondants de cellules immunitaires par μl dans lesdits échantillons de LCR de contrôle obtenus à partir desdits sujets ne souffrant pas de maladies neuro-inflammatoires auto-immunes;
iii) le niveau détecté de cellules NKT est réduit par rapport aux niveaux correspondants de cellules NKT dans lesdits échantillons de LCR de contrôle obtenus à partir desdits sujets ne souffrant pas de maladies auto-immunes neuro-inflammatoires;
iv) le niveau détecté de monocytes est réduit par rapport aux niveaux correspondants de monocytes dans lesdits échantillons de LCR de contrôle obtenus à partir desdits sujets ne souffrant pas de maladies auto-immunes neuro-inflammatoires; et

v) le niveau détecté de cellules NK $CD56^{dim}$ $CD16^{+}$ est réduit par rapport aux niveaux correspondants de cellules NK $CD56^{dim}$ $CD16^{+}$ dans les échantillons de PB de contrôle obtenus à partir desdits sujets ne souffrant pas de maladies auto-immunes neuro-inflammatoires,

la combinaison des niveaux détectés i) à v) de l'étape b) indique si le sujet est suspecté de souffrir d'une maladie auto-immune neuro-inflammatoire ou d'une autre maladie neurologique ou psychiatrique autre qu'une maladie auto-immune neuro-inflammatoire.

**15.** Kit comprenant un partenaire de liaison marqué par fluorescence pour CD1c, CD1d, CD3, CD4, CD5, CD8, CD11b, CD11c, CD14, CD16, CD19, CD20, CD21, CD27, CD24, CD25, CD27, CD38, CD40, CD45, CD56, CD62L, CD66b, CD78, CD80, CD86, CD94, CD115, CD116, CD121, CD122, CD138, CD159a, CD161, CD163, CD192, CD206, CD244, CD315, CD319, CD335, CD336, CD337, CXCR4, CXCR5, $CX_3CR1$, IgD, IgM, TACI, TCRγδ, Vα24-Jα18, Vβ11, Vδ1, Vδ2, KIR, Tbet, EOMES et HLA-DR.

Figure 1

A

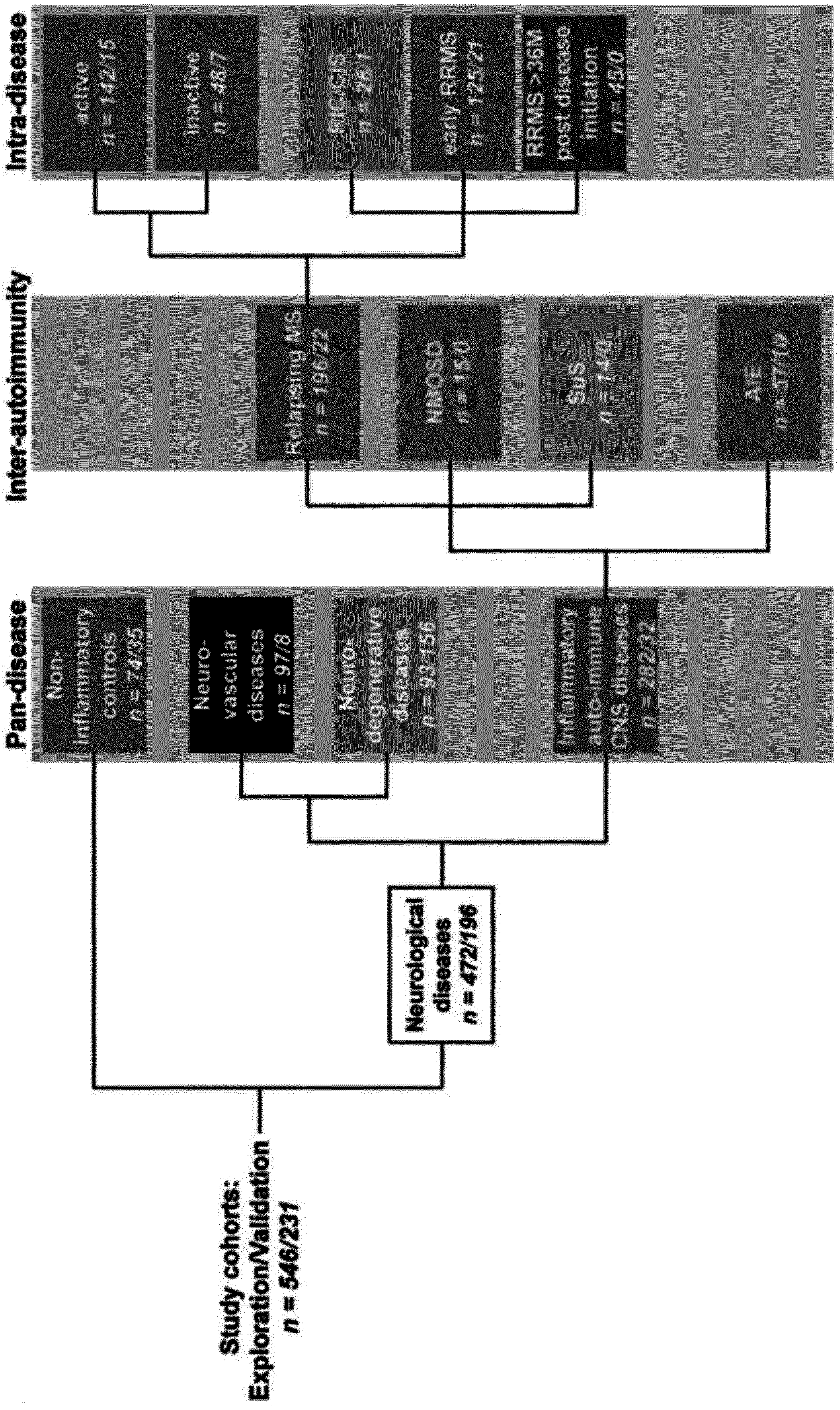
Intra-disease
active n = 142/15
inactive n = 48/7
RIC/CIS n = 26/1
early RRMS n = 125/21
RRMS >36M post disease initiation n = 45/0
Inter-autoimmunity
Relapsing MS n = 196/22
NMOSD n = 15/0
SuS n = 14/0
AIE n = 57/10
Pan-disease
Non-inflammatory controls n = 74/35
Neuro-vascular diseases n = 97/8
Neuro-degenerative diseases n = 93/156
Inflammatory auto-immune CNS diseases n = 282/32
Neurological diseases n = 472/196
Study cohorts: Exploration/Validation n = 546/231

Figure 1 (cont.)

B

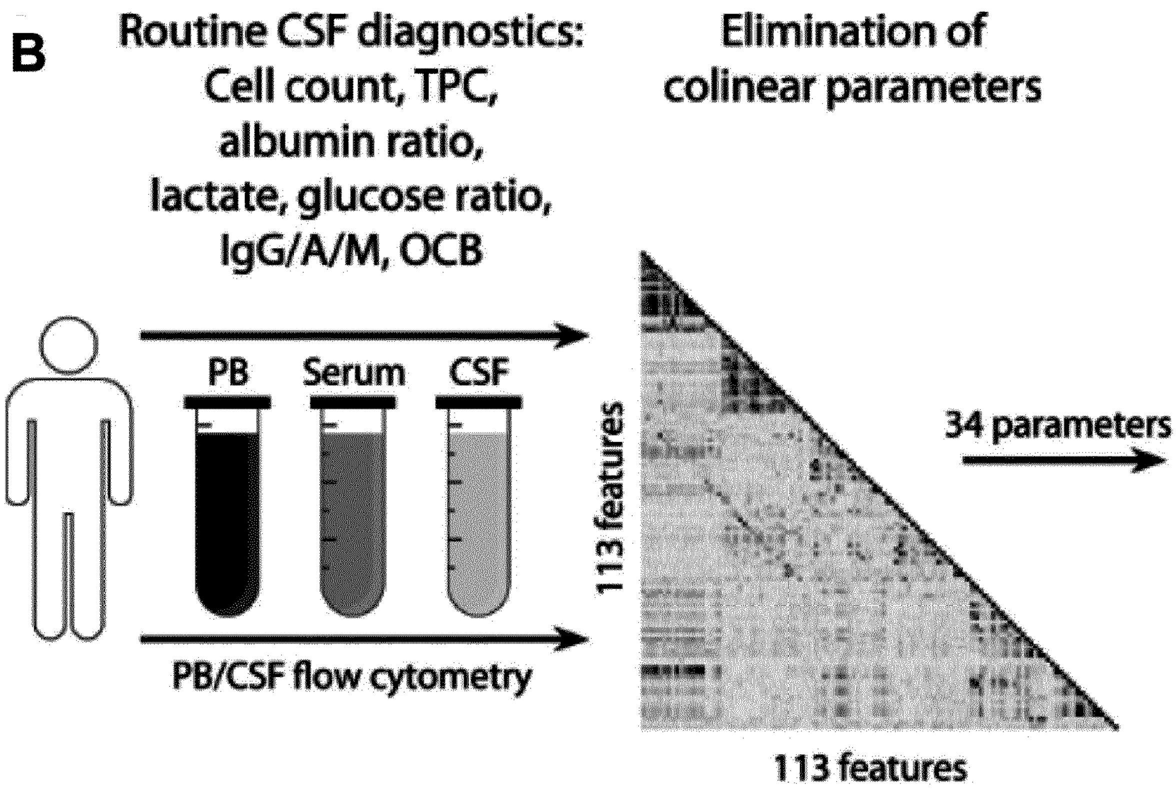

Figure 1 (cont.)

B

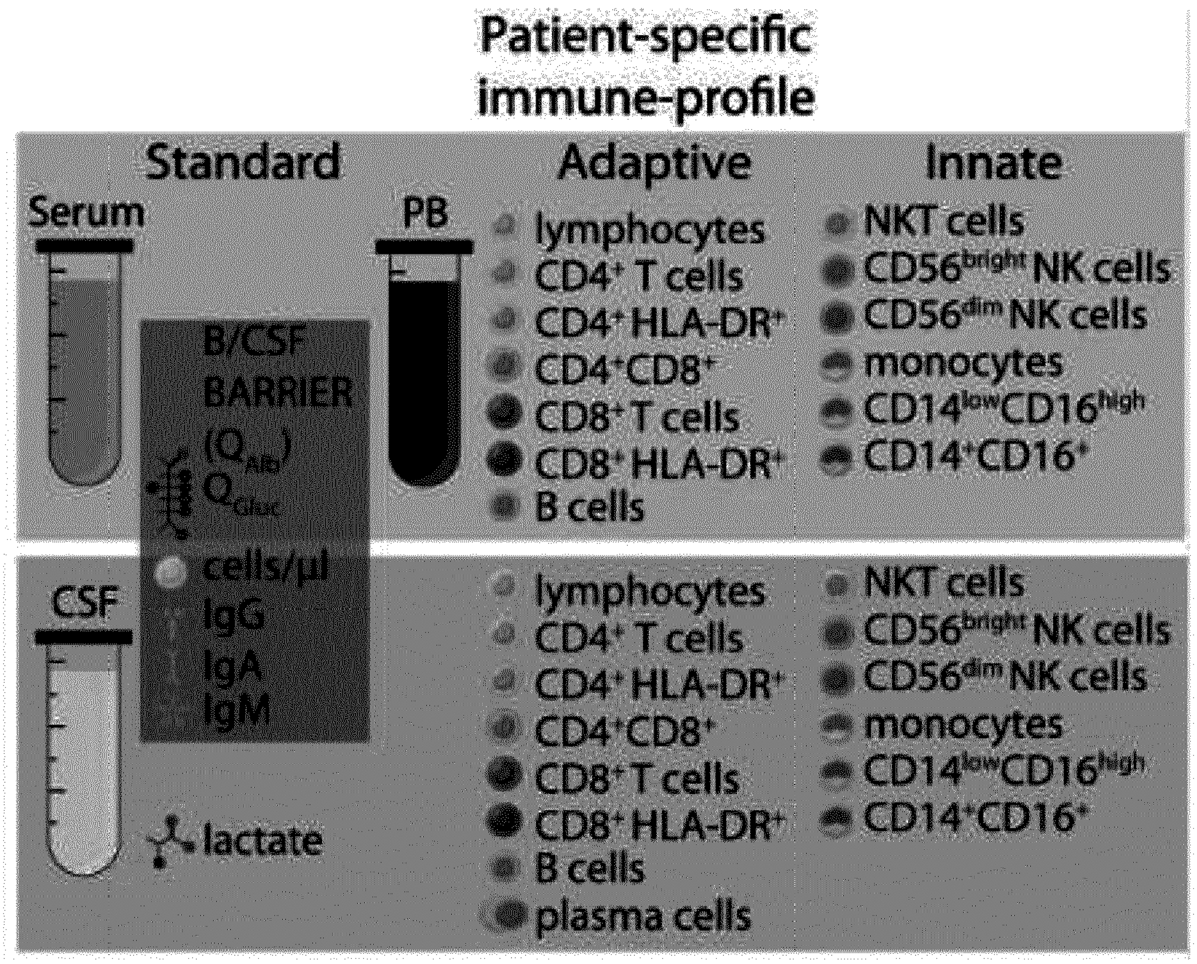

Figure 1 (cont.)

C

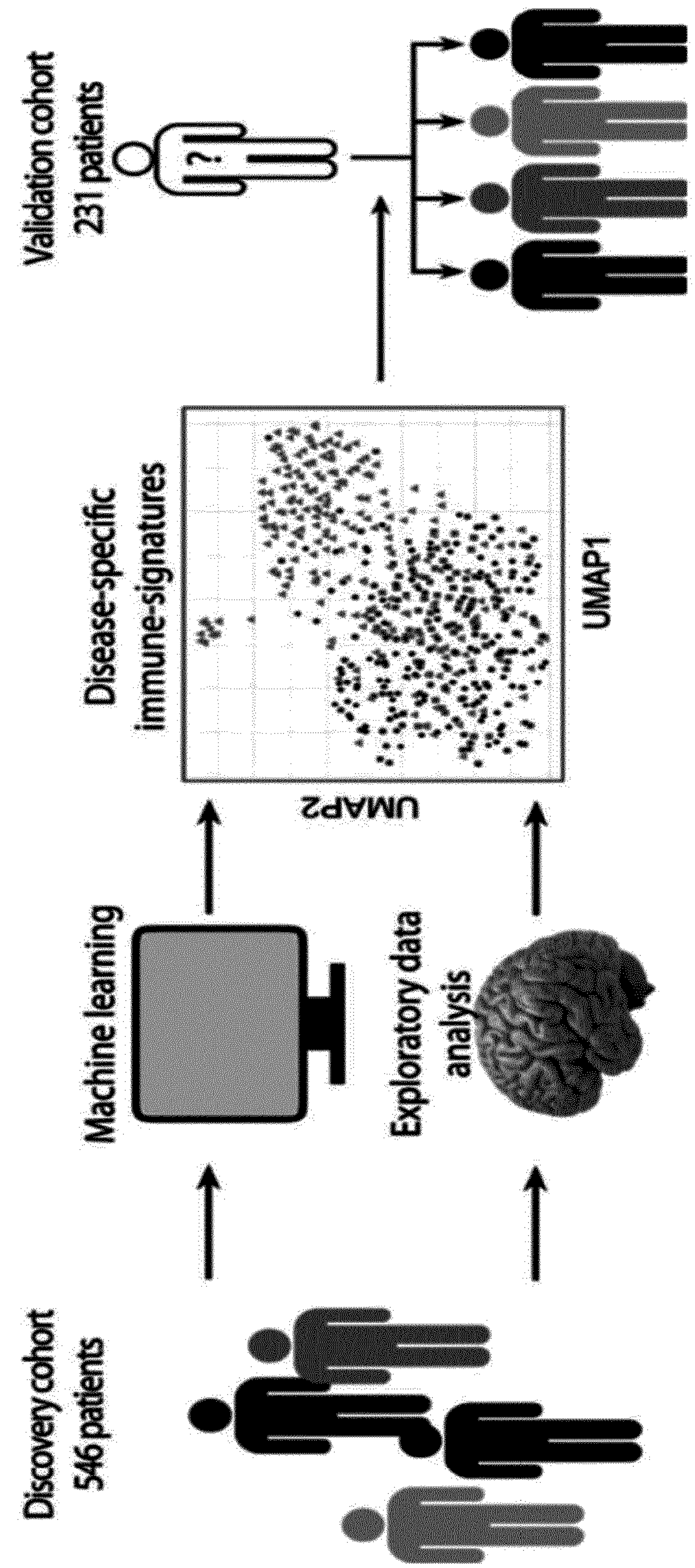

Figure 2

A

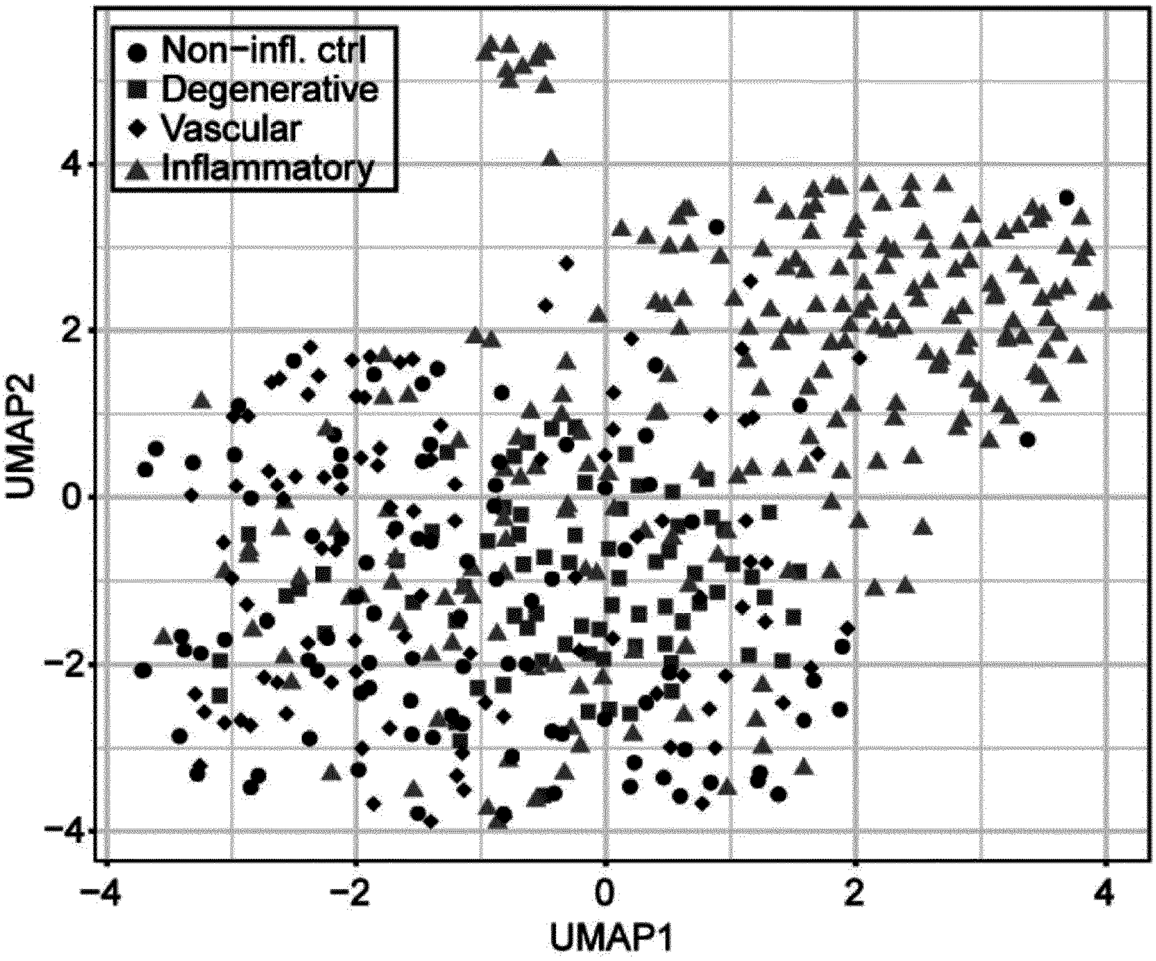
Non−infl. ctrl
Degenerative
Vascular
Inflammatory
4
2
0
−2
−4
UMAP2
−4
−2
0
2
4
UMAP1

Figure 2 (cont.)

B

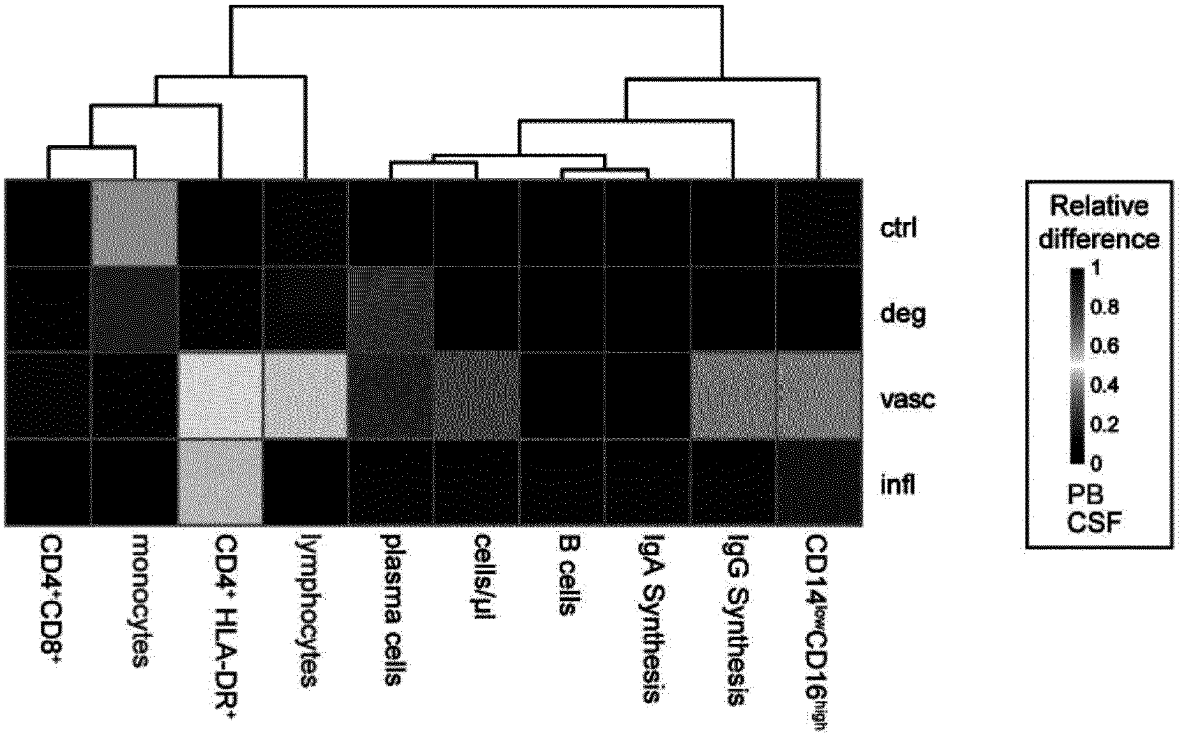


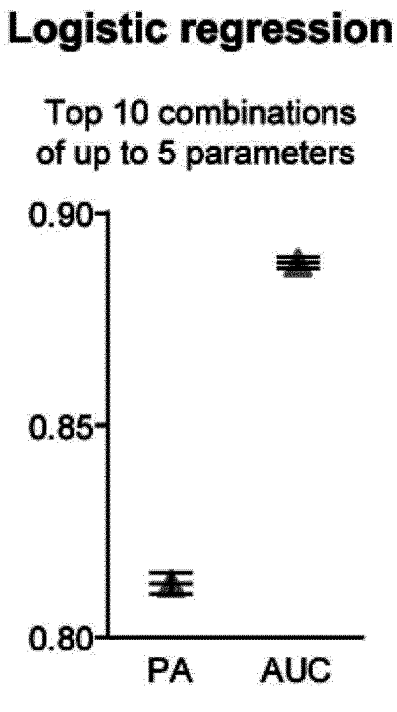

Figure 2 (cont.)

C

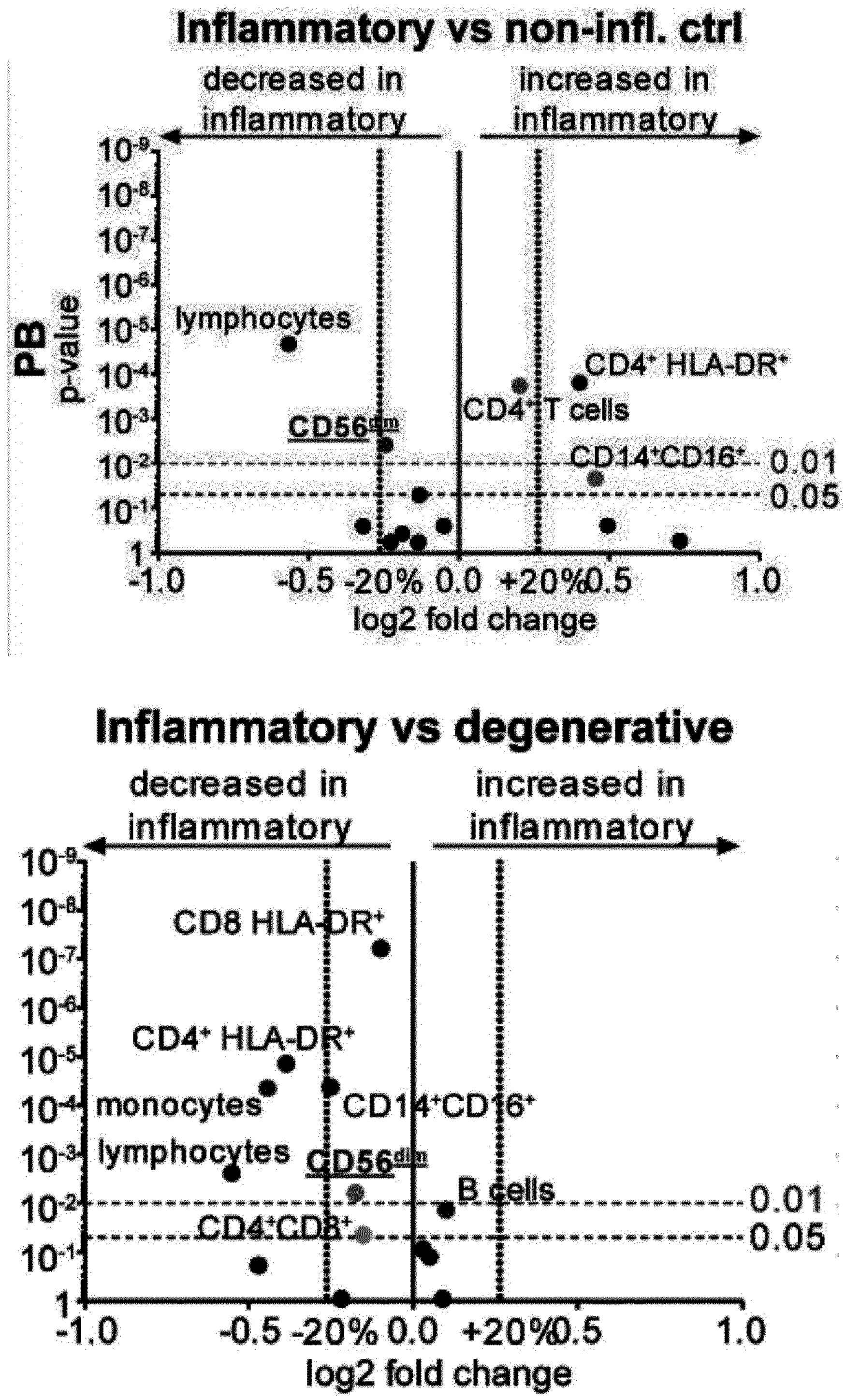

Figure 2 (cont.)

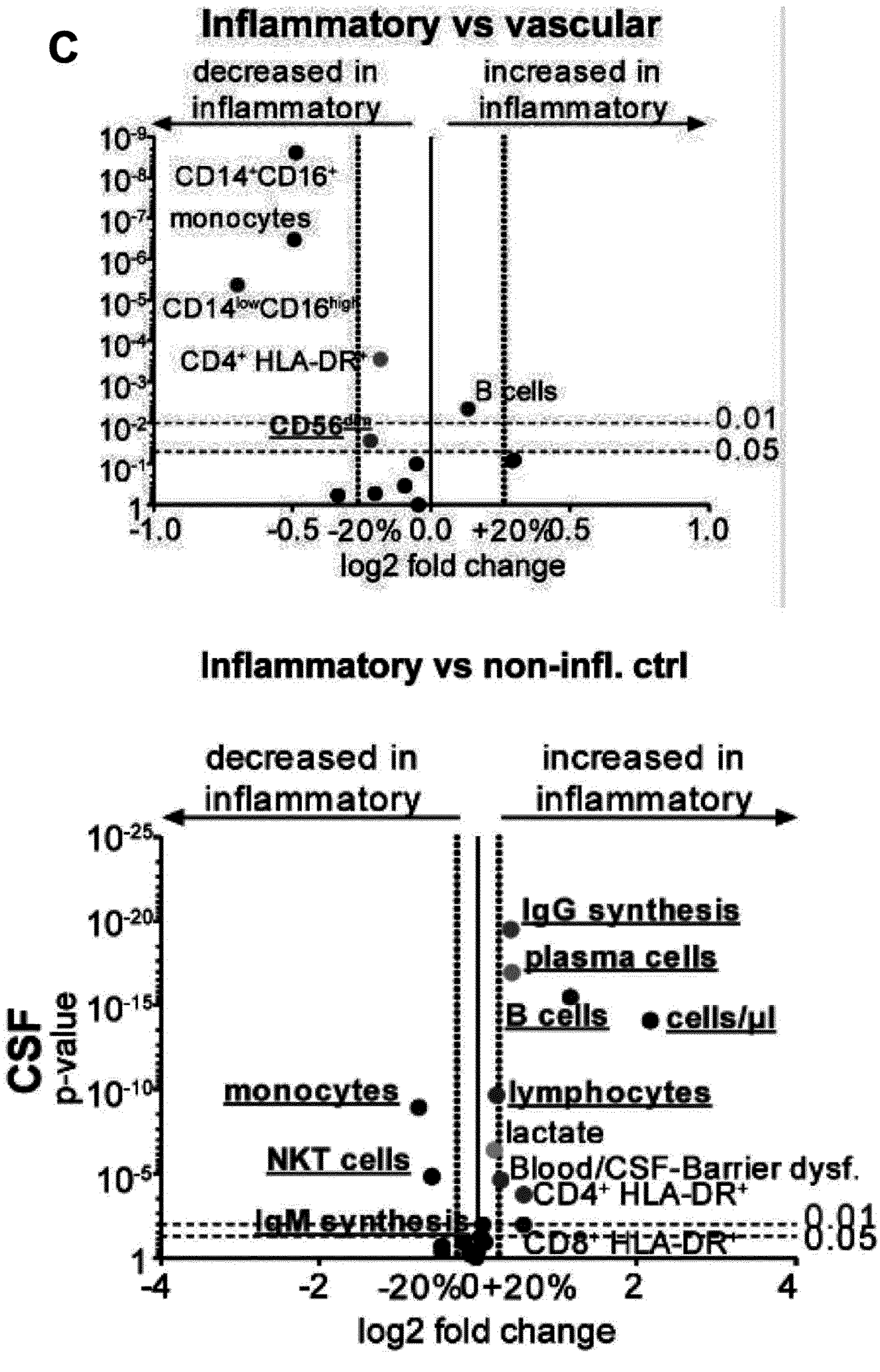

C

Figure 2 (cont.)

Inflammatory vs degenerative

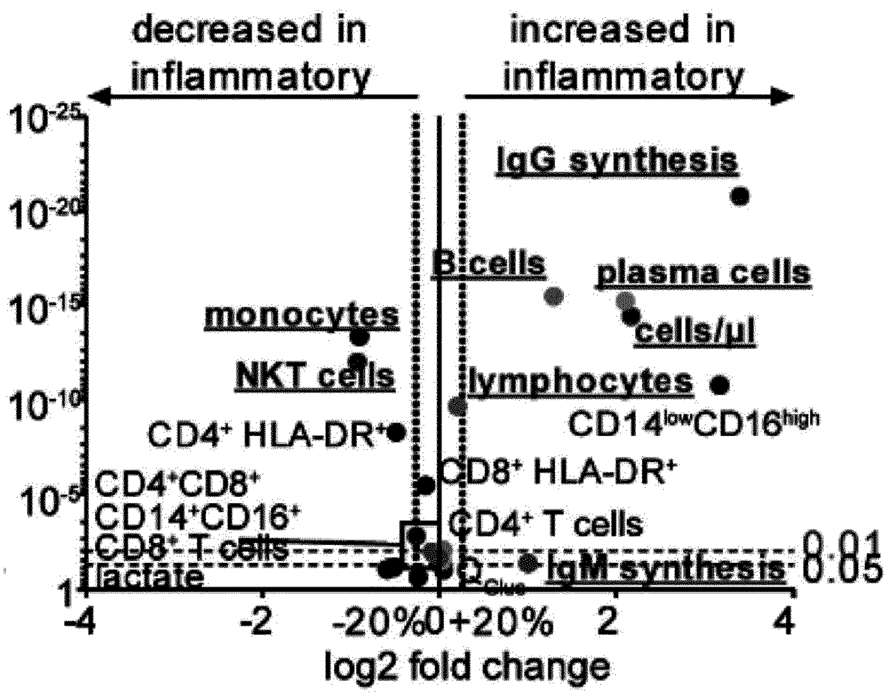


Inflammatory vs vascular

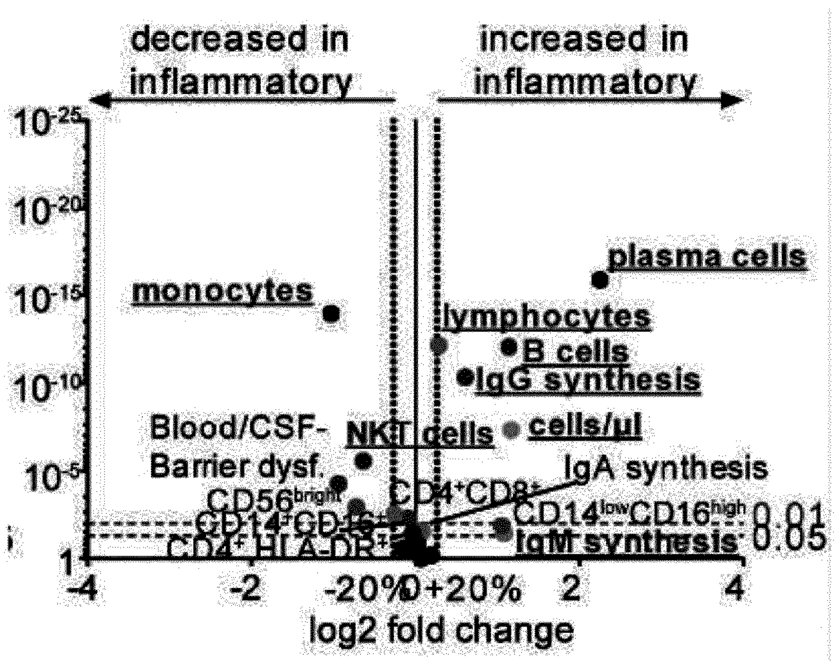

Figure 3

A

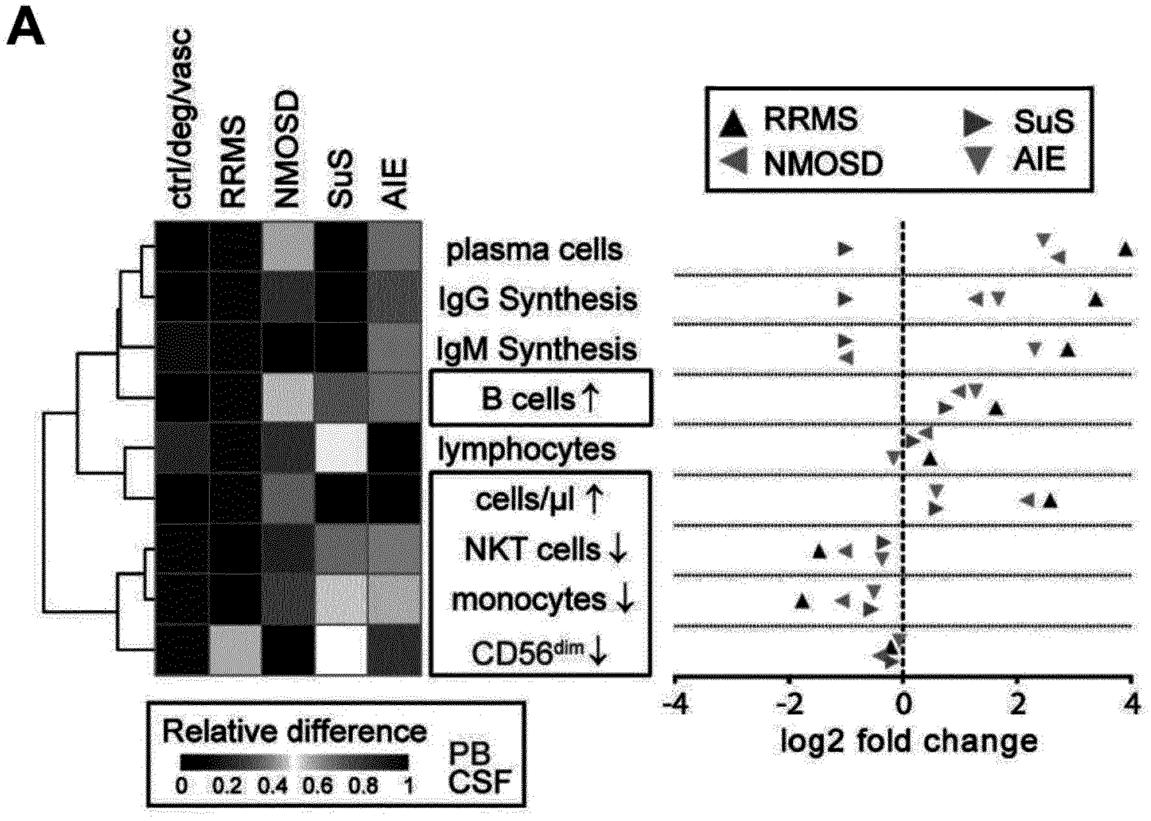
ctrl/deg/vasc
RRMS
NMOSD
SuS
AIE
plasma cells
IgG Synthesis
IgM Synthesis
B cells ↑
lymphocytes
cells/µl ↑
NKT cells ↓
monocytes ↓
CD56dim ↓
Relative difference
0 0.2 0.4 0.6 0.8 1
PB
CSF
RRMS
NMOSD
SuS
AIE
-4 -2 0 2 4
log2 fold change

B

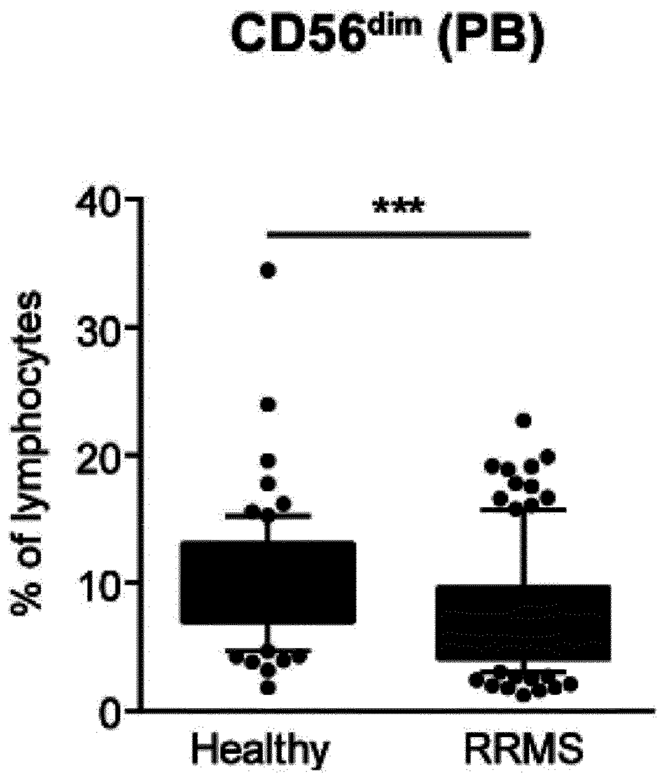
CD56dim (PB)
% of lymphocytes
0
10
20
30
40
***
Healthy
RRMS

## Figure 3 (cont.)

C

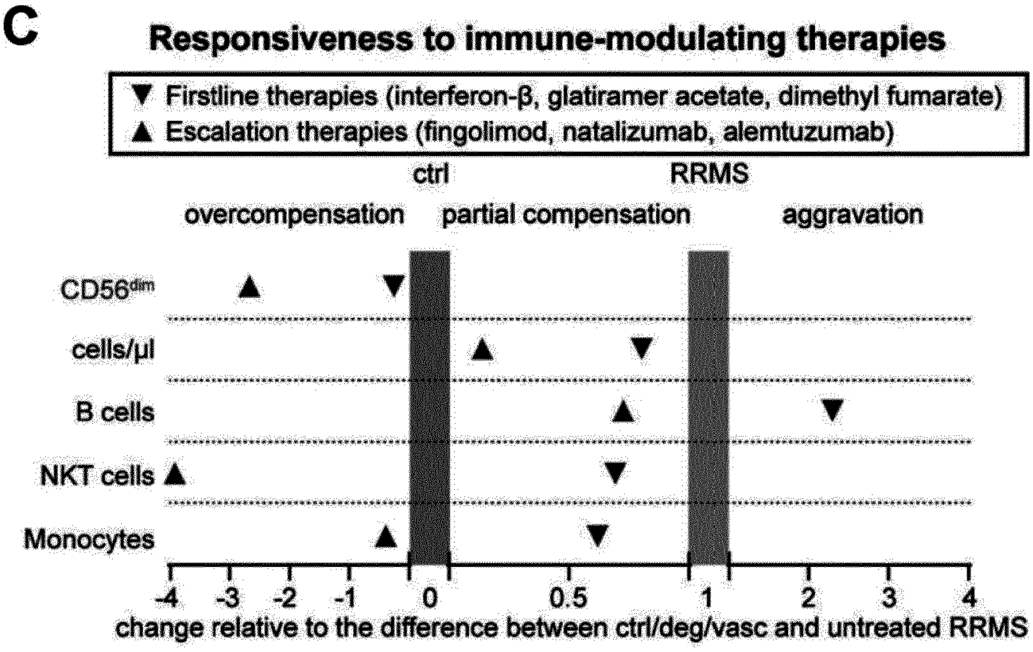


D

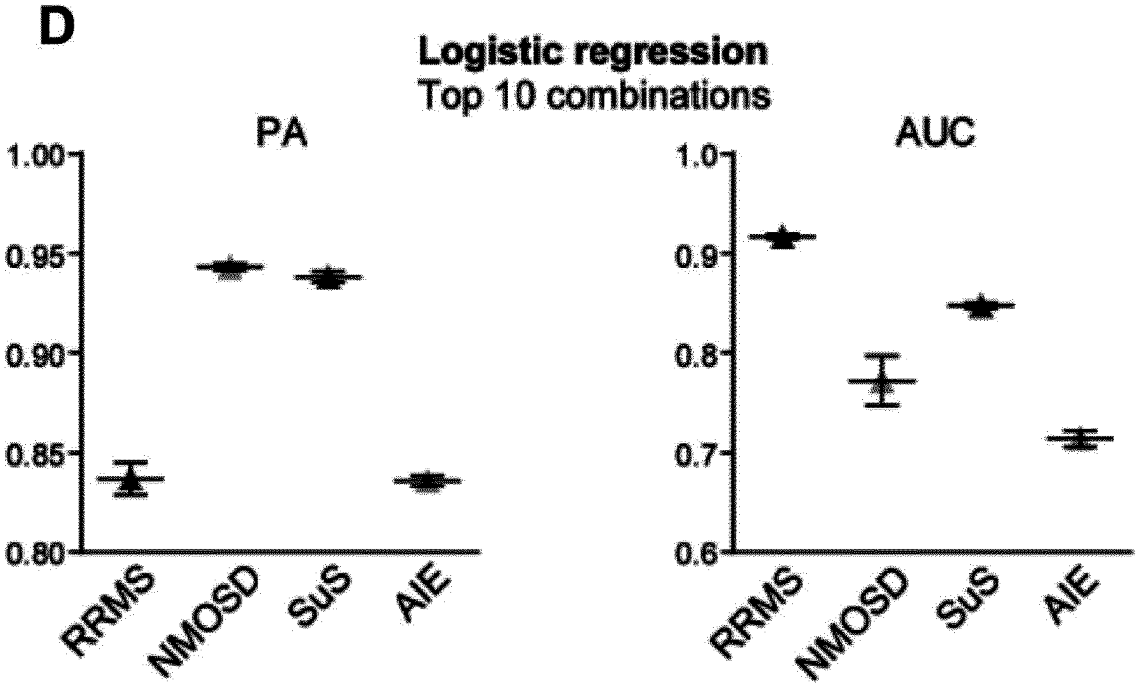

Figure 3 (cont)

E

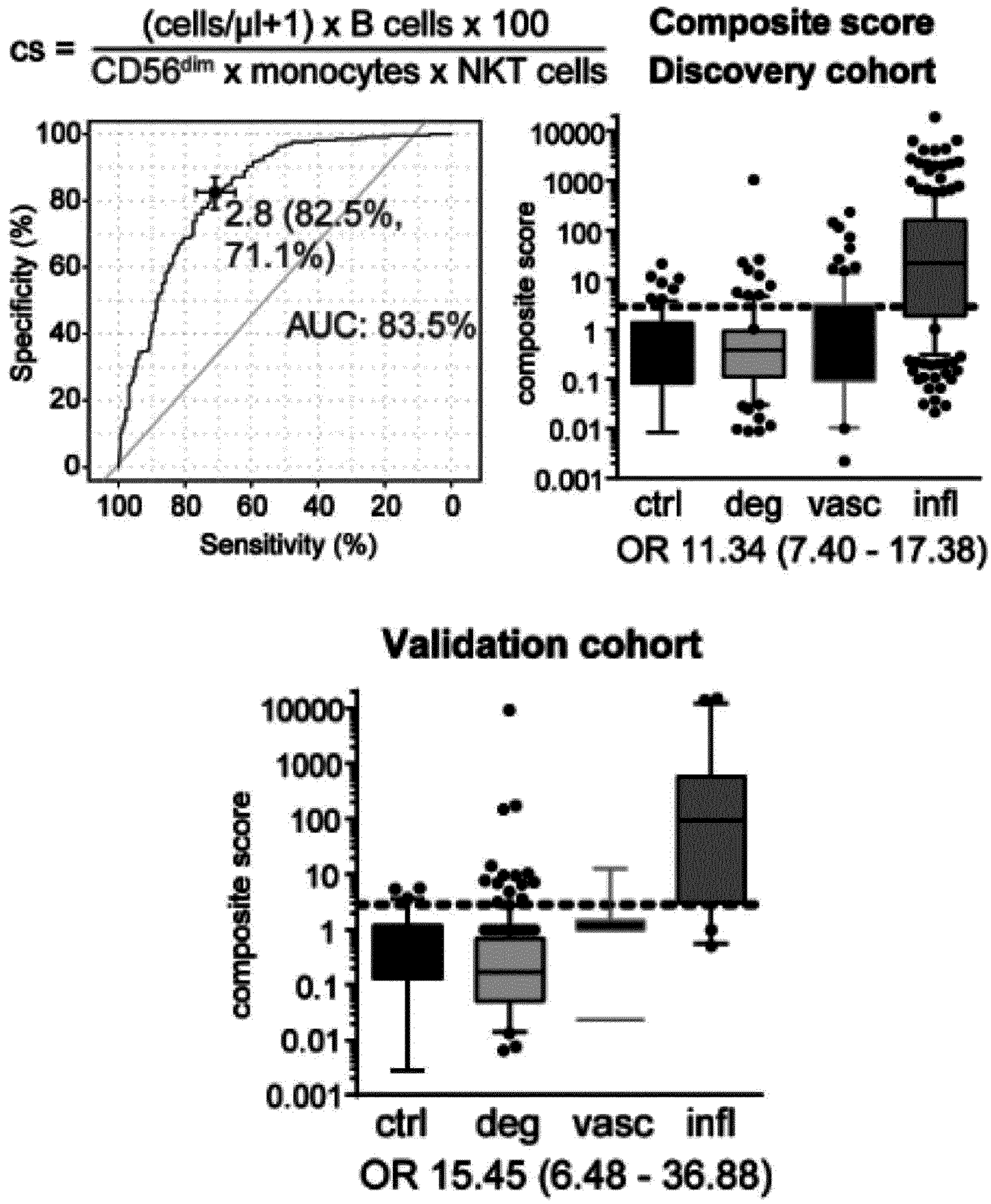

Figure 3 (cont.)

F

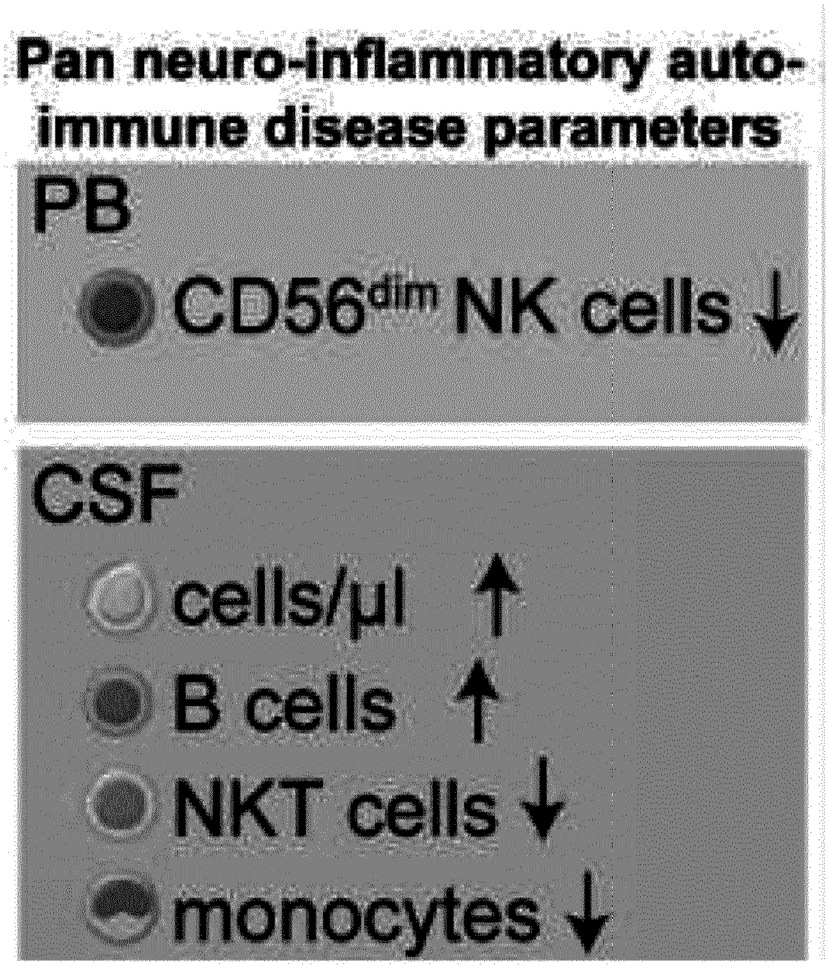

Figure 4

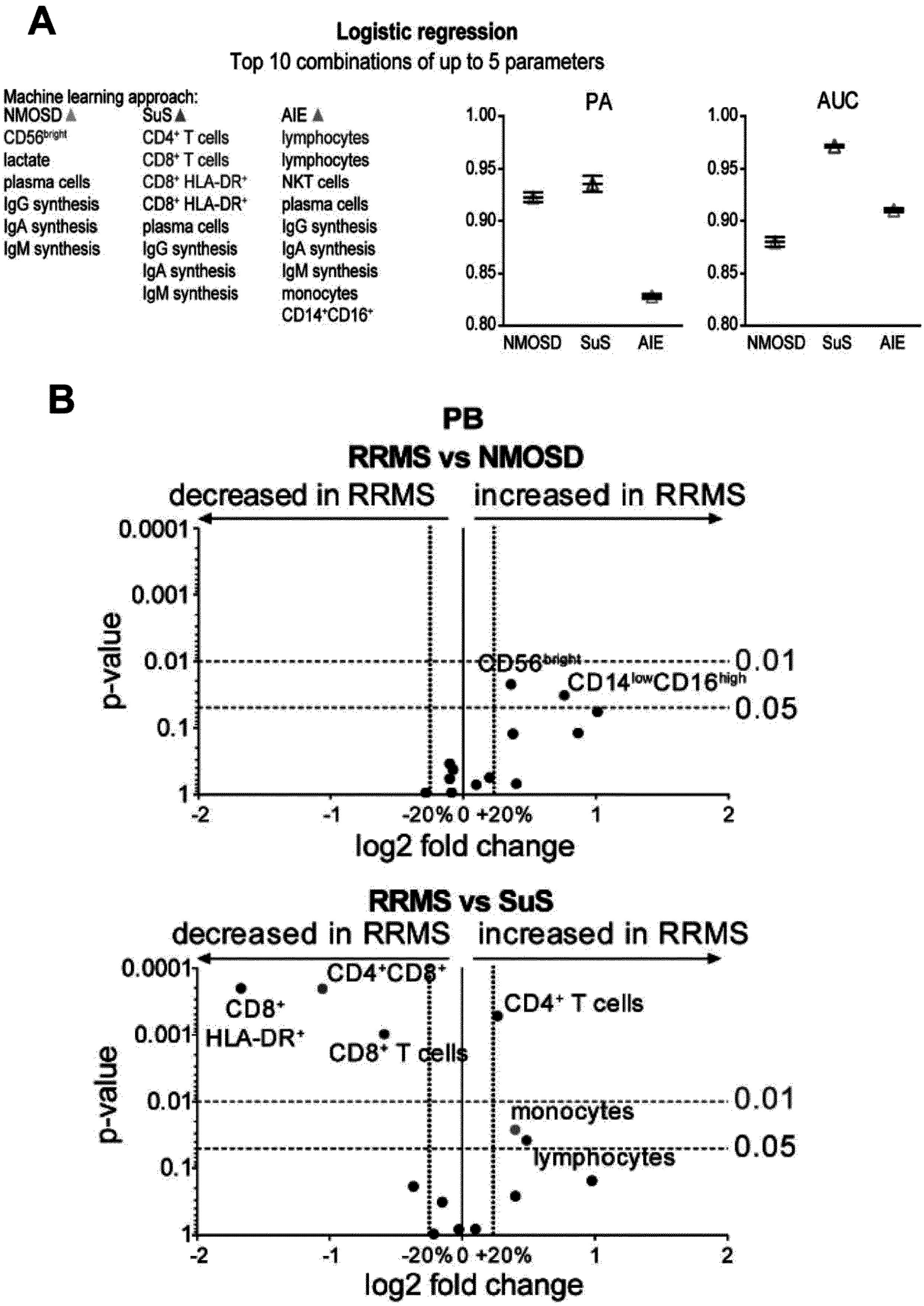
A
Logistic regression
Top 10 combinations of up to 5 parameters
Machine learning approach:
NMOSD ▲
CD56bright
lactate
plasma cells
IgG synthesis
IgA synthesis
IgM synthesis
SuS ▲
CD4+ T cells
CD8+ T cells
CD8+ HLA-DR+
CD8+ HLA-DR+
plasma cells
IgG synthesis
IgA synthesis
IgM synthesis
AIE ▲
lymphocytes
lymphocytes
NKT cells
plasma cells
IgG synthesis
IgA synthesis
IgM synthesis
monocytes
CD14+CD16+
PA
AUC
1.00
0.95
0.90
0.85
0.80
NMOSD
SuS
AIE
B
PB
RRMS vs NMOSD
decreased in RRMS
increased in RRMS
p-value
0.0001
0.001
0.01
0.1
1
CD56bright
CD14lowCD16high
0.01
0.05
-2
-1
-20%
0
+20%
1
2
log2 fold change
RRMS vs SuS
decreased in RRMS
increased in RRMS
CD4+CD8+
CD8+
HLA-DR+
CD8+ T cells
CD4+ T cells
monocytes
lymphocytes

Figure 4 (cont.)

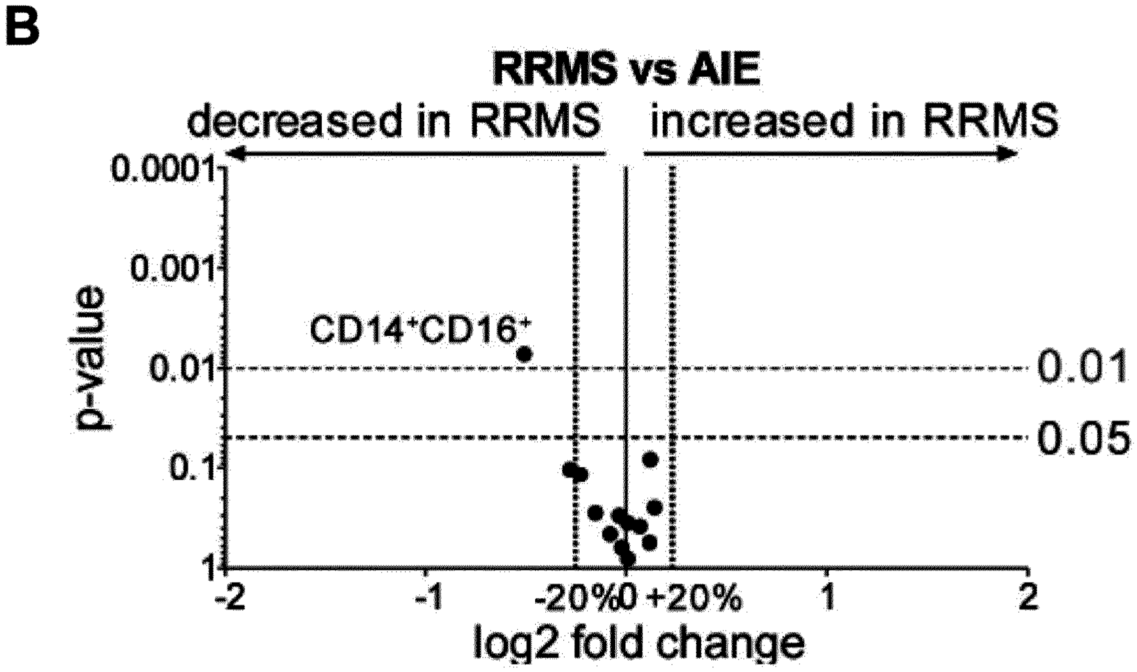


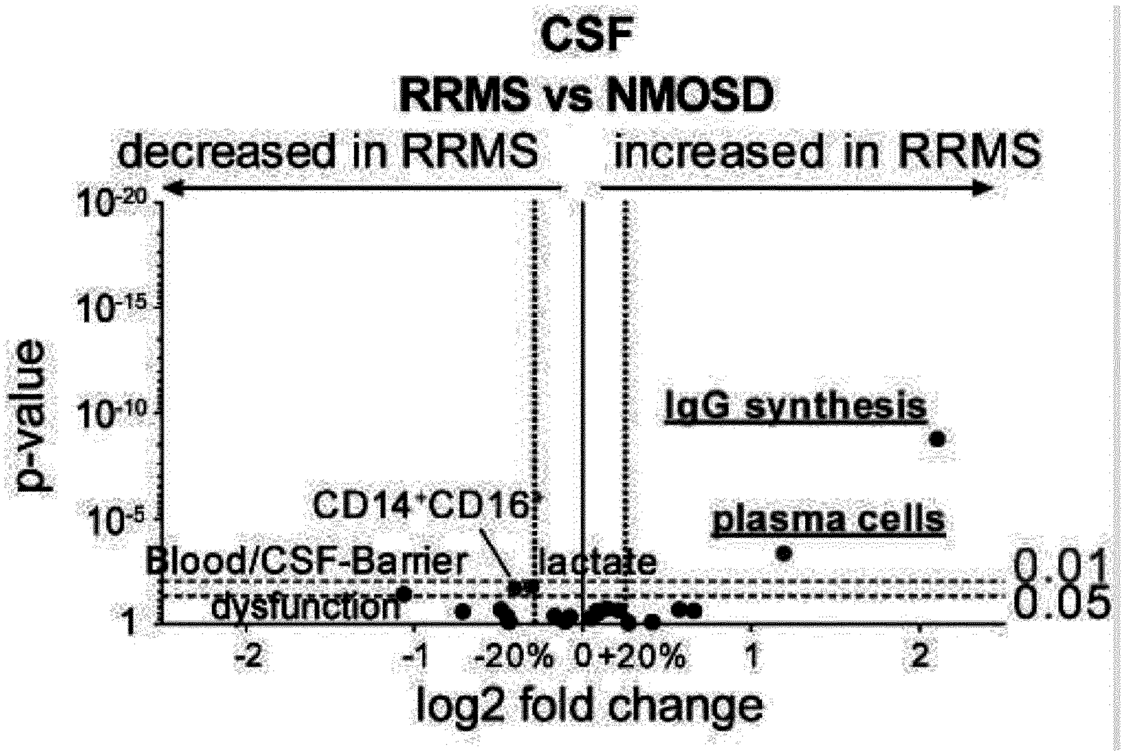

Figure 4 (cont.)

B

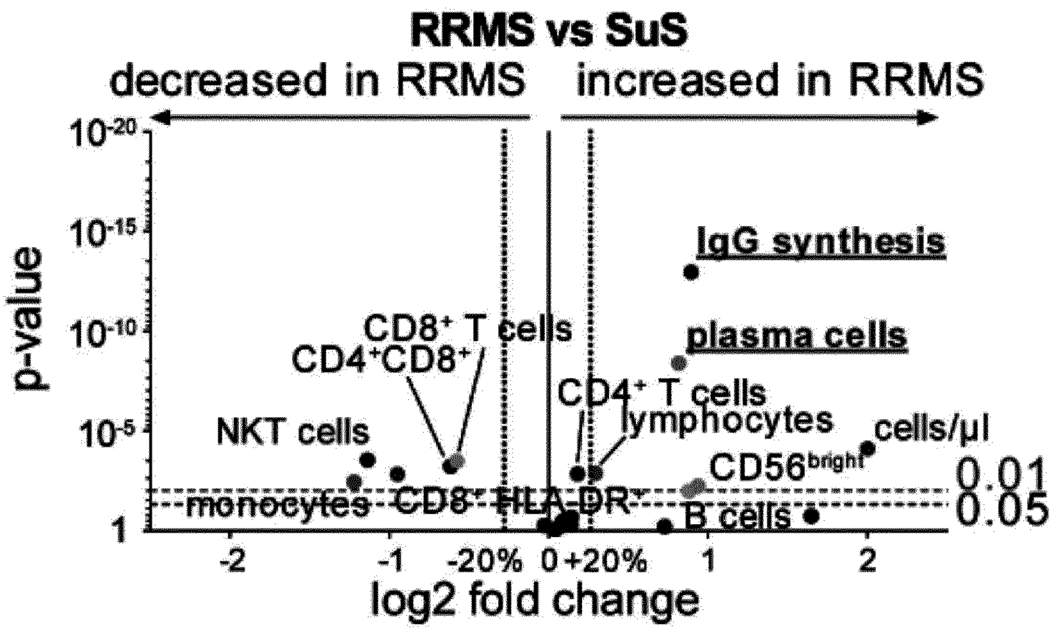


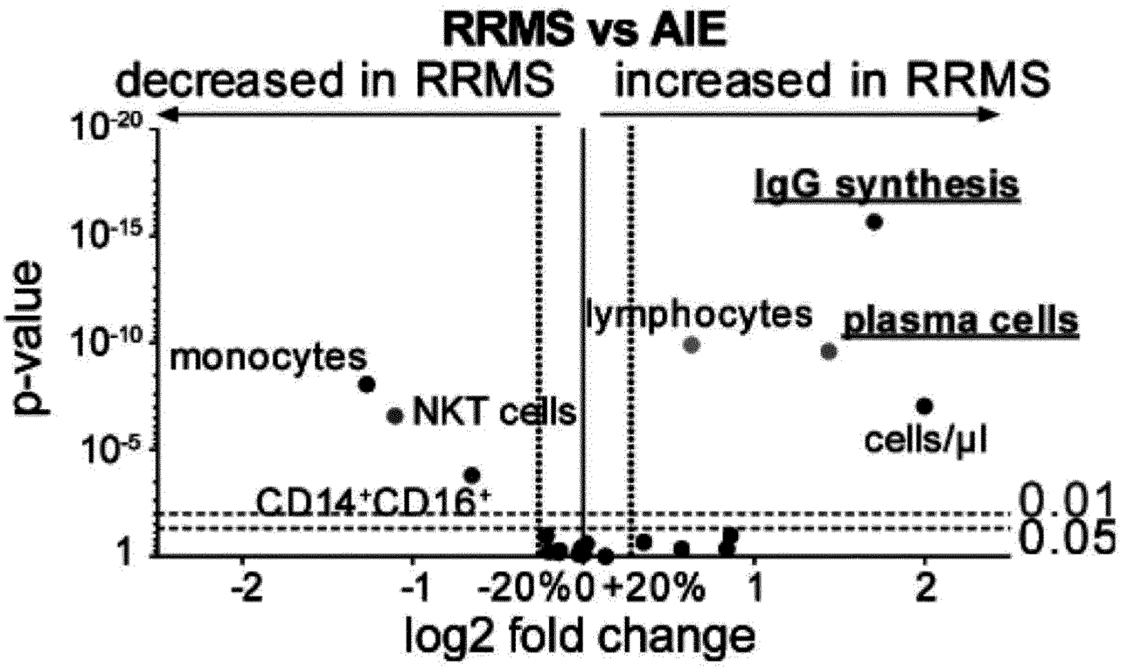

Figure 4 (cont.)

C

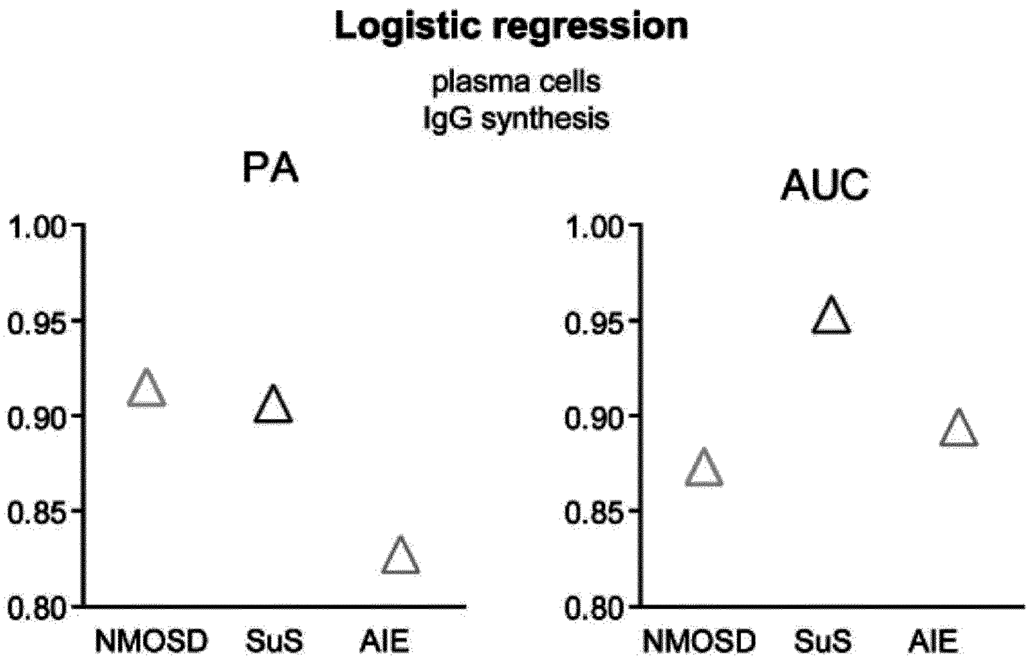


D

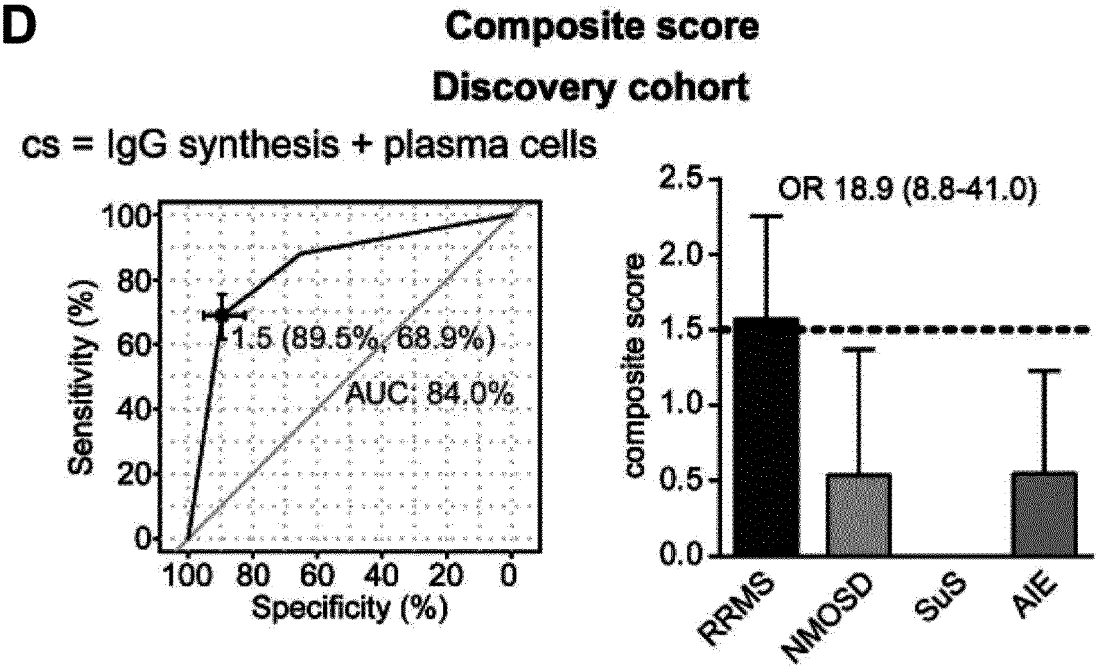

Figure 4 (cont.)

D

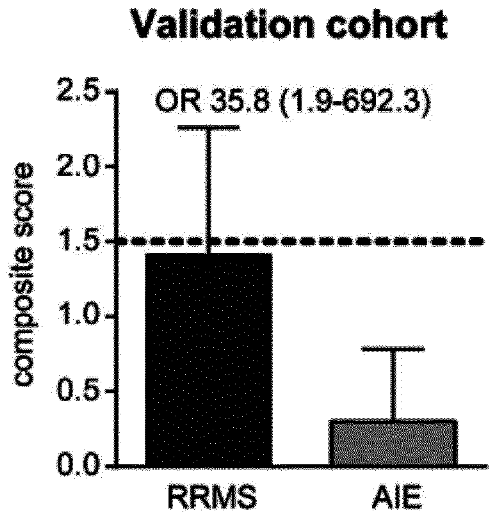


E

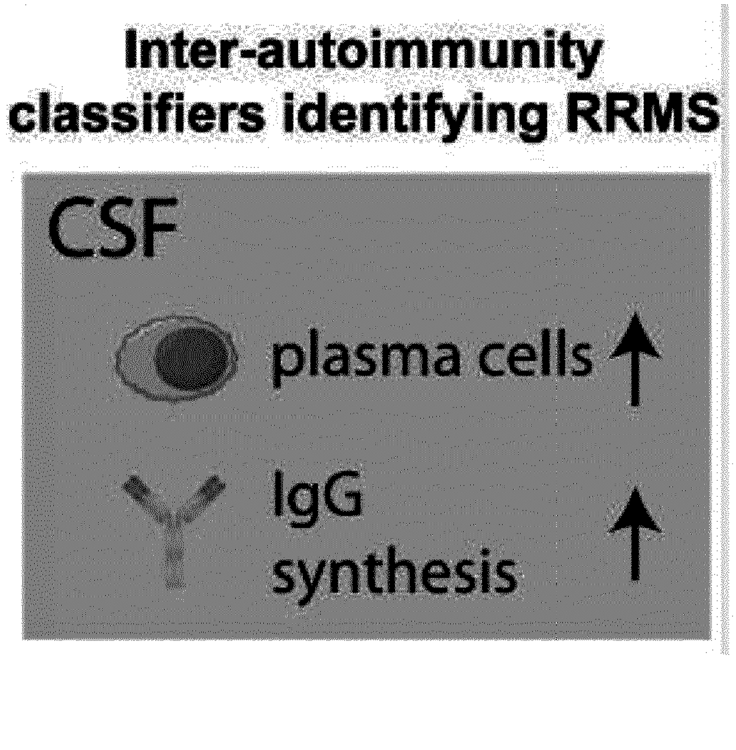

**Figure 5**

**A**

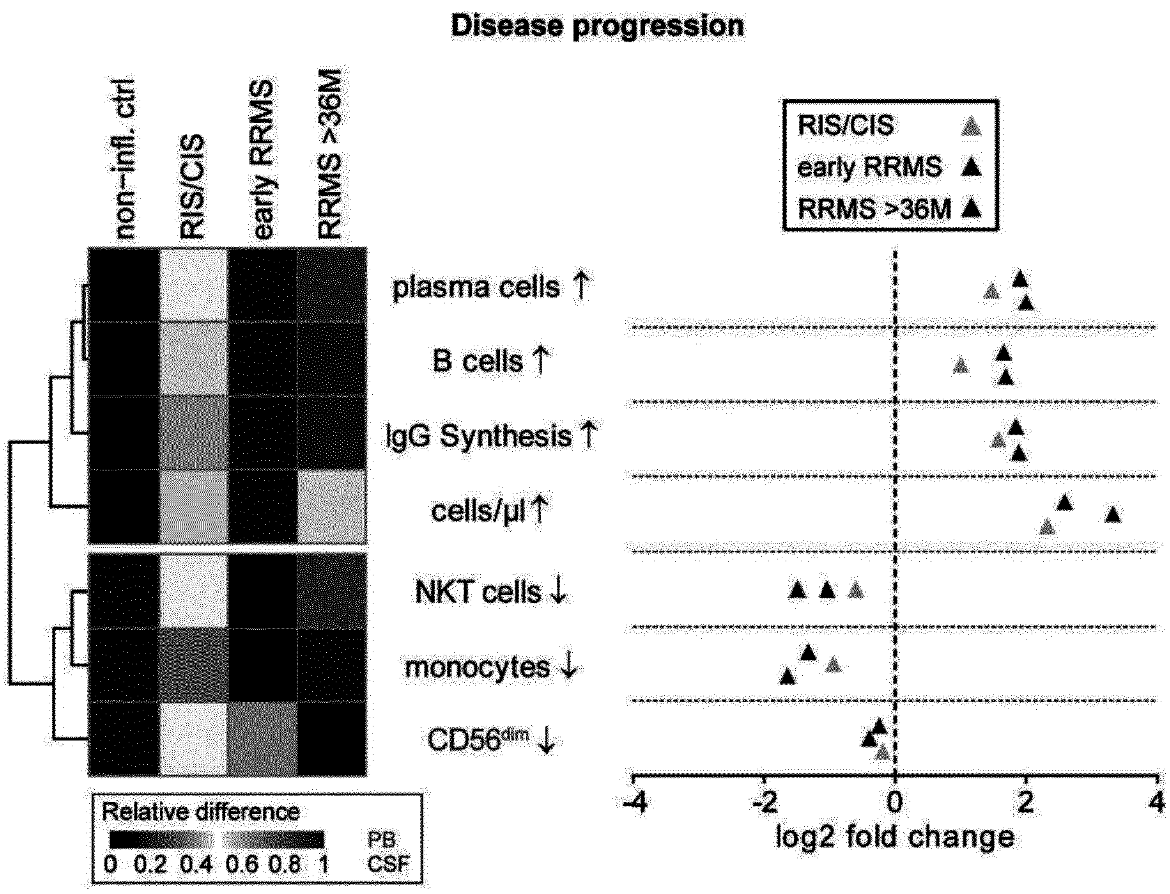
Disease progression
non-infl. ctrl
RIS/CIS
early RRMS
RRMS >36M
plasma cells ↑
B cells ↑
IgG Synthesis ↑
cells/µl ↑
NKT cells ↓
monocytes ↓
CD56dim ↓
Relative difference
0 0.2 0.4 0.6 0.8 1
PB
CSF
RIS/CIS
early RRMS
RRMS >36M
-4 -2 0 2 4
log2 fold change

Figure 5 (cont.)

B

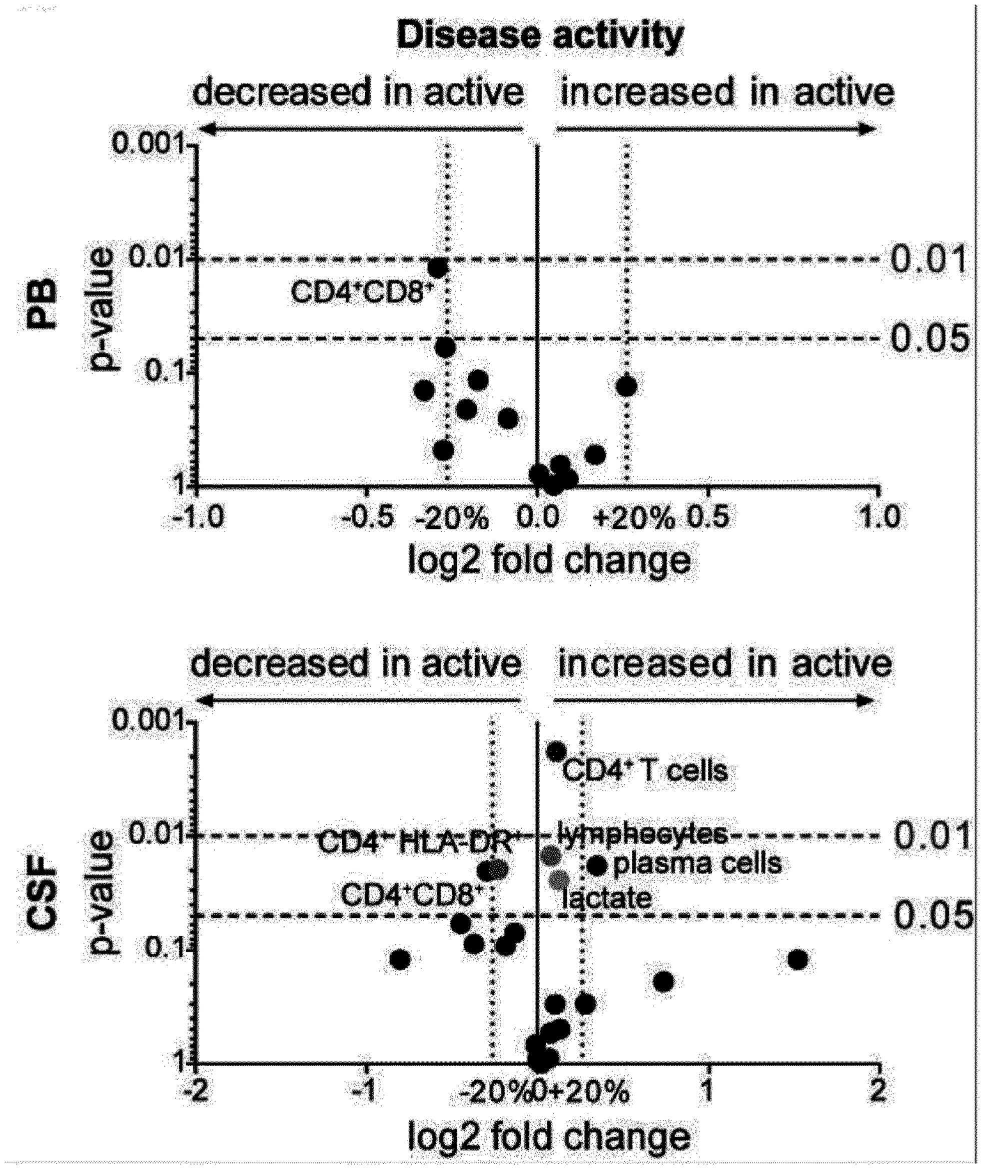

Figure 6

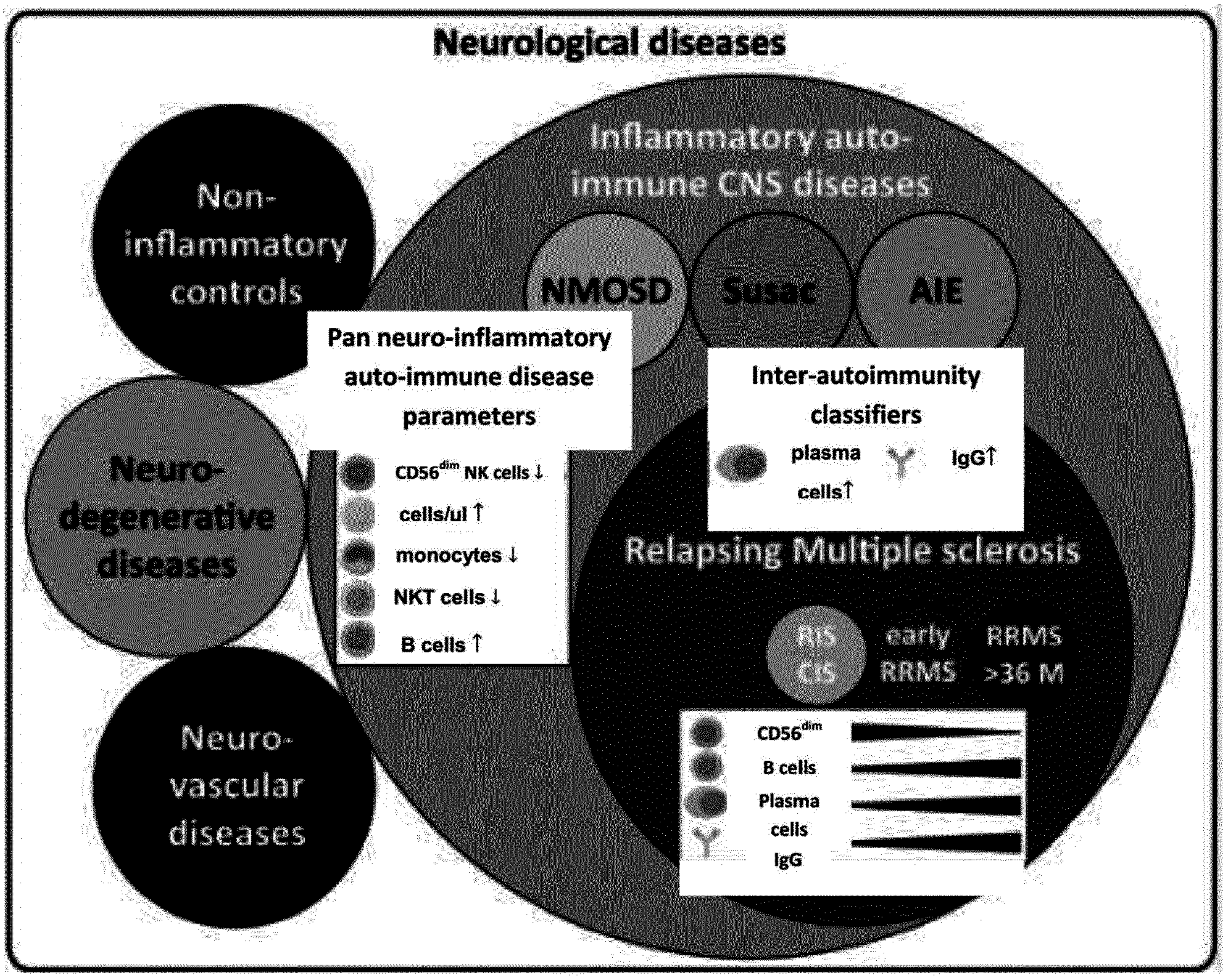
Neurological diseases
Inflammatory auto-immune CNS diseases
Non-inflammatory controls
NMOSD
Susac
AIE
Pan neuro-inflammatory auto-immune disease parameters
CD56dim NK cells ↓
cells/ul ↑
monocytes ↓
NKT cells ↓
B cells ↑
Inter-autoimmunity classifiers
plasma cells↑
IgG↑
Neuro-degenerative diseases
Relapsing Multiple sclerosis
RIS CIS
early RRMS
RRMS >36 M
CD56dim
B cells
Plasma cells
IgG
Neuro-vascular diseases

**Figure 7**

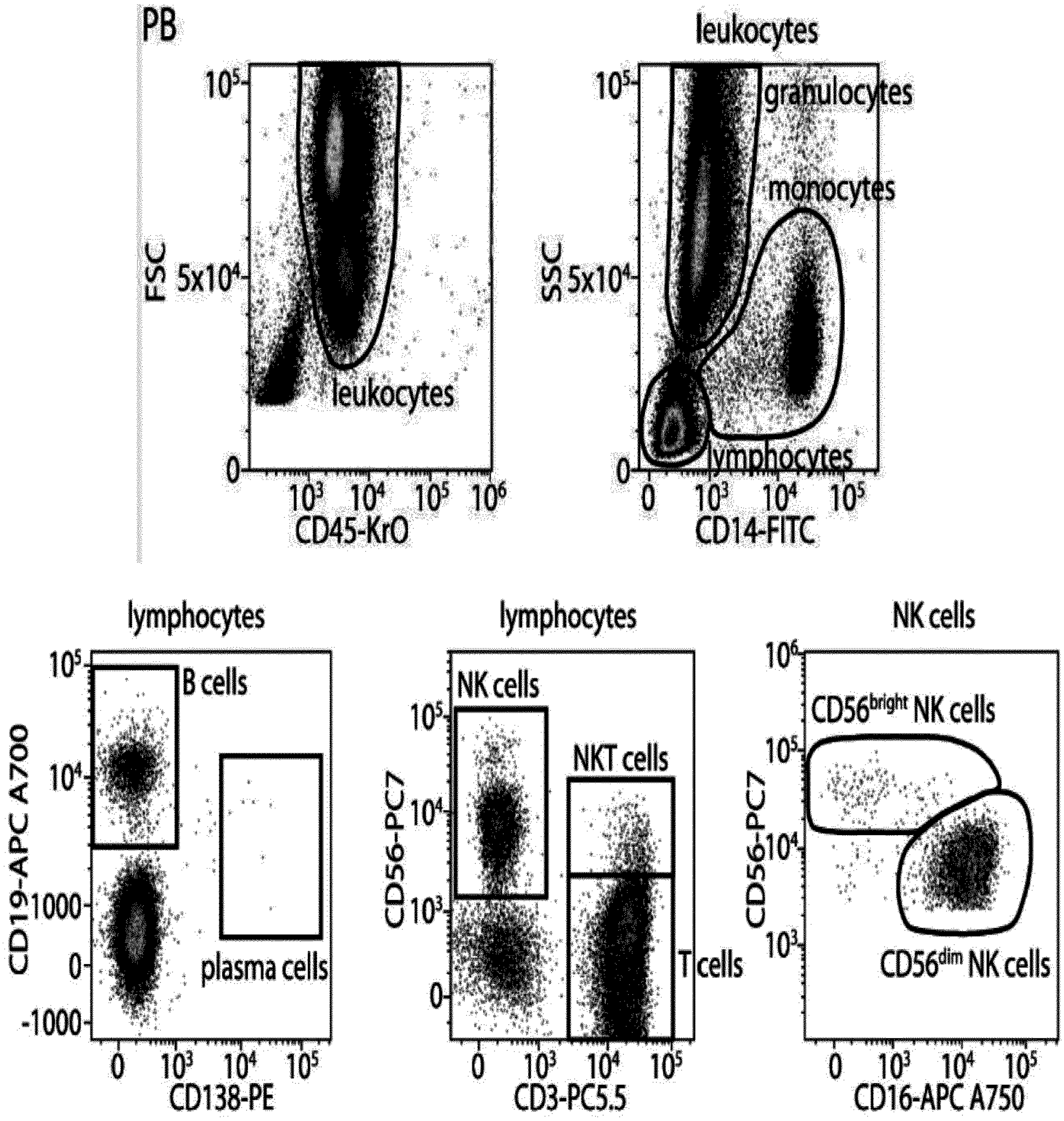
PB
FSC
10⁵
5x10⁴
0
leukocytes
10³ 10⁴ 10⁵ 10⁶
CD45-KrO
leukocytes
SSC
granulocytes
monocytes
lymphocytes
0 10³ 10⁴ 10⁵
CD14-FITC
lymphocytes
CD19-APC A700
B cells
plasma cells
10⁵
10⁴
1000
0
-1000
0 10³ 10⁴ 10⁵
CD138-PE
lymphocytes
CD56-PC7
NK cells
NKT cells
T cells
0 10³ 10⁴ 10⁵
CD3-PC5.5
NK cells
CD56-PC7
CD56^bright NK cells
CD56^dim NK cells
10⁶
10³
0 10³ 10⁴ 10⁵
CD16-APC A750

**Figure 7 (cont.)**

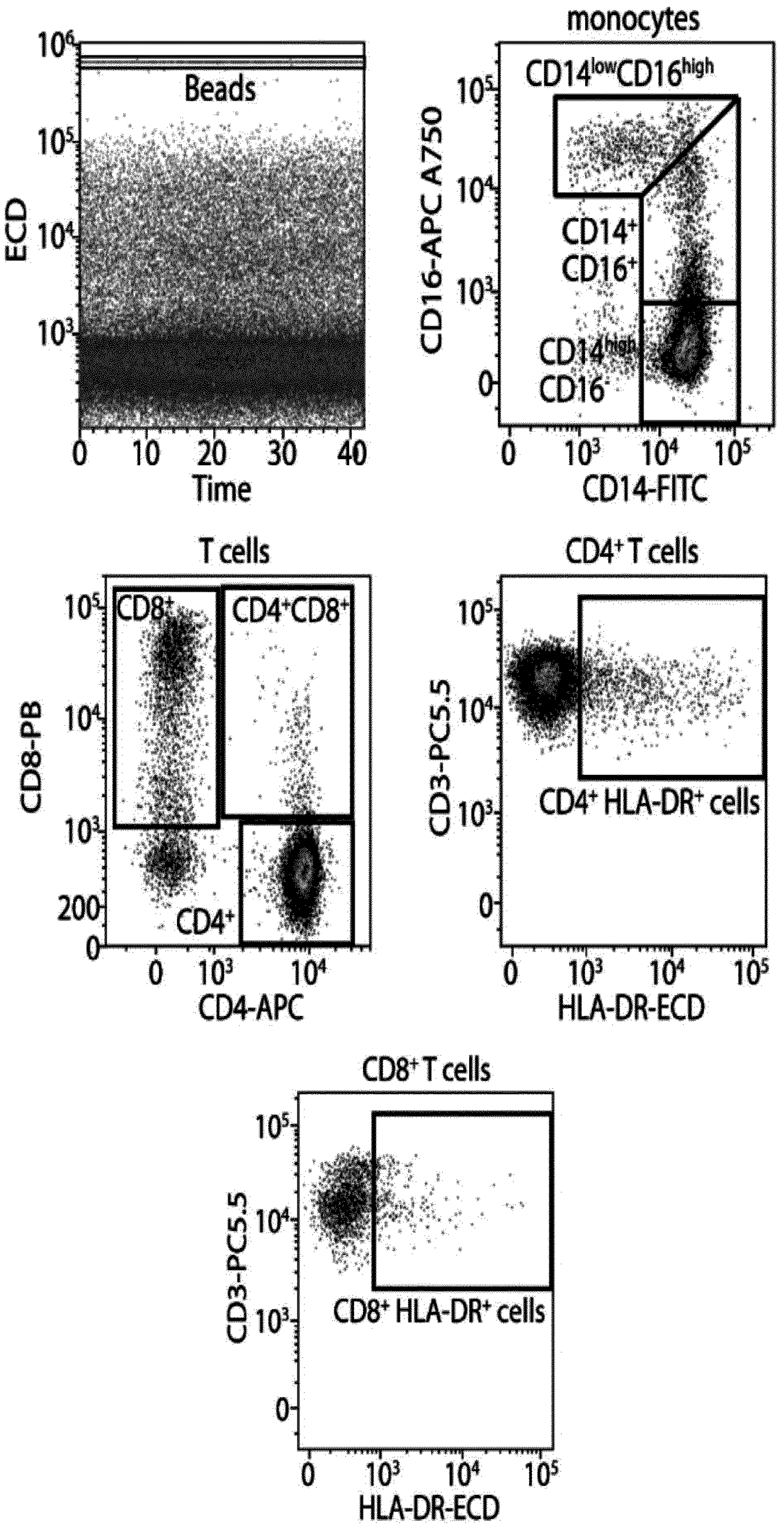

**Figure 7 (cont.)**

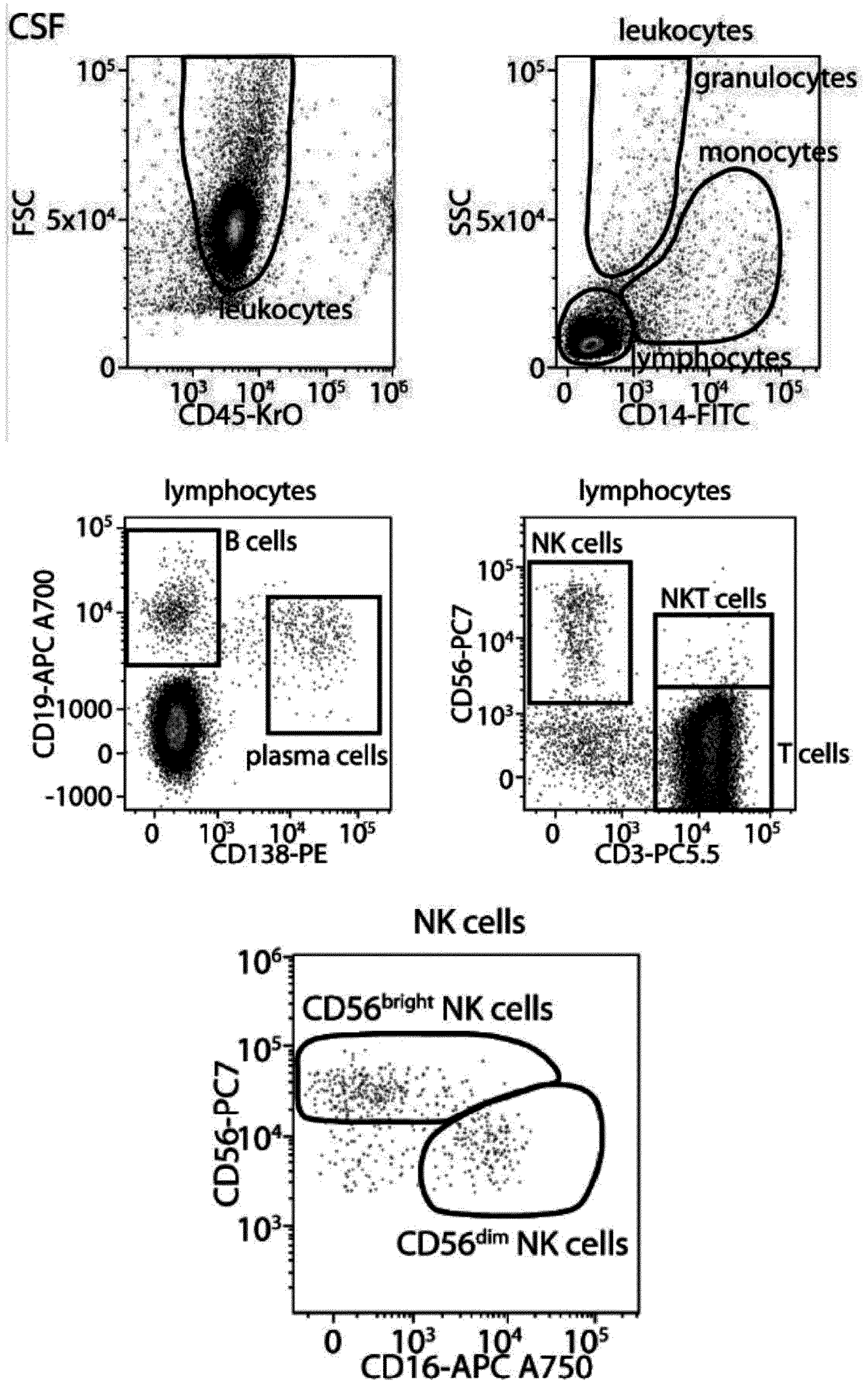

**Figure 7 (cont.)**

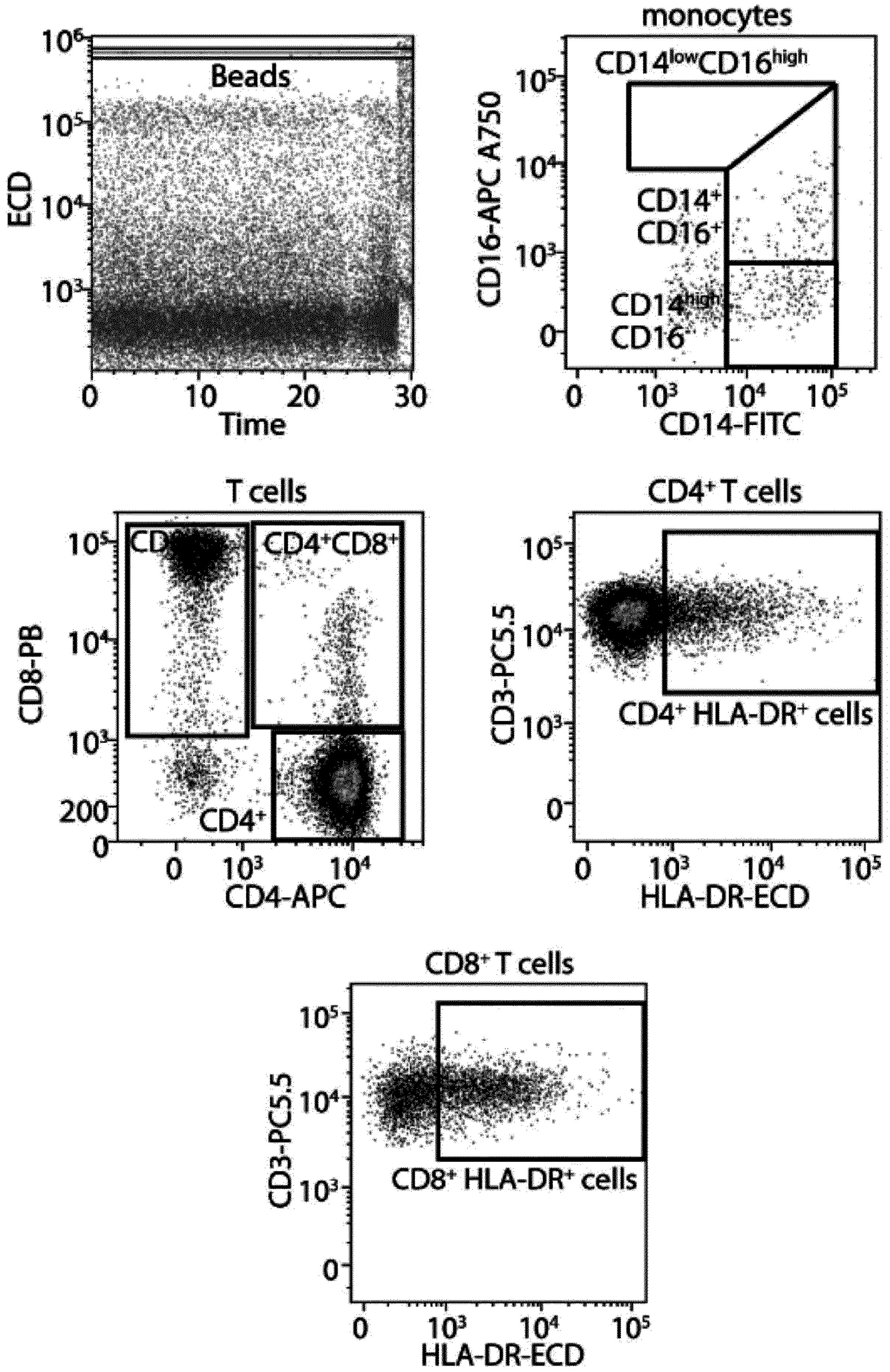

Figure 8

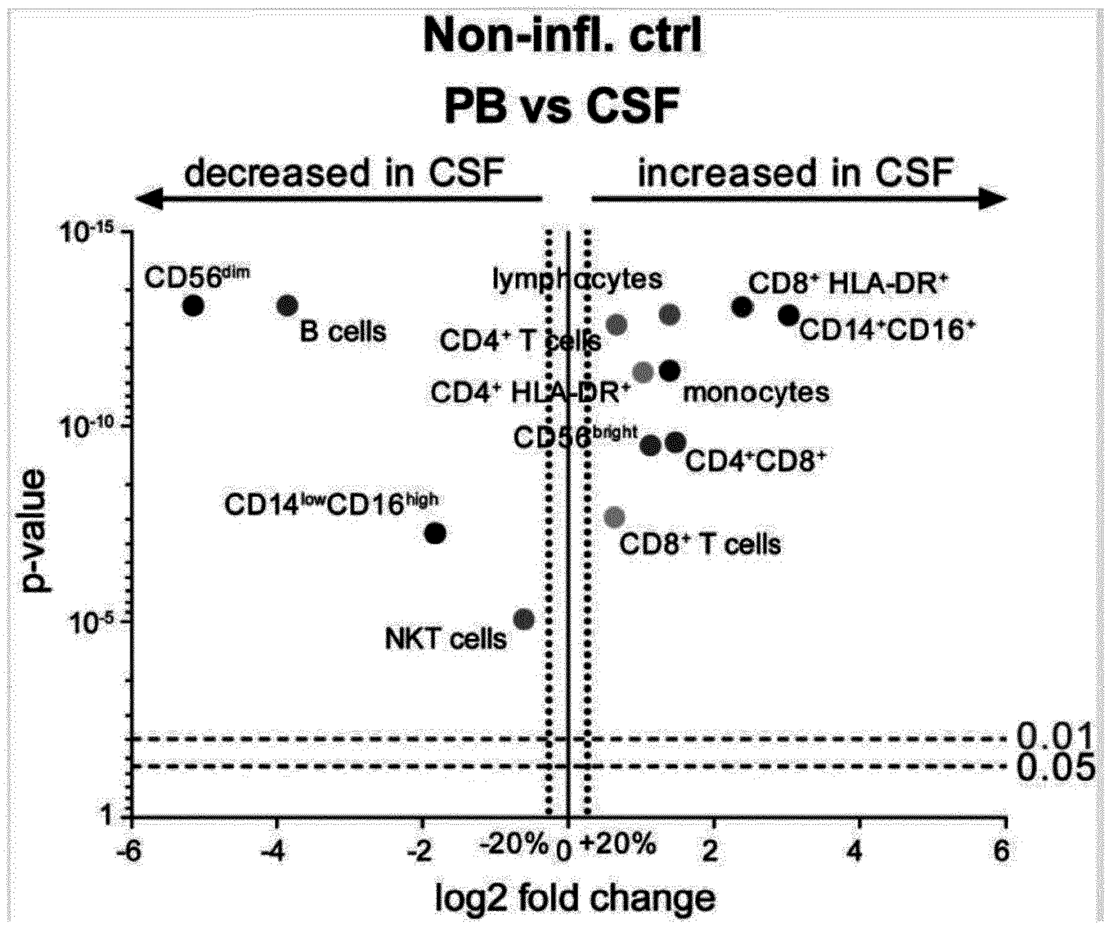
Non-infl. ctrl
PB vs CSF
decreased in CSF
increased in CSF
10-15
10-10
10-5
1
p-value
CD56dim
B cells
lymphocytes
CD4+ T cells
CD8+ HLA-DR+
CD14+CD16+
CD4+ HLA-DR+
monocytes
CD56bright
CD4+CD8+
CD14lowCD16high
CD8+ T cells
NKT cells
0.01
0.05
-6
-4
-2
-20%
0
+20%
2
4
6
log2 fold change

Figure 9

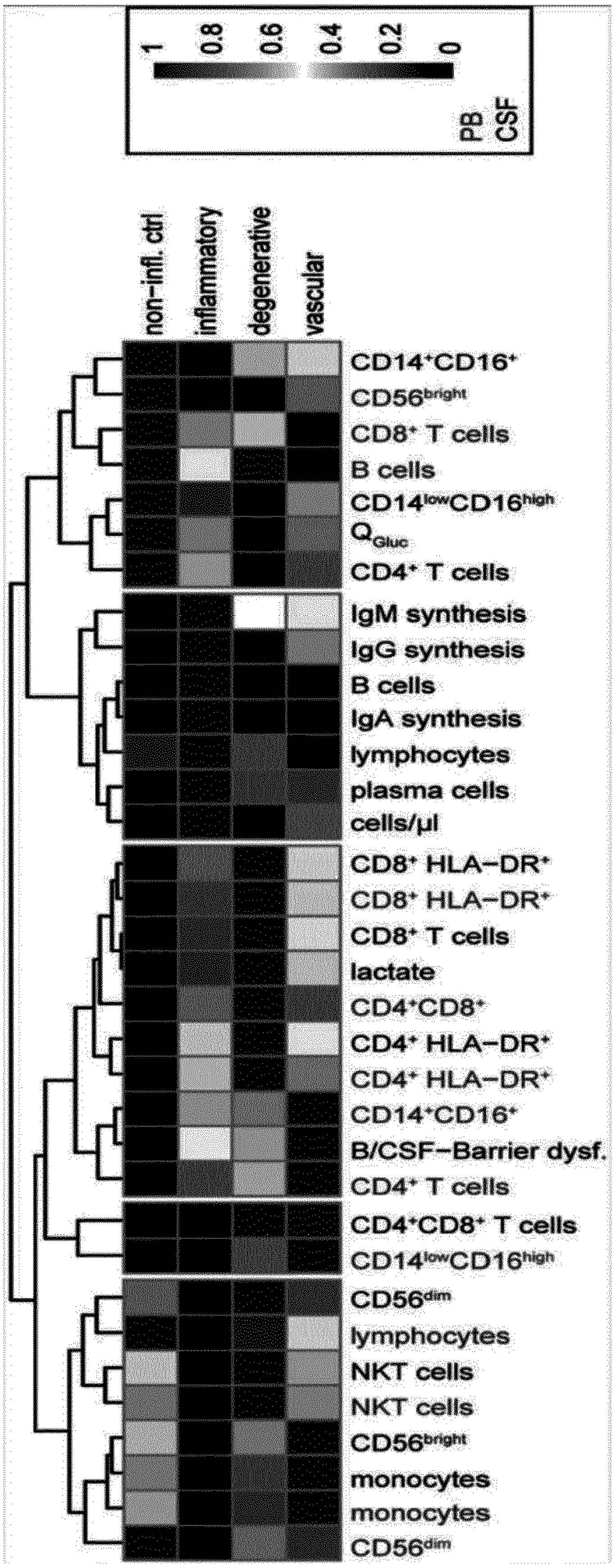
1
0.8
0.6
0.4
0.2
0
PB
CSF
non-infl. ctrl
inflammatory
degenerative
vascular
CD14+CD16+
CD56bright
CD8+ T cells
B cells
CD14lowCD16high
QGluc
CD4+ T cells
IgM synthesis
IgG synthesis
B cells
IgA synthesis
lymphocytes
plasma cells
cells/µl
CD8+ HLA-DR+
CD8+ HLA-DR+
CD8+ T cells
lactate
CD4+CD8+
CD4+ HLA-DR+
CD4+ HLA-DR+
CD14+CD16+
B/CSF-Barrier dysf.
CD4+ T cells
CD4+CD8+ T cells
CD14lowCD16high
CD56dim
lymphocytes
NKT cells
NKT cells
CD56bright
monocytes
monocytes
CD56dim

Figure 10

A

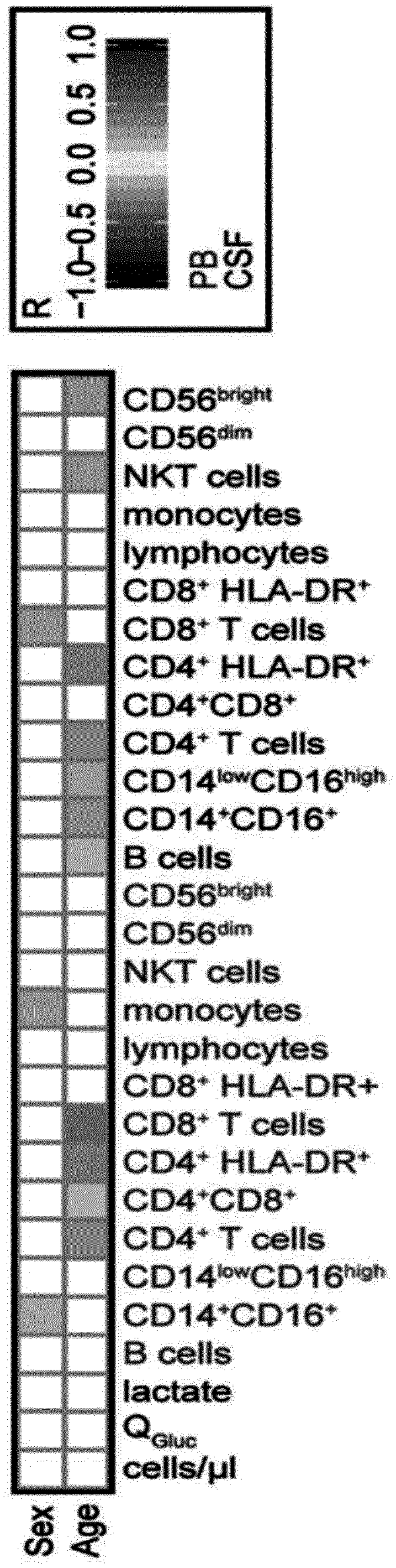
R
−1.0 −0.5 0.0 0.5 1.0
PB
CSF
CD56bright
CD56dim
NKT cells
monocytes
lymphocytes
CD8+ HLA-DR+
CD8+ T cells
CD4+ HLA-DR+
CD4+CD8+
CD4+ T cells
CD14lowCD16high
CD14+CD16+
B cells
CD56bright
CD56dim
NKT cells
monocytes
lymphocytes
CD8+ HLA-DR+
CD8+ T cells
CD4+ HLA-DR+
CD4+CD8+
CD4+ T cells
CD14lowCD16high
CD14+CD16+
B cells
lactate
QGluc
cells/µl
Sex
Age

Figure 10 (cont.)

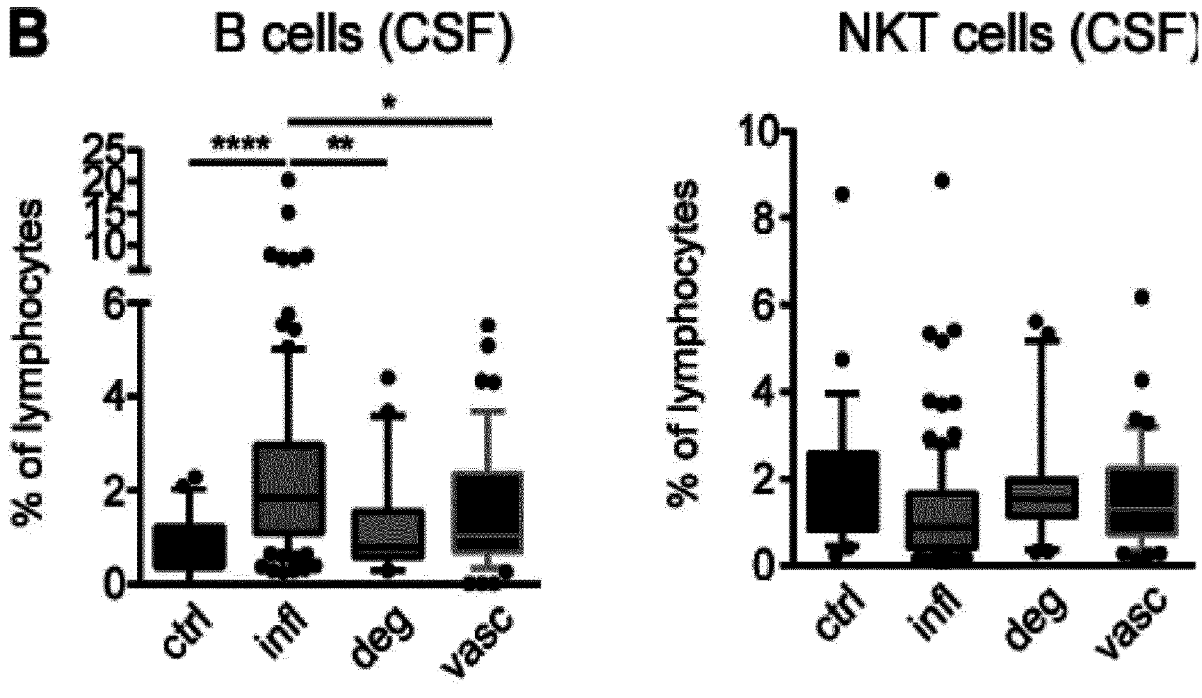

Figure 11

A

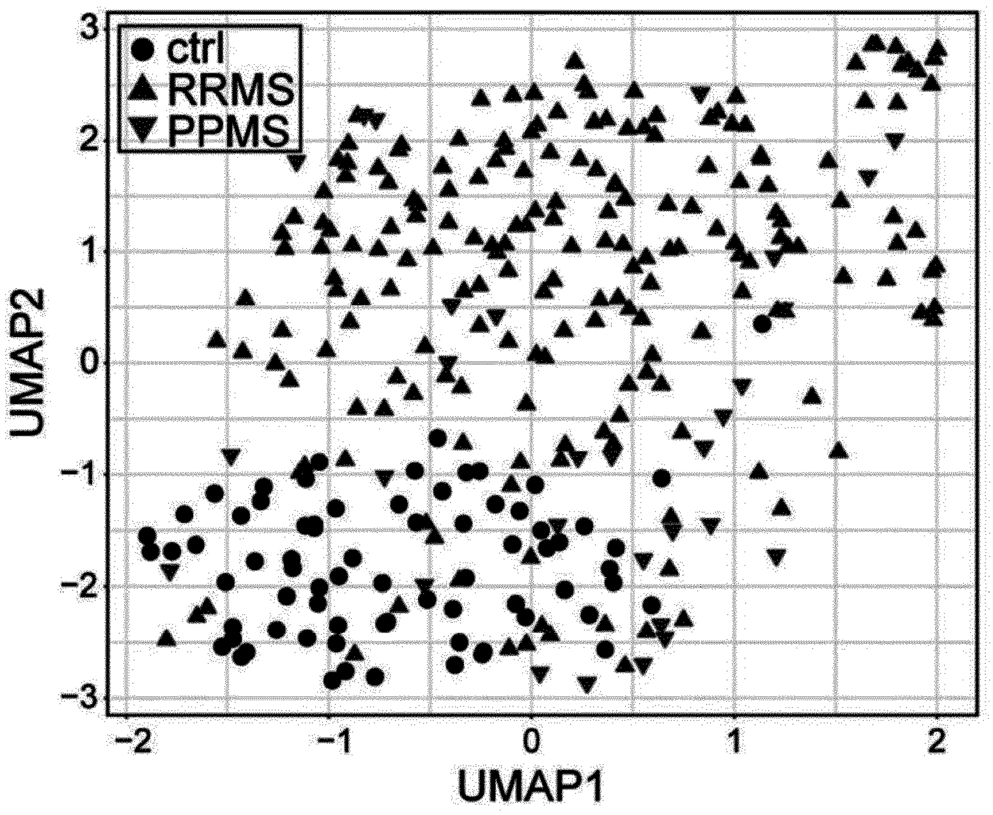
ctrl
RRMS
PPMS
UMAP2
UMAP1
3
2
1
0
−1
−2
−3
−2
−1
0
1
2

B

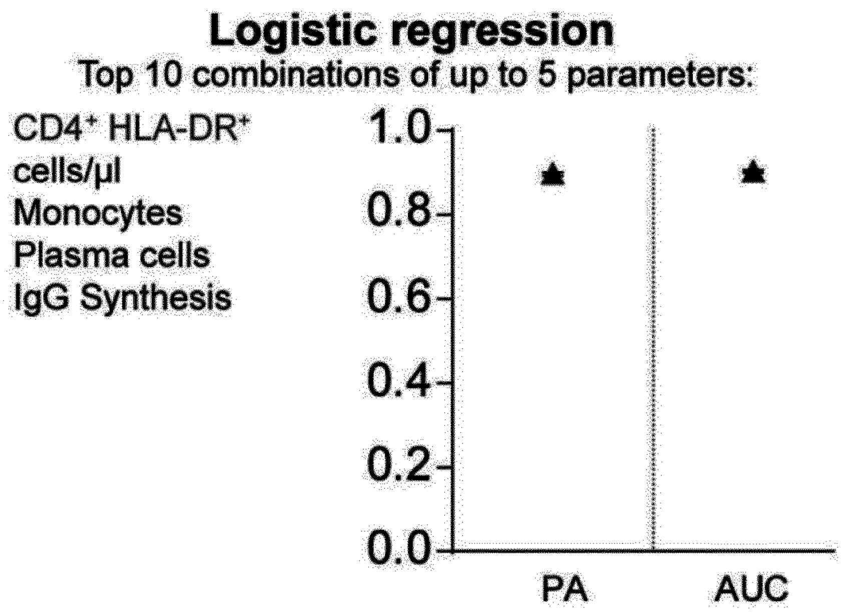
Logistic regression
Top 10 combinations of up to 5 parameters:
CD4+ HLA-DR+
cells/µl
Monocytes
Plasma cells
IgG Synthesis
1.0
0.8
0.6
0.4
0.2
0.0
PA
AUC

Figure 11 (cont.)

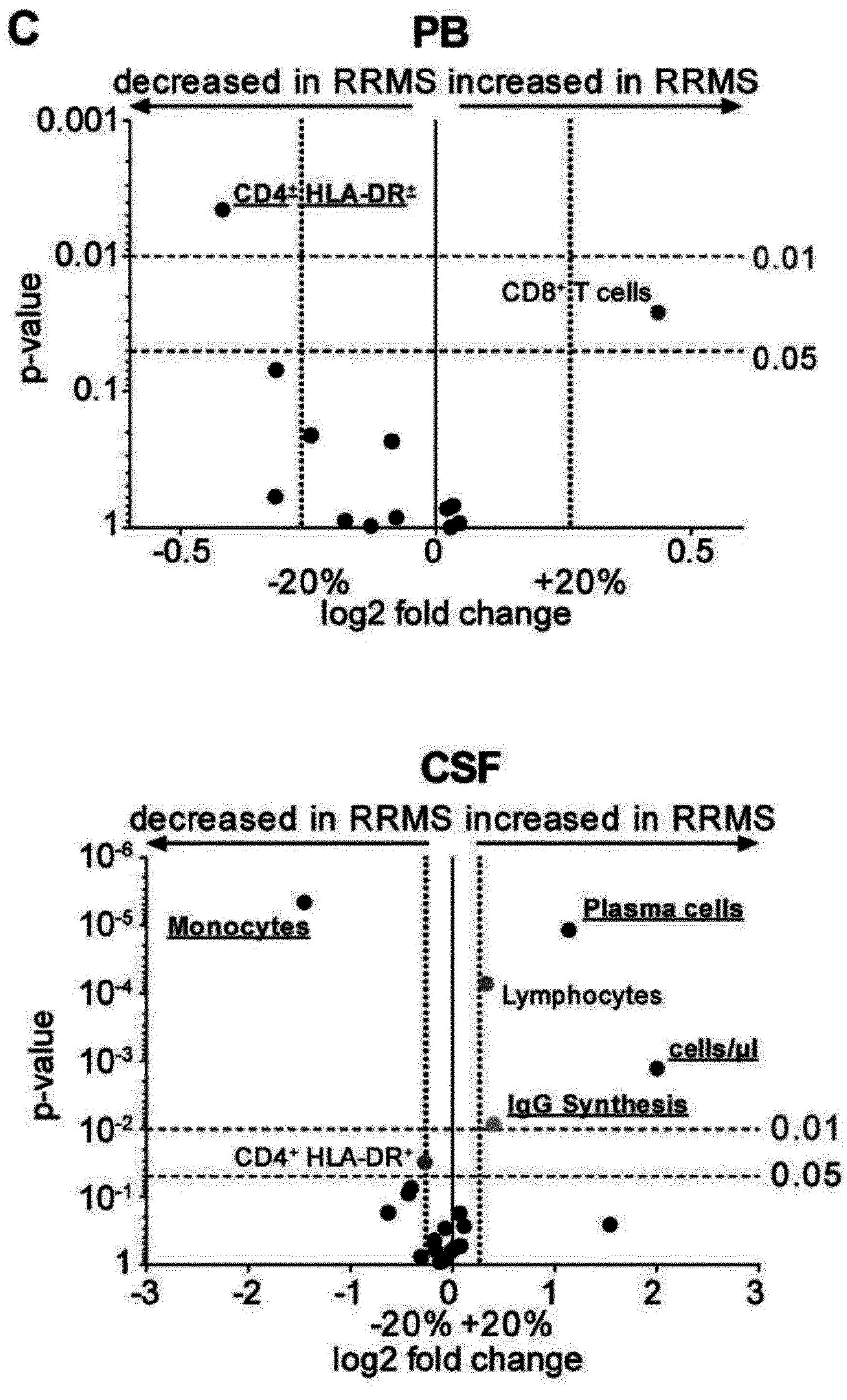

Figure 11 (cont.)

D

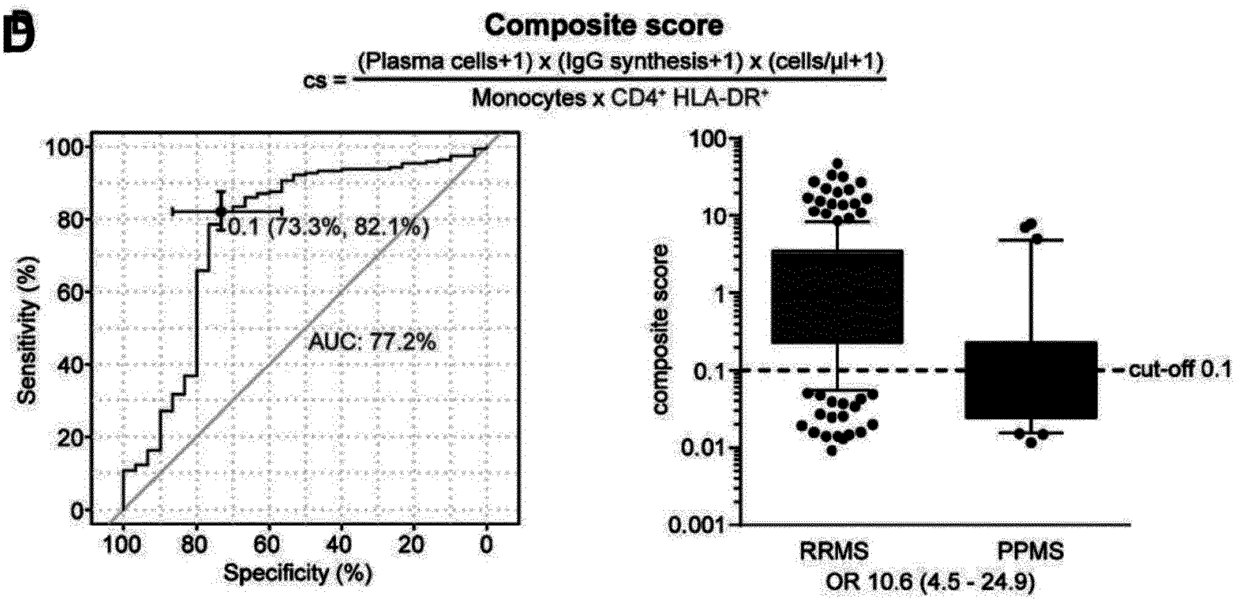


E

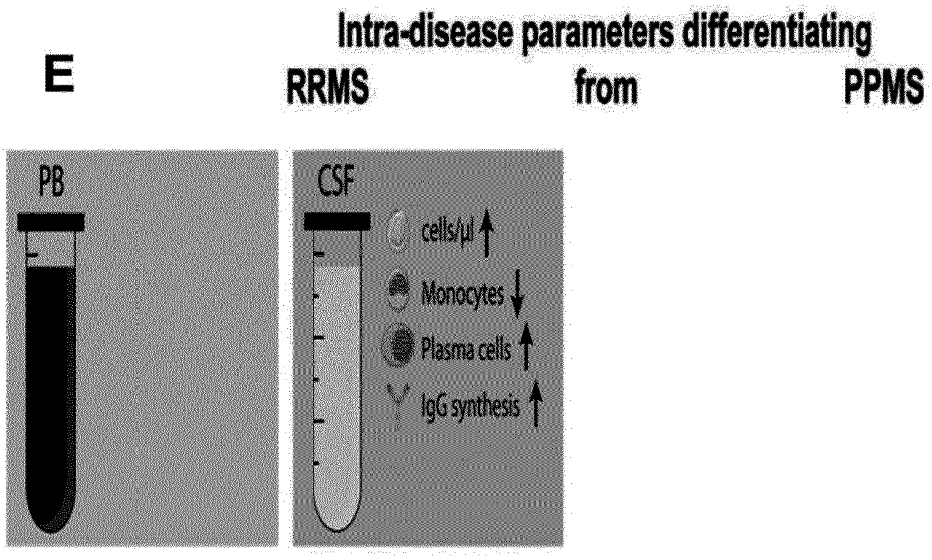

**Figure 12**

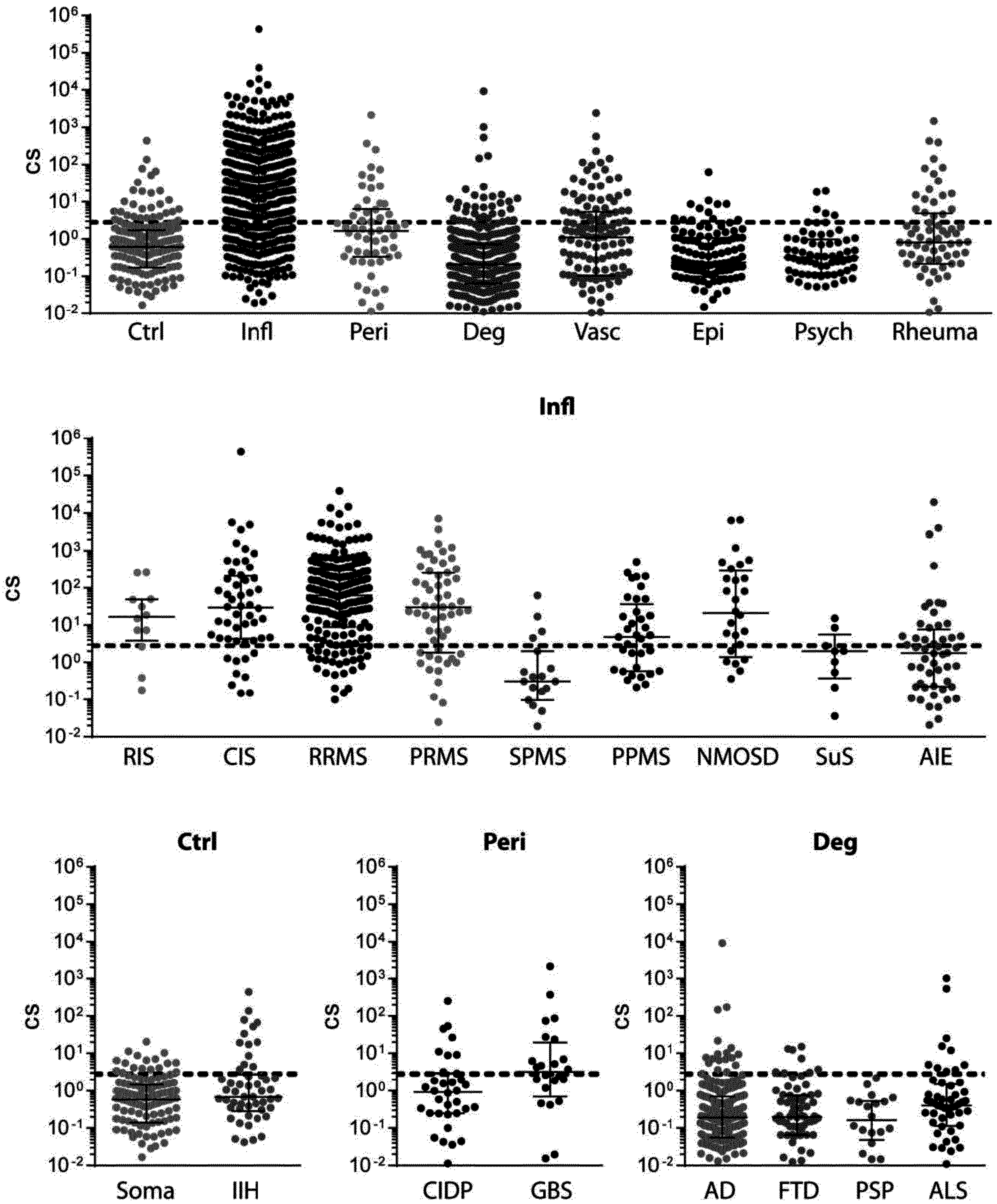
CS
$10^{6}$
$10^{5}$
$10^{4}$
$10^{3}$
$10^{2}$
$10^{1}$
$10^{0}$
$10^{-1}$
$10^{-2}$
Ctrl
Infl
Peri
Deg
Vasc
Epi
Psych
Rheuma
Infl
RIS
CIS
RRMS
PRMS
SPMS
PPMS
NMOSD
SuS
AIE
Ctrl
Soma
IIH
Peri
CIDP
GBS
Deg
AD
FTD
PSP
ALS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020043693 A
- WO 03029462 A
- WO 9623879 A
- WO 0104144 A
- US 4376110 A


### Non-patent literature cited in the description


- **S. JARIUS** ; **B. WILDEMANN** ; **F. PAUL**. *Clin Exp Immunol*, 2014, vol. 176, 149-164
- **J. DALMAUET**. *Lancet Neurol*, 2008, vol. 7, 1091-1098
- **C. C. GROSS et al.** *Nat Commun*, 2019, vol. 10, 5779
- **H. WIENDL**. *Curr Opin Neurol*, 2012, vol. 25, 302-305
- **J. J. ILIFF et al.** *Sci Transl Med*, 2012, vol. 4, 147-111
- **A. LLEO et al.** *Nat Rev Neurol*, 2015, vol. 11, 41-55
- **H. TUMANI et al.** *DGNeurologie*, 2019, vol. 2, 456-480
- **G. MEYER** ; **ZU HORSTE et al.** *Trends Immunol*, 2020, vol. 41, 341-354
- **S. ALVERMANN et al.** *JAMA Neurol*, 2014, vol. 71, 905-912
- **S, HAN et al.** *J Immunol*, 2014, vol. 192 (6), 2551-2563
- **HEMING, M. et al.** *Front Immunol*, 2019, vol. 10, 5151
- **HOLT, L.J. et al.** *Trends Biotechnol.*, 2003, vol. 21 (11), 484-490
- **BESTE et al.** *Proc Nat. Acad Sci*, 1999, vol. 96, 1898-1903
- **NAPOLITANO et al.** *Chemistry & Biology*, 1996, vol. 3 (5), 359-367
- **MOSAVI et al.** *Protein Science*, 2004, vol. 13 (6), 1435-1448
- **SKERRA**. *J. Mol. Recognit.*, 2000, vol. 13, 167-187
- **SILVERMAN et al.** *Nature Biotechnology*, 2005, vol. 23, 1556-1561
- **GILL** ; **DAMLE**. *Current Opinion in Biotechnology*, 2006, vol. 17, 653-658
- **KWON** ; **KODADEK**. *J. Am. Chem. Soc.*, 2007, vol. 129, 1508-1509
- **UNVERDORBEN et al.** *Protein Engineering, Design & Selection*, 2012, vol. 25, 81-88
- **KÖHLER et al.** *Nature*, 1975, vol. 256, 495-497
- **ANDRESEN, M et al.** *Nature Biotechnology*, 2008, vol. 26 (9), 1035
- **FISCHER et al.** *Intensive Care Med.*, 2003, vol. 29, 1043-51
- Ausgewählte Methoden der Liquordiagnostik und Klinische Neurochemie. *Deutschen Gesellschaft für Neurologie*, 2020, 20-25
- **FERRARO et al.** *Neuroimmunology.*, 2015, vol. 283, 64-69
- **DUPONT et al.** *International Journal of Molecular Sciences*, 2017, vol. 18 (4), 785
- **TOLEDANO et al.** *Curr Neurol Neurosci Rep*, 2015, vol. 15, 57
- **CEPOK et al.** *Brain*, 2001, 124
- **HAN et al.** *J Immunol*, 2014, 192
- **SCHAFFLICK et al.** *Nat Commun*, 2020, vol. 11
- **KRAUS J et al.** *Acta Neurol Scand*, 2000
- **GROSS CC et al.** *Proc Natl Acad Sci U S A*, 2016
- **TOLEDANO M** ; **WEINSHENKER BG** ; **SOLOMON AJ.** *Curr Neurol Neurosci Rep.*, 2015, vol. 15, 57
- **DORR J** ; **KRAUTWALD S** ; **WILDEMANN B et al.** *Nat Rev Neurol.*, 2013, vol. 9, 307-16
- **JARIUS et al.** *Nat Rev Dis Primers.*, 2020, vol. 6, 85
- **LGAHTANI H** ; **SHIRAH B** ; **AMIN M** ; **ALTARAZI E** ; **ALMARZOUKI H**. *Neuroradiol J.*, 2018, vol. 31, 207-212